(19) Europäisches Patentamt
European Patent Office
Office européen des brevets

(11) EP 3 921 422 B1

(12) EUROPEAN PATENT SPECIFICATION

(45) Date of publication and mention of the grant of the patent:
**22.11.2023 Bulletin 2023/47**

(21) Application number: **19709026.9**

(22) Date of filing: **04.03.2019**

(51) International Patent Classification (IPC):
***C12N 15/63*** *(2006.01)* ***C12N 15/74*** *(2006.01)*
***A61K 39/02*** *(2006.01)*

(52) Cooperative Patent Classification (CPC):
**C12N 15/63; C12N 15/74;** Y02A 50/30

(86) International application number:
**PCT/EP2019/055352**

(87) International publication number:
**WO 2020/177857 (10.09.2020 Gazette 2020/37)**

# (54) CHLORIDE-INDUCIBLE PROKARYOTIC EXPRESSION SYSTEM

CHLORIDINDUZIERBARES PROKARYOTISCHES EXPRESSIONSSYSTEM

SYSTÈME D'EXPRESSION PROCARYOTE INDUCTIBLE PAR CHLORURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(43) Date of publication of application:
**15.12.2021 Bulletin 2021/50**

(73) Proprietor: **Aurealis Therapeutics AG**
**4057 Basel (CH)**

(72) Inventors:
- **WIRTH, Thomas**
  **9174 Chernevo (BG)**
- **YRJÄNHEIKKI, Juha**
  **70840 Kuopio (FI)**
- **SAMARANAYAKE, Haritha**
  **70200 Kuopio (FI)**
- **KÄRKKÄINEN, Hanna-Riikka**
  **70820 Kuopio (FI)**
- **KURKIPURO, Jere**
  **70820 Kuopio (FI)**
- **MIERAU, Igor**
  **8191 TM Wapenveld (NL)**
- **SMITH, Wesley**
  **00410 Helsinki (FI)**

(74) Representative: **Louis Pöhlau Lohrentz**
**Patentanwälte**
**Postfach 30 55**
**90014 Nürnberg (DE)**

(56) References cited:
**WO-A2-97/14806**

- **KATHRYN GELDART ET AL: "Chloride-Inducible Expression Vector for Delivery of Antimicrobial Peptides Targeting Antibiotic-Resistant Enterococcus faecium.", APPLIED AND ENVIRONMENTAL MICROBIOLOGY, vol. 81, no. 11, 1 June 2015 (2015-06-01), pages 3889-3897, XP55583036, US ISSN: 0099-2240, DOI: 10.1128/AEM.00227-15**
- **CHANG SHIAO-MING ET AL: "Genetic engineering techniques for lactic acid bacteria: construction of a stable shuttle vector and expression vector for [beta]-glucuronidase.", BIOTECHNOLOGY LETTERS FEB 2014, vol. 36, no. 2, February 2014 (2014-02), pages 327-335, XP002792344, ISSN: 1573-6776**
- **SANDERS J W ET AL: "A chloride-inducible gene expression cassette and its use in induced lysis of Lactococcus lactis", APPLIED AND ENVIRONMENTAL MICROBIOLOGY, AMERICAN SOCIETY FOR MICROBIOLOGY, US, vol. 63, no. 12, 1 December 1997 (1997-12-01), pages 4877-4882, XP002523488, ISSN: 0099-2240 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SANDERS J W ET AL: "A chloride-inducible acid resistance mechanism in Lactococcus lactis and its regulation.", MOLECULAR MICROBIOLOGY JAN 1998, vol. 27, no. 2, January 1998 (1998-01), pages 299-310, XP002792345, ISSN: 0950-382X cited in the application**
- **DJORDJEVIC G M ET AL: "INDUCIBLE GENE EXPRESSION SYSTEMS IN LACTOCOCCUS LACTIS", MOLECULAR BIOTECHNOLOGY, HUMANA PRESS, BOSTON, vol. 9, 1 January 1998 (1998-01-01), pages 127-139, XP002902496, ISSN: 1073-6085, DOI: 10.1007/BF02760814**
- **VAN ASSELDONK M ET AL: "Cloning of usp45, a gene encoding a secreted protein from Lactococcus lactis subsp. lactis MG1363", GENE, ELSEVIER, AMSTERDAM, NL, vol. 95, no. 1, 30 October 1990 (1990-10-30), pages 155-160, XP023545781, ISSN: 0378-1119, DOI: 10.1016/0378-1119(90)90428-T [retrieved on 1990-10-30] cited in the application**
- **MAISCHBERGER THOMAS ET AL: "High-level expression of Lactobacillus beta-galactosidases in Lactococcus lactis using the food-grade, nisin-controlled expression system NICE.", JOURNAL OF AGRICULTURAL AND FOOD CHEMISTRY 24 FEB 2010, vol. 58, no. 4, 24 February 2010 (2010-02-24), pages 2279-2287, XP002792346, ISSN: 1520-5118**
- **BERLEC A ET AL: "Large increase in brazzein expression achieved by changing the plasmid /strain combination of the NICE system in Lactococcus lactis.", LETTERS IN APPLIED MICROBIOLOGY JUN 2009, vol. 48, no. 6, June 2009 (2009-06), pages 750-755, XP55002564, ISSN: 1472-765X**
- **CHRISTOPHER TAUER ET AL: "Tuning constitutive recombinant gene expression in Lactobacillus plantarum", MICROBIAL CELL FACTORIES,, vol. 13, no. 1, 20 November 2014 (2014-11-20), page 150, XP021204129, ISSN: 1475-2859, DOI: 10.1186/S12934-014-0150-Z cited in the application**
- **JORGE MASSO-SILVA ET AL: "Antimicrobial Peptides from Fish", PHARMACEUTICALS, vol. 7, no. 3, 3 March 2014 (2014-03-03), pages 265-310, XP055736996, DOI: 10.3390/ph7030265**


Remarks:

The complete document including Reference Table(s) and the Sequence Listing(s) can be downloaded from the EPO website

## Description

The invention is directed to recombinant bacteria, a recombinant plasmid, a pharmaceutical composition and a kit as well as the use of a reconstitution medium comprising chloride ions to reconstitute the recombinant bacteria.

Biopharmaceuticals or biologic therapeutics refer to a wide range of biological products in medicine that, for example, are produced by means of biological processes involving recombinant DNA technology.

Biopharmaceuticals or biologic therapeutics include, for example, polypeptides, that are identical or nearly identical to polypeptides of a subject to be treated, such as the blood-production stimulating protein erythropoietin, biosynthetic human insulin and its analogues, or monoclonal antibodies, that can be made specifically to counteract or block any given target, such as specific cell types, specific polypeptides or endogenous antigens, as well as RNA molecules, that are designed to modulate gene expression or translation, by neutralizing targeted mRNA molecules.

Biopharmaceuticals have a profound impact on many medical fields of medicine adding major therapeutic options for the treatment of many diseases, including some for which no effective therapies were available, and others where previously existing therapies were clearly inadequate.

However, the advent of biopharmaceuticals has also raised complex regulatory issues, and significant pharmacoeconomic concerns, because the cost for biologic therapies has been dramatically higher than for conventional medications. This factor has been particularly relevant since many biological medications are used for the treatment of chronic diseases or for the treatment of otherwise untreatable cancer during the remainder of life.

Furthermore, biopharmaceuticals often have the disadvantage of a limited stability against degradation, for example, by proteases or nucleases, especially after application to a subject. Partial or full degradation of molecules of a biopharmaceutical lead to a significantly reduced half-life of the biopharmaceutical after administration to a subject.

Instead of a direct application of the biopharmaceutical to a subject, alternative modes of application are often required in order to provide a sufficient amount of the respective biopharmaceutical to a subject.

For example, a biopharmaceutical such as a polypeptide can be either provided by autologous or heterologous cells, which secrete the respective polypeptide or by transferring the gene encoding for the required polypeptide to the target tissue by using gene transfer methods, e.g. viral vectors.

Commercially available examples are dermal substitutes containing human embryonic cells, which secrete various growth factors after application to a diseased wound area.

The dermal substitute Dermagraft is composed of fibroblasts, extracellular matrix and a bio-absorbable scaffold. Dermagraf is manufactured from human fibroblast cells derived from donated new-born foreskin tissue. During the manufacture process, the human fibroblasts are seeded onto a bio-absorbable polygalactin scaffold.

The commercially available dermal substitute Apligraf contains two cell types derived from neonatal foreskin. Living human keratinocytes and fibroblasts are embedded in a wound type 1 collagen matrix.

The disadvantage of the afore-mentioned dermal substitutes is a comparable high price which results from the manufacturing process. Furthermore, the respective cells used in the dermal substitutes have to be tested for evidence of infection with human viruses, such as immuno deficiency virus type 1 and 2, hepatitis B virus, hepatitis C virus, syphilis, human T-lymphotropic type 1 and 2 as well as Epstein Barr virus.

Another commercially available example is voretigene neparvovec-rzyl (LUXTURNA). LUXTURNA is a suspension of an adeno-associated virus vector-based gene therapy for subretinal injection and is designed to deliver a normal copy of the gene encoding the human retinal pigment epithelial 65 kDa protein (RPE65) to cells of the retina in persons with reduced or absent levels of biologically active RPE65.

The disadvantage of a viral product or any other viral vector-based gene therapy products, is the high manufacturing cost and the limitations to scale up the production.

For example, WO 9714806 A2 describes the delivery of biologically active polypeptides to a subject by of non-invasive bacteria.

WO 9611277 A1 is directed to the use of microorganisms as vehicles for delivery of therapeutic compounds to a subject.

WO 2011160062 A2 provides a method to treat inflammatory bowel disease comprising administering to the subject a recombinant microorganism capable of producing a therapeutically effective amount of interleukin 27 (IL-27) or a variant or fragment thereof in situ in the intestinal mucosa.

US 2013209407 A is directed to a commensal strain of E. coli which can colonize the genitourinary and/or gastrointestinal mucosa and which can block the infectious and/or disease-causing activity of a pathogen by secreting a heterologous antimicrobial polypeptide.

Several expression systems for heterologous polypeptides are known in bacteria using either inducible or constitutive promoters.

A constitutive promoter is preferably active in a cell in all circumstances allowing for a, preferably high level, production of a desired heterologous polypeptide, which, however, can also lead to a significantly increased metabolic burden and, thus, reduced viability and/or growth rate of the bacteria used. This again can have a direct impact on bacterial yield during the fermentation of the manufacturing process. The higher the metabolic burden, the lower the yield during the fermentation process can be.

In contrast, a regulated, preferably inducible, promoter becomes active or promoter activity is enhanced in response to a specific inducer. Upon application of the inducer to recombinant bacteria, which express a desired heterologous polypeptide under control of the respective regulated, preferably inducible, promoter, the promoter becomes active and the nucleic acid of interest encoding for the respective heterologous polypeptide is expressed. This is preferably of advantage during manufacturing of the recombinant bacteria. Therein higher yields of the recombinant bacteria can be achieved, because of the metabolic burden is minimized.

A widely used controlled gene expression system is, for example, the nisin controlled gene expression system (NICE) of *Lactococcus lactis.*

Nisin, which is well known to the skilled person, is a 34-amino acid lantibiotic polypeptide with a broad host spectrum produced by several *L. lactis* strains. Nisin is widely used as a preservative in food. Initially, nisin is ribosomally synthesized as precursor. After subsequent enzymatic modifications, the modified molecule is translocated across the cytoplasmic membrane and processed into its mature form.

Expression of a nucleic acid sequence of interest can be induced by addition of nisin when the gene of interest is placed behind the inducible promoter P*nisA* of the nisin system.

The nisin controlled gene expression system preferably provides for a high protein yield, however, it is dependent on an external inducer. The addition of nisin is costly when used for administration to a subject, as this requires the provision of nisin in pharmaceutical grade, which raises additional regulatory issues.

Since nisin is a polypeptide, it is also prone to degradation by proteases. Thus, after administration of recombinant bacteria, which express a nucleic acid sequence of interest under control of a nisin promoter, to a subject, nisin has to be supplied preferably repeatedly, if a continuous expression of the nucleic acid sequence of interest is intended.

The requirement of nisin addition for a continuous expression of a nucleic acid sequence of interest further limits the applicable route of administration of the respective recombinant bacteria.

For example, systemic administration of recombinant bacteria expressing a nucleic acid sequence of interest under control of a nisin promoter would additionally require administration of potentially a significant amount of nisin to the subject in order to achieve a concentration of nisin within the subject that induces expression of the respective nucleic acid sequence of interest. Furthermore, the toxicity profile of nisin as well as the necessity of the availability of pharma grade nisin must be taken into account.

WO 97/14806 A2 is directed to methods of delivering biologically active polypeptides and/or antigens, together with delivery means and pharmaceutical formulations comprising such delivery means.

Kathryn Geldart, et al., Applied and Environmental Microbiology, vol. 81, no. 11, 1 June 2015 (2015-06-01), pages 3889-3897, is directed to chloride-inducible expression vector for delivery of antimicrobial peptides targeting antibiotic-resistant Enterococcus faecium.

Chang Shiao-Ming, et al., Biotechnology Letters Feb 2014, vol. 36, no. 2, February 2014 (2014-02), pages 327-335, is directed to genetic engineering techniques for lactic acid bacteria and construction of a stable shuttle vector and expression vector for β-glucuronidase.

Sanders J. W. et al., APPLIED AND ENVIRONMENTAL MICROBIOLOGY, American Society for Microbiology, US, vol., 63, no. 12, 1 December 1997 (1997-12-01), pages 4877-4882, is directed to a chloride-inducible gene expression cassette and its use in induced lysis of Lactococcus lactis.

Sanders J. W. et al., Molecular Microbiology Jan 1998, vol. 27, no. 2, January 1998 (1998-01), pages 299-310, is directed to a chloride-inducible acid resistance mechanism in Lactoccocus lactis and its regulation.

Djordjevic G. M., et al., Molecular Miotechnology, Humana Press, Boston, vol. 9, 1 January 1998 (1998-01-01), pages 127-139, is directed to inducible gene expression systems in Lactococcus lactis.

Masso-Silva, J. et al., Pharmaceuticals, vol. 7, no.3, 3 March 2014 (2014-03-03), pages 265-310, is dirested to antimicrobial peptides from fish.

It is an object of the present invention to provide a bacterial expression system, that allows expression of at least one nucleic acid sequence encoding for at least one heterologous factor, which independently is a heterologous polypeptide, or a complex thereof and wherein the heterologous polypeptide comprises at least one eukaryotic polypeptide, at least one fragment thereof or a combination thereof, without negatively impacting the viability of the respective recombinant bacteria used before administration to a subject.

The bacterial expression system should provide for an inducible expression of the respective heterologous factor at least upon administration to a subject.

The bacterial expression system should also provide for an easy administration of at least one beneficial factor, which independently is a heterologous polypeptide, or a complex thereof, and wherein the heterologous polypeptide comprises at least one eukaryotic polypeptide, at least one fragment thereof or a combination thereof, to a subject, preferably over a prolonged period of time, without the necessity of supplying an additional inducer.

Furthermore, the bacterial expression system should provide a controlled amount of the respective beneficial factor, which is to be used in medicine.

The object of the present invention is solved by providing recombinant bacteria according to claim 1, comprising

a) at least one nucleic acid sequence functionally coupled to a prokaryotic, chloride-inducible promoter and encoding for at least one heterologous factor, said heterologous factor is independently a heterologous polypeptide, or a complex thereof, and
b) at least one prokaryotic regulator gene, which controls activity of said chloride-inducible promoter,

wherein said heterologous polypeptide comprises at least one eukaryotic polypeptide, at least one fragment thereof or a combination thereof, and
wherein said heterologous polypeptide is selected from the group consisting of growth factors, cytokines, chemokines, enzymes, polypeptide hormones, neuropeptides, antibodies, cell surface receptors, soluble receptors, receptor ligands, intrabodies, co-factors, transcription factors, adhesion molecules, tumor antigens, precursors thereof, biologically active fragments thereof and combinations thereof from an eukaryotic species.

Preferably, the recombinant bacteria of the present invention are to be used in medicine.

Preferred embodiments of the recombinant bacteria are disclosed in any one of dependent claims 3 to 11, 13, or 15 to 21.

The object of the present invention is further solved by providing a recombinant nucleic acid according to claim 2, comprising

a) at least one nucleic acid sequence functionally coupled to a prokaryotic, chloride-inducible promoter and encoding for at least one heterologous factor, said heterologous factor is independently a heterologous polypeptide, or a complex thereof, and
b) at least one prokaryotic regulator gene controlling activity of said chloride-inducible promoter,

wherein said heterologous polypeptide comprises a eukaryotic polypeptide, at least one fragment thereof or a combination thereof, and
wherein said heterologous polypeptide is selected from the group consisting of growth factors, cytokines, chemokines, enzymes, polypeptide hormones, neuropeptides, antibodies, cell surface receptors, soluble receptors, receptor ligands, intrabodies, co-factors, transcription factors, adhesion molecules, tumor antigens, precursors thereof, biologically active fragments thereof and combinations thereof from an eukaryotic species.

Preferably, the recombinant nucleic acid, preferably plasmid, of the present invention is used in a method for manufacturing the recombinant bacteria of the present invention.

Preferred embodiments of the recombinant nucleic acid are disclosed in any one of dependent claims 4 to 9, or 12 to 14.

The object of the present invention is further solved by providing a pharmaceutical composition according to claim 22, comprising recombinant bacteria according to any one of claims 1, 3 to 11, 13, or 15 to 19 and at least one pharmaceutically acceptable excipient.

Preferably, the pharmaceutical composition of the present invention is to be used in medicine, preferably in the treatment of a, preferably chronic, inflammatory wound or a degenerative condition, or in the treatment of a tumor, preferably a malignant tumor.

Preferred embodiments of the pharmaceutical composition are disclosed in any one of dependent claims 25 or 26.

The object of the present invention is further solved by a kit according to claim 23 for use in medicine, comprising

a) recombinant bacteria according to any one of claims 1, 3 to 11, 13, or 15 to 19, capable of expressing at least one heterologous factor under the control of the prokaryotic, chloride-inducible promoter, and
b) at least one inducer comprising chloride ions.

Preferred embodiments of the kit are disclosed in any one of dependent claims 25 to 28.

The object of the present invention is further solved by a medical device according to claim 24, comprising

a) recombinant bacteria according to any one of claims 1, 3 to 11, 13, or 15 to 19, capable of expressing at least one heterologous factor under the control of the prokaryotic, chloride-inducible promoter.

Preferred embodiments of the kit are disclosed in dependent claim 26.

The object of the present invention is further solved by the use of a reconstitution medium according to claim 29, comprising chloride ions to reconstitute recombinant bacteria according to any one of claims 1, 3 to 11, 13, or 15 to 18.

The inventors found that recombinant bacteria comprising the above-mentioned nucleic acid sequences could be used in medicine, preferably in the treatment of a, preferably chronic, inflammatory wound or a degenerative condition, or in the treatment of a tumor, preferably a malignant tumor, to provide suitable prophylactic and/or therapeutic factors to a subject in need thereof without additional administration of an exogenous inducer.

The inventors further found that the extracellular tissue environment or body fluids of a subject provide a sufficiently high concentration of chloride ions to initiate and/or maintain expression of the at least one nucleic acid sequence functionally coupled to the prokaryotic, chloride-inducible promoter and encoding for the at least one heterologous factor.

Suitable body fluids are, for example, extracellular fluids such as interstitial fluid, intravascular fluid, such as blood, plasma and serum, cerebrospinal fluid, peritoneal fluid, urine, tears and lymphatic fluid.

Furthermore, intracellular fluids of some phagocytic mammalian cells such as neutrophils and monocytes preferably have suficiently high resting intracellular chloride ion concentrations to initiate and/or maintain expression of the at least one nucleic acid sequence functionally coupled to the prokaryotic, chloride-inducible promoter and encoding for the at least one heterologous factor.

However, in the intracellular fluid of other mammalian cells intracellular chloride ion concentration is preferably insufficient for protein production and, thus, if the recombinant bacteria of the present invention would enter the cytoplasm of these cells the chloride concentration inside the cell, as a safety feature, would prevent initiation and/or maintainance of expression of the at least one nucleic acid sequence functionally coupled to the prokaryotic, chloride-inducible promoter and encoding for the at least one heterologous factor.

These findings enable the provision of recombinant bacteria, which can be designed, preferably as a single pharmaceutical entity, based on bacteria, further preferably non-pathogenic, lactic acid bacteria, that have been genetically engineered to produce the at least one heterologous factor.

According to the invention the term "heterologous factor" means a factor, preferably a polypeptide, or a complex thereof, which is not naturally occurring in or expressed by said bacteria used.

When referring to "heterologous factor(s)" in general or when referring to specific "heterologous factor(s)" such as, e.g. FGF-2, IL-4, CSF-1, etc., it is intended that this term includes also functional analog(s) thereof.

According to the invention the term "functional analog" of a factor means an agent that binds to identical receptor(s) as the respective factor and preferably activates identical second messengers in a target cell.

Preferably, "a functional analog" of said at least one heterologous factor has a sequence identity of the amino acid sequence of at least 50%, preferably of at least 80%, further preferably of at least 90%, further preferably of at least 93%, further preferably of at least 95%, further preferably of at least 97%, if the respective heterologous factor is a polypeptide or a complex thereof.

Preferably, "a functional analog" of said at least one heterologous factor has a sequence identity of the ribonucleic acid sequence of at least 80%, further preferably of at least 90%, further preferably of at least 93%, further preferably of at least 95%, further preferably of at least 97%, if the respective heterologous factor is a ribonucleic acid.

A "functional analog" can also be designated as biosimilar.

Upon induction by chloride ions, the recombinant bacteria of the present invention comprising the above-mentioned at least one nucleic acid sequence are able to produce the at least one heterologous factor at least by transcribing and preferably by translating the at least one nucleic acid sequences.

In the presence of chloride ions, the recombinant bacteria of the present invention preferably are able to deliver the at least one heterologous factor as recited in claim 1 to a subject, for example to diseased tissue, thereby mediating a beneficial effect and/or enabling healing of the subject. Preferably, the recombinant bacteria of the present invention release the at least one heterologous factor after administration to the subject.

In the presence of chloride ions, the recombinant bacteria of the present invention preferably further provide for a constant release of the at least one heterologous factor, further preferably after administration to a subject in need thereof.

Thereby, a much-improved, safer, and more cost-effective treatment option for subjects suffering from a medical condition, such as a, preferably chronic, inflammatory wound or a degenerative condition, or a tumor, preferably a malignant tumor, is available.

Preferably, the at least one heterologous factor, after release from the bacteria, exerts at least one biological active function supporting healing of said subject and/or preventing worsening of the medical condition.

Furthermore, the at least one heterologous factor, after release from the bacteria, can have a prophylactic and/or therapeutic effect, for example, by exerting paracrine and/or endocrine activities impacting local or whole-body metabolism and/or by regulating activities of cells of the body and/or by impacting the viability, growth and differentiation of a variety of cells in the body and/or by impacting the immune regulation or induction of acute phase inflammatory responses to injury and/or infection.

The recombinant bacteria of the present invention comprise:

a) at least one nucleic acid sequence functionally coupled to a prokaryotic, chloride-inducible promoter and encoding for at least one heterologous factor, said heterologous factor is independently a heterologous polypeptide, or a complex thereof, and
b) at least one prokaryotic regulator gene, which controls activity of said chloride-inducible promoter,

wherein said heterologous polypeptide comprises at least one eukaryotic polypeptide, at least one fragment thereof or a combination thereof, and
wherein said heterologous polypeptide is selected from the group consisting of growth factors, cytokines, chemokines, enzymes, polypeptide hormones, neuropeptides, antibodies, cell surface receptors, soluble receptors, receptor ligands, intrabodies, co-factors, transcription factors, adhesion molecules, tumor antigens, precursors thereof, biologically active fragments thereof and combinations thereof from an eukaryotic species.

The term "prokaryotic promoter" is known to the skilled person and refers to a nucleic acid sequence that controls initiation of transcription of a particular gene by preferably providing a binding site for RNA polymerase and/or for at least one transcription factor that recruits RNA polymerase.
A prokaryotic promoter is preferably located near the transcription start side of a gene, further preferably on the same strand and upstream of the gene, which is towards the 5' region of the same strand, which is to be transcribed.
By the term "functionally coupled to" it is meant that transcription of the at least one nucleic acid sequence encoding for at least one heterologous factor is preferably initiated and controlled by the respective prokaryotic promoter.
The prokaryotic promoter used according to the present invention to preferably initiate and control transcription of the at least one nucleic acid sequence encoding for at least one heterologous factor is a prokaryotic promoter, which is inducible by chloride ions.
Further preferably, activity of said prokaryotic, chloride-inducible promoter is dependent on the concentration of chloride ions in the environment of the recombinant bacteria, such as a reconstitution medium or body fluid after application to a subject.
The term "activity" of said prokaryotic, chloride-inducible promoter preferably refers to the amount of the at least one heterologous factor expressed from the at least one nucleic acid sequence functionally coupled to said promoter.
Preferably, the term "activity" of said prokaryotic, chloride-inducible promoter refers to the amount of protein(s) obtained from the transcription of the at least one nucleic acid sequence encoding for at least one heterologous factor.
Preferably, the at least one heterologous factor is subsequently generated from the transcribed mRNA inter alia by translation of the mRNA.
The activity of the promoter used according to the present invention to preferably initiate and control transcription of the at least one nucleic acid sequence encoding for at least one heterologous factor is further controlled by at least one prokaryotic regulator gene.
The term "regulator gene" is known to the skilled person and refers to a nucleic acid sequence, preferably gene, involved in controlling the expression of one or more other genes. A regulatory sequence, which preferably encodes for a regulatory gene, is preferably arranged 5' to the start site of transcription of a gene to be regulated. A regulatory sequence can also be arranged 3' to the transcription start site or on a distant site on a chromosom.
A regulator gene can preferably be located within an operon, adjacent to it, or far away from it, further preferably in the same bacterial cell.
In a preferred embodiment the regulator gene encodes for a regulator protein, such as a repressor protein or an activator protein, wherein further preferably expression of said regulator protein is initiated and controlled by an individual prokaryotic promoter, selected from at least one of a constitutive promoter or regulated, preferably inducible, promoter.
Further preferably expression of said regulator protein is controlled by a constitutive prokaryotic promoter and, thereby, the recombinant bacteria of the present invention are able to provide intercellularly a sufficient amount of the regulator protein to regulate activity of said chloride-inducible promoter.
A repressor protein preferably binds to an operator or promoter, preventing RNA polymerase from transcribing RNA. At least one inducer preferably can cause the repressor protein to change shape or otherwise become unable to bind DNA, allowing RNA polymerase to start and/or to continue transcription.
An activator protein, such as GadR, preferably binds to a site on the DNA molecule, preferably near the promoter to be controlled by the regulator protein and allows transcription and/or enhances rate of transcription.
According to the invention, the at least one nucleic acid sequence functionally coupled to said prokaryotic, chloride-inducible promoter and encoding for at least one heterologous factor and the at least one prokaryotic regulator gene controlling activity of said chloride-inducible promoter are each located in the same bacterial cell, preferably in each bacterial cell, of the recombinant bacteria of the present invention.

Preferably, said at least one nucleic acid sequence functionally coupled to said prokaryotic, chloride-inducible promoter and encoding for the at least one heterologous factor and said at least one prokaryotic regulator gene, which controls activity of said chloride-inducible promoter, are each independently located on a chromosome and/or at least one plasmid in the same bacterial cell of said recombinant bacteria.

For example, said at least one nucleic acid sequence functionally coupled to said prokaryotic, chloride-inducible promoter and encoding for the at least one heterologous factor is located on at least one of a chromosome and/or a plasmid in at least one bacterial cell of the recombinant bacteria of the present invention. Independently therefrom, the at least one prokaryotic regulator gene is preferably located on at least one of a chromosome and/or a plasmid of the same bacterial cell of said recombinant bacteria.

Preferably, the at least one nucleic acid sequence functionally coupled to a prokaryotic, chloride-inducible promoter and encoding for at least one heterologous factor and the at least one prokaryotic regulator gene controlling activity of said chloride-inducible promoter, are each located on the same recombinant nucleic acid molecule, which is least one of a chromosome and/or a plasmid.

Further preferably, said at least one nucleic acid sequence functionally coupled to said prokaryotic, chloride-inducible promoter and encoding for the at least one heterologous factor and said at least one prokaryotic regulator gene, which controls activity of said chloride-inducible promoter, are at least both located on a recombinant nucleic acid, further preferably on a recombinant plasmid of the present invention.

The recombinant nucleic acid, preferably recombinant plasmid, of the present invention, comprises

a) at least one nucleic acid sequence functionally coupled to a prokaryotic, chloride-inducible promoter and encoding for at least one heterologous factor, said heterologous factor is independently a heterologous polypeptide, or a complex thereof, and
b) at least one prokaryotic regulator gene controlling activity of said chloride-inducible promoter,

wherein said heterologous polypeptide comprises a eukaryotic polypeptide, at least one fragment thereof or a combination thereof, and wherein said heterologous polypeptide is selected from the group consisting of growth factors, cytokines, chemokines, enzymes, polypeptide hormones, neuropeptides, antibodies, cell surface receptors, soluble receptors, receptor ligands, intrabodies, co-factors, transcription factors, adhesion molecules, tumor antigens, precursors thereof, biologically active fragments thereof and combinations thereof from an eukaryotic species.

The term "plasmid" is known to the skilled person and refers to a preferably circular, further preferably double-stranded, DNA molecule within a bacterial cell that is physically separated from chromosomal DNA and can replicate independently.

This is to say, a recombinant nucleic acid of the present invention is preferably a circular, further preferably double-stranded, DNA molecule, comprising

a) at least one nucleic acid sequence functionally coupled to a prokaryotic, chloride-inducible promoter and encoding for at least one heterologous factor, said heterologous factor is independently a heterologous polypeptide, or a complex thereof, and
b) at least one prokaryotic regulator gene controlling activity of said chloride-inducible promoter,

wherein said heterologous polypeptide comprises a eukaryotic polypeptide, at least one fragment thereof or a combination thereof, and wherein said heterologous polypeptide is selected from the group consisting of growth factors, cytokines, chemokines, enzymes, polypeptide hormones, neuropeptides, antibodies, cell surface receptors, soluble receptors, receptor ligands, intrabodies, co-factors, transcription factors, adhesion molecules, tumor antigens, precursors thereof, biologically active fragments thereof and combinations thereof from an eukaryotic species.

The recombinant bacteria of the present invention comprise preferably at least one copy of a recombinant nucleic acid, preferably recombinant plasmid, of the present invention.

The at least one nucleic acid sequence, which is functionally coupled to the prokaryotic, chloride-inducible promoter, encodes for at least one heterologous factor.

Preferably, the at least one nucleic acid sequence encodes for one or more factor(s), which each is/are not naturally occurring in or expressed by said bacteria used.

Further preferably, the at least one nucleic acid sequence encodes for 1, 2, 3, 4 or more factors, which each are not naturally occurring in or expressed by said bacteria used and which independently are a heterologous polypeptide, or a complex thereof, preferably a heterologous polypeptide or a complex thereof.

The term "complex" refers to a protein complex of two or more polypeptide chains, which can be designated as "subunits" and which preferably are associated or linked by at least one non-covalent protein-protein interaction, such as hydrogen bond, ionic interaction, Van der Waals force, and/or hydrophobic bond, and/or by at least one covalent protein-protein bond, which is not a peptide bond, for example a disulfide bond.

The subunits of a multimeric protein complex may be identical as in a homomultimeric protein complex or different as in a heteromultimeric protein complex.

Preferably, a complex of a heterologous polypeptide is formed before and/or after release of the at least one heterologous polypeptide from the recombinant bacteria of the present invention.

For example, the recombinant bacteria of the present invention express one heterologous polypeptide, which after release from the bacteria forms a homomultimeric protein complex having two or more identical subunits.

Alternatively, the recombinant bacteria of the present invention express two or more heterologous polypeptides, which are different from each other and which after release from the bacteria form a heteromultimeric protein complex having two or more different subunits.

Preferably, the at least one heterologous factor has a therapeutic and/or preventive effect in a subject, preferably after application of the recombinant bacteria of the present invention to said subject.

In a further preferred embodiment of the present invention the at least one heterologous factor is a heterologous polypeptide or complex thereof each comprising or consisting of at least one eukaryotic polypeptide, at least one fragment thereof or a combination thereof.

Further preferably, the term "fragment of a eukaryotic polypeptide" referes to a biologically active fragment of said eukaryotic polypeptide.

Preferably, the recombinant bacteria of the present invention express, after application to a subject, the at least one heterologous polypeptide or complex thereof. Further preferably, the recombinant bacteria of the present invention release, preferably secrete, the at least one heterologous polypeptide or complex thereof to the surrounding environment, for example the site of application and/or a body fluid of said subject.

The at least one heterologous polypeptide or complex thereof can itself have a biological effect on at least one cell of the subject, for example by stimulating or inhibiting cellular growth, proliferation, and/or cellular differentiation, by inducing or suppressing of apoptosis, by activating or inhibiting the immune system, by regulating metabolism, by controlling migration of cells, and/or by regulating production and/or release of endogenous factors of the subject, thereby mediating a therapeutic and/or preventive effect in the subject, preferably after application of the recombinant bacteria of the present invention to said subject and preferably after release, futher preferably after secretion, from the recombinant bacteria of the present invention.

A therapeutic and/or preventive effect in a subject can also be mediated by binding of the at least one heterologous polypeptide or complex thereof to at least one endogenous or exogenous target molecule present in the subject, such as a bacterial antigen, a viral antigen, a tumour antigen and/or endogenous polypeptide.

For example, the at least one heterologous polypeptide or complex thereof is an antibody and/or at least one biologically active fragment thereof, which, after application of the recombinant bacteria of the present invention to a subject, is released from the bacteria and subsequently binds to at least one target molecule, preferably an exogeneous antigen, such as a part of a bacterial cell or a virus, or to an endogenous antigen or polypeptide, which otherwise would mediate a harmful effect, such as an overactive immune response.

By binding to the at least one target molecule present in the subject, the at least one heterologous polypeptide or complex thereof preferably reduces the amount of said molecule in said subject and/or reduces and/or prevents the biological activity of said molecule in said subject, for example by reducing or inhibiting enzyme activity of said molecule and/or by reducing or inhibiting binding of said molecule to an endogenous receptor.

Preferably, said at least one heterologous factor is a heterologous polypeptide, at least one fragment thereof, preferably having at least 5, preferably at least 7, amino acids joined by peptide bonds, and/or a complex thereof.

Examples of suitable polypeptides include polypeptides, precursors thereof, fragments thereof and combinations thereof from an eukaryotic species, preferably a mammalian species, further preferably human, which are capable of acting locally and/or systemically.

According to the invention, said at least one heterologous polypeptide is selected from the group consisting of growth factors, cytokines, chemokines, enzymes, polypeptide hormones, neuropeptides, antibodies, cell surface receptors, soluble receptors, receptor ligands, intrabodies, which can also be designated as intracellular antibodies, co-factors, transcription factors, adhesion molecules, tumor antigens, precursors thereof, biologically active fragments thereof and combinations thereof from an eukaryotic species, preferably a mammalian species, further preferably human.

Suitable tumor antigens are known to the skilled person and, preferably are disclosed in Cheever, M.A. et al. (2009) ("The Prioritization of Cancer Antigens: A National Cancer Institute Pilot Project for the Acceleration of Translational Research", Clin. Cancer Res. 15(17), pages 5323 ot 5337; DOI: 10.1158/1078-0432.CCR-09-0737).

Of course, functional analogues of the afore mentioned or below mentioned polypeptides or biosimilars thereof can also be used within the scope of the invention as claimed.

Growth factors are preferably polypeptides capable of stimulating cellular growth, proliferation, healing, and/or cellular differentiation.

Preferably, the growth factor is selected from the group consisting of fibroblast growth factors (FGF), vascular endothelial growth factors (VEGF), epidermal growth factors (EGF), insulin-like growth factors (IGF), platelet-derived growth factors (PDGF), transforming growth factor beta (TGF-beta), nerve growth factor (NGF), activins, functional analogues thereof, biosimilars thereof, and mixtures thereof.

Fibroblast growth factors are a family of growth factors, which are involved in angiogenesis, wound healing, and various endocrine signalling pathways. In humans, 22 members of the FGF family have been identified, FGF-1 to FGF-14 and FGF-16 to FGF-23, which can be used in the present invention. FGF-1 through FGF-10 bind the fibroblast growth factor receptors (FGFRs).

In a preferred embodiment, the fibroblast growth factor is selected from the group consisting of FGF-1, FGF-2, FGF-3, FGF-4, FGF-5, FGF-6, FGF-7, FGF-8, FGF-9, FGF-10, and mixtures thereof, further preferably FGF-1, FGF-2, FGF-7, FGF-10, and mixtures thereof, further preferably, FGF-2, FGF-7, functional analogues thereof, biosimilars thereof, and mixtures thereof, further preferably FGF-2.

For example, FGF-1 and FGF-2 can stimulate angiogenesis and are mitogenic for several cell types present at the site of an inflammatory skin dysfunction, including fibroblasts and keratinocytes. Furthermore, FGF-7 can stimulate wound reepithelization in a paracrine manner.

The nucleic acid sequence of the mRNA of the human fibroblast growth factor 2 (hFGF-2) is available under the NCBI accession number NM_002006.4. The respective amino acid sequence of the AUG-isomer is available under the NCBI accession number NP_001997.5 as well as the UniProt accession number P09038 - version 182.

The precursor includes a propeptide, which spans the amino acids 1 to 142 of the precursor, and the mature human fibroblast growth factor 2 peptide, which spans the amino acids 143 to 288 of the precursor.

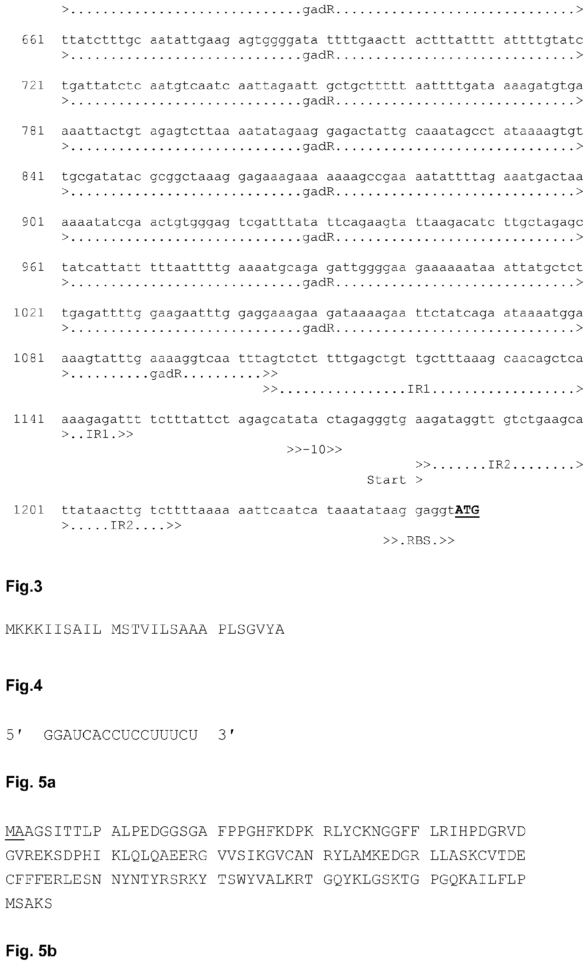

In a preferred embodiment, fibroblast growth factor 2 comprises one or at least one of the amino acid sequences of SEQ ID Nos 5 to 7. The amino acid sequences of SEQ ID Nos 5 to 7 are depicted in Figures 5a to 5c, respectively.

The insulin-like growth factors (IGFs) are proteins with a high sequence similarity to insulin. The insulin-like growth factors comprise two proteins IGF-1 and IGF-2, which can be used in the present invention.

The family of epidermal growth factors (EGFs) are proteins with highly similar structural and functional characteristics and comprises the proteins epidermal growth factor (EGF), heparin-binding EGF-like growth factor (HB-EGF), transforming growth factor-α (TGF-α), amphiregulin (AR), epiregulin (EPR), epigen (EPGN), betacellulin (BTC), neuregulin-1 (NRG1), neuregulin-2 (NRG2), neuregulin-3 (NRG3), and neuregulin-4 (NRG4), preferably epidermal growth factor (EGF), heparin-binding EGF-like growth factor (HB-EGF), transforming growth factor-α (TGF-α), amphiregulin (AR), epiregulin (EPR), epigen (EPGN), and betacellulin (BTC), further preferably epidermal growth factor (EGF), heparin-binding EGF-like growth factor (HB-EGF), and transforming growth factor-α (TGF-α), which can be used in the present invention.

Transforming growth factor-α (TGF-α), preferably human transforming growth factor-α (hTGF-α), can be produced in macrophages, brain cells, and keratinocytes. hTGF-α induces epithelial development. hTGF-α and hEGF bind to the same receptor, epidermal growth factor receptor (EGFR; ErbB-1; HER1 in humans). When TGF-α binds to EGFR it can initiate multiple cell proliferation events including wound healing.

Human transforming growth factor-α exists in at least five isoforms produced by alternative splicing.

The amino acid sequence of the human transforming growth factor alpha isoform 1 precursor is available under the NCBI accession number NP_003227.1. The respective nucleic acid sequence of the mRNA is available under the NCBI accession number NM_003236.2.

The precursor of human transforming growth factor alpha isoform 1 includes a signal peptide, which spans the amino acids 1 to 23 of the precursor, protransforming growth factor alpha isoform 1 which spans the amino acids 24 to 160 of the precursor and the mature transforming growth factor alpha peptide, which spans the amino acids 40 to 89 of the precursor.

The amino acid sequence of the human transforming growth factor alpha isoform 2 precursor is available under the NCBI accession number NP_001093161.1. The respective nucleic acid sequence of the mRNA is available under the NCBI accession number NM_001099691.1.

The amino acid sequence of the human transforming growth factor alpha isoform 3 precursor is available under the NCBI accession number NP_001295087.1. The respective nucleic acid sequence of the mRNA is available under the NCBI accession number NM_001308158.1.

The amino acid sequence of the human transforming growth factor alpha isoform 4 precursor is available under the NCBI accession number NP_001295088.1. The respective nucleic acid sequence of the mRNA is available under the NCBI accession number NM_001308159.1.

The amino acid sequence of the human transforming growth factor alpha isoform 5 precursor is available under the NCBI accession number AAF05090.1. The respective nucleic acid sequence of the mRNA is available under the NCBI accession number AF149097.1.

Amphiregulin (AREG), preferably human amphiregulin (hAREG), is another ligand of the EGF receptor. Human amphiregulin is an autocrine growth factor as well as a mitogen for a broad range of target cells including astrocytes, schwann cells and fibroblasts. Human amphiregulin promotes the growth of epithelial cells.

The amino acid sequence of the human amphiregulin precursor is available under the NCBI accession number NP_001648.1. The respective nucleic acid sequence of the mRNA is available under the NCBI accession number NM_001657.3.

Epiregulin (EPR), preferably human epiregulin (hEPR), is a ligand of the EGF receptor which can stimulate cell proliferation and/or angiogenesis.

The amino acid sequence of the human epiregulin precursor is available under the NCBI accession number NP_001423.1. The respective nucleic acid sequence of the mRNA is available under the NCBI accession number NM_001432.1.

Epigen (EPGN), preferably human epigen (hEPGN), promotes the growth of epithelial cells. Human epigen exists in at least seven isoforms produced by alternative splicing.

The amino acid sequence of the human epigen isoform 1 precursor is available under the NCBI accession number NP_001257918.1. The respective nucleic acid sequence of the mRNA is available under the NCBI accession number NM_001270989.1.

The amino acid sequence of the human epigen isoforms 1 to 7 precursors is also available under UniProt accession number Q6UW88 - version 101.

Betacellulin (BTC), preferably human betacellulin (hBTC), is a growth factor that also binds to epidermal growth factor receptor and that is synthesized by a wide range of adult tissues and in many cultured cells, including smooth muscle cells and epithelial cells. The amino acid sequence of the human probetacellulin precursor is available under the NCBI accession number NP_001720.1. The respective nucleic acid sequence of the mRNA is available under the NCBI accession number NM_001729.1.

Insulin-like growth factor 1 (IGF-1) is also called somatomedin C. The nucleic acid sequence of the mRNA of the human IGF-1 is available under the NCBI accession number NM_000618.2. The respective amino acid sequence is available under NCBI accession number NP_000609.1 as well as the UniProt accession number P05019 - version 178.

The nucleic acid sequence of the mRNA of the human insulin-like growth factor 2 (hIGF-2) is available under the NCBI accession number NM_000612.4. The respective amino acid sequence of the human insulin-like growth factor 2 precursor is available under the NCBI accession number NP_000603.1 as well as the UniProt accession number P01344 - version 192.

The family of vascular endothelial growth factors (VEGF) is a group of growth factors which include VEGF-A, VEGF-B, VEGF-C, VEGF-D and placental growth factor (PGF), which can be used in the present invention.

In a preferred embodiment, the vascular endothelial growth factor is the vascular endothelial growth factor A (VEGF-A). VEGF-A can induce angiogenesis, vasculogenesis and endothelial cell growth.

The nucleic acid sequence of the mRNA of the human vascular endothelial growth factor A (hVEGF-A) is available under the NCBI accession number NM_001025366.1. The respective amino acid sequence of the human vascular endothelial growth factor A is available under the NCBI accession number NP_001020537.2 as well as the UniProt accession number P15692 - version 197.

Platelet-derived growth factor (PDGF) regulates cell growth and division. Human platelet-derived growth factor (hPDGF) has four subunits, PDGF-A, PDGF-B, PDGF-C and PDGF-D, which form either homo- or heterodimers of the respective subunits, which can be used in the present invention.

Preferably, the platelet-derived growth factor is PDGF-AA, PDGF-BB, PDGF-AB, PDGF-CC, PDGF-DD, or a mixture thereof.

Further preferably, the platelet-derived growth factor is a dimeric protein composed of two PDGF-A subunits, a dimeric protein composed of two PDGF-B subunits, a dimeric protein composed of a PDGF-A subunit and a PDGF-B subunit, or a mixture thereof.

The nucleic acid sequence of the mRNA of the human platelet-derived growth factor subunit A (hPDGF-A) is available under the NCBI accession number NM_002607.4. The respective amino acid sequence is available under the NCBI accession number NP_002598.4 as well as the UniProt accession number P04085 - version 159.

The nucleic acid sequence of the mRNA of the human platelet-derived growth factor subunit B (hPDGF) is available under the NCBI accession number NM_002608.1. The respective amino acid sequence of the human platelet-derived growth factor subunit precursor is available under the NCBI accession number NP_002599.1 as well as the UniProt accession number P01127 - version 181.

Hepatocyte growth factor (HGF) is a growth factor which is secreted by mesenchymal cells and acts primarily upon epithelial cells and endothelial cells but also on hemapoeitic progenitor cells and can be used in the present invention.

The nucleic acid sequence of the mRNA of the human hepatocyte growth factor (hHGF) is available under the NCBI accession number NM_000601.3. The respective amino acid sequence of the human hepatocyte growth factor precursor is available under the NCBI accession number NP_000592.3 as well as the UniProt accession number P14210 - version 186.

Transforming growth factor β (TGF-β), preferably human transforming growth factor β (hTGF-β), is a cytokine which is secreted by many cell types, including macrophages.

TGF-β exists in at least three isoforms, TGF-β1, TFG-β2 and TGF-β3, which can be used in the present invention.

Human transforming growth factor β 1 is a secreted protein that is cleaved into a latency-associated peptide (LAP) and a mature TGF-β1 peptide. The mature peptide may either form TGF-β1 homodimers or heterodimers with other TGF-β family members.

The nucleic acid sequence of the mRNA of the human transforming growth factor β1 precursor can be obtained by the NCBI accession number NM_000660.4. The respective amino acid sequence is available under the NCBI accession number NP_000651.3 or the UniProt accession number P01137 - version 199.

Transforming growth factor β2 (TGF-β2), preferably human transforming growth factor β2 (hTGF-β2), is a multifunctional cytokine that regulates proliferation, differentiation, adhesion, and migration of many cell types.

Alternatively, spliced transcript variants of the human transforming growth factor β2 gene have been identified, which encode two different isoforms.

The nucleic acid sequence of the mRNA of the human transforming growth factor beta 2 isoform 1 precursor is available under the NCBI accession number NM_001135599.3. The respective amino acid sequence is available under the NCBI accession number NP_001129071.1.

The nucleic acid sequence of the mRNA of the human transforming growth factor β2 isoform 2 precursor is available under the NCBI accession number NM_003238.3. The respective amino acid sequence is available under the NCBI accession number NP_003229.1. The amino acid sequence of transforming growth factor β2 is further available under the UniProt accession number P61812 - version 128.

Transforming growth factor β3 (TGF-β3), preferably human transforming growth factor β3 (hTGF-β3), is a secreted cytokine that is involved in embryogenesis and cell differentiation.

The nucleic acid sequence of the mRNA of the human transforming growth factor β3 precursor protein is available under the NCBI accession number NM_003239.3. The corresponding amino acid sequence is available under the NCBI accession number NP_003230.1 as well as the UniProt accession number P10600 - version 170.

Activins are disulfide-linked dimeric proteins originally purified from gonadal fluids as proteins that stimulated pituitary follicle stimulating hormone (FSH) release. Activin proteins have a wide range of biological activities, including mesoderm induction, neural cell differentiation, bone remodelling, haematopoiesis and roles in reproductive physiology.

Activins are homodimers or heterodimers of the various beta subunit isoforms, while inhibins are heterodimers of a unique alpha subunit and one of the four beta subunits, beta A, beta B, beta C, and beta E.

Cytokines are preferably polypeptides that are involved in autocrine signalling, paracrine signalling and endocrine signalling as immunomodulating agents.

Preferably, cytokines are selected from the group consisting of interferons, interleukins, lymphokines, tumour necrosis factors, colony-stimulating factors, functional analogues thereof, biosimilars thereof, and mixtures thereof.

Preferably, interferons, further preferably human interferons, are selected from the group consisting of interferon alpha (IFN-α), interferon beta (IFN-β), interferon epsilon (IFN-ε), interferon kappa (IFN-κ), interferon gamma (IFN-γ), interferon omega (IFN-ω), interferon lambda (IFN-λ), functional analogues thereof, biosimilars thereof, and mixtures thereof.

Interferon alpha, preferably human interferon alpha, is preferably selected from the group consisting of interferon alpha-1 (IFN-α1), interferon alpha-2 (IFN-α2), interferon alpha-4 (IFN-α4), interferon alpha-5 (IFN-α5), interferon alpha-6 (IFN-α6), interferon alpha-7 (IFN-α7), interferon alpha-8 (IFN-α8), interferon alpha-10 (IFN-α10), interferon alpha-13 (IFN-α13)interferon alpha-14 (IFN-α14), interferon alpha-16 (IFN-α16), interferon alpha-17 (IFN-α17), interferon alpha-21 (IFN-α21), functional analogues thereof, biosimilars thereof, and mixtures thereof, preferably interferon alpha-2 (IFN-α2), functional analogues thereof, biosimilars thereof, and mixtures thereof.

The nucleic acid sequence of the mRNA of human interferon alpha 1 (IFN-α1) is available under the NCBI accession number NM_024013.2. The nucleic acid sequence of the mRNA of human interferon alpha 13 (IFN-α13) is available under the NCBI accession number NM_006900.3.

Human interferons alpha-1 and alpha-13 have identical protein sequences. The respective amino acid sequences of the human interferon alpha-1/13 precursor is available under the NCBI accession number NP_076918.1 as well as NP_008831.3 as well as the UniProt accession number P01562 - version 180.

The precursor of human interferon alpha-1/13 includes a signal peptide, which spans the amino acids 1 to 23 of the precursor, and the mature interferon alpha-2, which spans the amino acids 24 to 189 of the precursor.

The nucleic acid sequence of the mRNA of human interferon alpha 2 (IFN-α2) is available under the NCBI accession number NM_000605.3. The respective amino acid sequence of the human interferon alpha-2 precursor is available under the NCBI accession number NP_000596.2 as well as the UniProt accession number P01563 - version 176.

The precursor of human interferon alpha-2 includes a signal peptide, which spans the amino acids 1 to 23 of the precursor, and the mature interferon alpha-2, which spans the amino acids 24 to 188 of the precursor.

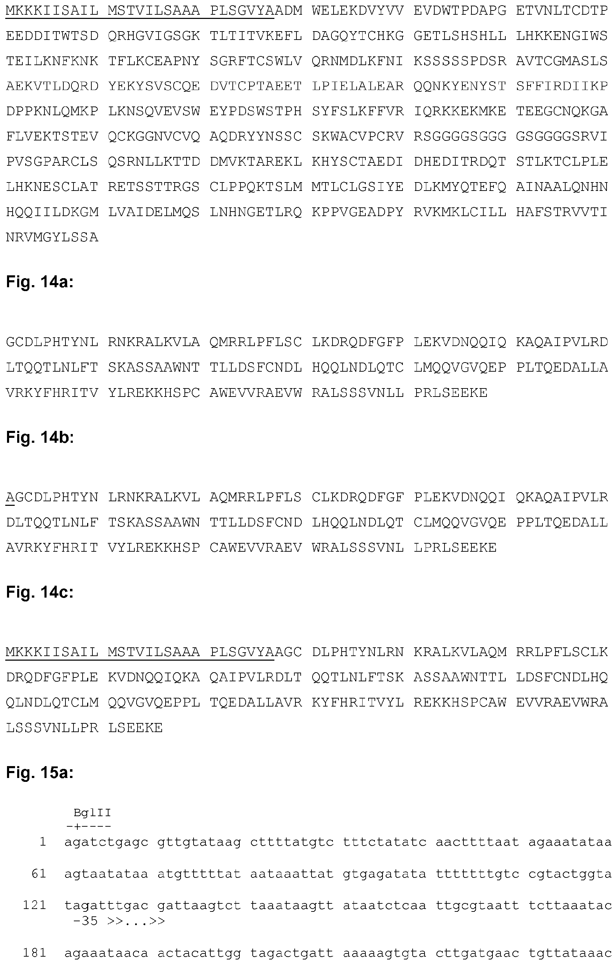

Further preferably, interferon alpha-2 comprises one or at least one of the amino acid sequences of SEQ ID Nos 28 to 30, which are also depicted in Figures 14a to 14c, respectively.

The nucleic acid sequence of the mRNA of human interferon alpha 4 (IFN-α4) is available under the NCBI accession number NM_021068.2. The respective amino acid sequence of the human interferon alpha-4 precursor is available under the NCBI accession number NP_066546.1 as well as the UniProt accession number P05014 - version 168.

The precursor of human interferon alpha-4 includes a signal peptide, which spans the amino acids 1 to 23 of the precursor, and the mature interferon alpha-4, which spans the amino acids 24 to 189 of the precursor.

The nucleic acid sequence of the mRNA of human interferon alpha 5 (IFN-$\alpha$5) is available under the NCBI accession number NM_002169.2. The respective amino acid sequence of the human interferon alpha-5 precursor is available under the NCBI accession number NP_002160.1 as well as the UniProt accession number P01569 - version 158.

The precursor of human interferon alpha-5 includes a signal peptide, which spans the amino acids 1 to 21 of the precursor, and the mature interferon alpha-5, which spans the amino acids 22 to 189 of the precursor.

The nucleic acid sequence of the mRNA of human interferon alpha 6 (IFN-$\alpha$6) is available under the NCBI accession number NM_021002.2. The respective amino acid sequence of the human interferon alpha-6 precursor is available under the NCBI accession number NP_066282.1 as well as the UniProt accession number P05013 - version 158.

The precursor of human interferon alpha-6 includes a signal peptide, which spans the amino acids 1 to 20 of the precursor, and the mature interferon alpha-6, which spans the amino acids 21 to 189 of the precursor.

The nucleic acid sequence of the mRNA of human interferon alpha 7 (IFN-$\alpha$7) is available under the NCBI accession number NM_021057.2. The respective amino acid sequence of the human interferon alpha-7 precursor is available under the NCBI accession number NP_066401.2 as well as the UniProt accession number P01567 - version 160.

The precursor of human interferon alpha-7 includes a signal peptide, which spans the amino acids 1 to 23 of the precursor, and the mature interferon alpha-7, which spans the amino acids 24 to 189 of the precursor.

The nucleic acid sequence of the mRNA of human interferon alpha 8 (IFN-$\alpha$8) is available under the NCBI accession number NM_002170.3. The respective amino acid sequence of the human interferon alpha-8 precursor is available under the NCBI accession number NP_002161.2 as well as the UniProt accession number P32881 - version 157.

The precursor of human interferon alpha-8 includes a signal peptide, which spans the amino acids 1 to 23 of the precursor, and the mature interferon alpha-8, which spans the amino acids 24 to 189 of the precursor.

The nucleic acid sequence of the mRNA of human interferon alpha 10 (IFN-$\alpha$10) is available under the NCBI accession number NM_002171.2. The respective amino acid sequence of the human interferon alpha-10 precursor is available under the NCBI accession number NP_002162.1 as well as the UniProt accession number P01566 - version 160.

The precursor of human interferon alpha-10 includes a signal peptide, which spans the amino acids 1 to 23 of the precursor, and the mature interferon alpha-10, which spans the amino acids 24 to 189 of the precursor.

The nucleic acid sequence of the mRNA of human interferon alpha 14 (IFN-$\alpha$14) is available under the NCBI accession number NM_002172.2. The respective amino acid sequence of the human interferon alpha-14 precursor is available under the NCBI accession number NP_002163.2 as well as the UniProt accession number P01570 - version 172.

The precursor of human interferon alpha-14 includes a signal peptide, which spans the amino acids 1 to 23 of the precursor, and the mature interferon alpha-14, which spans the amino acids 24 to 189 of the precursor

The nucleic acid sequence of the mRNA of human interferon alpha 16 (IFN-$\alpha$16) is available under the NCBI accession number NM_002173.3. The respective amino acid sequence of the human interferon alpha-16 precursor is available under the NCBI accession number NP_002164.1 as well as the UniProt accession number P05015 - version 161.

The precursor of human interferon alpha-16 includes a signal peptide, which spans the amino acids 1 to 23 of the precursor, and the mature interferon alpha-16, which spans the amino acids 24 to 189 of the precursor.

The nucleic acid sequence of the mRNA of human interferon alpha 17 (IFN-$\alpha$17) is available under the NCBI accession number NM_021268.2. The respective amino acid sequence of the human interferon alpha-17 precursor is available under the NCBI accession number NP_067091.1 as well as the UniProt accession number P01571 - version 162.

The precursor of human interferon alpha-17 includes a signal peptide, which spans the amino acids 1 to 23 of the precursor, and the mature interferon alpha-17, which spans the amino acids 24 to 189 of the precursor.

The nucleic acid sequence of the mRNA of human interferon alpha 21 (IFN-$\alpha$21) is available under the NCBI accession number NM_002175.2. The respective amino acid sequence of the human interferon alpha-21 precursor is available under the NCBI accession number NP_002166.2 as well as the UniProt accession number P01568 - version 171.

The precursor of human interferon alpha-21 includes a signal peptide, which spans the amino acids 1 to 23 of the precursor, and the mature interferon alpha-21, which spans the amino acids 24 to 189 of the precursor.

The nucleic acid sequence of the mRNA of human interferon beta 1 (IFN-$\beta$1) is available under the NCBI accession number NM_002176.3. The respective amino acid sequence of the human interferon beta precursor is available under the NCBI accession number NP_002167.1 as well as the UniProt accession number P01574 - version 196.

The precursor of human interferon beta includes a signal peptide, which spans the amino acids 1 to 21 of the precursor, and the mature interferon beta, which spans the amino acids 22 to 187 of the precursor.

The nucleic acid sequence of the mRNA of human interferon gamma (IFN-$\gamma$) is available under the NCBI accession number NM_000619.2. The respective amino acid sequence of the human interferon gamma precursor is available under the NCBI accession number NP_000610.2 as well as the UniProt accession number P01579 - version 205.

The precursor of human interferon gamma includes a signal peptide, which spans the amino acids 1 to 23 of the precursor, and the mature interferon gamma, which spans the amino acids 24 to 161 of the precursor, and a propeptide, which spans the amino acids 162 to 166 of the precursor.

The nucleic acid sequence of the mRNA of human interferon kappa (IFN-κ) is available under the NCBI accession number NM_020124.2. The respective amino acid sequence of the human interferon kappa precursor is available under the NCBI accession number NP_064509.2 as well as the UniProt accession number Q9P0W0 - version 120.

The precursor of human interferon kappa includes a signal peptide, which spans the amino acids 1 to 27 of the precursor, and the mature interferon gamma, which spans the amino acids 28 to 207 of the precursor.

The nucleic acid sequence of the mRNA of human interferon epsilon (IFN-ε) is available under the NCBI accession number NM_176891.4. The respective amino acid sequence of the human interferon epsilon precursor is available under the NCBI accession number NP_795372.1 as well as the UniProt accession number Q86WN2 - version 124.

The precursor of human interferon epsilon includes a signal peptide, which spans the amino acids 1 to 21 of the precursor, and the mature interferon epsilon, which spans the amino acids 22 to 208 of the precursor.

The nucleic acid sequence of the mRNA of human interferon omega 1 (IFN-ω1) is available under the NCBI accession number NM_002177.2. The respective amino acid sequence of the human interferon omega precursor is available under the NCBI accession number NP_002168.1 as well as the UniProt accession number P05000 - version 165.

The precursor of human interferon omega includes a signal peptide, which spans the amino acids 1 to 21 of the precursor, and the mature interferon omega, which spans the amino acids 22 to 195 of the precursor.

Human interferon lambda (IFN-λ) is preferably selected from the group consisting of interferon lambda-1 (IFN-λ1), interferon lambda-2 (IFN-λ2), interferon lambda-3 (IFN-λ3), interferon lambda-4 (IFN-λ4), functional analogues thereof, biosimilars thereof, and mixtures thereof.

The nucleic acid sequence of the mRNA of human interferon lambda 1 (IFN-λ1) is available under the NCBI accession number NM_172140.1. The respective amino acid sequence of the human interferon lambda-1 precursor is available under the NCBI accession number NP_742152.1 as well as the UniProt accession number Q8IU54 - version 125.

The precursor of human interferon lambda-1 includes a signal peptide, which spans the amino acids 1 to 19 of the precursor, and the mature interferon lambda-1, which spans the amino acids 20 to 200 of the precursor.

The nucleic acid sequence of the mRNA of human interferon lambda 2 (IFN-λ2) is available under the NCBI accession number NM_172138.1. The respective amino acid sequence of the human interferon lambda-2 precursor is available under the NCBI accession number NP_742150.1 as well as the UniProt accession number Q8IZJ0 - version 105.

The precursor of human interferon lambda-2 precursor includes a signal peptide, which spans the amino acids 1 to 25 of the precursor, and the mature interferon lambda-2, which spans the amino acids 26 to 200 of the precursor.

The nucleic acid sequence of the mRNA of human interferon lambda 3 (IFN-λ3) comprise two transcript variants. The sequence of the mRNA of human interferon lambda 3 transcript variant 1 is available under the NCBI accession number NM_001346937.1. The sequence of the mRNA of human interferon lambda 3 transcript variant 2 is available under the NCBI accession number NM_172139.3.

The respective amino acid sequence of the human interferon lambda-3 isoform 1 precursor is available under the NCBI accession number NP_001333866.1. The respective amino acid sequence of the human interferon lambda-3 isoform 2 precursor is available under the NCBI accession number NP_742151.2 as well as the UniProt accession number Q8IZI9 - version 113.

The precursor of human interferon lambda-3 isoform 2 includes a signal peptide, which spans the amino acids 1 to 21 of the precursor, and the mature interferon lambda-3, which spans the amino acids 22 to 196 of the precursor.

The nucleic acid sequence of the mRNA of human interferon lambda 4 (IFN-λ4) is available under the NCBI accession number NM_001276254.2. The respective amino acid sequence of the human interferon lambda-4 precursor is available under the NCBI accession number NP_001263183.2 as well as the UniProt accession number K9M1U5 - version 24.

The precursor of human interferon lambda-4 includes a signal peptide, which spans the amino acids 1 to 21 of the precursor, and the mature interferon lambda-4, which spans the amino acids 22 to 179 of the precursor.

Preferably, interleukins, further preferably human interleukins, are selected from the group consisting of interleukin-1 (IL-1), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), interleukin-13 (IL-13), interleukin-14 (IL-14), interleukin-15 (IL-15), interleukin-16 (IL-16), interleukin-17 (IL-17), interleukin-18 (IL-18), interleukin-19 (IL-19), interleukin-20 (IL-20), interleukin-21 (IL-21), interleukin-22 (IL-22), interleukin-23 (IL-23), interleukin-24 (IL-24), interleukin-25 (IL-25), interleukin-26 (IL-26), interleukin-27 (IL-27), interleukin-28 (IL-28), interleukin-29 (IL-29), interleukin-30 (IL-30), interleukin-31 (IL-31), interleukin-32 (IL-32), interleukin-33 (IL-33), interleukin 34 (IL-34), interleukin-36 alpha (IL-36α), interleukin-36 beta (IL-36β), interleukin-36 gamma (IL-36γ), interleukin-37 (IL-37), cardiotrophin-like cytokine factor 1 (CLCF1), ciliary neurotrophic factor (CNTF), leukemia inhibitory factor (LIF), oncostatin-M (OSM), interleukin receptor antagonist proteins, interleukin binding proteins, functional analogues thereof, biosimilars thereof, and mixtures thereof, preferably interleukin-2 (IL-2), interleukin-12 (IL-12), interleukin-18 (IL-18), functional analogues thereof, biosimilars thereof, and mixtures thereof.

Preferably, interleukin-1 (IL-1), further preferably human interleukin-1, is selected from the group consisting of interleukin-1 alpha (IL-1α), interleukin-1 beta (IL-1β), functional analogues thereof, biosimilars thereof, and mixtures thereof.

The nucleic acid sequence of the mRNA of human interleukin-1 alpha (IL-1α) precursor is available under the NCBI accession number NM_000575.4. The respective amino acid sequence of the precursor is available under the NCBI accession number NP_000566.3.

The amino acid sequence of human interleukin-1 alpha precursor is also available under the UniProt accession number P01583 - version 1908. The amino acid sequence comprises a propeptide, which spans the amino acids 1 to 112 of the interleukin-1 alpha precursor, and the mature interleukin-1 alpha, which spans the amino acids 113 to 271 of the precursor.

The nucleic acid sequence of the mRNA of human interleukin-2 precursor is available under the NCBI accession number NM_000586.3. The respective amino acid sequence is available under the NCBI accession number NP_000577.2.

The amino acid sequence of human interleukin-2 precursor is also available under the UniProt accession number P60568 - version 161. The amino acid sequence comprises a signal peptide, which spans the amino acids 1 to 20 of the interleukin-2 precursor, and the mature interleukin-2, which spans the amino acids 21 to 153 of the precursor.

The nucleic acid sequence of the mRNA of human interleukin-3 precursor is available under the NCBI accession number NM_000586.3. The respective amino acid sequence is available under the NCBI accession number NP_000577.2.

The amino acid sequence of human interleukin-3 precursor is also available under the UniProt accession number P08700 - version 177. The amino acid sequences comprise a signal peptide, which spans the amino acids 1 to 19 of the interleukin-3 precursor, and the mature interleukin 3, which spans the amino acids 20 to 152 of the precursor.

Interleukin-4 (IL-4), preferably human interleukin-4 (hIL-4), is a pleiotropic cytokine. Interleukin-4 is a ligand for the interleukin-4 receptor.

The nucleic acid sequence of the mRNA of human interleukin-4 isoform 1 precursor is available under the NCBI accession number NM_000589.3. The respective amino acid sequence of interleukin-4 isoform 1 precursor is available under the NCBI accession number NP _000580.1.

The nucleic acid sequence of the mRNA of the human interleukin-4 isoform 2 precursor is available under the NCBI accession number NM_172348.2. The respective amino acid sequence of interleukin-4 isoform 2 precursor is available under the NCBI accession number NP_758858.1

The amino acid sequence of human interleukin-4 precursors is also available under the UniProt accession number P05112 - version 178. The amino acid sequence comprises a signal peptide which spans the amino acids 1 to 24 of the interleukin 4 isoform 1 and isoform 2 precursors.

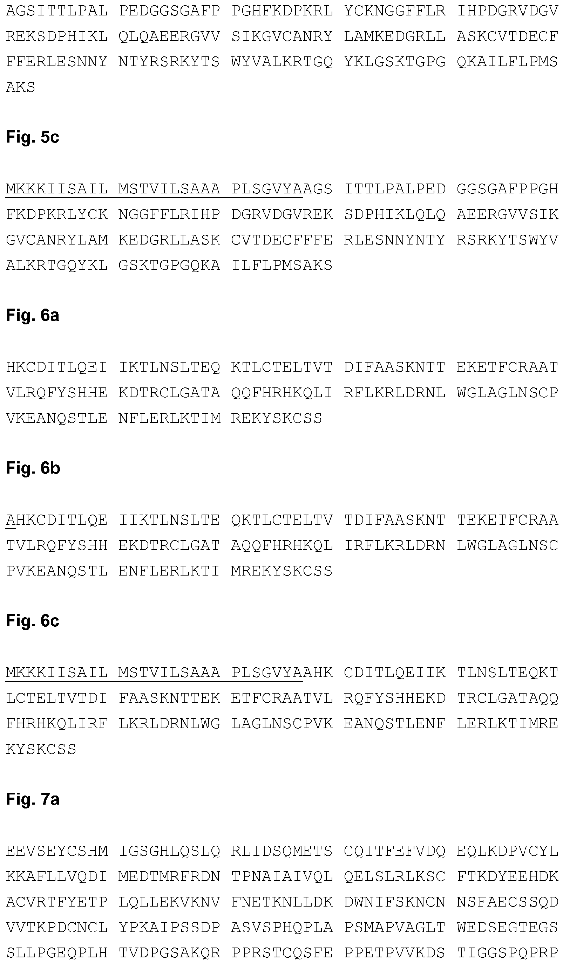

Preferably, interleukin-4 comprises one or at least one of the amino acid sequences of SEQ ID Nos 8 to 10, which are also depicted in Figures 6a to 6c, respectively.

The nucleic acid sequence of the mRNA of the human interleukin-5 precursor is available under the NCBI accession number NM_000879.2. The respective amino acid sequence is available under the NCBI accession number NP_000870.1.

The amino acid sequence of human interleukin-5 precursor is also available under the UniProt accession number P05113 - version 189. The amino acid sequences comprise a signal peptide, which spans the amino acids 1 to 19 of the interleukin-5 precursor, and the mature interleukin-5, which spans the amino acids 20 to 134 of the precursor.

The nucleic acid sequence of the mRNA of transcript variant 1 of human interleukin-6 precursor is available under the NCBI accession number NM_000600.4. The respective amino acid sequence of interleukin-6 isoform 1 precursor is available under the NCBI accession number NP_000591.1.

The amino acid sequence of human interleukin-6 precursor is also available under the UniProt accession number P05231 - version 212. The amino acid sequences comprise a signal peptide, which spans the amino acids 1 to 29 of the interleukin-6 precursor, and the mature interleukin-6, which spans the amino acids 30 to 212 of the precursor.

The nucleic acid sequence of the mRNA of transcript variant 1 of human interleukin-7 precursor is available under the NCBI accession number NM_000880.3. The respective amino acid sequence of interleukin-7 isoform 1 precursor is available under the NCBI accession number NP_000871.1.

The amino acid sequence of human interleukin-7 precursor is also available under the UniProt accession number P05231 - version 212. The amino acid sequence of human interleukin-7 isoform 1 precursor comprises a signal peptide, which spans the amino acids 1 to 25 of the isoform 1 precursor, and the mature interleukin-7, which spans the amino acids 26 to 177 of the isoform 1 precursor.

The nucleic acid sequence of the mRNA of transcript variant 1 of human interleukin-8 precursor is available under the NCBI accession number NM_000584.3. The respective amino acid sequence of interleukin-8 isoform 1 precursor is available under the NCBI accession number NP_000575.1.

The amino acid sequence of human interleukin-8 precursor is also available under the UniProt accession number P10145 - version 210. The amino acid sequence of human interleukin-8 isoform 1 precursor comprises a signal peptide, which spans the amino acids 1 to 20 of the isoform 1 precursor. The precursor peptide of 99 amino acids undergoes cleavage to create several active IL8 isoforms.

Preferably the mature human interleukin-8 comprises the amino acids 31 to 99 of the interleukin-8 isoform 1 precursor.

Interleukin-9 (IL-9), preferably human interleukin-9 (hIL-9), is produced by variety of cells. The nucleic acid sequence of the mRNA of the human interleukin-9 precursor can be found under the NCBI accession number NM_000590.1. The respective amino acid sequence can be found under the NCBI accession number NP_000581.1 as well as the UniProt accession number P15248 - version 153.

The amino acid sequence of the human interleukin-9 precursor includes a signal peptide spanning the amino acids 1 to 18 of the human interleukin-9 precursor protein and the mature interleukin-9, which spans the amino acids 19 to 144 of the precursor.

Interleukin-10 (IL-10), preferably human interleukin-10 (hIL-10), is a cytokine produced primarily by monocytes. The nucleic acid sequence of the mRNA of the human interleukin-10 precursor can be found under the NCBI accession number NM_000572.2. The respective amino acid sequence can be found under the NCBI accession number NP_000563.1 as well as the UniProt accession number P22301 - version 156.

The amino acid sequence of the human interleukin-10 precursor includes a signal peptide spanning the amino acids 1 to 18 of the human interleukin-10 precursor protein.

Interleukin-12 is a heterodimeric cytokine encoded by two separate genes, interleukin-12 subunit alpha (IL-12A), also designated p35, and interleukin-12 subunit beta (IL-12B), also designated p40. The active heterodimer, which is referred to as "p70", and a homodimer of p40 are formed following protein synthesis.

The nucleic acid sequence of the mRNA of the human interleukin-12 subunit alpha precursor comprises three transcript variants, which are available under the NCBI accession numbers NM_000882.4 (variant 1), NM_001354582.1 (variant 2) and NM_001354583.1 (variant 3).

The respective amino acid sequences of human interleukin-12 subunit alpha precursor isoforms 1, 2, and 3 are available under the NCBI accession number NP_000873.2, NP_001341511.1, and NP_001341512.1, respectively, as well as the UniProt accession number P29459 - version 170.

The human interleukin-12 subunit alpha isoform 1 precursor comprises a signal peptide which spans the amino acids 1 to 56 of the precursor protein deposited under NCBI Reference Sequence: NP_000873.2.

Human IL12A variants 2 and 3 lack an alternate in-frame exon compared to variant 1. The resulting isoforms 2 and 3 have the same N- and C-termini but are shorter compared to isoform 1.

The nucleic acid sequence of the mRNA of the mouse interleukin-12 subunit alpha precursor comprises two transcript variants, which are available under the NCBI accession numbers NM_001159424.2 (variant 1), and NM_008351.3 (variant 2).

The respective amino acid sequences of mouse interleukin-12 subunit alpha precursor isoforms 1, and 2 are available under the NCBI accession number NP_001152896.1, and NP_032377.1, respectively, as well as the UniProt accession number P43431- version 140.

The mouse interleukin-12 subunit alpha isoform 2 precursor comprises a signal peptide which spans the amino acids 1 to 22 of the precursor protein deposited under NCBI Reference Sequence: NP_032377.1.

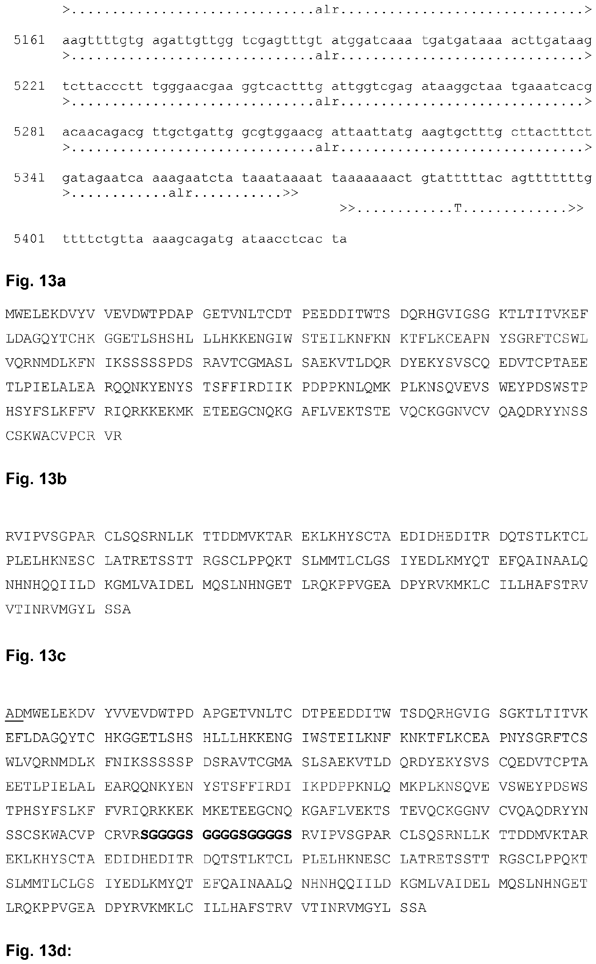

Preferably, interleukin-12 subunit alpha comprises the amino acid sequences of the mature mouse interleukin-12 subunit alpha isoform 2, which is depicted in Figure 13b and SEQ ID No 25.

The nucleic acid sequence of the mRNA of the human interleukin-12 subunit beta precursor is available under the NCBI accession number NM_002187.2. The respective amino acid sequence is available under the NCBI accession number NP_002178.2 as well as the UniProt accession number P29460 - version 210.

The human interleukin-12 subunit beta precursor precursor comprises a signal peptide which spans the amino acids 1 to 22 of the amino acid sequence deposited under NCBI Reference Sequence NP_002178.2.

The nucleic acid sequence of the mRNA of the mouse interleukin-12 subunit beta precursor is available under the NCBI accession number NM_001303244.1. The respective amino acid sequence is available under the NCBI accession number NP_001290173.1 as well as the UniProt accession number P43432 - version 163.

The mouse interleukin-12 subunit beta precursor precursor comprises a signal peptide which spans the amino acids 1 to 22 of the amino acid sequence deposited under NCBI Reference Sequence NP_001290173.1.

Preferably, interleukin-12 subunit beta comprises the amino acid sequences of the mature mouse interleukin-12 subunit beta, which is depicted in Figure 13a and SEQ ID No 24.

Further preferably, interleukin-12 is expressed as recombinant fusion protein comprising the mature form of interleukin-12 subunit alpha and the mature form of interleukin-12 subunit beta, preferably linked by a linker sequence, further preferably comprising at least one amino acid.

Further preferably, interleukin-12 is expressed as recombinant fusion protein comprising one or at least one of the amino acid sequences of SEQ ID Nos 26 to 27, which are also depicted in Figures 13c and 13d, respectively.

Interleukin-13 (IL-13), preferably human interleukin-13 (hIL-13), is an immuno-regulatory cytokine. The nucleic acid sequence of the mRNA of the human interleukin-13 precursor is available under the NCBI accession number NM_002188.2. The respective amino acid sequence is available under the NCBI accession number NP_002179.2 as well as the UniProt accession number P35225 - version 157.

The interleukin-13 precursor comprises a signal peptide which spans the amino acids 1 to 24 of the interleukin-13 precursor protein deposited under UniProt accession number P35225 - version 157.

Interleukin-18 (IL-18) is a cytokine that belongs to the IL-1 superfamily and is produced for example by macrophages.

The nucleic acid sequence of the mRNA of the human interleukin-18 (hIL-18) precursor comprises two transcript variants, which are available under the NCBI accession numbers NM_001562.4 (variant 1) and NM_001243211.1 (variant 2).

Transcript variant 1 represents the predominant and longer transcript, and encodes the longer hIL-18 isoform 1.

Transcript variant 2, which is also known as Delta3pro-IL-18, lacks an in-frame coding exon compared to variant 1. The encoded hIL-18 isoform 2 is shorter and may be resistant to proteolytic activation, compared to hIL-18 isoform 1.

The respective amino acid sequences of human interleukin-18 precursor isoforms 1, and 2 are available under the NCBI accession number NP_001553.1, and NP_001230140.1, respectively, as well as the UniProt accession number Q14116 - version 1174.

The human interleukin-18 isoform 1 precursor comprises a propeptide which spans the amino acids 1 to 36 of the precursor protein deposited under NCBI Reference Sequence: NP_001553.1 and which is processed *in vivo* by caspase 1 (CASP1) or caspase 4 (CASP4) to yield the active form.

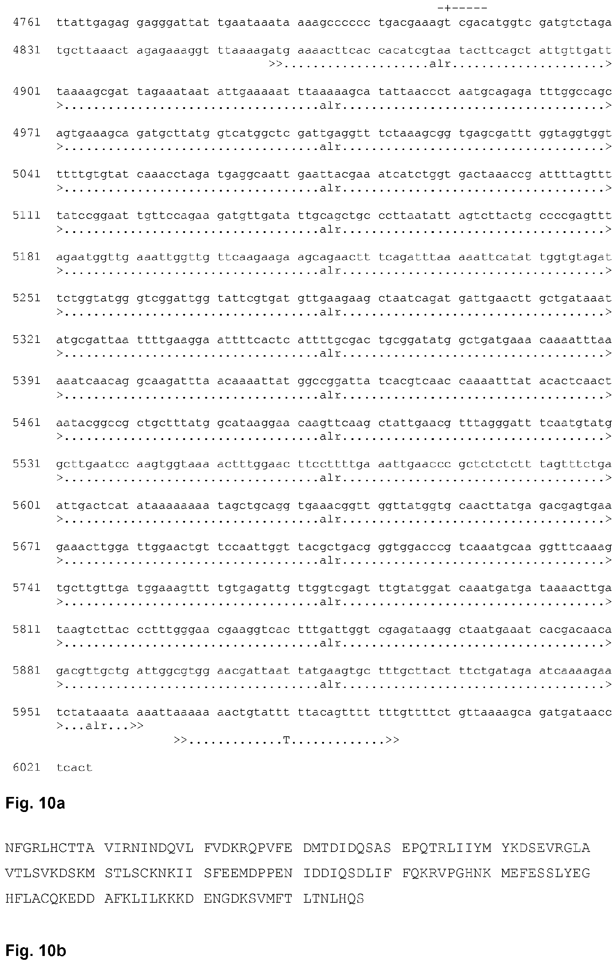

Preferably, interleukin-18 comprises one or at least one of the amino acid sequences of SEQ ID Nos 16 to 18, which are also depicted in Figures 10a to 10c, respectively.

Interleukin-34 (IL-34) is a cytokine that also promotes the differentiation and viability of monocytes and macrophages. Due to alternative splicing, human interleukin-34 exists in two isoforms, which can be used in the present invention.

The nucleic acid sequence of the mRNA of the human interleukin-34 isoform 1 precursor is available under the NCBI accession number NM_001172772.1. The corresponding amino acid sequence is available under the NCBI accession number NP_001166243.1.

The nucleic acid sequence of the mRNA of the human interleukin-34 isoform 2 precursor is available under the NCBI accession number NM_001172771.1. The corresponding amino acid sequence is available under the NCBI accession number NP_001166242.1.

The respective amino acid sequence is also available under the UniProt accession number Q6ZMJ4 - version 80. The human interleukin-34 precursor includes a signal peptide, which spans the amino acids 1 to 20 of the respective amino acid sequences of the precursor proteins.

Interleukin receptor antagonist proteins are preferably selected from interleukin-1 receptor antagonist protein (IL-1 RA), interleukin-36 receptor antagonist protein (IL-36RA) and mixtures thereof.

A suitable interleukin binding protein is preferably interleukin-18 binding protein (IL-18BP).

Colony-stimulating factors are preferably polypeptides capable of stimulating proliferation, healing, and/or cellular differentiation.

Preferably, the colony-stimulating factor is selected from the group consisting of colony-stimulating factor 1 (CSF-1), granulocyte macrophage colony-stimulating factor (GM-CSF), granulocyte colony-stimulating factor (G-CSF), erythropoietin, thrombopoietin, functional analogues thereof, biosimilars thereof, and mixtures thereof, preferably colony-stimulating factor 1 (CSF-1), granulocyte macrophage colony-stimulating factor (GM-CSF), granulocyte colony-stimulating factor (G-CSF), erythropoietin, functional analogues thereof, biosimilars thereof, and mixtures thereof.

Colony stimulating factor-1 (CSF-1), which is also known as macrophage colony-stimulating factor (M-CSF), is a cytokine that controls the production, differentiation, and function of macrophages.

Due to alternative splicing, the human CSF-1 exists in different isoforms, which can be used in the present invention.

The nucleic acid sequence of the mRNA of the human CSF-1 isoform 1, which is also designated as macrophage colony-stimulating factor 1 isoform A precursor, is available under the NCBI accession number NM_000757.5. The corresponding amino acid sequence is available under the NCBI accession number NP_000748.3.

The nucleic acid sequence of the mRNA of the human CSF-1 isoform 2 precursor, which is also designated human macrophage colony-stimulating factor 1 isoform B precursor, is available under the NCBI accession number NM_172210.2. The corresponding amino acid sequence is available under the NCBI accession number NP_757349.1.

The nucleic acid sequence of the mRNA of the human CSF-1 isoform 3 precursor, which is also designated as macrophage colony-stimulating factor 1 isoform C precursor, is available under the NCBI accession number NM_172211.3. The corresponding amino acid sequence is available under the NCBI accession number NP_757350.1.

The respective amino acid sequences are also available under the UniProt accession number P09603 - version 158. Isoform 1 has been chosen as canonical UniProt sequence.

The respective protein sequences of the human CSF-1 precursor isoforms 1 to 3 include an N-terminal signal peptide, which spans the amino acid number 1 to amino acid number 32 of the respective amino acid sequences.

The active form of human CSF-1 can be found extracellularly as a disulfide-linked homodimer. The active form is produced by proteolytic cleavage of a membrane-bound precursor resulting in the loss of the N-terminal signal peptide.

Preferably, colony stimulating factor 1 comprises one or at least one of the amino acid sequences of SEQ ID Nos 11 to 13, which are also depicted in Figures 7a to 7c, respectively.

Granulocyte-macrophage colony-stimulating factor (GM-CSF) is also known as colony-stimulating factor 2 (CSF-2)

The nucleic acid sequence of the mRNA of human granulocyte-macrophage colony-stimulating factor precursor is available under the NCBI accession number NM_000758.3. The respective amino acid sequence of GM-CSF precursor is available under the NCBI accession number NP_000749.2.

The amino acid sequence of human GM-CSF precursor is also available under the UniProt accession number P04141 - version 180. The amino acid sequence of human GM-CSF precursor comprises a signal peptide, which spans the amino acids 1 to 17 of the precursor, and the mature GM-CSF, which spans the amino acids 18 to 144 of the GM-CSF precursor.

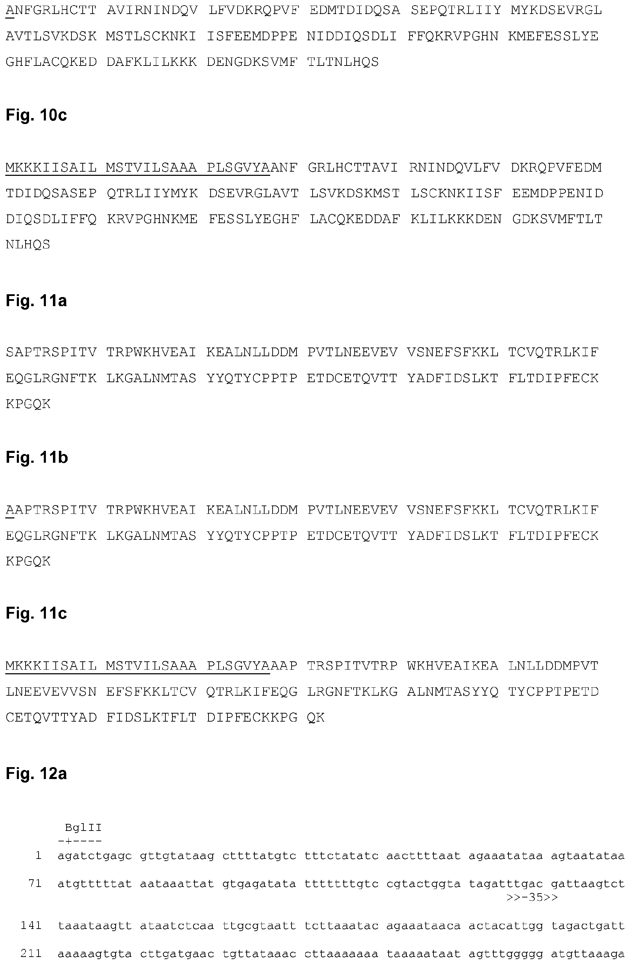

Preferably, granulocyte-macrophage colony-stimulating factor (GM-CSF) comprises one or at least one of the amino acid sequences of SEQ ID Nos 19 to 21, which are also depicted in Figures 11a to 11c, respectively.

Granulocyte-colony stimulating factor (G-CSF or GCSF) is also known as colony-stimulating factor 3 (CSF 3).

In humans preferably 2 different G-CSF polypeptides, designated isoform 1 and isoform 2, are synthesized from the same gene by differential splicing of mRNA. The 2 polypeptides differ by the presence or absence of 3 amino acids. Expression studies indicate that both preferably have G-CSF activity.

The nucleic acid sequence of the mRNA of human G-CSF isoform 1 precursor, which is also designated as G-CSF isoform a precursor, is available under the NCBI accession number NM_000759.3. The corresponding amino acid sequence is available under the NCBI accession number NP_000750.1.

The nucleic acid sequence of the mRNA of human G-CSF isoform 2 precursor, which is also designated as G-CSF isoform b precursor, is available under the NCBI accession number NM_172219.2. The corresponding amino acid sequence is available under the NCBI accession number NP_757373.1.

The respective amino acid sequences of human G-CSF are also available under the UniProt accession number P09919 - version 193. Isoform 1 has been chosen as canonical UniProt sequence.

The respective protein sequences of the human G-CSF precursor isoforms 1 to 2 include an N-terminal signal peptide, which spans the amino acid number 1 to amino acid number 29 of the respective amino acid sequences.

Erythropoietin (EPO) stimulates red blood cell production (erythropoiesis) in the bone marrow.

The nucleic acid sequence of the mRNA of human erythropoietin precursor is available under the NCBI accession number NM_000799.3. The respective amino acid sequence of erythropoietin precursor is available under the NCBI accession number NP_000790.2.

The amino acid sequence of human erythropoietin precursor is also available under the UniProt accession number P01588 - version 183. The amino acid sequence of human erythropoietin precursor comprises a signal peptide, which spans the amino acids 1 to 27 of the precursor, and the mature erythropoietin, which spans the amino acids 28 to 193 of the precursor.

Chemokines are a group of chemotactic polypeptides that preferably govern the recruitment of various leukocytes to inflammation site. Based on homology of the respective polypeptide sequence and the number of amino acids between the first two cysteines, chemokines can be subdivided into C, CC, CXC, and $CX_3C$ subgroups.

Preferably, a chemokine is selected from the group consisting of CCL1 to CCL28, CXCL1 to CXCL17, XCL1, XCL2, CX3CL1, functional analogues thereof, biosimilars thereof, and mixtures thereof.

Examples of polypeptide hormons are known to the skilled person and include, for example corticotropin-releasing hormone, islet amyloid polypeptide, gastric inhibitory polypeptide, ghrelins, glucagon-like peptide-1, growth hormone-releasing hormone, appetite-regulating hormone, insulin, leptin, motilin, thyrotropin-releasing hormone, urocortin, pancreatic polypeptide, somatostatin, natriuretic peptides, growth hormone, prolactin, calcitonin, luteinising hormone, parathyroid hormone, thyroid stimulating hormone, vasoactive intestinal polypeptide, functional analogues thereof, biosimilars thereof, and mixtures thereof,

Examples of neuropeptides are known to the skilled person and include, for example, agouti-related protein (AgRP), gastrin releasing peptide (GRP), calcitonin gene-related peptide (CGRP), cocaine- and amphetamine-regulated transcript peptide (CART), dynorphins, endorphins, enterostatin, galanin peptide (GAL), galanin-like peptide (GALP), hypocretin/orexin, melanin concentrating hormone (MCH), neuromedins, neuropeptide B, neuropeptide K, neuropeptide S, neuropeptide W, neuropeptide Y, neurotensin, oxytocin, prolactin releasing peptide, pro-opiomelanocortin and melanocortins derived thereof, , apolipoprotein A-IV, oxyntomodulin, gastrin-releasing peptide, glucose-dependent insulinotrophic polypeptide, orphanin FQ, enkephalins, functional analogues thereof, biosimilars thereof, and mixtures thereof.

Examples of enzymes are known to the skilled person and include, for example, eukaryotic thrombolytic enzymes, including tissue plasminogen activator, urokinase, or other enzymes such as factor VII-activating protease.

An antibody as described herein can be a full-size antibody or a functional fragment thereof such as Fab, a fusion protein or a multimeric protein.

As used herein, the term "functional" refers to an antibody fragment, which can still exert its intended function, i.e. antigen binding. The term antibody, as used here, includes, but is not limited to conventional antibodies, chimeric antibodies, dAb, bispecific antibody, trispecific antibody, multispecific antibody, bivalent antibody, trivalent antibody, multivalent antibody, VHH, nanobody, Fab, Fab', F(ab')2 scFv, Fv, dAb, Fd, diabody, triabody, single chain antibody, single domain antibody, single antibody variable domain.

In the present context, the term "antibody" is used to describe an immunoglobulin whether natural or partly or wholly engineered. As antibodies can be modified in a number of ways, the term "antibody" covers antibody fragments, derivatives, functional equivalents and homologues of antibodies, as well as single chain antibodies, bifunctional antibodies, bivalent antibodies, VHH, nanobodies, Fab, Fab', F(ab')2, scFv, Fv, dAb, Fd, diabodies, triabodies and camelid antibodies, including any polypeptide comprising an immunoglobulin binding domain, whether natural or wholly or partially engineered. Chimeric molecules comprising an immunoglobulin binding domain, or equivalent, fused to another polypeptide are therefore included. The term also covers any polypeptide or protein having a binding domain which is, or is homologous to, an antibody binding domain, e.g. antibody mimics.

Examples of antibodies are the immunoglobulin isotypes and their isotypic subclasses, including IgG (IgG1, IgG2a, IgG2b, IgG3, IgG4), IgA, IgD, IgM and IgE. The person in the art will thus appreciate that the present invention also relates to antibody fragments, comprising an antigen binding domain such as VHH, nanobodies Fab, scFv, Fv, dAb, Fd, diabodies and triabodies. In an embodiment, the invention relates to a gram-positive bacterium or a recombinant nucleic acid as described herein, wherein one exogenous gene encodes the light chain (VL) of an antibody or of a functional fragment thereof, and another exogenous gene encodes the heavy chain (VH) of the antibody or of a functional fragment thereof, more preferably wherein the functional fragment is Fab. In an embodiment, the exogenous gene encoding VL or functional fragment thereof is transcriptionally coupled to the 3' end of the exogenous gene encoding VH or functional fragment thereof.

In a preferred embodiment, the recombinant bacteria of the present invention express 1, 2, 3, 4 or more heterologous factors, which each are not naturally occurring in or expressed by said bacteria used and which independently are a heterologous polypeptide, or a complex thereof, preferably a heterologous polypeptide or a complex thereof.

For example, if the recombinant bacteria of the present invention express 2, 3, 4 or more heterologous factors, expression of each of the respective heterologous factors can be controlled by an individual prokaryotic, chloride-inducible promoter or by a single prokaryotic, chloride-inducible promoter, further preferably controlling expression of said heterologous factors from a single operon.

For example, the respective nucleic acid sequences encoding for the respective heterologous factors can each be located in separate sub-populations of the recombinant bacteria, which are combined to obtain the recombinant bacteria of the present invention.

The respective individual sub-population comprising the respective nucleic acid sequences can, for example, be from the same species or from different species of bacteria.

For example, different species of recombinant bacteria expressing different heterologous factors can be used to adapt the expression and, preferably the release, of the respective heterologous factors from the recombinant bacteria.

In a preferred embodiment of the present invention, the respective nucleic acid sequence(s) encoding for at least one heterologous factor are present in the recombinant bacteria in various numbers of copies. For example, the recombinant bacteria comprise at least one copy of the respective nucleic acid sequences encoding for said at least one heterologous factor per bacterial cell.

The numbers of copies of the respective nucleic acid sequence, that is present in a, preferably each, bacterial cell of the recombinant bacteria, can independently be increased in order to enhance the expression of the respective heterologous factor.

If, for example, two or more heterologous factors are to be expressed by the recombinant bacteria various ratios of copies of the respective nucleic acid sequences encoding for said heterologous factors can be present in a bacterial cell of the recombinant bacteria.

In a preferred embodiment, the nucleic acid sequences encoding for the at least one, preferably 1, 2, 3, 4, or more, heterologous factor(s) is/are located in one population of the recombinant bacteria.

In a further preferred embodiment, the respective nucleic acid sequence(s) encoding for at least one heterologous factor is/are located on at least one of a chromosome and a plasmid of said recombinant bacteria.

Locating nucleic acid sequence on a chromosome of the recombinant bacteria or on a plasmid of said recombinant bacteria determines inter alia the amount of protein translated by the bacteria, since expression levels on a plasmid are higher compared to expression levels on a chromosome.

In a further preferred embodiment of the present invention, at least one nucleic acid sequence encoding for the respective heterologous factor is provided on a chromosome of the bacteria and at least one nucleic acid sequence encoding for another heterologous factor is provided on a plasmid of said recombinant bacteria.

In an alternative embodiment of the present invention, the respective nucleic acid sequences are either provided on individual portions of the chromosome of the recombinant bacteria or on different plasmids of said recombinant bacteria.

In a preferred embodiment of the invention, all nucleic acid sequences encoding for the at least one, preferably 1, 2, 3, 4, or more, heterologous factor(s) is/are provided on a single recombinant nucleic acid, preferably recombinant plasmid.

Further preferably, the recombinant nucleic acid, preferably recombinant plasmid, of the present invention comprises 1, 2, 3, 4 or more nucleic acid sequence each encoding for 1, 2, 3, 4 or more factors, which each are not naturally occurring in or expressed by said bacteria used and which independently are a heterologous polypeptide, or a complex thereof.

In another particular preferred embodiment of the invention, the at least one nucleic acid sequence encoding for the at least one, preferably 1, 2, 3, 4, or more, heterologous factor(s) is/are located in a single operon which is operatively linked to and controlled by a single prokaryotic, chloride-inducible promoter, which activity is controlled by at least one prokaryotic regulator gene.

Further preferably said chloride-inducible promoter and said at least one regulator gene are arranged in a chloride-inducible gene expression cassette, wherein said chloride-inducible promoter is arranged downstream from said at least one regulator gene, and wherein said chloride-inducible gene expression cassette controls transcription of the at least one nucleic acid sequence encoding for the at least one heterologous factor, which further preferably is located in a single operon.

An operon is a functioning unit of DNA containing a cluster of genes under the control of a single promoter. The genes are transcribed together in mRNA strands and are translated together in the cytoplasm.

Preferably, the respective nucleic acid sequences encoding for the respective heterologous factors located in said single operon are provided with a nucleic acid sequence encoding a ribosome binding site for translational initiation.

Preferably, the nucleic acid sequences encoding a ribosome binding site is located upstream of the start codon, which is towards the 5' region of the same strand which is to be transcribed. The sequence is preferably complementary to the 3' end of the 16S ribosomal RNA.

Preferably, the respective nucleic acid sequences encoding for the respective heterologous factors located in said single operon are provided with a nucleic acid sequence encoding for a secretory signal sequence at the 5'-end of the open reading frame (ORF) of the heterologous factor generating a fusion protein comprising the secretory signal sequence and the heterologous factor.

In a preferred embodiment of the recombinant bacteria of the present invention or the recombinant plasmid of the present invention said chloride-inducible promoter and/or said at least one regulator gene is/are each independently from the same bacterial species.

In a preferred embodiment of the present invention said chloride-inducible promoter is PgadC from a bacterial species of the taxonomic order Lactobacillales, preferably *Lactococcus lactis*

In a preferred embodiment of the present invention said regulator gene encodes for GadR from a bacterial species of the taxonomic order Lactobacillales, preferably *Lactococcus lactis,* preferably encoding for a polypeptide comprising the amino acid sequence available under GenBank accession number AAC46186.1.

Further preferably, expression of said regulator gene, which further preferably encodes for GadR from a bacterial species of the taxonomic order Lactobacillales, preferably *Lactococcus lactis,* is regulated by a constitutive promoter.

Preferably, the nucleic acid sequences encoding the *Lactococcus lactis* positive regulator GadR (gadR), GadC (gadC) and glutamate decarboxylase (gadB) available under GenBank accession number AAC46187.1.

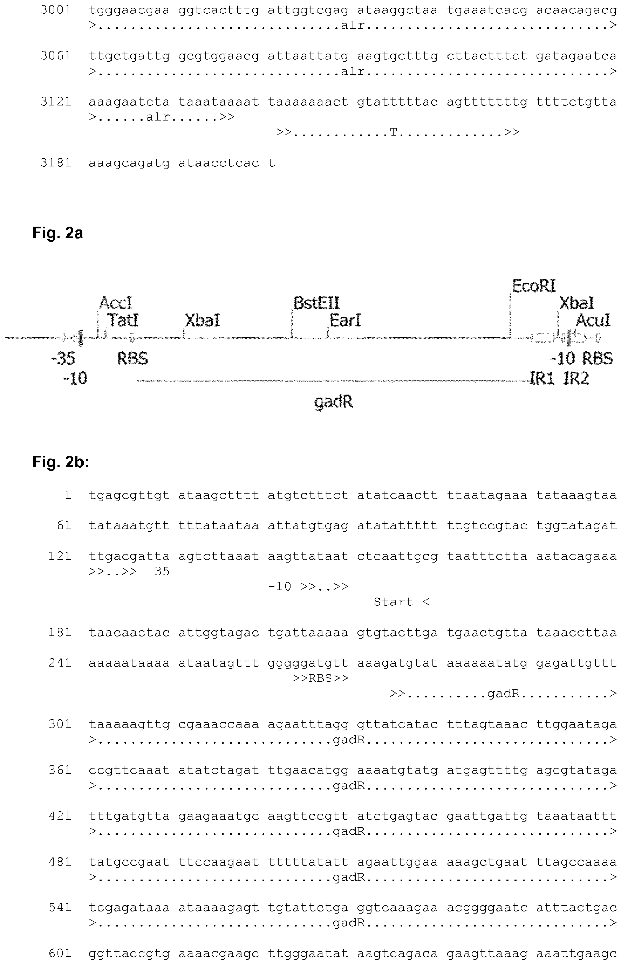

In a preferred embodiment of the present invention said chloride-inducible gene expression cassette comprises or consists of the nucleic acid sequence of SEQ ID No 2, which is also depicted in Figure 2b, and controls transcription of the at least one nucleic acid sequence encoding for the at least one heterologous factor, which further preferably is located in a single operon.

The recombinant bacteria of the present invention are preferably obtained by transforming bacteria, preferably selected from the taxonomic phylum firmicutes, the taxonomic phylum actinobacteria or the taxonomic phylum proteobacteria, with at least one nucleic acid sequence functionally coupled to a prokaryotic, chloride-inducible promoter and encoding for at least one heterologous factor, said heterologous factor is independently a heterologous polypeptide, or a complex thereof, wherein said heterologous polypeptide comprises at least one eukaryotic polypeptide, at least one fragment thereof or a combination thereof, and at least one prokaryotic regulator gene, which controls activity of said chloride-inducible promoter.

Proteobacteria is a major phylum of gram-negative bacteria that include a wide variety of known bacterial genera, such as Escherichia, Salmonella, Vibrio, Helicobacter, Yersinia, Legionellales.

Suitable bacteria for obtaining the recombinant bacteria of the present invention are preferably non-pathogenic bacteria. Preferably, the non-pathogenic bacteria are non-invasive bacteria.

In a further preferred embodiment, the recombinant bacteria are gram-positive bacteria, preferably gram-positive non-sporulating bacteria. Further preferably, said bacteria are non-colonizing bacteria lacking the ability to colonize in human gastrointestinal tract.

In a further preferred embodiment, the recombinant bacteria comprise a gram-positive food grade bacterial strain, preferably a facultative anaerobic bacterial strain.

According to a preferred embodiment of the present invention, the recombinant bacteria can be from the same bacterial strain or a mixture of different bacterial strains.

In another embodiment, the bacteria are classified as "generally recognized as safe" (GRAS) by the United States Food and Drug Administration (FDA).

In another embodiment, the bacteria have a "qualified presumption of safety" (QPS-status) as defined by the European Food Safety Authority (EFSA). An introduction of the qualified presumption of safety (QPS) approach for the assessment of selected microorganisms is described in the EFSA Journal, Vol. 587, 2007, pages 1-16.

In a further preferred embodiment, the bacteria are non-pathogenic bacteria belonging to the taxonomic phylum firmicutes or actinobacteria. Preferably, the bacteria are non-pathogenic bacteria from at least one genus selected form the group consisting of *Bifidobacterium, Corynebacterium, Enterococcus, Lactobacillus, Lactococcus, Leuconostoc, Pediococcus, Propionibacterium,* and *Streptococcus*.

In another preferred embodiment, the recombinant bacteria are lactic acid bacteria (LAB). Lactic acid bacteria are a clade of gram-positive, acid-tolerant, generally non-sporulating, non-respiring bacteria that share common metabolic and physiological characteristics. These bacteria produce lactic acid as a major metabolic end product of carbohydrate degradation.

Lactic acid bacteria are known and are used, for example, in food fermentation. Furthermore, proteinaceous bacteriocins are produced by several lactic acid bacteria strains.

In a further preferred embodiment, the bacteria used for obtaining the recombinant bacteria of the present invention exclude pathogenic and/or opportunistic bacteria.

In another embodiment, the bacteria are from the genus Bifidobacterium sp., including but not limited to, Bifidobacterium actinocoloniiforme, Bifidobacterium adolescentis, Bifidobacterium aesculapii, Bifidobacterium angulatum, Bifidobacterium Bifidobacterium animalis, for example Bifidobacterium animalis subsp. animalis or Bifidobacterium animalis subsp. lactis, Bifidobacterium asteroides, Bifidobacterium biavatii, Bifidobacterium bifidum, Bifidobacterium bohemicum, Bifidobacterium bombi, Bifidobacterium boum, Bifidobacterium breve, Bifidobacterium callitrichos, Bifidobacterium catenulatum, Bifidobacterium choerinum, Bifidobacterium coryneforme, Bifidobacterium crudilactis, Bifidobacterium cuniculi, Bifidobacterium denticolens, Bifidobacterium dentium, Bifidobacterium faecale, Bifidobacterium gallicum, Bifidobacterium gallinarum, Bifidobacterium globosum, Bifidobacterium indicum, Bifidobacterium infantis, Bifidobacterium inopinatum, Bifidobacterium kashiwanohense, Bifidobacterium lactis, Bifidobacterium longum, for example Bifidobacterium longum subsp. infantis, Bifidobacterium longum subsp. longum, or Bifidobacterium longum subsp. suis, Bifidobacterium magnum, Bifidobacterium merycicum, Bifidobacterium minimum, Bifidobacterium mongoliense, Bifidobacterium moukalabense, Bifidobacterium pseudocatenulatum, Bifidobacterium pseudolongum, for example Bifidobacterium pseudolongum subsp. globosum or Bifidobacterium pseudolongum subsp. pseudolongum, Bifidobacterium psychraerophilum, Bifidobacterium pullorum, Bifidobacterium reuteri, Bifidobacterium ruminantium, Bifidobacterium saeculare, Bifidobacterium saguini, Bifidobacterium scardovii, Bifidobacterium stellenboschense, Bifidobacterium subtile, Bifidobacterium stercoris, Bifidobacterium suis, Bifidobacterium thermacidophilum, for example Bifidobacterium thermacidophilum subsp. porcinum or Bifidobacterium thermacidophilum subsp. thermacidophilum, Bifidobacterium thermophilum, or Bifidobacterium tsurumiense.

Preferably, the bacteria are not Bifidobacterium dentium.

Preferably, the bacteria are bacteria having a "Qualified Presumption of Safety" (QPS) status in the genus Bifidobacterium sp., including but not limited to, Bifidobacterium adolescentis, Bifidobacterium animalis, Bifidobacterium longum, Bifidobacterium breve, or Bifidobacterium bifidum.

In one embodiment, the bacteria are from the genus Corynebacterium sp., including but not limited to, Corynebacterium accolens, Corynebacterium afermentans, for example Corynebacterium afermentans subsp. afermentans or Corynebacterium afermentans subsp. lipophilum, Corynebacterium ammoniagenes, Corynebacterium amycolatum, Corynebacterium appendices, Corynebacterium aquatimens, Corynebacterium aquilae, Corynebacterium argentoratense, Corynebacterium atypicum, Corynebacterium aurimucosum, Corynebacterium auris, Corynebacterium auriscanis, Corynebacterium betae, Corynebacterium beticola, Corynebacterium bovis, Corynebacterium callunae, Corynebacterium camporealensis, Corynebacterium canis, Corynebacterium capitovis, Corynebacterium casei, Corynebacterium caspium, Corynebacterium ciconiae, Corynebacterium confusum, Corynebacterium coyleae, Corynebacterium cystitidis, Corynebacterium deserti, Corynebacterium diphtheriae, Corynebacterium doosanense, Corynebacterium durum, Corynebacterium efficiens, Corynebacterium epidermidicanis, Corynebacterium equi, Corynebacterium falsenii, Corynebacterium fascians, Corynebacterium felinum, Corynebacterium flaccumfaciens, for example Corynebacterium flaccumfaciens subsp. betae, Corynebacterium flaccumfaciens subsp. flaccumfaciens, Corynebacterium flaccumfaciens subsp. oortii, or Corynebacterium flaccumfaciens subsp. poinsettiae, Corynebacterium flavescens, Corynebacterium frankenforstense, Corynebacterium freiburgense, Corynebacterium freneyi, Corynebacterium glaucum, Corynebacterium glucuronolyticum, Corynebacterium glutamicum, Corynebacterium halotolerans, Corynebacterium hansenii, Corynebacterium hoagie, Corynebacterium humireducens, Corynebacterium ilicis, Corynebacterium imitans, Corynebacterium insidiosum, Corynebacterium iranicum, Corynebacterium jeikeium, Corynebacterium kroppenstedtii, Corynebacterium kutscheri, Corynebacterium lactis, Corynebacterium lilium, Corynebacterium lipophiloflavum, Corynebacterium liquefaciens, Corynebacterium lubricantis, Corynebacterium macginleyi, Corynebacterium marinum, Corynebacterium maris, Corynebacterium massiliense, Corynebacterium mastitidis, Corynebacterium matruchotii, Corynebacterium michiganense, for example Corynebacterium michiganense subsp. insidiosum, Corynebacterium michiganense subsp. michiganense, Corynebacterium michiganense subsp. nebraskense, Corynebacterium michiganense subsp. sepedonicum, or Corynebacterium michiganense subsp. tessellarius, Corynebacterium minutissimum, Corynebacterium mooreparkense, Corynebacterium mucifaciens, Corynebacterium mustelae, Corynebacterium mycetoides, Corynebacterium nebraskense, Corynebacterium nigricans, Corynebacterium nuruki, Corynebacterium oortii, Corynebacterium paurometabolum, Corynebacterium phocae, Corynebacterium pilbarense, Corynebacterium pilosum, Corynebacterium poinsettiae, Corynebacterium propinquum, Corynebacterium pseudodiphtheriticum, Corynebacterium pseudotuberculosis, Corynebacterium pyogenes, Corynebacterium pyruviciproducens, Corynebacterium rathayi, Corynebacterium renale, Corynebacterium resistens, Corynebacterium riegelii, Corynebacterium seminale, Corynebacterium sepedonicum, Corynebacterium simulans, Corynebacterium singulare, Corynebacterium sphenisci, Corynebacterium spheniscorum, Corynebacterium sputi, Corynebacterium stationis, Corynebacterium striatum, Corynebacterium suicordis, Corynebacterium sundsvallense, Corynebacterium terpenotabidum, Corynebacterium testudinoris, Corynebacterium thomssenii, Corynebacterium timonense, Corynebacterium tritici, Corynebacterium tuberculostearicum, Corynebacterium tuscaniense, Corynebacterium ulcerans, Corynebacterium ulceribovis, Corynebacterium urealyticum, Corynebacterium ureicelerivorans, Corynebacterium uterequi, Corynebacterium variabile, Corynebacterium vitaeruminis, or Corynebacterium xerosis.

Preferably, the bacteria are not Corynebacterium diphtheriae, Corynebacterium amicolatum, Corynebacterium striatum, Corynebacterium jeikeium, Corynebacterium urealyticum, Corynebacterium xerosis, Corynebacterium pseudotuberculosis, Corynebacterium tenuis, Corynebacterium striatum, or Corynebacterium minutissimum.

Preferably, the bacteria are bacteria classified as "generally regarded as safe" (GRAS) in the Corynebacterium genus, including but not limited to Corynebacterium ammoniagenes, Corynebacterium casei, Corynebacterium flavescens, or Corynebacterium variabile.

In another embodiment, the bacteria are from the genus Enterococcus sp., including but not limited to, Enterococcus alcedinis, Enterococcus aquimarinus, Enterococcus asini, Enterococcus avium, Enterococcus caccae, Enterococcus camelliae, Enterococcus canintestini, Enterococcus canis, Enterococcus casseliflavus, Enterococcus cecorum, Enterococcus columbae, Enterococcus devriesei, Enterococcus diestrammenae, Enterococcus dispar, Enterococcus durans, Enterococcus eurekensis, Enterococcus faecalis, Enterococcus faecium, Enterococcus flavescens, Enterococcus gallinarum, Enterococcus gilvus, Enterococcus haemoperoxidus, Enterococcus hermanniensis, Enterococcus hirae, Enterococcus italicus, Enterococcus lactis, Enterococcus lemanii, Enterococcus malodoratus, Enterococcus moraviensis, Enterococcus mundtii, Enterococcus olivae, Enterococcus pallens, Enterococcus phoeniculicola, Enterococcus plantarum, Enterococcus porcinus, Enterococcus pseudoavium, Enterococcus quebecensis, Enterococcus raffinosus, Enterococcus ratti, Enterococcus rivorum, Enterococcus rotai, Enterococcus saccharolyticus, for example Enterococcus saccharolyticus subsp. saccharolyticus or Enterococcus saccharolyticus subsp. taiwanensis, Enterococcus saccharominimus, Enterococcus seriolicida, Enterococcus silesiacus, Enterococcus solitarius, Enterococcus sulfureus, Enterococcus termitis, Enterococcus thailandicus, Enterococcus ureilyticus, Enterococcus viikkiensis, Enterococcus villorum, or Enterococcus xiangfangensis.

Preferably, the bacteria are bacteria classified as "generally regarded as safe" (GRAS) in the Enterococcus genus, including but not limited to Enterococcus durans, Enterococcus faecalis, or Enterococcus faecium.

In another embodiment, the bacteria are from the genus Lactobacillus sp., including but not limited to, Lactobacillus acetotolerans, Lactobacillus acidifarinae, Lactobacillus acidipiscis, Lactobacillus acidophilus, Lactobacillus agilis, Lactobacillus algidus, Lactobacillus alimentarius, Lactobacillus amylolyticus, Lactobacillus amylophilus, Lactobacillus amylotrophicus, Lactobacillus amylovorus, Lactobacillus animalis, Lactobacillus antri, Lactobacillus apinorum, Lactobacillus apis, Lactobacillus apodemi, Lactobacillus aquaticus, Lactobacillus arizonensis, Lactobacillus aviaries, for example Lactobacillus aviarius subsp. araffinosus or Lactobacillus aviarius subsp. aviarius, Lactobacillus backii, Lactobacillus bavaricus, Lactobacillus bifermentans, Lactobacillus bobalius, Lactobacillus bombi, Lactobacillus brantae, Lactobacillus brevis, Lactobacillus buchneri, Lactobacillus bulgaricus, Lactobacillus cacaonum, Lactobacillus camelliae, Lactobacillus capillatus, Lactobacillus carnis, Lactobacillus casei, for example Lactobacillus casei subsp. alactosus, Lactobacillus casei subsp. casei, Lactobacillus casei subsp. pseudoplantarum, Lactobacillus casei subsp. rhamnosus, or Lactobacillus casei subsp. tolerans, Lactobacillus catenaformis, Lactobacillus cellobiosus, Lactobacillus ceti, Lactobacillus coleohominis, Lactobacillus collinoides, Lactobacillus composti, Lactobacillus concavus, Lactobacillus confusus, Lactobacillus coryniformis, for example Lactobacillus coryniformis subsp. coryniformis or Lactobacillus coryniformis subsp. torquens, Lactobacillus crispatus, Lactobacillus crustorum, Lactobacillus curieae, Lactobacillus curvatus, for example Lactobacillus curvatus subsp. curvatus or Lactobacillus curvatus subsp. melibiosus, Lactobacillus cypricasei, Lactobacillus delbrueckii, for example Lactobacillus delbrueckii subsp. bulgaricus, Lactobacillus delbrueckii subsp. delbrueckii, Lactobacillus delbrueckii subsp. indicus, Lactobacillus delbrueckii subsp. jakobsenii, Lactobacillus delbrueckii subsp. lactis, or Lactobacillus delbrueckii subsp. sunkii, Lactobacillus dextrinicus, Lactobacillus diolivorans, Lactobacillus divergens, Lactobacillus durianis, Lactobacillus equi, Lactobacillus equicursoris, Lactobacillus equigenerosi, Lactobacillus fabifermentans, Lactobacillus faecis, Lactobacillus farciminis, Lactobacillus farraginis, Lactobacillus ferintoshensis, Lactobacillus fermentum, Lactobacillus floricola, Lactobacillus florum, Lactobacillus fornicalis, Lactobacillus fructivorans, Lactobacillus fructosus, Lactobacillus frumenti, Lactobacillus fuchuensis, Lactobacillus furfuricola, Lactobacillus futsaii, Lactobacillus gallinarum, Lactobacillus gasseri, Lactobacillus gastricus, Lactobacillus ghanensis, Lactobacillus gigeriorum, Lactobacillus graminis, Lactobacillus halotolerans, Lactobacillus hammesii, Lactobacillus hamsteri, Lactobacillus harbinensis, Lactobacillus hayakitensis, Lactobacillus heilongjiangensis, Lactobacillus helsingborgensis, Lactobacillus helveticus, Lactobacillus heterohiochii, Lactobacillus hilgardii, Lactobacillus hokkaidonensis, Lactobacillus hominis, Lactobacillus homohiochii, Lactobacillus hordei, Lactobacillus iners, Lactobacillus ingluviei, Lactobacillus intestinalis, Lactobacillus iwatensis, Lactobacillus jensenii, Lactobacillus johnsonii, Lactobacillus kalixensis, Lactobacillus kandleri, Lactobacillus kefiranofaciens, for example Lactobacillus kefiranofaciens subsp. kefiranofaciens or Lactobacillus kefiranofaciens subsp. kefirgranum, Lactobacillus kefiri, Lactobacillus kefirgranum, Lactobacillus kimbladii, Lactobacillus kimchicus, Lactobacillus kimchiensis, Lactobacillus kimchii, Lactobacillus kisonensis, Lactobacillus kitasatonis, Lactobacillus koreensis, Lactobacillus kullabergensis, Lactobacillus kunkeei, Lactobacillus lactis, Lactobacillus leichmannii, Lactobacillus lindneri, Lactobacillus malefermentans, Lactobacillus mali, Lactobacillus maltaromicus, Lactobacillus manihotivorans, Lactobacillus mellifer, Lactobacillus mellis, Lactobacillus melliventris, Lactobacillus mindensis, Lactobacillus minor, Lactobacillus minutus, Lactobacillus mucosae, Lactobacillus murinus, Lactobacillus nagelii, Lactobacillus namurensis, Lactobacillus nantensis, Lactobacillus nasuensis, Lactobacillus nenjiangensis, Lactobacillus nodensis, Lactobacillus odoratitofui, Lactobacillus oeni, Lactobacillus oligofermentans, Lactobacillus oris, Lactobacillus oryzae, Lactobacillus otakiensis, Lactobacillus ozensis, Lactobacillus panis, Lactobacillus pantheris, Lactobacillus parabrevis, Lactobacillus parabuchneri, Lactobacillus paracasei, for example Lactobacillus paracasei subsp. paracasei or Lactobacillus paracasei subsp. tolerans, Lactobacillus paracollinoides, Lactobacillus parafarraginis, Lactobacillus parakefiri, Lactobacillus paralimentarius, Lactobacillus paraplantarum, Lactobacillus pasteurii, Lactobacillus paucivorans, Lactobacillus pentosus, Lactobacillus perolens, Lactobacillus piscicola, Lactobacillus plantarum, for example Lactobacillus plantarum subsp. argentoratensis or Lactobacillus plantarum subsp. plantarum, Lactobacillus pobuzihii, Lactobacillus pontis, Lactobacillus porcinae, Lactobacillus psittaci, Lactobacillus rapi, Lactobacillus rennini, Lactobacillus reuteri, Lactobacillus rhamnosus, Lactobacillus rimae, Lactobacillus rodentium, Lactobacillus rogosae, Lactobacillus rossiae, Lactobacillus ruminis, Lactobacillus saerimneri, Lactobacillus sakei, for example Lactobacillus sakei subsp. carnosus or Lactobacillus sakei subsp. sakei, Lactobacillus salivarius, for example Lactobacillus salivarius subsp. salicinius or Lactobacillus salivarius subsp. salivarius, Lactobacillus sanfranciscensis, Lactobacillus saniviri, Lactobacillus satsumensis, Lactobacillus secaliphilus, Lactobacillus selangorensis, Lactobacillus senioris, Lactobacillus senmaizukei, Lactobacillus sharpeae, Lactobacillus shenzhenensis, Lactobacillus sicerae, Lactobacillus silagei, Lactobacillus siliginis, Lactobacillus similes, Lactobacillus sobrius, Lactobacillus songhuajiangensis, Lactobacillus spicheri, Lactobacillus sucicola, Lactobacillus suebicus, Lactobacillus sunkii, Lactobacillus suntoryeus, Lactobacillus taiwanensis, Lactobacillus thailandensis, Lactobacillus thermotolerans, Lactobacillus trichodes, Lactobacillus tucceti, Lactobacillus uli, Lactobacillus ultunensis, Lactobacillus uvarum, Lactobacillus vaccinostercus, Lactobacillus vaginalis, Lactobacillus versmoldensis, Lactobacillus vini, Lactobacillus viridescens, Lactobacillus vitulinus, Lactobacillus xiangfangensis, Lactobacillus xylosus, Lactobacillus yamanashiensis, for example Lactobacillus yamanashiensis subsp. mali or Lactobacillus yamanashiensis subsp. yamanashiensis, Lactobacillus yonginensis, Lactobacillus zeae, or Lactobacillus zymae.

Prefereably, the bacteria are bacteria classified as "generally regarded as safe" (GRAS) in the genus Lactobacillus sp., including but not limited to, Lactobacillus acidophilus strain NP 28, Lactobacillus acidophilus strain NP51 , Lactobacillus subsp. lactis strain NP7, Lactobacillus reuteri strain NCIMB 30242, Lactobacillus casei strain Shirota, Lactobacillus reuteri strain DSM 17938, Lactobacillus reuteri strain NCIMB 30242, Lactobacillus acidophilus strain NCFM, Lactobacillus rhamnosus strain HN001 , Lactobacillus rhamnosus strain HN001, Lactobacillus reuteri strain DSM 17938, Lactobacillus casei subsp. rhamnosus strain GG, Lactobacillus acidophilus, Lactobacillus lactis, Lactobacillus acetotolerans, Lactobacillus acidifarinae, Lactobacillus acidipiscis, Lactobacillus acidophilus, Lactobacillus alimentarius, Lactobacillus amylolyticus, Lactobacillus amylovorus, Lactobacillus brevis, Lactobacillus buchneri, Lactobacillus cacaonum, Lactobacillus casei subsp. casei, Lactobacillus collinoides, Lactobacillus composti, Lactobacillus coryniformis subsp. coryniformis, Lactobacillus crispatus, Lactobacillus crustorum, Lactobacillus curvatus subps. curvatus, Lactobacillus delbrueckii subsp. bulgaricus, Lactobacillus delbrueckii subsp. delbrueckii, Lactobacillus delbrueckii subsp. lactis, Lactobacillus dextrinicus, Lactobacillus diolivorans, Lactobacillus fabifermentans, Lactobacillus farciminis, Lactobacillus fermentum, Lactobacillus fructivorans, Lactobacillus frumenti, Lactobacillus gasseri, Lactobacillus ghanensis, Lactobacillus hammesii, Lactobacillus harbinensis, Lactobacillus helveticus, Lactobacillus hilgardii, Lactobacillus homohiochii, Lactobacillus hordei, Lactobacillus jensenii, Lactobacillus johnsonii, Lactobacillus kefiri, Lactobacillus kefiranofadens subsp. kefiranofaciens, Lactobacillus kefiranofadens subsp. kefirgranum, Lactobacillus kimchii, Lactobacillus kisonensis, Lactobacillus mail, Lactobacillus manihotivorans, Lactobacillus mindensis, Lactobacillus mucosae, Lactobacillus nagelii, Lactobacillus namurensis, Lactobacillus nantensis, Lactobacillus nodensis, Lactobacillus oeni, Lactobacillus otakiensis, Lactobacillus panis, Lactobacillus parabrevis, Lactobacillus parabuchneri, Lactobacillus paracasei subsp. paracasei, Lactobacillus parakefiri, Lactobacillus paralimentarius, Lactobacillus paraplantarum, Lactobacillus pentosus, Lactobacillus perolens, Lactobacillus plantarum subsp. plantarum, Lactobacillus pobuzihii, Lactobacillus pontis, Lactobacillus rapi, Lactobacillus reuteri, Lactobacillus rhamnosus, Lactobacillus rossiae, Lactobacillus sakei subsp carnosus, Lactobacillus sakei subsp. sakei, Lactobacillus sali varius subsp. salivarius, Lactobacillus sanfranciscensis, Lactobacillus satsumensis, Lactobacillus secaliphilus, Lactobacillus senmaizukei, Lactobacillus siliginis, Lactobacillus spicheri, Lactobacillus suebicus, Lactobacillus sunkii, Lactobacillus tucceti, Lactobacillus vaccinostercus, Lactobacillus versmoldensis, or Lactobacillus yamanashiensis.

Preferably, the bacteria are bacteria having a "Qualified Presumption of Safety" (QPS) status in the genus Lactobacillus sp., including but not limited to, Lactobacillus acidophilus, Lactobacillus amylolyticus, Lactobacillus amylovorus, Lactobacillus alimentarius, Lactobacillus aviaries, Lactobacillus brevis, Lactobacillus buchneri, Lactobacillus casei, Lactobacillus crispatus, Lactobacillus curvatus, Lactobacillus delbrueckii, Lactobacillus farciminis, Lactobacillus fermentum, Lactobacillus gallinarum, Lactobacillus gasseri, Lactobacillus helveticus, Lactobacillus hilgardii, Lactobacillus johnsonii, Lactobacillus kefiranofaciens, Lactobacillus kefiri, Lactobacillus mucosae, Lactobacillus panis, Lactobacillus paracasei, Lactobacillus paraplantarum, Lactobacillus pentosus, Lactobacillus plantarum, Lactobacillus pontis, Lactobacillus reuteri, Lactobacillus rhamnosus, Lactobacillus sakei, Lactobacillus salivarius, Lactobacillus sanfranciscensis, or Lactobacillus zeae.

In another embodiment, the bacteria are from the genus Lactococcus sp., including but not limited to Lactococcus chungangensis, Lactococcus formosensis, Lactococcus fujiensis, Lactococcus garvieae, Lactococcus lactis, for example Lactococcus lactis subsp. cremoris, Lactococcus lactis subsp. hordniae, Lactococcus lactis subsp. lactis, or Lactococcus lactis subsp. tructae, Lactococcus piscium, Lactococcus plantarum, Lactococcus raffinolactis, or Lactococcus taiwanensis.

Preferably, the bacteria are bacteria classified as "generally regarded as safe" (GRAS) in the genus Lactococcus, including but not limited to, Lactococcus lactis subsp. cremoris, Lactococcus lactis subsp. lactis, and Lactococcus raffinolactis.

Preferably, the bacteria are Lactococcus lactis, preferably Lactococcus lactis subsp. lactis, Lactococcus lactis subsp. lactis biovariant diacetylactis, or Lactococcus lactis subsp. cremoris, further preferably Lactococcus lactis subsp. cremoris.

In another embodiment, the bacteria are from the genus Leuconostoc sp., including but not limited to, Leuconostoc amelibiosum, Leuconostoc argentinum, Leuconostoc carnosum, Leuconostoc citreum, Leuconostoc cremoris, Leuconostoc dextranicum, Leuconostoc durionis, Leuconostoc fallax, Leuconostoc ficulneum, Leuconostoc fructosum, Leuconostoc gasicomitatum, Leuconostoc gelidum, for example Leuconostoc gelidum subsp. aenigmaticum, Leuconostoc gelidum subsp. gasicomitatum, or Leuconostoc gelidum subsp. gelidum, Leuconostoc holzapfelii, Leuconostoc inhae, Leuconostoc kimchii, Leuconostoc lactis, Leuconostoc mesenteroides, for example Leuconostoc mesenteroides subsp. cremoris, Leuconostoc mesenteroides subsp. dextranicums, Leuconostoc mesenteroides subsp. mesenteroides, or Leuconostoc mesenteroides subsp. suionicum, Leuconostoc miyukkimchii, Leuconostoc oeni, Leuconostoc paramesenteroides, Leuconostoc pseudoficulneum, or Leuconostoc pseudomesenteroides.

Preferably, the bacteria are bacteria classified as "generally regarded as safe" (GRAS) in the genus Leuconostoc sp., including but not limited to, Leuconostoc carnosum, Leuconostoc citreum, Leuconostoc fallax, Leuconostoc holzapfelii, Leuconostoc inhae, Leuconostoc kimchii, Leuconostoc lactis, Leuconostoc mesenteroides subsp. cremoris, Leuconostoc mesenteroides subsp. dextranicums, Leuconostoc mesenteroides subsp. mesenteroides, Leuconostoc palmae, or Leuconostoc pseudomesenteroides.

Preferably, the bacteria are bacteria having a "Qualified Presumption of Safety" (QPS) status in the genus Leuconostoc sp., including but not limited to, Leuconostoc citreum, Leuconostoc lactis, Leuconostoc mesenteroides subsp. cremoris, Leuconostoc mesenteroides subsp. dextranicum, or Leuconostoc mesenteroides subsp. mesenteroides.

In another embodiment, the bacteria are from the genus of Pediococcus sp., including but not limited to, Pediococcus acidilactici, Pediococcus argentinicus, Pediococcus cellicola, Pediococcus claussenii, Pediococcus damnosus, Pediococcus dextrinicus. Pediococcus ethanolidurans, Pediococcus halophilus, Pediococcus inopinatus, Pediococcus lolii, Pediococcus parvulus, Pediococcus pentosaceus, Pediococcus siamensis, Pediococcus stilesii, or Pediococcus urinaeequi

Preferably, the bacteria are bacteria having a "Qualified Presumption of Safety" (QPS) status in the genus Pediococcus sp., including but not limited to, Pediococcus acidilactici, Pediococcus dextrinicus, or Pediococcus pentosaceus.

In another embodiment, the bacteria are from the genus Propionibacterium sp., including but not limited to, Propionibacterium acidifaciens, Propionibacterium acidipropionici, Propionibacterium acnes, Propionibacterium australiense, Propionibacterium avidum, Propionibacterium cyclohexanicum, Propionibacterium damnosum, Propionibacterium freudenreichii, for example Propionibacterium freudenreichii subsp. freudenreichii or Propionibacterium freudenreichii subsp. shermanii, Propionibacterium granulosum, Propionibacterium innocuum, Propionibacterium jensenii, Propionibacterium lymphophilum, Propionibacterium microaerophilum, Propionibacterium olivae, Propionibacterium propionicum, or Propionibacterium thoenii.

According to an embodiment, the bacteria are not Propionibacterium acnes.

Further preferably, the bacteria are bacteria classified as "generally regarded as safe" (GRAS) in the genus Propionibacterium sp., including but not limited to Propionibacterium acidipropionici, Propionibacterium freudenreichii subsp. Freudenreichii, Propionibacterium freudenreichii subsp. shermanii, Propionibacterium jensenii, or Propionibacterium thoenii.

Further preferably, the bacteria are Propionibacterium freudenreichii, further preferably Propionibacterium freudenreichii subsp. freudenreichii or Propionibacterium freudenreichii subsp. shermanii.

In another embodiment, the bacteria are from the genus Streptococcus sp., including but not limited to, Streptococcus acidominimus, Streptococcus adjacens, Streptococcus agalactiae, Streptococcus alactolyticus, Streptococcus anginosus, Streptococcus australis, Streptococcus bovis, Streptococcus caballi, Streptococcus canis, Streptococcus caprinus, Streptococcus castoreus, Streptococcus cecorum, Streptococcus constellatus, for example Streptococcus constellatus subsp. constellatus, Streptococcus constellatus subsp. pharynges, or Streptococcus constellatus subsp. viborgensis, Streptococcus cremoris, Streptococcus criceti, Streptococcus cristatus, Streptococcus cuniculi, Streptococcus danieliae, Streptococcus defectivus, Streptococcus dentapri, Streptococcus dentirousetti, Streptococcus dentasini, Streptococcus dentisani, Streptococcus devriesei, Streptococcus didelphis, Streptococcus difficilis, Streptococcus downei, Streptococcus durans, Streptococcus dysgalactiae, for example Streptococcus dysgalactiae subsp. dysgalactiae or Streptococcus dysgalactiae subsp. equisimilis, Streptococcus entericus, Streptococcus equi, for example Streptococcus equi subsp. equi, Streptococcus equi subsp. ruminatorum, or Streptococcus equi subsp. zooepidemicus, Streptococcus equinus, Streptococcus faecalis, Streptococcus faecium, Streptococcus ferus, Streptococcus gallinaceus, Streptococcus gallinarum, Streptococcus gallolyticus, for example Streptococcus gallolyticus subsp. gallolyticus, Streptococcus gallolyticus subsp. macedonicus, or Streptococcus gallolyticus subsp. pasteurianus, Streptococcus garvieae, Streptococcus gordonii, Streptococcus halichoeri, Streptococcus hansenii, Streptococcus henryi, Streptococcus hongkongensis, Streptococcus hyointestinalis, Streptococcus hyovaginalis, Streptococcus ictaluri, Streptococcus infantarius, for example Streptococcus infantarius subsp. coli or Streptococcus infantarius subsp. infantarius, Streptococcus infantis, Streptococcus iniae, Streptococcus intermedius, Streptococcus intestinalis, Streptococcus lactarius, Streptococcus lactis, for example Streptococcus lactis subsp. cremoris, Streptococcus lactis subsp. diacetilactis, or Streptococcus lactis subsp. lactis, Streptococcus loxodontisalivarius, Streptococcus lutetiensis, Streptococcus macacae, Streptococcus macedonicus, Streptococcus marimammalium, Streptococcus massiliensis, Streptococcus merionis, Streptococcus minor, Streptococcus mitis, Streptococcus morbillorum, Streptococcus moroccensis, Streptococcus mutans, Streptococcus oligofermentans, Streptococcus oralis, Streptococcus orisasini, Streptococcus orisuis, Streptococcus ovis, Streptococcus parasanguinis, Streptococcus parauberis, Streptococcus parvulus, Streptococcus pasteurianus, Streptococcus peroris, Streptococcus phocae, for example Streptococcus phocae subsp. phocae or Streptococcus phocae subsp. salmonis, Streptococcus plantarum, Streptococcus pleomorphus, Streptococcus pluranimalium, Streptococcus plurextorum, Streptococcus pneumonia, Streptococcus porci, Streptococcus porcinus, Streptococcus porcorum, Streptococcus pseudopneumoniae, Streptococcus pseudoporcinus, Streptococcus pyogenes, Streptococcus raffinolactis, Streptococcus ratti, Streptococcus rifensis, Streptococcus rubneri, Streptococcus rupicaprae, Streptococcus saccharolyticus, Streptococcus salivarius, for example Streptococcus salivarius subsp. salivarius or Streptococcus salivarius subsp. thermophilus, Streptococcus saliviloxodontae, Streptococcus sanguinis, Streptococcus shiloi, Streptococcus sinensis, Streptococcus sobrinus, Streptococcus suis, Streptococcus thermophilus, Streptococcus thoraltensis, Streptococcus tigurinus, Streptococcus troglodytae, Streptococcus uberis, Streptococcus urinalis, Streptococcus ursoris, Streptococcus vestibularis, or Streptococcus waius.

Preferably, the bacteria are classified as "generally regarded as safe" (GRAS) in the genus Streptococcus sp., including but not limited to, Streptococcus thermophilus strain Th4, Streptococcus gallolyticus subsp. macedonicus, Streptococcus salivarius subsp. salivarius, or Streptocorus salivarius subsp. thermophilus.

Preferably, the bacteria are bacteria having a "Qualified Presumption of Safety" (QPS) status in the genus Streptococcus sp., including but not limited to, Streptococcus thermophilus.

In a preferred embodiment, said bacteria produce no endotoxins or other potentially toxic substances. Thereby, said bacteria are safe to use and are not harmful to a subject after application.

Preferably, said bacteria produce no spores. Bacterial spores are not part of a sexual cycle but are resistant structures used for survival under unfavourable conditions. Since said recombinant bacteria preferably produce no spores, said recombinant bacteria can not survive, for example, without nutrients or if an auxotrophic factor is missing.

Preferably, said bacteria produce no inclusion bodies. Inclusion bodies often contain over-expressed proteins and aggregation of said over-expressed proteins in inclusion bodies can be irreversible.

Since bacteria used for obtaining the recombinant bacteria of the present invention preferably produce no inclusion bodies, the amount of said at least one heterologous factor after transcribing and preferably translating the respective nucleic acid sequences is not diminished by intracellular accumulation in inclusion bodies.

Further preferably, said bacteria also do not produce extracellular proteinases. Extracellular proteinases may be secreted from bacteria to destroy extracellular structures, such as proteins, to generate nutrients, such as carbon, nitrogen, or sulfur. Extracellular proteinases may also act as an exotoxin and be an example of a virulence factor in bacterial pathogenesis.

Due to the absence of extracellular proteinases the safety of said bacteria after application to a subject is preferably increased. Furthermore, said bacteria preferably do not degrade said at least one heterologous factor after release from said recombinant bacteria.

In a further preferred embodiment, said recombinant bacteria are lactic acid bacteria, preferably a *Lactobacillus* or a *Lactococcus* species. In a further preferred embodiment, said *Lactococcus* species is *Lactococcus lactis* subsp. *cremoris.*

Lactic acid bacteria further release lactic acid as the major metabolic end-product of carbohydrate fermentation. Lactic acid is known to also stimulate endothelial growth and proliferation. Furthermore, lactic acid has an antibacterial effect which reduces the probability of a bacterial infection at the site of the skin dysfunction.

Techniques for transforming the above-mentioned bacteria are known to the skilled person, and, for example, are described in Green and Sambrook (2012): "Molecular cloning: a laboratory manual", fourth edition, Cold Spring Harbour Laboratory Press (Cold Spring Harbor, NY, US).

After transformation, the recombinant bacteria comprise at least one nucleic acid sequence functionally coupled to a prokaryotic, chloride-inducible promoter and encoding for at least one heterologous factor, said heterologous factor is independently a heterologous polypeptide, or a complex thereof, and at least one prokaryotic regulator gene, which controls activity of said chloride-inducible promoter, wherein said heterologous polypeptide comprises at least one eukaryotic polypeptide, at least one fragment thereof or a combination thereof.

Further preferably, the recombinant bacteria comprise after transformation at least one copy of a recombinant plasmid of the present invention.

Suitable strains for obtaining the recombinant bacteria of the present invention are commercially available, for example, from MoBiTec GmbH (Göttingen, Germany) or from NIZO food research BV (Ede, NL). Suitable strains are, for example, *Lactococcus lactis* strain NZ1330, which preferably includes a plasmid selection system which is not based on antibiotic resistance.

In a further preferred embodiment, the nucleic acid sequences encoding for the respective heterologous factors are modified in order to increase expression, secretion and/or stability of the encoded at least one heterologous factor.

Suitable modifications are known to the skilled person and include, for example, adapting the codon usage of the at least one nucleic acid sequence to the bacteria used.

For example, the at least one nucleic acid sequence can be designed to fit the codon usage pattern of the bacteria used. Furthermore, in addition to a general codon optimization, specific codon tables can be used, such as a codon table for the highly expressed ribosomal protein genes of the respective bacteria, to further increase translation of the encoded at least one heterologous factor.

Suitable modifications include also, for example, the incorporation of a nucleic acid sequence encoding for a secretory signal sequence at the 5'-end of the open reading frame (ORF) of the heterologous factor generating a fusion protein comprising the secretory signal sequence and the heterologous factor. The secretory signal sequence is preferably arranged at the N-terminal side of the respective heterologous factor.

The secretory signal sequence preferably directs the newly synthesized protein to the plasma membrane. At the end of the secretory signal sequence, preferably signal peptide, there is preferably a stretch of amino acids that is recognized and cleaved by a signal peptidase in order to generate a free secretory signal sequence, preferably signal peptide, and the mature heterologous factor, which is secreted extracellularly.

Suitable secretory signal sequences include, for example, a signal peptide.

Preferably, the secretory signal sequence is the native signal peptide of the respective heterologous factor encoded by the nucleic acid sequence, which preferably is a precursorof the respective factor.

In a further preferred embodiment, said secretory signal sequence is a homologous secretory sequence from said recombinant bacteria, preferably said secretory signal sequence is the unknown secreted protein 45 (Usp 45) signal sequence of *Lactococcus* sp.. The Usp45 secretion signal has very high secretion efficiency. Other secretion signals are known to the skilled person and include, for example, PrtP, SlpA, SP310, SPEXP4 and AL9 of Lactococcus sp..

The amino acid sequence of of the Usp45 protein of *Lactococcus lactis* subsp. *cremoris* MG1363 is, for example, available under GenBank accession number CAL99070.1. The signal sequence spans the amino acids 1 to 27 of the sequence deposited under GenBank accession number CAL99070.1.

For example, the native signal peptide of the heterologous factor can be replaced by a homologous secretory signal sequence from the recombinant bacteria used to express said at least one heterologous factor.

A secretory signal sequence preferably improves the secretion efficiency of the respective heterologous factor. For example, in *Lactococcus lactis,* most proteins are secreted via the Sec-pathway. Proteins are synthesized as precursors containing the mature moiety of the proteins with an N-terminal signal peptide. The signal peptide targets the protein to the cytoplasmic membrane and facilitates the secretion of the protein. Following cleavage of the signal peptide, the mature protein is released extracellularly.

Further preferably, the secretory signal comprises the amino acid sequence of SEQ ID No. 3, which is also depicted in Figure 3.

In a further preferred embodiment, the at least one heterologous factor, preferably 1, 2, 3, 4, or more, heterologous factor(s) is/are expressed as a propeptide with or without a secretion enhancing factor. The propeptide can be cleaved by proteases to release the mature form of the at least one heterologous factor, preferably 1, 2, 3, 4, or more, heterologous factor(s).

Preferably, the at least one heterologous factor, preferably 1, 2, 3, 4, or more, heterologous factor(s) is/are releasable from said recombinant bacteria.

For example, in gram-positive bacteria, such as lactic acid bacteria, proteins to be secreted from the bacteria are preferably synthesized as a precursor containing an N-terminal signal peptide and the mature moiety of the protein. Precursors are recognized by the secretion machinery of the bacteria and are translocated across the cytoplasmic membrane. The signal peptide is then cleaved and degraded and the mature protein is released from the bacteria, for example, into the culture supernatant or into the area of an inflammatory skin dysfunction.

For example, *Lactococcus lactis* is able to secrete proteins ranging from low molecular mass, for example smaller than 10 kDa, to high molecular mass, for example molecular weight above 160 kDa, through a Sec-dependent pathway.

Alternatively, the at least one heterologous factor, preferably 1, 2, 3, 4, or more, heterologous factor(s) is/are released from said recombinant bacteria through a leaky cytoplasmic membrane.

For example, the cytoplasmic membrane of said recombinant bacteria becomes leaky due to an impaired synthesis of cell wall components.

Preferably, said recombinant bacteria comprise an inactivated alanine racemase (*alr*) gene. In the absence of D-alanine, said recombinant bacteria cannot maintain the integrity of the cytoplasmic membrane and, subsequently, the at least one heterologous factor, preferably 1, 2, 3, 4, or more, heterologous factor(s) is/are released from said recombinant bacteria.

In a further preferred embodiment, the recombinant bacteria of the present invention comprise at least one inactivated gene encoding for an essential protein necessary for viability of said bacteria.

In a preferred embodiment, the essential protein necessary for viability of said recombinant bacteria is a protein, further preferably an enzyme, necessary for synthesis of an organic compound necessary for viability of said recombinant bacteria.

After inactivation of said essential protein, preferably enzyme, the respective organic compound is an auxotrophic factor required for the viability of the recombinant bacteria and which has to be supplemented in order to sustain the viability of the recombinant bacteria.

Preferably, said auxotrophic factor is a vitamin, amino acid, nucleic acid, and/or a fatty acid.

For example, amino acids and nucleotides are biologically important organic compounds, which are precursors to proteins and nucleic acids, respectively.

Preferably, said gene encoding for an essential protein necessary for viability of said bacteria is provided on a recombinant nucleic acid and/or recombinant plasmid according to the present invention.

In a further preferred embodiment, the at least one gene necessary for viability of the recombinant bacteria is selected from the group consisting of alanine racemase (*alr*), thymidylate synthase (*thyA*), asparagine synthase (asnH), CTP synthase (*pyrG*)*,* tryptophan synthase (*trbBA*), and combinations thereof.

For example, alanine racemase is an enzyme that catalyzes the racemization of L-alanine into L- and D-alanine. D-alanine produced by alanine racemase is used for peptidoglycan synthesis. Peptidoglycan is found in the cell walls of all bacteria.

It is known to the skilled person that inactivating the alanine racemase (*alr*) gene in bacteria results in the need of the bacteria to use an external source of D-alanine in order to maintain cell wall integrity.

It is known to the skilled person that, for example, *Lactococcus lactis* and *Lactobacillus plantarum* contain a single *alr* gene. Inactivation of the gene effects the incorporation of D-alanine into the cell wall.

*Lactococcus lactis* bacteria, for example, with an inactivated alanine racemase (*alr*) gene are dependent on external supply of D-alanine to be able to synthesize peptidoglycan and to incorporate D-alanine in the lipoteichoic acid (LTA). These bacteria lyse rapidly when D-alanine is removed, for example, at mid-exponential growth.

Alanine racemase deficient strains of lactic acid bacteria could, for example, be generated by methods known in the art.

Thymidylate synthase is an enzyme that catalyzes the conversion of deoxyuridine monophosphate (dUMP) to deoxythymidylate monophosphate (dTMP). dTPM is one of the three nucleotides that form thymine (dTMP, dTDP, and dTTP). Thymidine is a nucleic acid in DNA.

It is known to the skilled person that thymidylate synthase plays a crucial role in DNA biosynthesis. Furthermore, for proliferation of the bacteria it is necessary to synthesize DNA.

Inactivation of the thymidylate synthase (*thyA*) gene in bacteria results in the need of the bacteria to use an external source of thymidine in order to maintain DNA integrity.

Asparagine synthetase is an enzyme that generates the amino acid asparagine from aspartate. Inactivation of the asparagines synthetase (asnH) gene results in an inability to synthesize the respective amino acid, which becomes an auxotrophic factor.

CTP-synthase is an enzyme involved in pyrimidine synthesis. CTP synthase interconverts uridine-5'-triphosphate (UTP) and cytidine-5'-triphosphate (CTP). Inactivation of the CTP-synthase (*pyrG*) gene renders the bacteria unable to synthesize cytosine nucleotides from both the *de novo* synthesis as well as the uridine cell wall pathway.

CTP becomes an auxotrophic factor, which has to be supplemented for the synthesis of RNA and DNA.

Tryptophan synthase is an enzyme that catalyzes the final two steps in the biosynthesis of the amino acid tryptophan.

Inactivation of the tryptophan synthase (*trpBA*) gene renders the bacteria unable to synthesize the respective amino acid, which becomes an auxotrophic factor.

Methods for inactivation of said gene necessary for the viability of the bacteria are known to the skilled person and include deletion of the gene, mutation of the gene, RNA interference (RNAi) mediated gene silencing of said gene, translational inhibition of said gene, or combinations thereof.

In a further preferred embodiment, the respective auxotrophic factor is provided together with said recombinant bacteria.

In a further preferred embodiment of the invention, said inactivated gene necessary for viability of the recombinant bacteria is used for environmental containment.

After application of the recombinant bacteria comprising at least one inactivated gene necessary for viability of said bacteria, the respective auxotrophic factor has to be provided externally, for example, with a reconstitution medium, application medium or a growth medium.

In case the recombinant bacteria are released into the environment, the auxotrophic factor is missing and, preferably, the recombinant bacteria die.

Furthermore, application of the externally supplemented auxotrophic factor enables for a control of the biosynthesis and release of the respective heterologous factor(s).

Further preferably, said gene encoding for an essential protein necessary for viability of said bacteria, which preferably is selected from the group consisting of alanine racemase (alr), thymidylate synthase (thyA), asparagine synthase (asnH), CTP synthase (pyrG), tryptophan synthase (trbBA), and combinations thereof, is provided on a recombinant nucleic acid and/or recombinant plasmid according to the present invention.

Thereby it is possible to provide a highly stable system for maintaining the recombinant nucleic acid and/or recombinant plasmid according to the present invention in the recombinant bacteria even during in vivo growth, as a selection pressure can be achieved to maintain the recombinant nucleic acid and/or recombinant plasmid according to the present invention, preferably in the absence of the externally supplemented auxothrophic factor.

Expression of said gene encoding for an essential protein necessary for viability of said bacteria, which preferably is selected from the group consisting of alanine racemase (alr), thymidylate synthase (thyA), asparagine synthase (asnH), CTP synthase (pyrG), tryptophan synthase (trbBA), and combinations thereof, on a recombinant nucleic acid and/or recombinant plasmid according to the present invention not only allowes for an auxotrophic complementation, but can also be used as a selection marker for the presence of the recombinant nucleic acid and/or recombinant plasmid according to the present invention in the respective bacteria.

According to a preferred embodiment, the recombinant bacteria are to be used in medicine, further preferably for use in treatment of a, preferably chronic, inflammatory wound or a degenerative condition, or for use in treatment of a tumor, preferably a maligant tumor.

During administration of the recombinant bacteria of the present invention to a subject, the released at least one heterologous factor, preferably 1, 2, 3, 4, or more, heterologous factor(s) can undergo degradation, for example by proteinase cleavage, which may lead to a loss of the biological activity of said heterologous factor(s).

The degradation or depletion of said heterologous factor(s) can be compensated by a sustained release of the at least one heterologous factor, preferably 1, 2, 3, 4, or more, heterologous factor(s) from said recombinant bacteria of the present invention in the presence of chloride ions.

Preferably, said recombinant bacteria express the at least one heterologous factor, preferably 1, 2, 3, 4, or more, heterologous factor(s) in a constant manner. Preferably, said recombinant bacteria release said heterologous factor(s) constantly.

The pharmaceutical composition of the present invention comprises recombinant bacteria according of the present invention and at least one pharmaceutically acceptable excipient.

In a preferred embodiment, the recombinant bacteria of the present invention are provided in a pharmaceutical composition for use in medicine, further preferably for use in treatment of a, preferably chronic, inflammatory wound or a degenerative condition, or for use in treatment of a tumor, preferably a malignant tumor.

An excipient is a substance formulated alongside the active ingredient of a medication, included for the purpose of long-term stabilization, bulking up solid formulations that contain potent active ingredients in small amounts (thus often referred to as "bulking agents", "fillers", or "diluents"), or to confer a therapeutic enhancement on the active ingredient in the final dosage form, such as facilitating drug absorption, reducing viscosity, or enhancing solubility.

The term "excipient" preferably also includes at least one diluent.

Excipients can also be useful in the manufacturing process, to aid in the handling of the active substance concerned such as by facilitating powder flowability or non-stick properties, in addition to aiding in vitro stability such as prevention of denaturation or aggregation over the expected shelf life, and/or to contribute to the viability of the bacterial cells of the recombinant bacteria during freezing and thawing.

Preferably, said pharmaceutical composition comprises at least one pharmaceutically acceptable excipient which, further preferably, is suitable for the intended route of administration.

Preferably, said pharmaceutically acceptable excipient are known to the skilled person and comprises at least one polymeric carrier, preferably selected from the group consisting of polysaccharides, polyesters, polymethacrylamide, and mixtures thereof. For example, said pharmaceutically acceptable excipient is a hydrogel, which, for example, provide additionally optimal moisture environment for promoting wound healing. Furthermore, the pharmaceutically acceptable excipient assists in the adhesion of said recombinant bacteria to the site of a, preferably chronic, inflammatory wound or a degenerative condition, or a tumor, preferably a malignant tumor, after application.

Preferably, said pharmaceutical composition further comprises at least one nutrient for said recombinant bacteria, preferably selected from the group consisting of carbohydrates, vitamins, minerals, amino acids, trace elements and mixtures thereof.

Further preferably, said pharmaceutical composition further comprises at least one component for stabilizing the at least one, preferably 1, 2, 3, 4, or more, heterologous factor(s) and/or the recombinant bacteria of the present invention. Said stabilizing compound is preferably selected from the group consisting of metal cations, preferably divalent metal cations such as $Mg^{2+}$, $Ca^{2+}$ and mixtures thereof, anti-microbial agents, cryoprotectants, protease inhibitors, reducing agents, metal chelators, and mixtures thereof.

In a preferred embodiment, the recombinant bacteria of the present invention are provided in form of a kit according to the present invention for use in medicine, further preferably for use in treatment of a, preferably chronic, inflammatory wound or a degenerative condition, or for treatment of a tumor, preferably a malignant tumor, wherein said recombinant bacteria are capable of expressing the at least one heterologous factor under the control of the prokaryotic, chloride-inducible promoter, and wherein the kit further comprises at least one inducer comprising chloride ions.

Preferably, the kit is provided as a combined preparation for separate, sequential or simultaneous use in medicine, further preferably for use in treatment of a, preferably chronic, inflammatory wound or a degenerative condition, or for treatment of a tumor, preferably a malignant tumor.

Preferably, the inducer comprising chloride ions is provided in form of a liquid, preferably culture medium, comprising chloride ions, further preferably together with at least one of the aforementioned pharmaceutically acceptable excipient.

In a preferred embodiment, the recombinant bacteria of the present invention are provided in form of a medical device of the present invention for use in medicine, further preferably for use in treatment of a, preferably chronic, inflammatory wound or a degenerative condition, or for treatment of a tumor, preferably a malignant tumor, wherein said recombinant bacteria are capable of expressing the at least one heterologous factor under the control of the prokaryotic, chloride-inducible promoter.

A suitable medical device preferably further comprises at least one support and/or container, for the provision of the said recombinant bacteria, for example a cell encapsulation system and/or microbeads.

A suitable medical device is preferably a stent, an implant, an inhaler, an encampsulation system or a medical dressing.

Preferably, the pharmaceutical composition and/or the kit and/or the medical device according to the present invention comprises said recombinant bacteria in solution, frozen, or dried, preferably lyophilised or spray dried. Further preferably, said recombinant bacteria are reconstituted in a liquid, preferably culture medium, comprising chloride ions prior to administration of the recombinant bacteria and/or pharmaceutical composition and/or kit to a subject.

The recombinant bacteria of the present invention might be contacted with said at least one inducer, preferably to induce expression of the at least one, preferably 1, 2, 3, 4, or more, heterologous factor(s).

Further, preferably, after induction of the expression, the at least one, preferably 1, 2, 3, 4, or more, heterologous factor(s) is/are released from said bacteria.

Preferably, said at least one, preferably 1, 2, 3, 4, or more, heterologous factor(s) is/are expressed with a secretory signal sequence, preferably N-terminal signal peptide. After expression of the respective factor, the secretory signal sequence, preferably N-terminal signal peptide, can be removed. Alternatively, said first, second, and/or third heterologous factor might be expressed in mature form, preferably without a secretory signal sequence, preferably N-terminal signal peptide.

A reconstitution medium comprising chloride ions, which is used to reconstitute recombinant bacteria according to the present invention, can be in the form of a solution or a dispersion, such as an emulsion, a suspension, a gel, preferably hydrogel, or a colloidal solution.

Preferably the reconstitution medium comprises chloride ions in an amount sufficient to initiate expression of the at least one heterologous factor under the control of the prokaryotic, chloride-inducible promoter.

Suitable polymers for obtaining a gel, preferably hydrogel, are known to the skilled person and include natural and/or synthetic polymers.

Preferably, the recombinant bacteria of the present invention are provided in an effective amount to treat and/or alleviate the course of a disease, such as a, preferably chronic, inflammatory wound, or a degenerative condition, or a tumor, preferably a maligant tumor, in a subject.

Preferably the pharmaceutical composition and/or the kit and/or medical device according to the present invention comprise an effective amount of the recombinant bacteria of the present invention.

In a preferred embodiment of the present invention, the recombinant bacteria of the invention and/or the pharmaceutical composition and/or the kit and/or medical device according to the present invention are to be used in treatment of an inflammatory wound, preferably a chronic inflammatory wound.

In a preferred embodiment, said inflammatory, preferably a chronic inflammatory, wound includes preferably frostbites, dermatitis, ulcer, and combinations thereof, further preferably ulcer.

Said inflammatory wound can also include an inflammatory skin trauma, which may progress into a chronic inflammatory state.

Frostbite is the medical condition in which localized damage is caused to skin and other tissues due to freezing and can involve tissue destruction. Said frostbite can also be chilblains (perniones), which are superficial ulcers of the skin that result from exposure to cold and humidity. Damage to capillary beds in the skin causes redness, itching, inflammation, and sometimes blisters. Further preferably, said frostbites are chilblains.

Chronic inflammatory wounds are known to the skilled person and include, for example, chronic venous ulcers, chronic arterial ulcers, chronic diabetic ulcers, and chronic pressure ulcers. Preferably said chronic wound is at least one of a chronic venous ulcer, a chronic arterial ulcer, a chronic pressure ulcer and a chronic preulceration stage thereof, preferably a chronic venous ulcer, a chronic arterial ulcer and a chronic pressure ulcer.

Chronic wounds occur in various forms and prevalences. A chronic wound does not heal in an orderly set of stages and in a predictable amount of time compared to an acute wound. Wounds that do not heal within 1-3 months or do not respond to initial treatments are typically classified as chronic. In acute wounds, there is a precise balance between production and degradation of molecules as well as the activation level of immune cells in the micro-environment leading to a well-controlled, step-wise healing process. In chronic wounds, this balance is lost resulting in degradation and aberrant activation of the local immune system. Commonly, chronic wounds remain in one or more of the phases of the wound healing process, for example in the inflammatory stage. This type of wounds is referred to as chronic inflammatory wounds.

Chronic wounds are usually results of various underlying diseases and medical conditions such as a tumor, diabetes (resulting in DFU), poor circulation (resulting in VLU), surgery and burns. Ischemia (i.e. the lack of oxygen in the extremities), bacterial infection and colonization, increase of proteolytic enzymes and inflammation are common underlying pathophysiological causes for non-healing wounds.

Said ulcer can be a decubitus ulcer or an ulcus cruris. Said ulcer can also be a venous ulcer, an arterial ulcer, a diabetic ulcer, or a pressure ulcer. Said ulcer can also be a preulceration stage of the above-mentioned ulcers without any visible sign of open skin wound. Without medical intervention, the preulceration stage may progress to ulceration.

Chronic venous ulcers usually may occur in the legs and account for about 70% to 90% of chronic wounds, mostly affecting elderly patients.

Another major cause of chronic inflammatory wounds is diabetes. Patients suffering from diabetes have a 15% higher risk for amputation than the general population due to chronic ulcers. Diabetes causes neuropathy, which inhibits nociception and the perception of pain. Thus, patients may not notice small wounds to legs and feet and may therefore fail to prevent infection or repeated injury.

A further problem is that diabetes causes immune compromise and damage to small blood vessels resulting in a reduced oxygenation of tissue. Preventing adequate oxygenation of tissue significantly increase the prevalence for chronic inflammatory wounds.

Pressure ulcers, which are also known as decubitus ulcers or bed sore, may occur with or without a diabetic condition. Pressure ulcers are localized injuries to the skin and/or underlying tissue that can occur over a bony prominence as a result of pressure, or pressure in combination with shear or friction.

Currently, the standard therapeutic management of chronic inflammatory wounds including diabetic wounds such as lower extremity ulceration is focussed primarily on controlling infection and promoting revascularisation. Despite these strategies, the rate of amputation remains unacceptably high in patients suffering from lower extremity ulceration.

Furthermore, when an underlying disease condition or cause of an ulceration, such as diabetes mellitus or chronic venous insufficiency, is improved and/or treated, for example by controlling blood sugar levels or administering blood pressure medication, respectively, existing ulcers may still take a very long time to heal.

Thus, in order to overcome the lack of definitive, non-invasive treatments of chronic inflammatory wounds including diabetic wounds such as lower extremity ulceration new strategies to reactivate and promote wound healing in patients suffering from chronic wounds are urgently needed.

Preferably, in case of an inflammatory wound, preferably chronic inflammatory wound, the unique combination of factors, preferably released from said bacteria, allows for a reprogramming of said chronic inflammatory wound into an acute wound, which subsequently undergoes wound closure.

In a preferred embodiment, in case of an inflammatory wound, preferably chronic inflammatory wound, the recombinant bacteria are to be administered systemically, topically and/or by subcutaneous injection, further preferably topically.

The recombinant bacteria preferably are to be administered topically, to the inflammatory, preferably chronic inflammatory, wound to be treated.

The recombinant bacteria can be administered topically to the inflammatory, preferably chronic inflammatory, wound and/or by subcutaneous injection into the vicinity of the wound, preferably into the edges or cavity of the inflammatory, preferably chronic inflammatory wound.

Furthermore, by topical application or subcutaneous injection of the recombinant bacteria of the present invention to and/or into the site of the preulceration a progression of the preulceration to an open inflammatory wound can be avoided.

In a preferred embodiment, in case of a tumor, preferably a malignant tumor, the recombinant bacteria are to be administered systemically, for example by intravenous injection, or locally, preferably by intratumoral injection and/or by intraperitonal injection.

The recombinant bacteria are preferably administered to the tumor, preferably malignant tumor, by intratumoral injection into the tumor and/or by injection into the vicinity of the tumor and/or by intraperitoneal injection of the recombinant bacteria of the present invention.

In a preferred embodiment, said recombinant bacteria according to claim 1 comprise at least one nucleic acid sequence encoding for a first heterologous factor, at least one nucleic acid sequence encoding for a second heterologous factor, and at least one nucleic acid sequence encoding for a third heterologous factor, with the proviso that said first factor, said second factor, and said third factor are functionally different from each other,
wherein said first factor is a growth factor, wherein said second factor is selected from the group consisting of M2-polarizing factors and wherein said third factor is selected from the group consisting of M2-polarizing factors and growth factors.

In a preferred embodiment, said second heterologous factor and said third heterologous factor are selected from the group consisting of M2-polarizing factors, wherein said second factor and said third factor are functionally different M2-polarizing factors.

This is to say, said second heterologous factor is a first M2-polarizing factor and said third heterologous factor is a second M2-polarizing factor functionally different from said first M2-polarizing factor.

Preferably, said first M2-polarizing factor is a M2-polarizing factor selected from the group consisting of colony stimulating factor-1 (CSF-1), interleukin 34 (IL-34), interleukin 4 (IL-4), and interleukin 13 (IL-13), and said second M2-polarizing factor is a M2-polarizing factor selected from the group consisting of colony stimulating factor-1 (CSF-1), interleukin 34 (IL-34), interleukin 4 (IL-4), interleukin 10 (IL-10), and interleukin 13 (IL-13), with the proviso that said second M2-polarizing factor is functionally different from said first M2-polarizing factor.

Further preferably, said first M2-polarizing factor is a colony stimulating factor-1 receptor (CSF1R) ligand and said second M2-polarizing factor is a M2-polarizing factor selected from the group consisting of interleukin 4 (IL-4), interleukin 10 (IL-10), interleukin 13 (IL-13), functional analogs thereof, biosimilars thereof, and mixtures thereof.

Further preferred combinations of M2-polarizing factors are:

- colony stimulating factor-1 and interleukin-4,
- colony stimulating factor-1 and interleukin-13,
- colony stimulating factor-1 and interleukin-10,
- interleukin-34 and interleukin-4,

- interleukin-34 and interleukin-13,
- interleukin-34 and interleukin-10,
- interleukin-4 and interleukin-10, or
- interleukin-13 and interleukin-10.

The above mentioned further preferred combinations of M2-polarizing factors are combined with at least one of the above mentioned growth factors, preferably with a growth factor selected from the group consisting of fibroblast growth factor 1, fibroblast growth factor 2, fibroblast growth factor 7, fibroblast growth factor 10, hepatocyte growth factor, transforming growth factor beta (TGF-beta), epidermal growth factor (EGF), heparin-binding EGF-like growth factor (HB-EGF), transforming growth factor-$\alpha$ (TGF-$\alpha$), and platelet derived growth factor BB.
Preferably, said first, second, and third heterologous factor is a combination of

- fibroblast growth factor 2, colony stimulating factor-1 and interleukin-4,
- fibroblast growth factor 2, interleukin-34 and interleukin-4,
- fibroblast growth factor 2, colony stimulating factor-1 and interleukin-13,
- fibroblast growth factor 2, interleukin-34 and interleukin-13,
- fibroblast growth factor 2, colony stimulating factor-1 and interleukin-10,
- fibroblast growth factor 2, interleukin-34 and interleukin-10,
- fibroblast growth factor 7, colony stimulating factor-1 and interleukin-4,
- fibroblast growth factor 7, interleukin-34 and interleukin-4,
- fibroblast growth factor 7, colony stimulating factor-1 and interleukin-13,
- fibroblast growth factor 7, interleukin-34 and interleukin-13,
- fibroblast growth factor 7, colony stimulating factor-1 and interleukin-10,
- fibroblast growth factor 7, interleukin-34 and interleukin-10,
- transforming growth factor beta, colony stimulating factor-1 and interleukin-4,
- transforming growth factor beta, interleukin-34 and interleukin-4,
- transforming growth factor beta, colony stimulating factor-1 and interleukin-13,
- transforming growth factor beta, interleukin-34 and interleukin-13
- transforming growth factor beta, colony stimulating factor-1 and interleukin-10,
- transforming growth factor beta, interleukin-34 and interleukin-10,
- transforming growth factor alpha, colony stimulating factor-1 and interleukin-4,
- transforming growth factor alpha, interleukin-34 and interleukin-4,
- transforming growth factor alpha, colony stimulating factor-1 and interleukin-13,
- transforming growth factor alpha, interleukin-34 and interleukin-13
- transforming growth factor alpha, colony stimulating factor-1 and interleukin-10,
- transforming growth factor alpha, interleukin-34 and interleukin 10
- platelet derived growth factor BB, colony stimulating factor-1 and interleukin-4,
- platelet derived growth factor BB, interleukin-34 and interleukin-4,
- platelet derived growth factor BB, colony stimulating factor-1 and interleukin-13,
- platelet derived growth factor BB, interleukin-34 and interleukin-13
- platelet derived growth factor BB, colony stimulating factor-1 and interleukin-10, or
- platelet derived growth factor BB, interleukin-34 and interleukin-10.

Further preferably, said first, second, and third heterologous factor is a combination of fibroblast growth factor 2, colony stimulating factor-1 and interleukin-4, functional analogs thereof, and biosimilars thereof.
Preferably, by release of two or more M2-polarizing factors from the bacteria of the present invention M2-polarization of unpolarized macrophages, M1-polarized macrophages as well as undifferentiated monocytes, and other macrophage progenitor cells is further fostered.
In an alternative embodiment, said first factor is a first growth factor selected from the group consisting from the above-mentioned growth factors, and said third factor is a second growth factor selected from the group consisting from the above-mentioned growth factors and which is functionally different from said first growth factor. Preferably, said second growth factor is transforming growth factor beta (TGF-beta).
Further preferred combinations of growth factors are:

- fibroblast growth factor 1 and transforming growth factor beta,
- fibroblast growth factor 2 and transforming growth factor beta,
- fibroblast growth factor 7 and transforming growth factor beta,
- fibroblast growth factor 10 and transforming growth factor beta,

- platelet derived growth factor BB and transforming growth factor beta,
- transforming growth factor alpha and transforming growth factor beta,
- epidermal growth factor and transforming growth factor beta,
- heparin-binding EGF-like growth factor and transforming growth factor beta,
- hepatocyte growth factor and transforming growth factor beta, or
- vascular endothelial growth factor A and transforming growth factor beta.

The above mentioned further preferred combinations of growth factors are preferably combined with a M2-polarizing factor selected from the group consisting of colony stimulating factor-1 (CSF-1), interleukin-34 (IL-34), interleukin-4 (IL-4), interleukin-10 (IL-10), interleukin-13 (IL-13), and mixtures thereof, preferably colony stimulating factor-1 (CSF-1), interleukin-34 (IL-34), interleukin-4 (IL-4), interleukin-13 (IL-13), and mixtures thereof.

In another preferred embodiment of the present invention, the recombinant bacteria of the invention and/or the pharmaceutical composition and/or the kit and/or medical device according to the present invention are to be used in treatment of a tumor, preferably a malignant tumor, preferably a peritoneal cancer, further preferably a peritoneal carcinomatosis, further preferably metastatic ovarian cancer, colorectal carcinoma, pancreatic cancer, stomach cancer, hepatocellular carcinoma, gallbladder carcinoma, renal cell carcinoma, transitional cell carcinoma, endometrial cervical cancers and/or extra-abdominal conditions such as breast cancer, lung cancer and malignant melanoma .

Tumor is an abnormal growth of cells/tissue that possesses no physiological function and arises from uncontrolled usually rapid cellular proliferation. Tumors may be benign, or malignant. Different types of tumors are named for the type of cells that form them. Examples of solid tumors are sarcomas, carcinomas, and lymphomas.

Peritoneal cancers can be divided into two categories; primary peritoneal cancers, wherein the primary tumor originates within the peritoneum, and peritoneal carcinomatosis (PC), defined as intraperitoneal dissemination of any tumor originating elsewhere.

Peritoneal carcinomatosis is one of the most common diffuse peritoneal diseases with ovarian cancer being the most common cause of it (46%), followed by colorectal carcinoma (31%), pancreatic cancer, stomach cancer and other malignancies, including the hepatocellular carcinoma, gallbladder carcinoma, renal cell carcinoma, transitional cell carcinoma, endometrial, cervical cancers and unknown primary. Extra-abdominal conditions such as breast cancer, lung cancer and malignant melanoma can involve the peritoneal cavity through the haematogenous spread (Singh, S. et al. "Peritoneal Carcinomatosis: Pictorial Review of Computed Tomography Findings", International Journal of Advanced Research (2016), Volume 4, Issue 7, pages 735 to 748; doi:10.21474/IJAR01/936).

In 10%-35% of patients with recurrent colorectal cancer (CRC) and in up to 50% of patients with recurrent gastric cancer (GC), tumor recurrence is confined to the peritoneal cavity; (Coccolini, F. et al., World J Gastroenterol. 2013; 19(41): pages 6979 to 6994, doi: 10.3748/wjg.v19.i41.6979).

Peritoneal metastasis can also be formed due to distant metastases of extra-peritoneal cancers, e.g., pleural mesothelioma, breast and lung. Lodging of tumor cells in diaphragmatic or abdominal lymphatic ducts causes obstruction of lymphatic drainage and decreased outflow of peritoneal fluid, leading to formation of carcinomatosis and/or ascites.

The immune system preferably plays a major role in the pathophysiology of cancers. Many tumors are heavily infiltrated by different types of immune cells. The composition of these cells within a tumor, however, may vary. Cells from both, the adaptive immune system, such as cytotoxic T-cells and T-regulatory cells, and cells of the innate immune system, such as macrophages, dendritic cells and natural killer (NK) cells, are key players in the progression of the disease. These immune cells seem to be connected to tumor growth, invasion and metastasis.

In particular, tumor growth has been paralleled with recruitment and accumulation of macrophages, wherein these cells acquire functions supporting tumor growth and dissemination of cancer cells from the primary tumor. Furthermore, there is clinical evidence demonstrating a strict correlation between the increased numbers of macrophages with progression of disease and poor prognosis. Since macrophages are able to produce a broad spectrum of different cytokines (Th1 and Th2 related cytokines) they are key players in the orchestration of the immune microenvironment.

Macrophages can be divided into M1 (classically activated) or M2 (alternatively activated) phenotypes. M1 macrophages exhibit a pro-inflammatory phenotype and are characterized by expression of pro-inflammatory cytokines such as IL-1, IL-6, TNF-$\alpha$ and IFN-$\gamma$. Monocytes can be differentiated into M1 type macrophages by bacterial components such as lipopolysaccharide (LPS) and interferon $\gamma$ (INF-y).

By contrast, M2 macrophages have an immunosuppressive phenotype releasing factors that promote a Th2 response such as IL-10, TGF-$\beta$ and VEGF.

Macrophages in tumors, which are also called as tumor associated macrophages (TAM's), often express many genes typical of the M2 phenotype. Chemo-attractants secreted by tumor and stromal cells recruit monocytes into the tumor tissue and drive them to differentiate into M2 tumor associated macrophages, thereby supporting tumor growth and metastasis.

After administration of the recombinant bacteria of the present invention to a tumor, the recombinant bacteria produce and release the therapeutic protein(s) into the tumor environment.

Preferably, the the therapeutic protein(s) released enhance and drive these immune cells towards an $T_H1$ immune response., which then results in a reduction of the production of vascular endothelial growth factor (VEGF).

Studies indicate that the expression of VEGF and their receptors on macrophages play a role in modulating the immune system and its surrounding microenvironment. Particularly M2 tumor associated macrophages do express large amounts of VEGF, supporting angiogenesis and immunomodulation.

VEGF has also been described to inhibit the differentiation of dendritic cells. In contrast, also Furthermore, VEGF has been shown to inhibit T cell development, thereby contributing to tumor-induced immune suppression. F

A reduction of the production of VEGF, preferably, further supports an anti-tumor effect by preventing immunosuppression and angiogenesis.

In a preferred embodiment, said recombinant bacteria according to claim 1 comprise at least one nucleic acid sequence encoding for a at least one heterologous factor, preferably selected from the group sonsisting of cell receptor activators, such as glucocorticoid-induced TNFR-related protein (GITR), tumor necrosis factor receptor superfamily member 9 (TNFRSF9), which is also known as 4-1BB, cluster of differentiation 40 (CD40), tumor necrosis factor receptor superfamily, member 4 (TNFRSF4), also known as CD134 or OX40 receptor, and combinations thereof, co-stimulatorty receptor activators, immune check point inhibitors, such as cytotoxic T-lymphocyte-associated protein 4 (CTLA-4), programmed cell death protein 1 (PD-1), programmed death-ligand 1 (PD-1L), programmed death-ligand 2 (PD-1L2), and combinations thereof, chimeric antigen receptors, such as CAR-T, and combinations thereof, pro-drug activating enzymes, such as HSV-tk, cyticine deaminase and combinations thereof, cytokines, chemokines, growth factors, decoy receptor ligands, antibodies, soluble receptors, decoy receptors, and combinations thereof, preferably granulocyte-macrophage-colony-stimulating factor (GM-CSF), interferon alpha, preferably interferon alpha 2 (IFNA2), interferon beta, interferon gamma, granulocyte-colony stimulating factor (G-CSF), interleukin-2 (IL-2), interleukin-6 (IL-6), interleukin-7 (IL-7), interleukin-8 (IL-8), interleukin-12 (IL-12), interleukin-15 (IL-15), interleukin-17 (IL-17), interleukin-18 (IL-18), interleukin-21 (IL-21), interleukin-23 (IL-23), interleukin-24 (IL-24), interleukin-32 (IL-32), and combinations thereof.

## Sequences

SEQ ID No 1 corresponds to the nucleic acid sequence of expression plasmid pAUC1010, which is also shown in Fig. 1. SEQ ID No. 2 corresponds to the nucleic acid sequence of the PgadC promoter used in the Examples and which is also shown in Fig. 2b. SEQ ID No. 3 corresponds to the amino acid sequence of Usp45 signal sequence of *L. lactis,* which is also shown in Fig. 3. SEQ ID No. 4 corresponds to the 3' end of 16S RNA of *L. lactis,* which is also shown in Fig.4.

SEQ ID No. 5 corresponds to the amino acid sequence of the mature form of hFGF-2-155, which is also shown in Fig. 5a. SEQ ID No. 6 correspomds to the amino acid sequence of the hFGF-2-153 variant used in the Examples and which is also shown in Fig. 5b. SEQ ID No. 7 corresponds to the amino acid sequence of the recombinant hFGF-2-153 precursor protein used in the Examples and which is also shown in Fig. 5c.

SEQ ID No. 8 corresponds to the amino acid sequence of the mature form of hIL-4, which is also shown in Fig. 6a. SEQ ID No. 9 correspomds to the amino acid sequence of the hIL-4 variant used in the Examples and which is also shown in Fig. 6b. SEQ ID No. 10 corresponds to the amino acid sequence of the recombinant hIL-4 precursor protein used in the Examples and which is also shown in Fig. 6c.

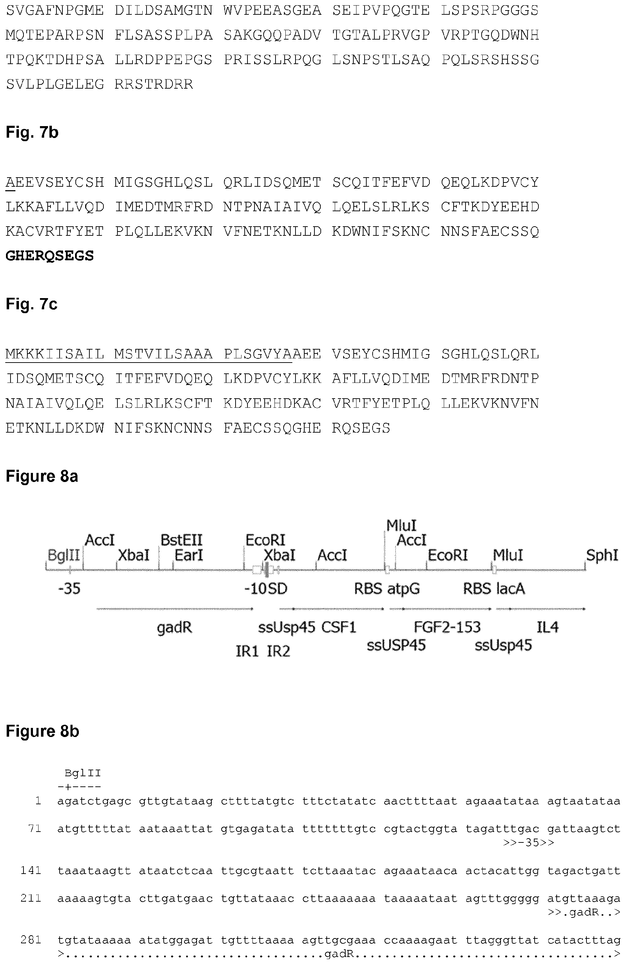

SEQ ID No. 11 corresponds to the amino acid sequence of the mature form of hCSF1, which is also shown in Fig. 7a. SEQ ID No. 12 correspomds to the amino acid sequence of the hCSF1 variant used in the Examples and which is also shown in Fig. 7b. SEQ ID No. 13 corresponds to the amino acid sequence of the recombinant hCSF1 precursor protein used in the Examples and which is also shown in Fig. 7c.

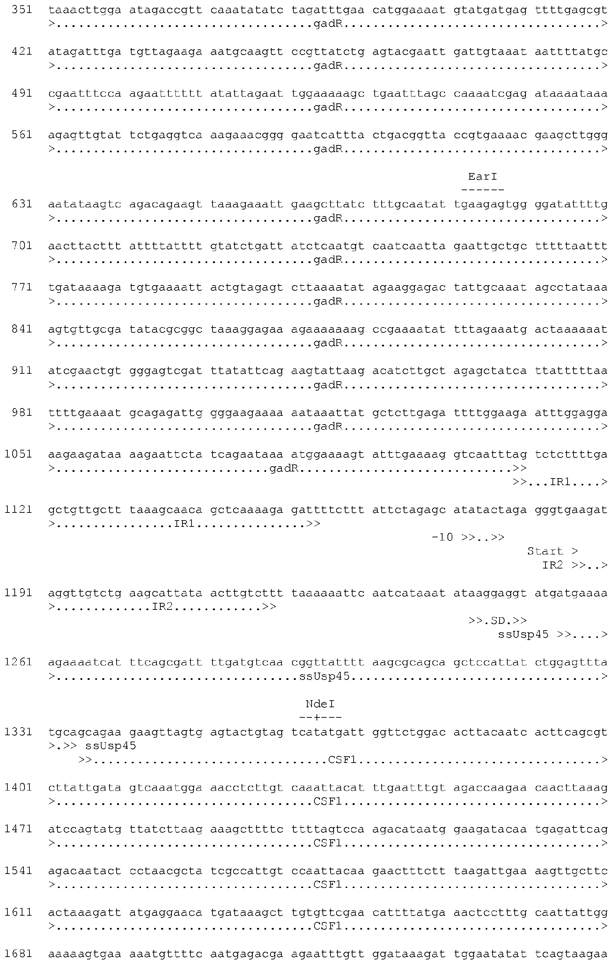

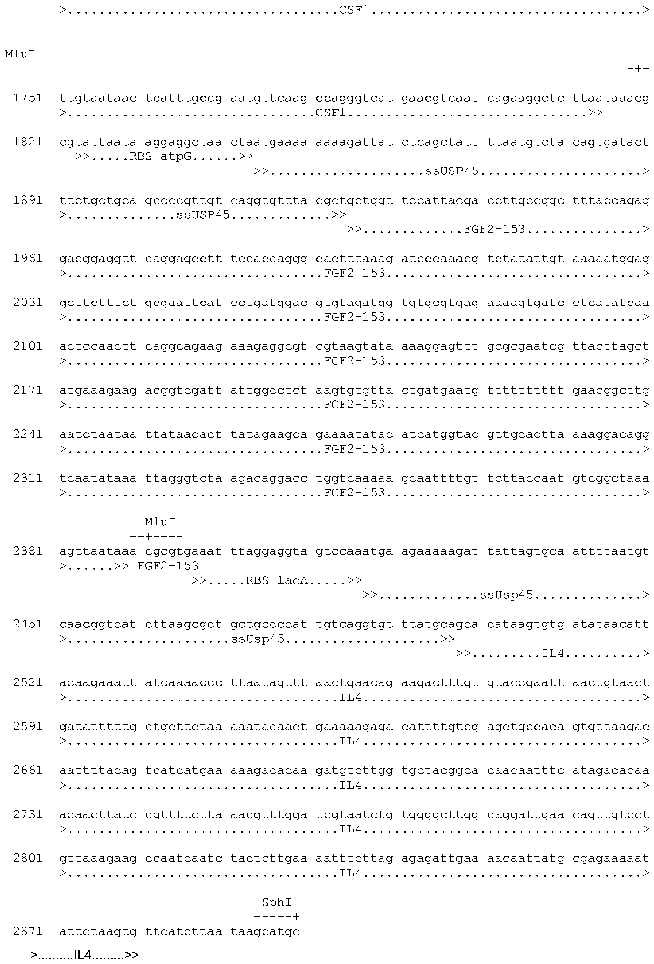

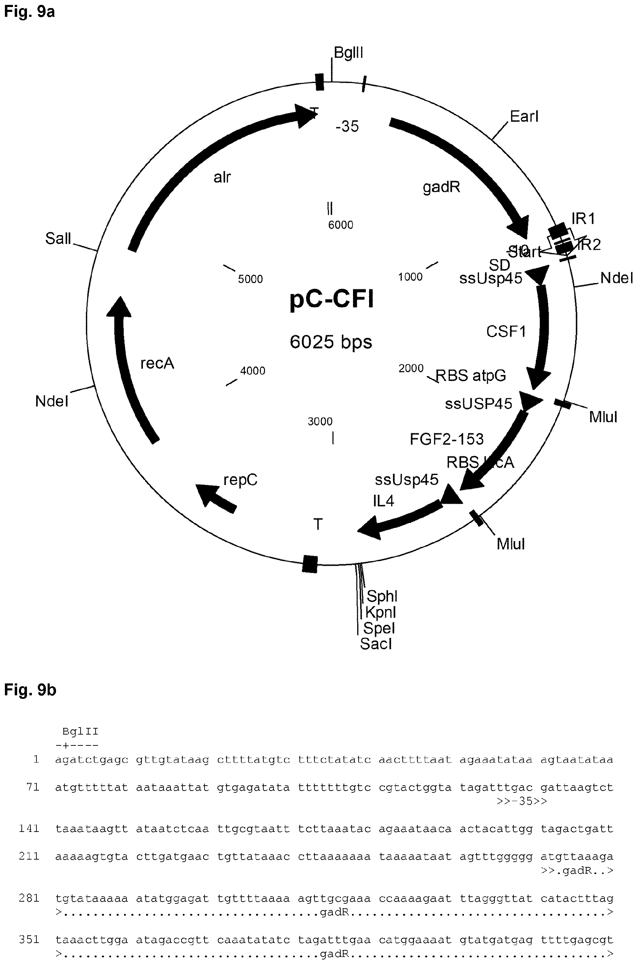

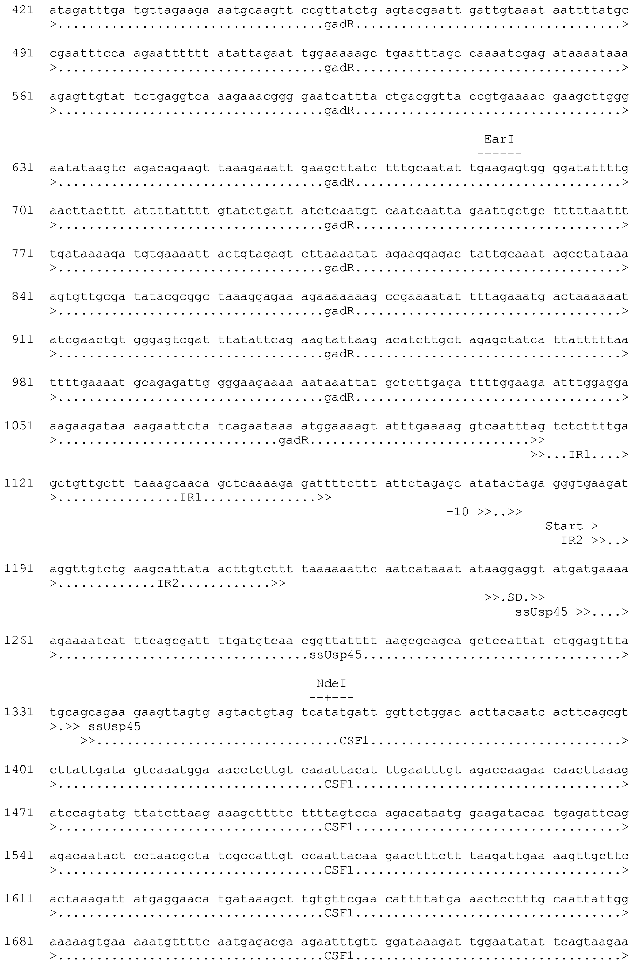

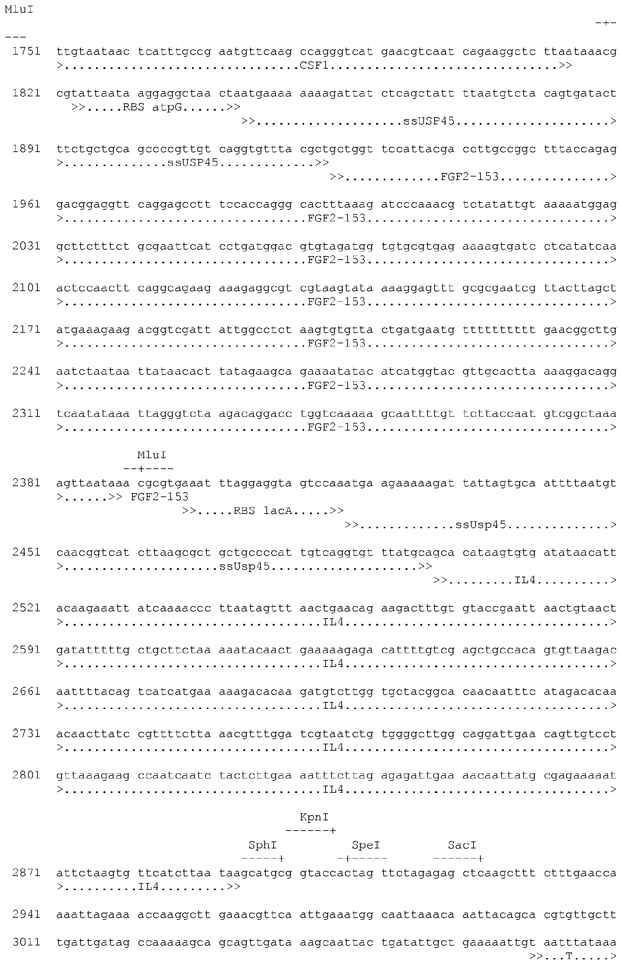

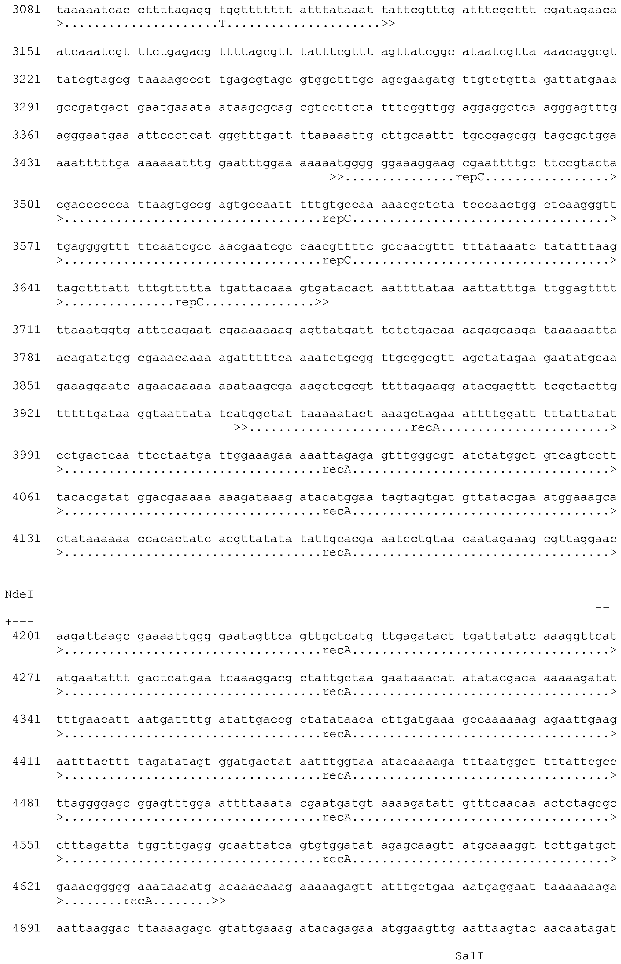

SEQ ID No. 14 corresponds to the nucleic acid sequence of synthetic CFI construct used in the Examples and which is also shown in Fig. 8b. SEQ ID No. 15 corresponds to the nucleic acid sequence of expression plasmid pC-CFI used in the Examples and which is also shown in Fig. 9b.

SEQ ID No. 16 corresponds to the amino acid sequence of the mature form of mIL-18, which is also shown in Fig. 10a. SEQ ID No. 17 correspomds to the amino acid sequence of the mIL-18 variant used in the Examples and which is also shown in Fig. 10b. SEQ ID No. 18 corresponds to the amino acid sequence of the recombinant mIL-18 precursor protein used in the Examples and which is also shown in Fig. 10c.

SEQ ID No. 19 corresponds to the amino acid sequence of the mature form of mGM-CSF, which is also shown in Fig. 10a. SEQ ID No. 20 correspomds to the amino acid sequence of the mGM-CSF variant used in the Examples and which is also shown in Fig. 10b. SEQ ID No. 21 corresponds to the amino acid sequence of the recombinant mGM-CSF precursor protein used in the Examples and which is also shown in Fig. 10c.

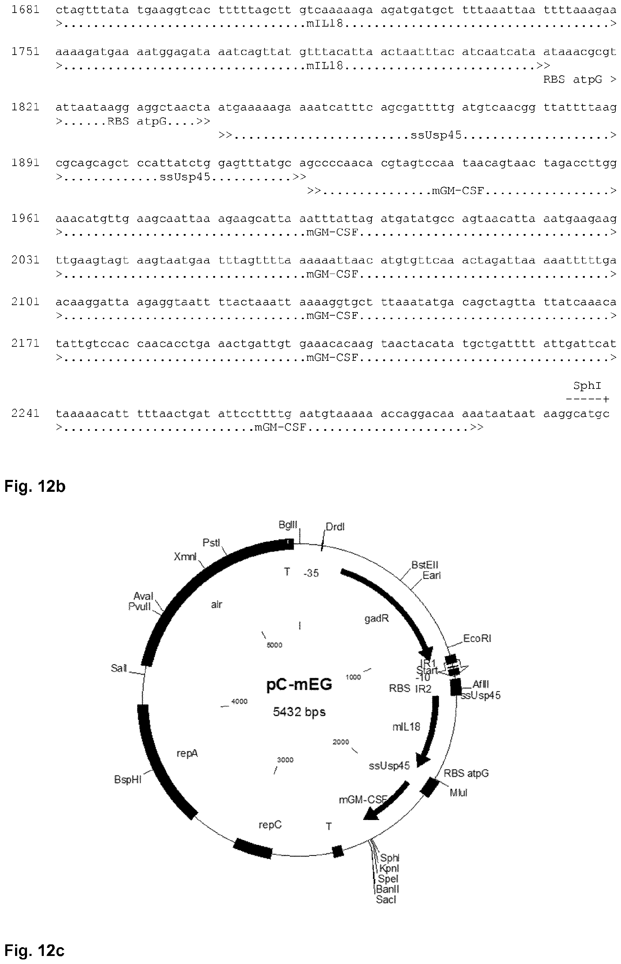

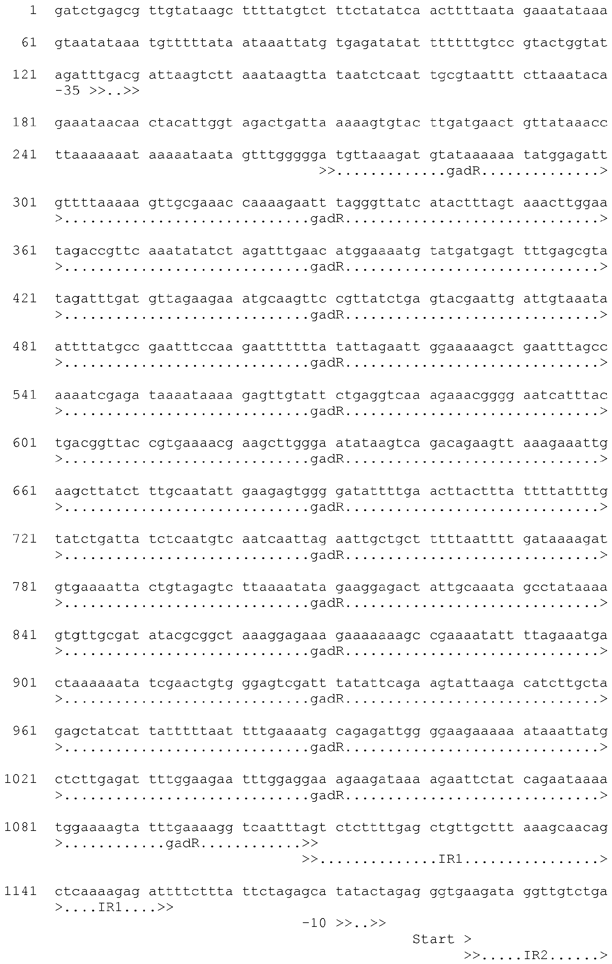

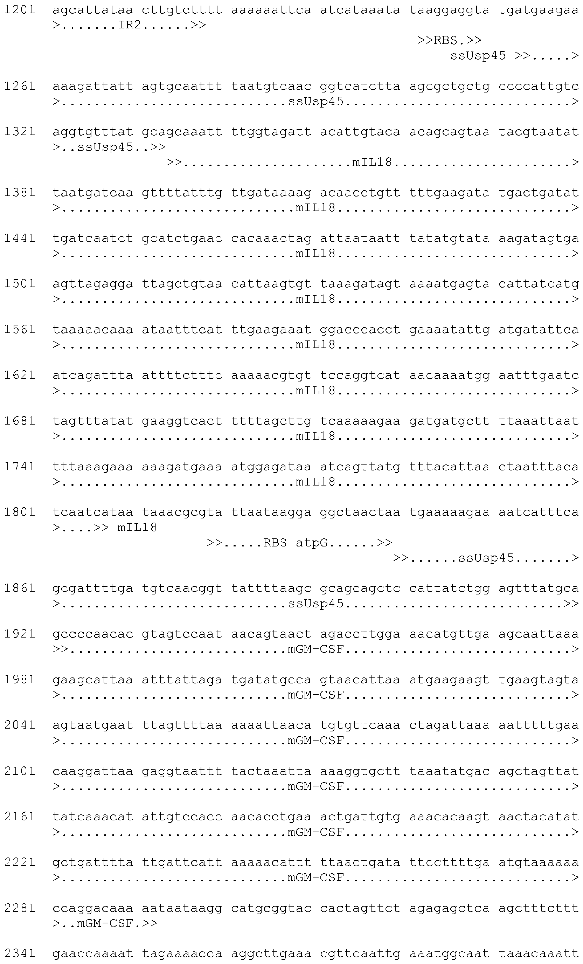

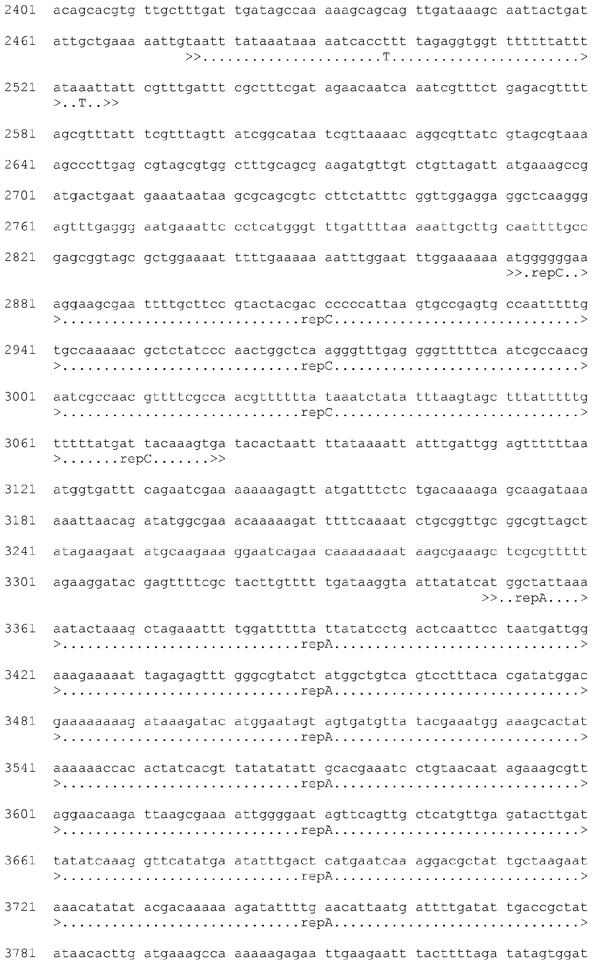

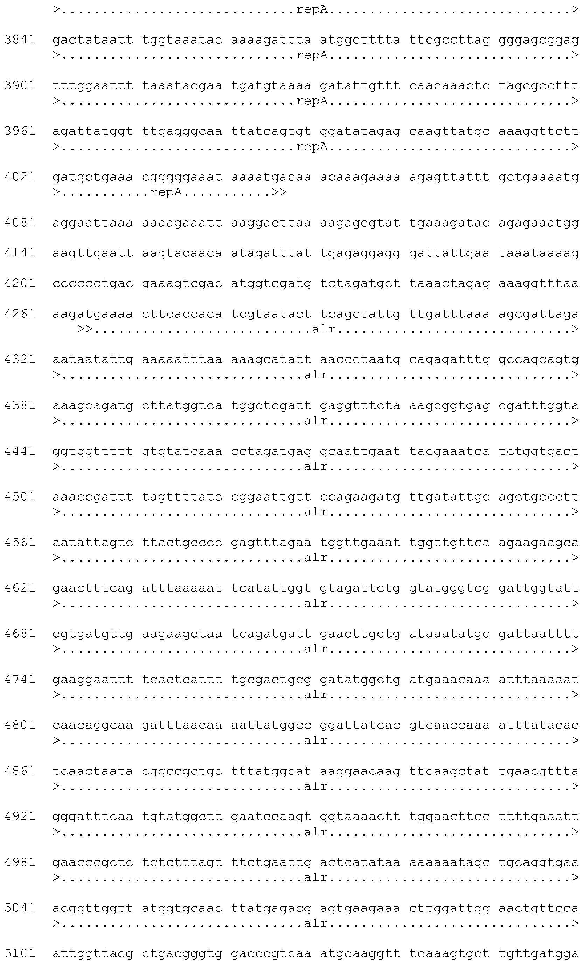

SEQ ID No. 22 corresponds to the nucleic acid sequence of synthetic mEG construct used in the Examples and which is also shown in Fig. 12b. SEQ ID No. 23 corresponds to the nucleic acid sequence of expression plasmid pC-mEG used in the Examples and which is also shown in Fig. 12c.

SEQ ID No. 24 corresponds to the amino acid sequence of the mature form of mIL-12 beta, which is also shown in Fig. 13a. SEQ ID No. 25 corresponds to the amino acid sequence of the mature form of mIL-12 alpha isoform 2, which is also shown in Fig. 13b. SEQ ID No. 26 correspomds to the amino acid sequence of the mature form of the recombinant mouse interleukin-12 fusion protein used in the Examples and which is also shown in Fig. 13c. SEQ ID No. 27 corresponds to the amino acid sequence of the recombinant mouse interleukin-12 precursor protein used in the Examples and which is also shown in Fig. 13d.

SEQ ID No. 28 corresponds to the amino acid sequence of the mature form of mouse interferon alpha-2, which is also shown in Fig. 14a. SEQ ID No. 29 correspomds to the amino acid sequence of the mouse interferon alpha-2 variant used in the Examples and which is also shown in Fig. 14b. SEQ ID No. 30 corresponds to the amino acid sequence of the recombinant mouse interferon alpha-2 precursor protein used in the Examples and which is also shown in Fig. 14c.

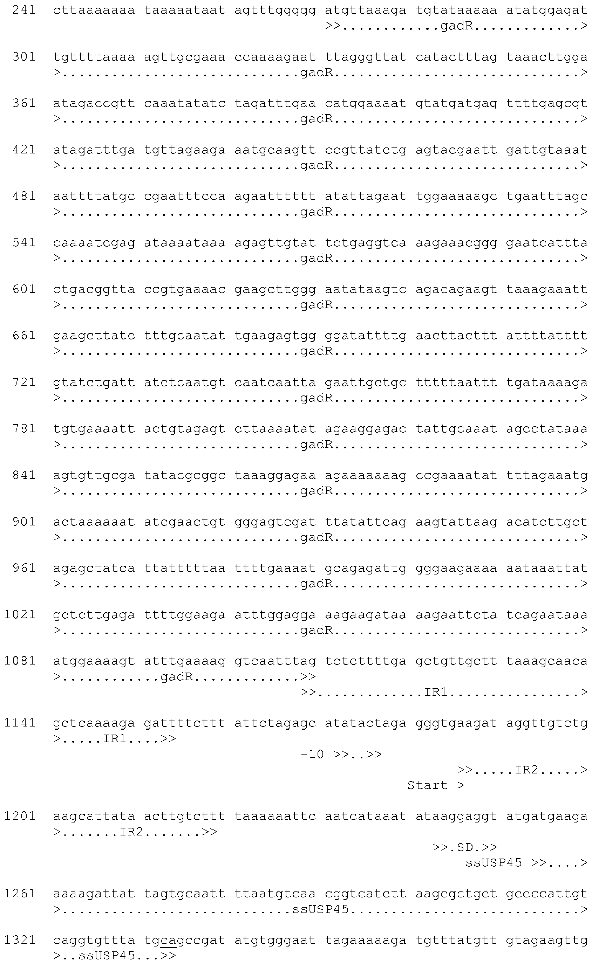

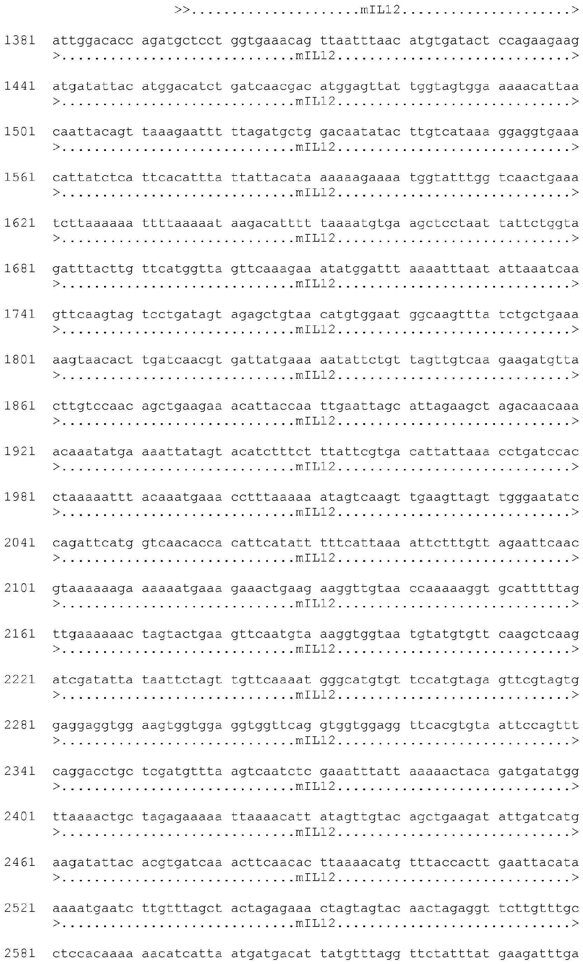

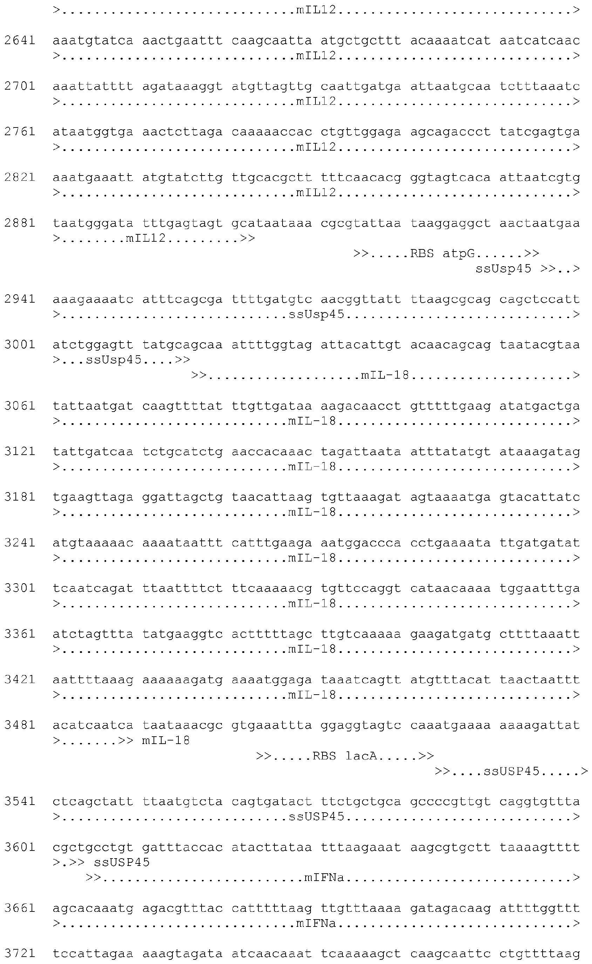

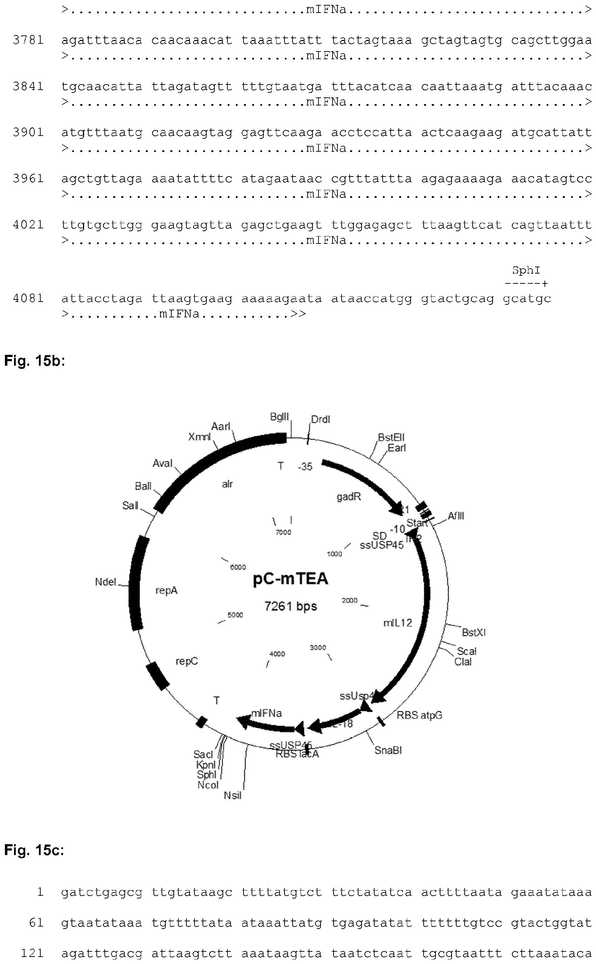

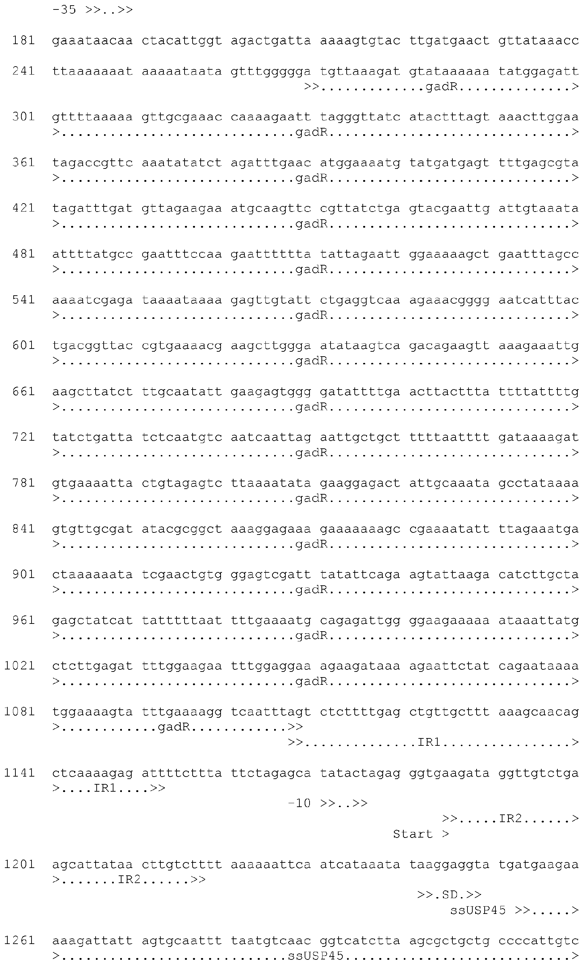

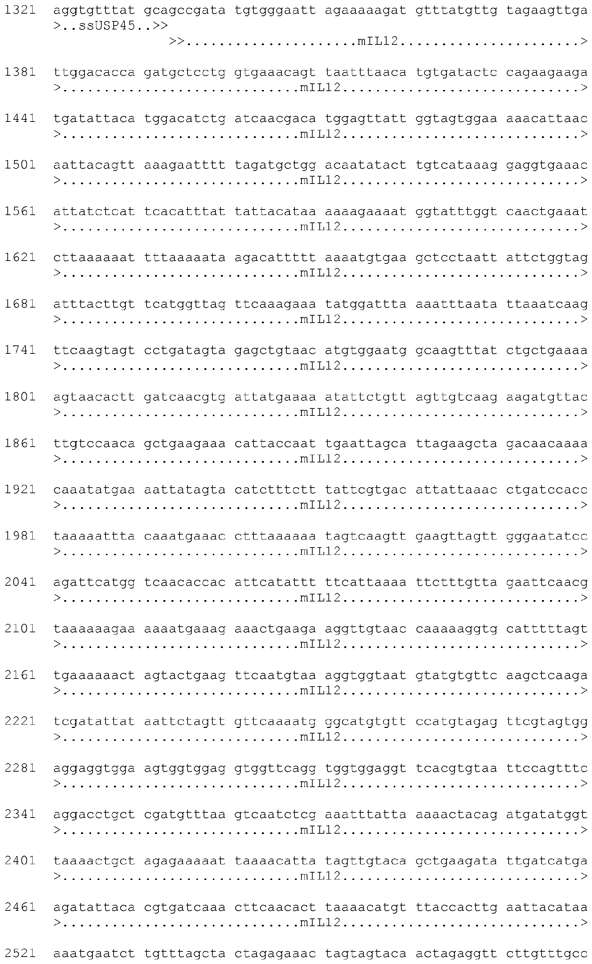

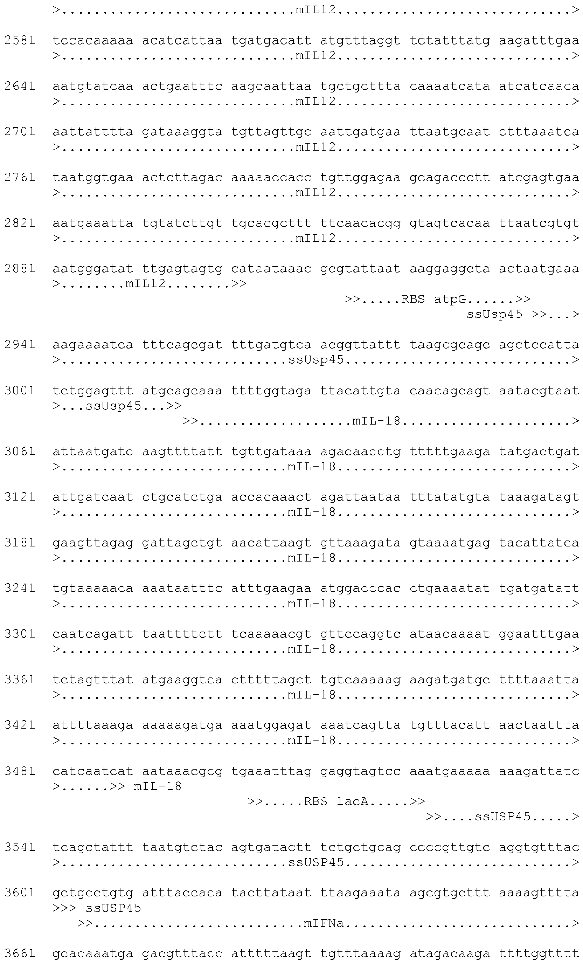

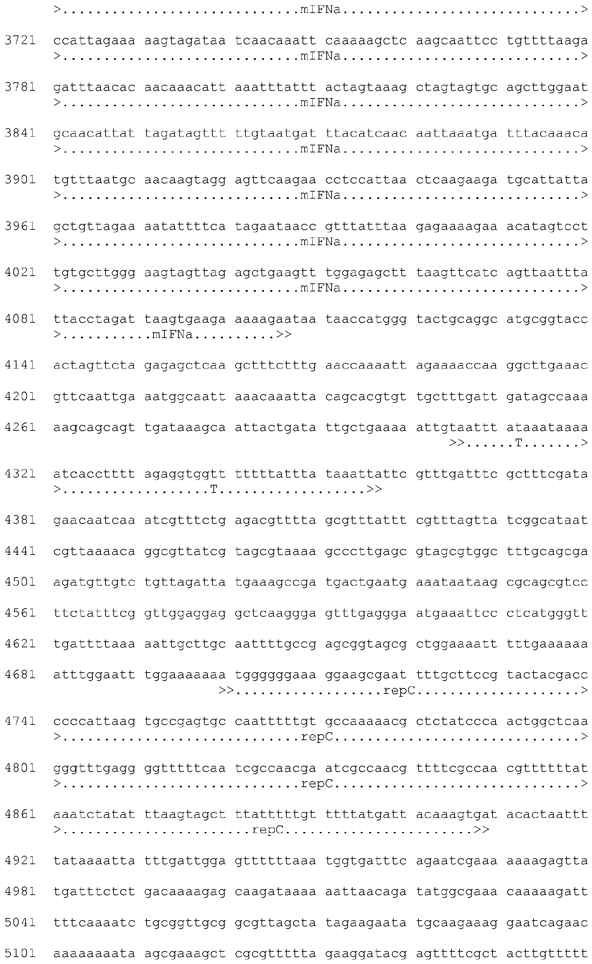

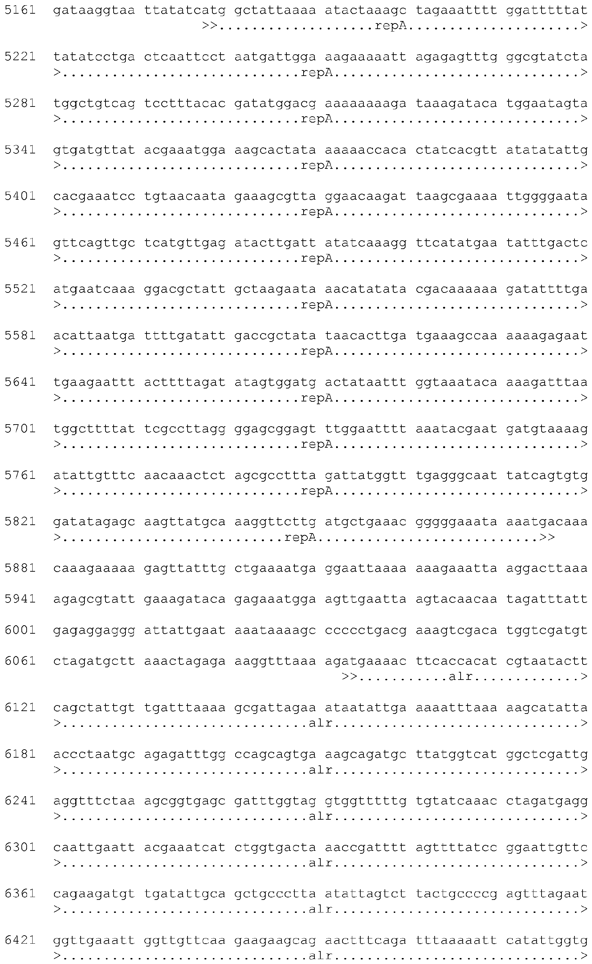

SEQ ID No. 31 corresponds to the nucleic acid sequence of synthetic mTEA construct used in the Examples and which is also shown in Fig. 15a. SEQ ID No. 32 corresponds to the nucleic acid sequence of expression plasmid pC-mTEA used in the Examples and which is also shown in Fig. 15c.

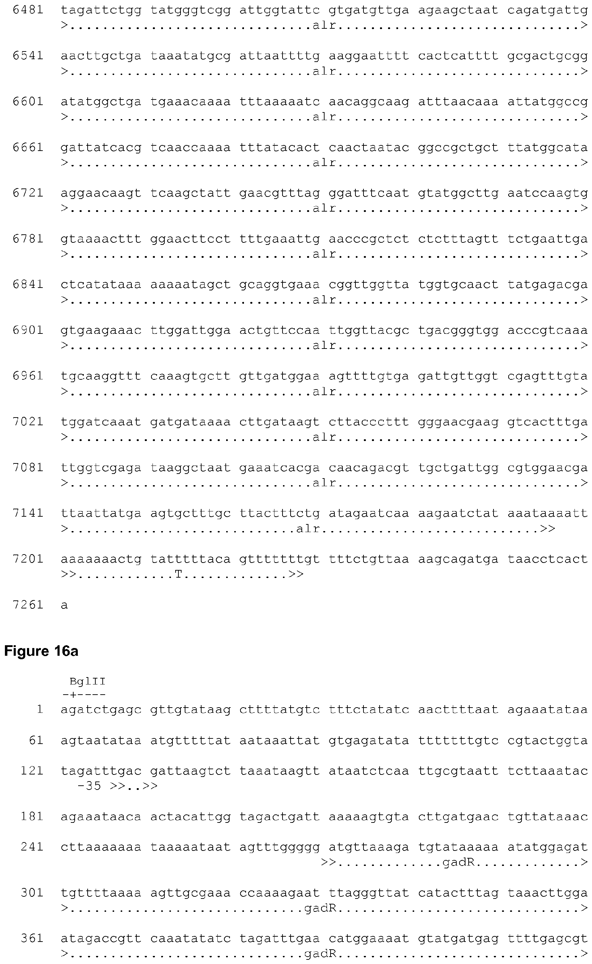

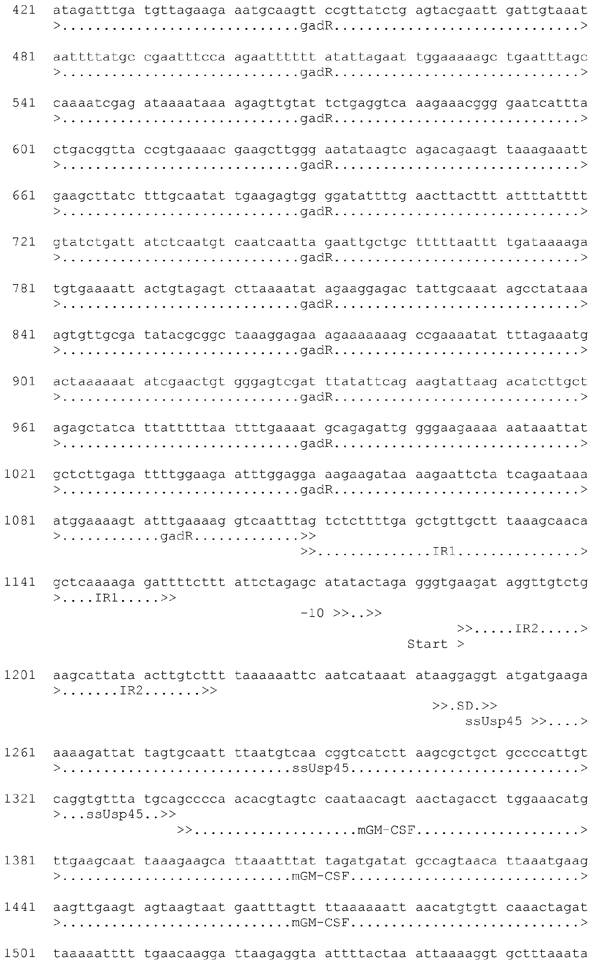

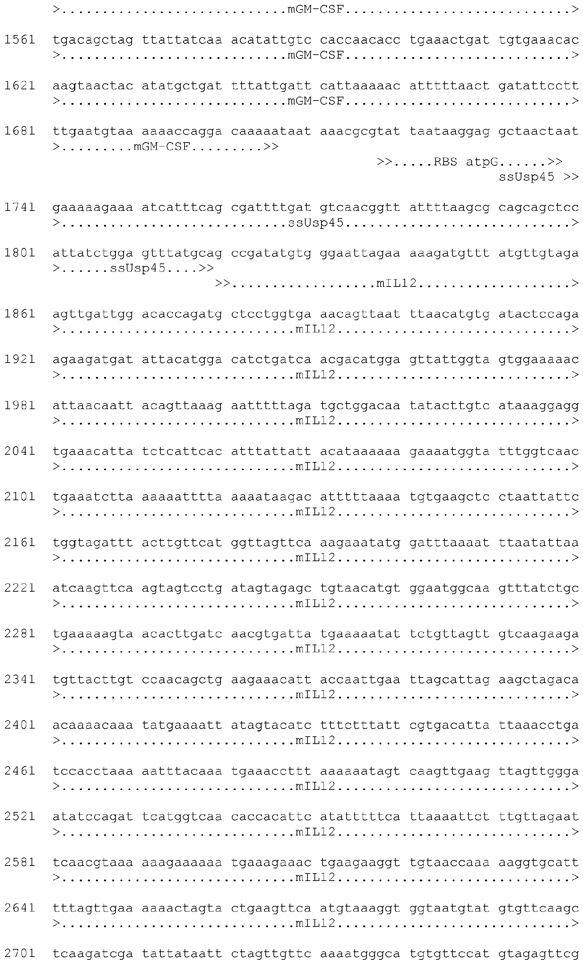

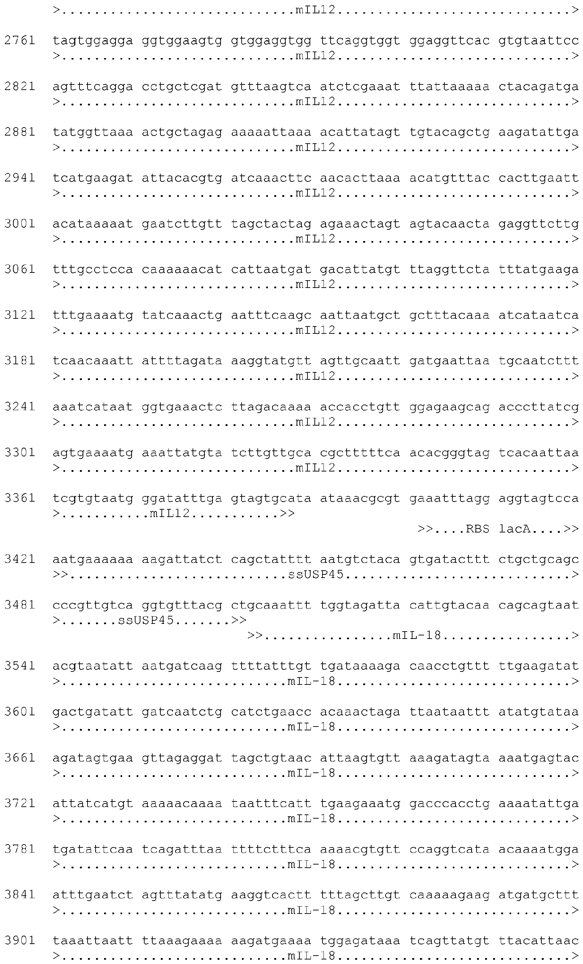

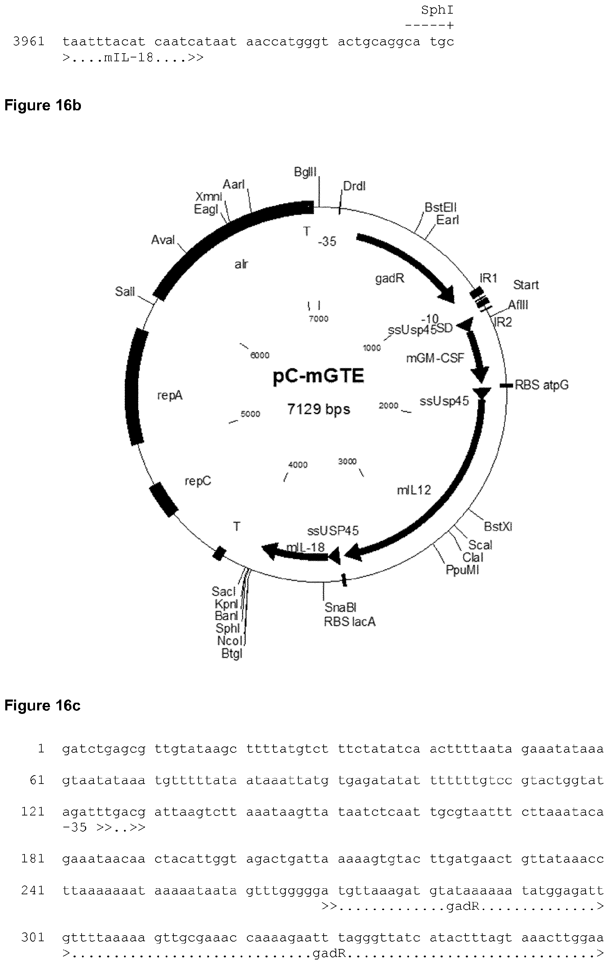

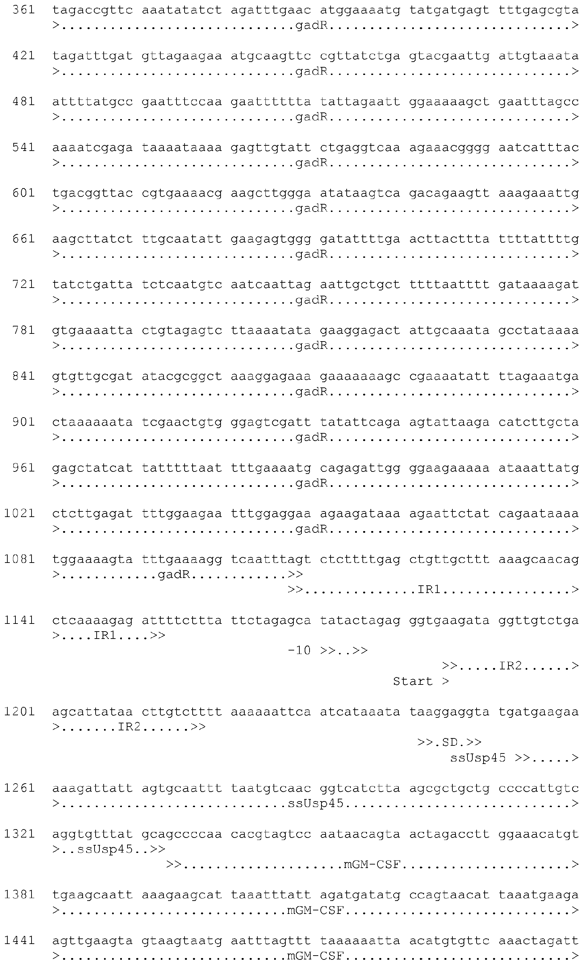

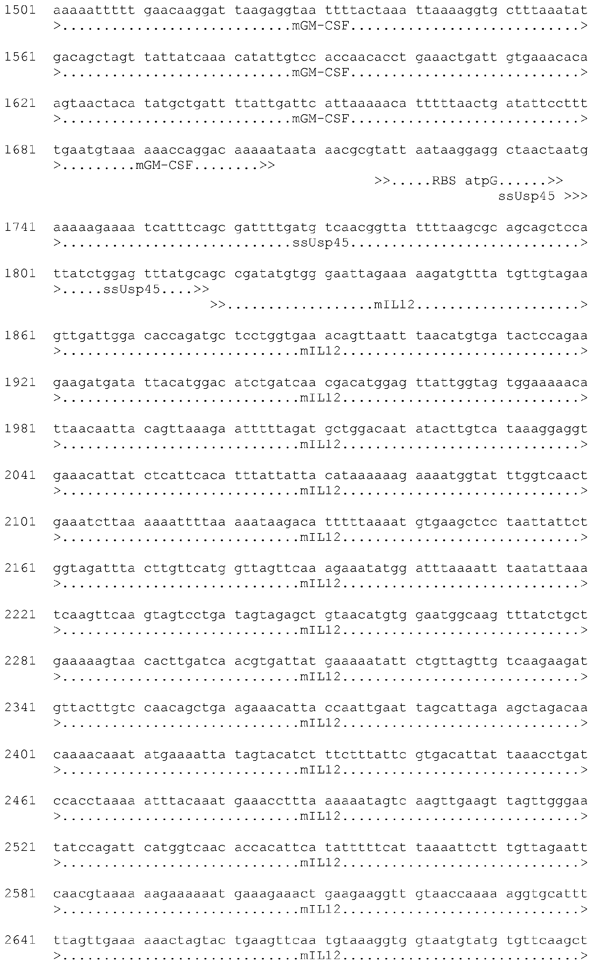

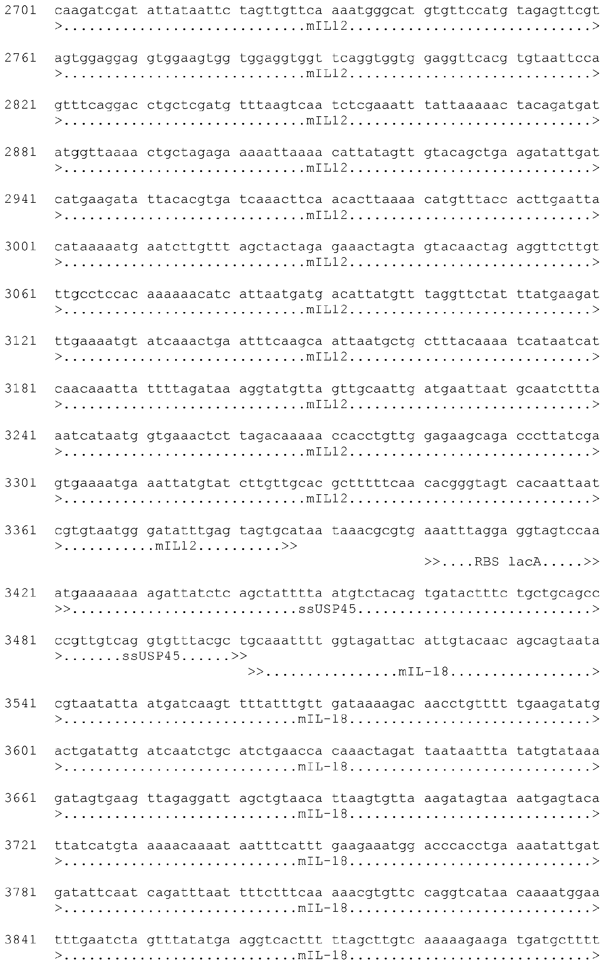

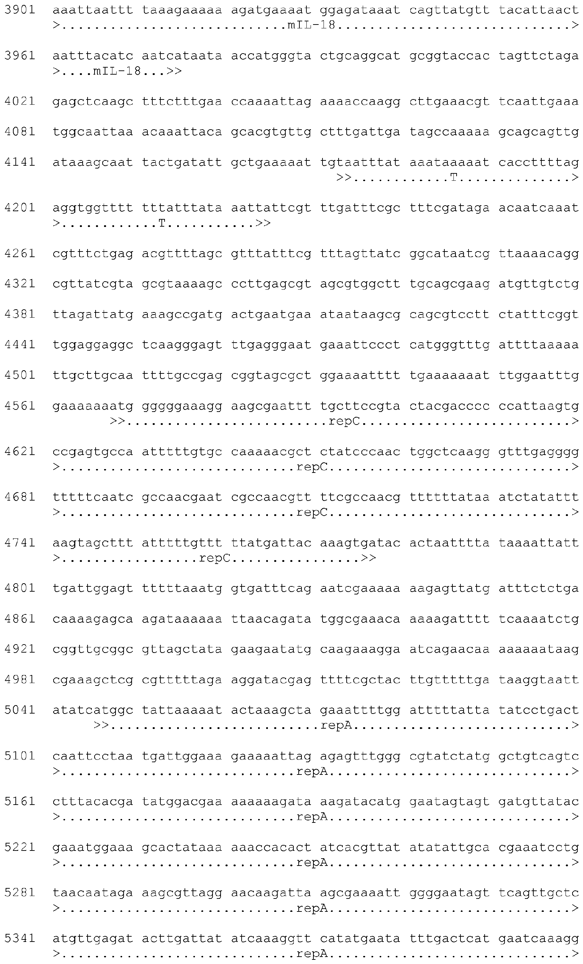

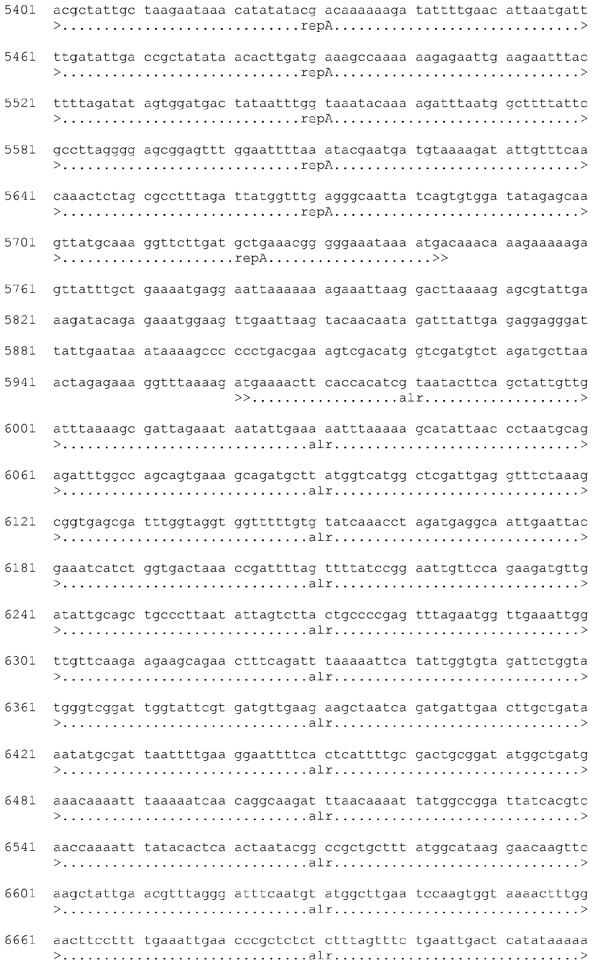

SEQ ID No. 33 corresponds to the nucleic acid sequence of synthetic mGTE construct used in the Examples and which is also shown in Fig. 16a. SEQ ID No. 34 corresponds to the nucleic acid sequence of expression plasmid pC-mGTE used in the Examples and which is also shown in Fig. 16c.

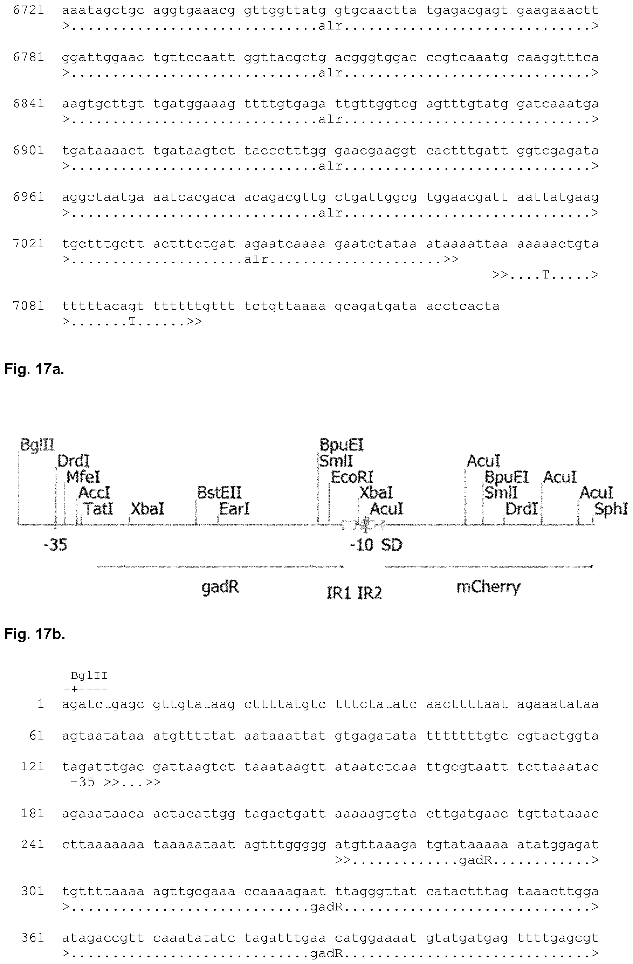

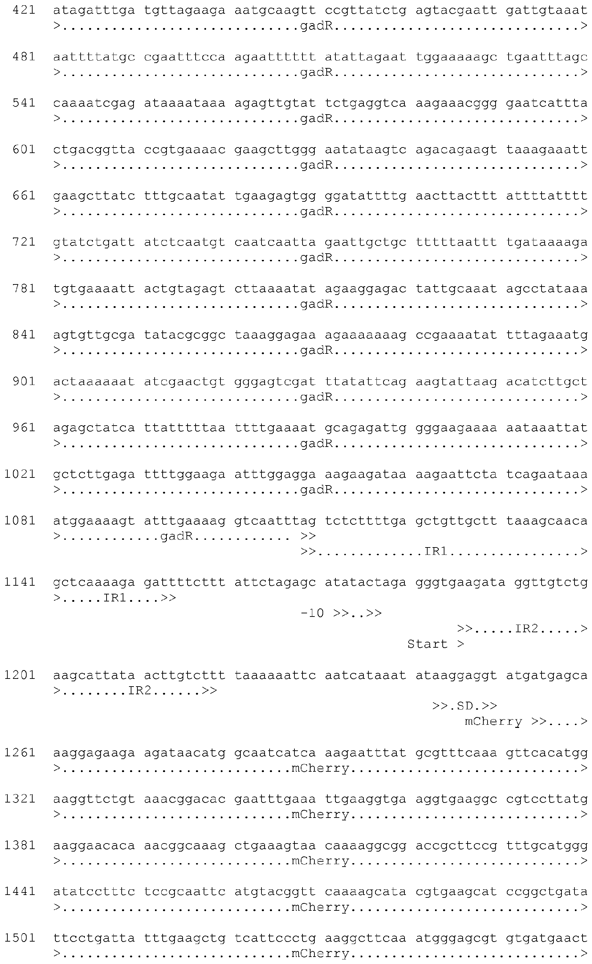

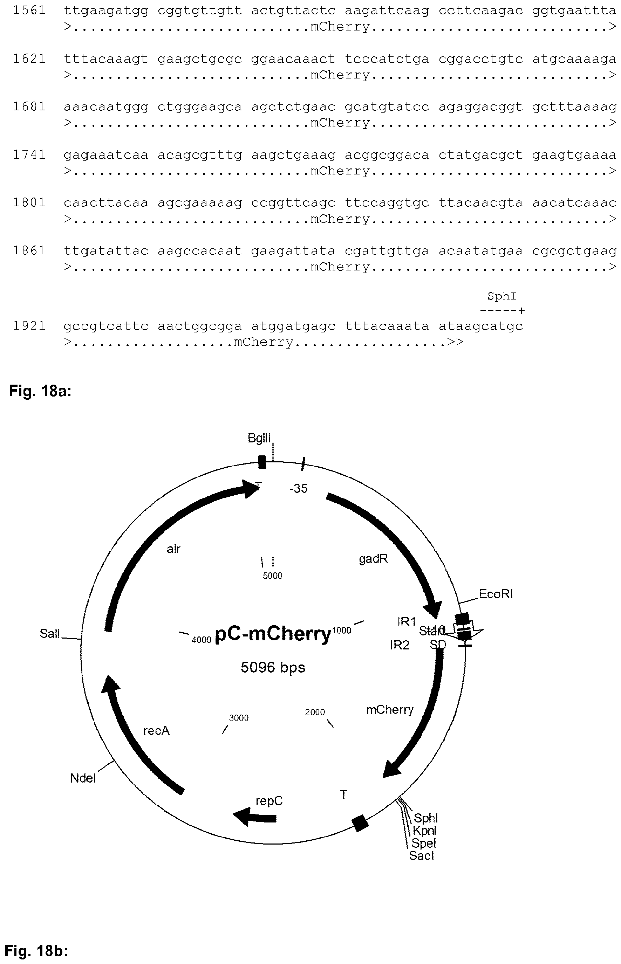

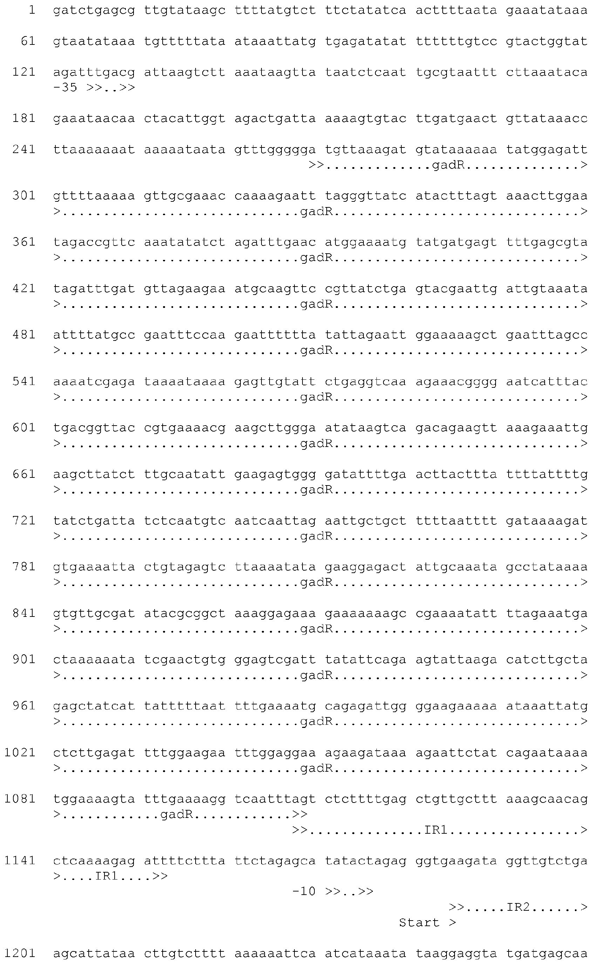

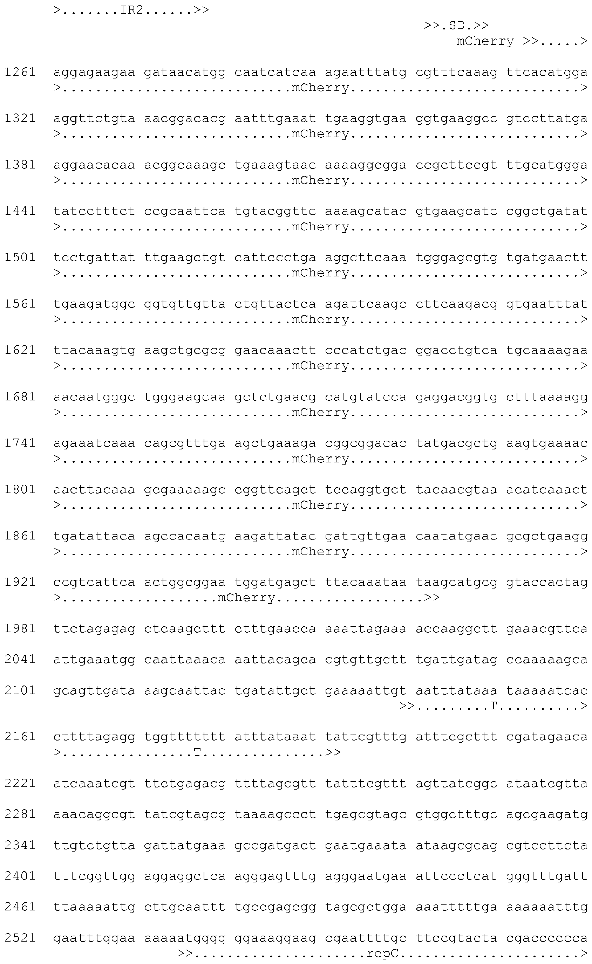

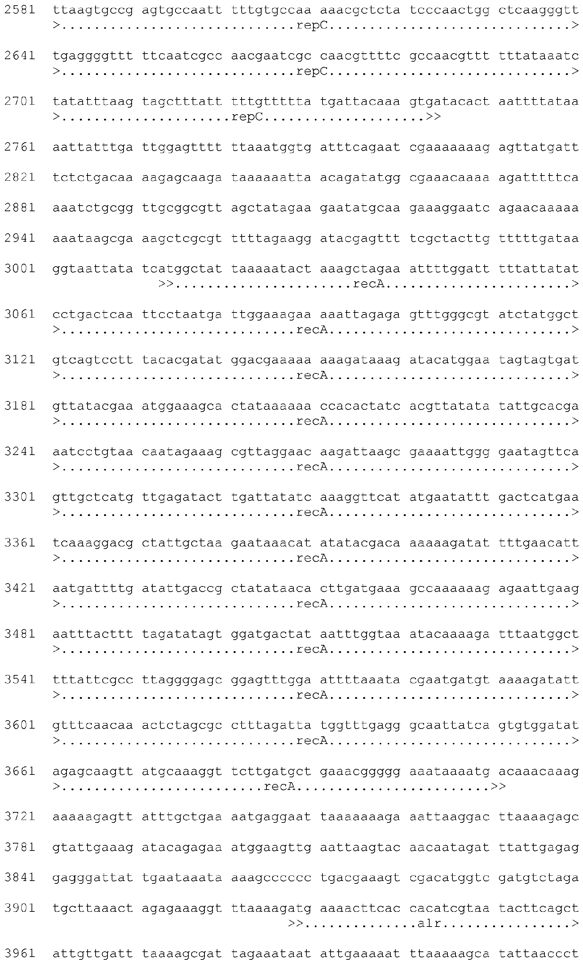

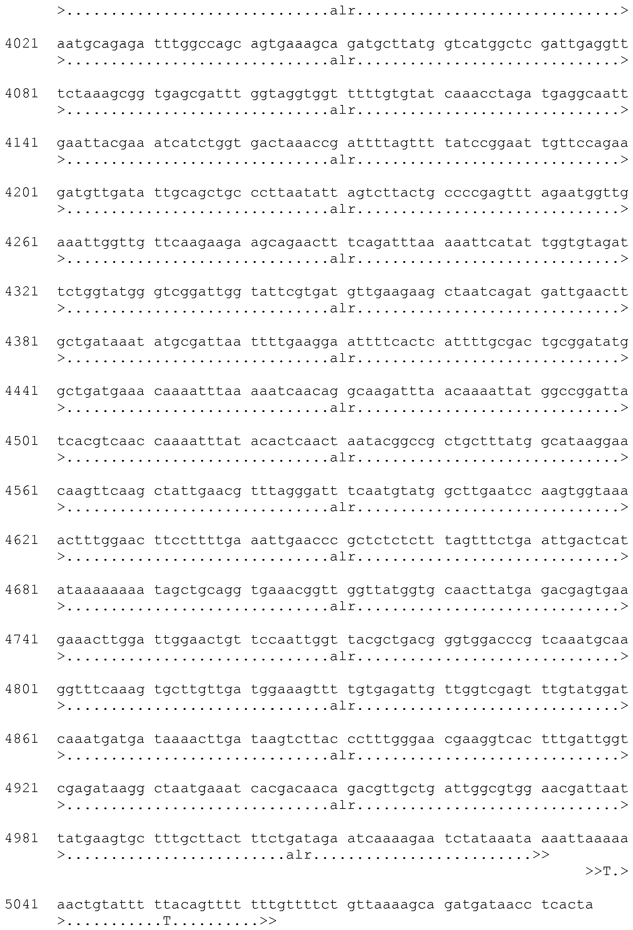

SEQ ID No. 35 corresponds to the nucleic acid sequence of synthetic mCherry construct used in the Examples and which is also shown in Fig. 17b. SEQ ID No. 36 corresponds to the nucleic acid sequence of expression plasmid pC-mCherry used in the Examples and which is also shown in Fig. 18b.

The following Figures and Examples are given for illustrative purpose only. The invention is not to be construed to be limited to the following examples:

**Figures:**

In the Figures the following abbreviations are used: "air" denotes the alanine racemase gene used for growth of the host strain in the absence of d-alanine; "T" denotes a terminator sequence; "-35" and "-10" denote the the -35 element and the -10 element, respectively, of a promoter; "IR" denotes an inverted repeat; "repC" and "repA" are prokaryotic genes necessary for replication of the plasmid in a bacterial cell, "gadR" denotes the gadR gene, "RBS" denotes a ribosome binding site of the indicated gene, for example of the ATP synthase subunit gamma (atpG) gene and/or the galactoside O-acetyltransferase (lacA) gene; "ssUsp45" denotes the Usp45 secretion signal;

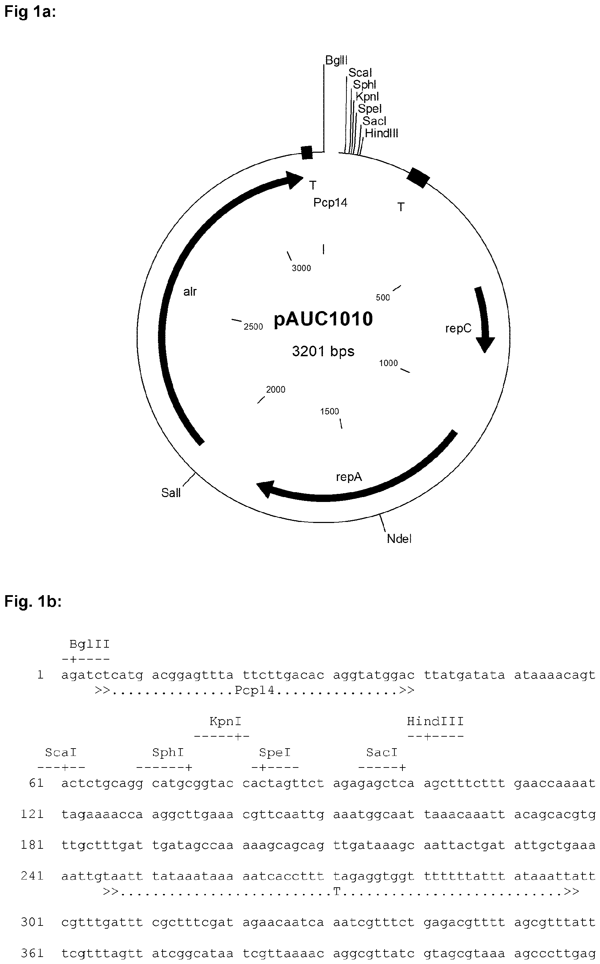

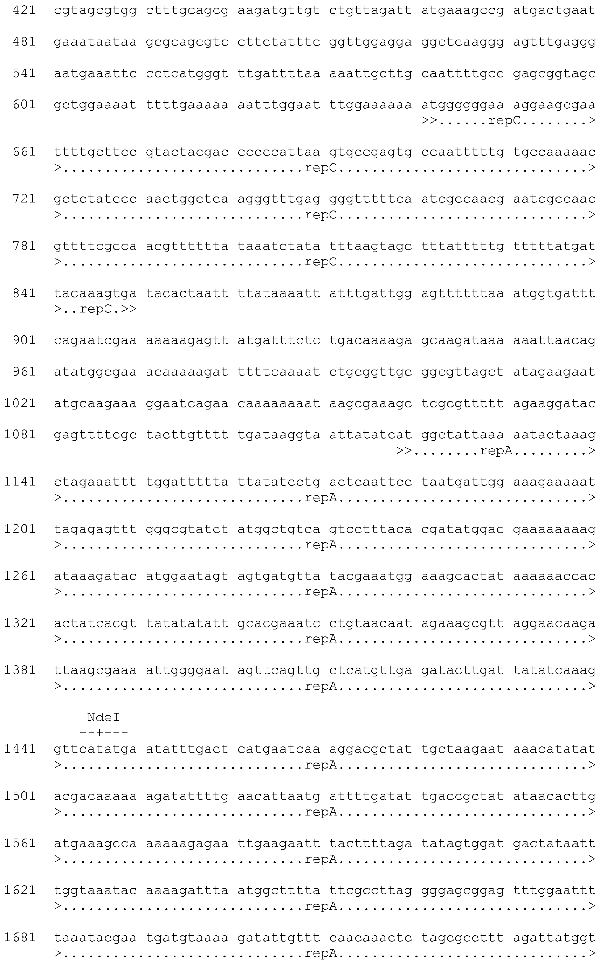

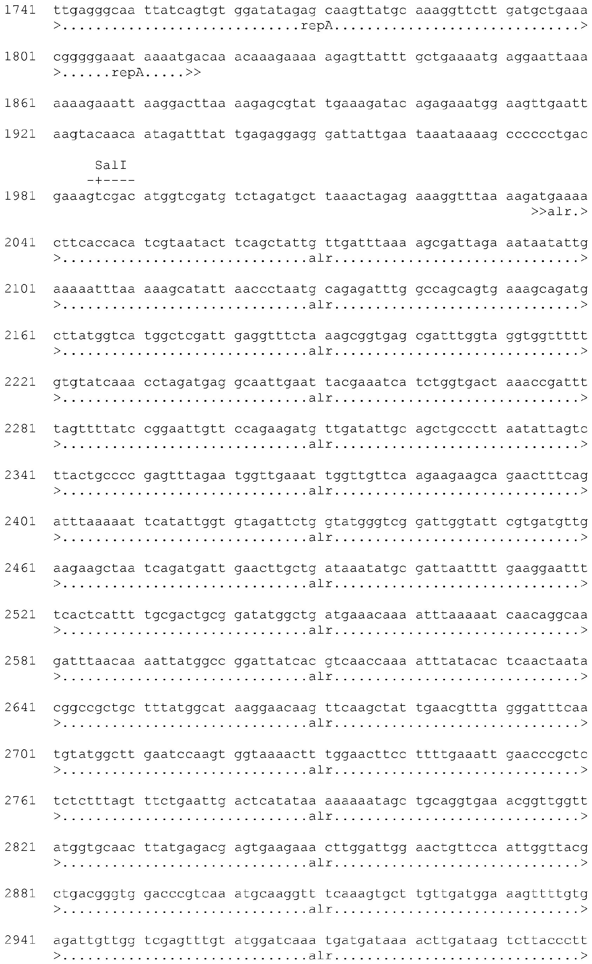

Fig. 1a shows a plasmid map of expression plamid pAUC1010. The corresponding nucleic acid sequence is shown in Fig. 1b.

Fig. 2a shows a schematic representation of the chloride-inducible promoter PgadC including regulatory elements. The corresponding nucleic acid sequence is shown in Fig. 2b, which includes the PgadC promoter region including regulatory elements, the gadR gene and the ribosome binding site (RBS) and start codon of the gadC gene.

Fig. 3 shows the amino acid sequence of the secretion signal of the Lactococcus protein Usp45.

Fig. 4 shows the nucleic acid sequence of the 3’ end of 16S rRNA of L. lactis.

Fig. 5a shows the amino acid sequence of the mature form of human FGF-2-155. The amino acid sequence of the variant of hFGF2-153 used in the Examples is depicted in Figure 5b. The amino acid sequence of the recombinant hFGF-2-153 precursor protein used in the Examples is shown in Figure 5c.

Fig. 6a shows the amino acid sequence of the mature form of human IL-4. The amino acid sequence of the variant of hIL-4 used in the Examples is depicted in Figure 6b. The amino acid sequence of the recombinant hIL-4 precursor protein used in the Examples is shown in Figure 6c.

Fig. 7a shows the amino acid sequence of the mature form of human CSF-1. The amino acid sequence of the variant of hCSF-1 used in the Examples is depicted in Figure 7b. The amino acid sequence of the recombinant hCSF-1 precursor protein used in the Examples is shown in Figure 7c.

Fig. 8a shows a schematic representation of the de novo synthesized CFI construct used in the Examples. The corresponding nucleic acid sequence of the de novo synthesized CFI construct is shown in Figure 8b. The nucleic acid sequence of the expression plasmid designated pC-CFI is shown in Figure 9b. A schematic representation of the expression plasmid pC-CFI is depicted in Figure 9a.

Fig. 10a shows the amino acid sequence of the mature form of mouse IL-18. The amino acid sequence of the variant of mIL-18 used in the Examples is depicted in Figure 10b. The amino acid sequence of the recombinant mIL-18 precursor protein used in the Examples is shown in Figure 10c.

Fig. 11a shows the amino acid sequence of the mature form of mouse GM-CSF. The amino acid sequence of the variant of mGM-CSF used in the Examples is depicted in Figure 11b. The amino acid sequence of the recombinant mGM-CSF precursor protein used in the Examples is shown in Figure 11c.

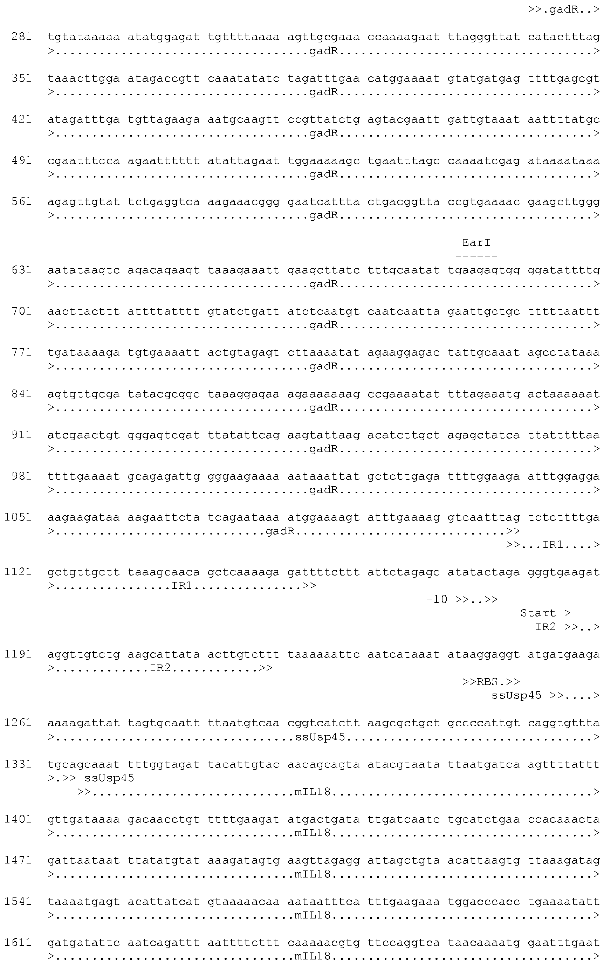

Fig. 12a shows the nucleic acid sequence of the synthetic mEG construct used in the Examples. The nucleic acid sequence of the expression plasmid designated pC-mEG is shown in Figure 12c and a schematic representation of the corresponding expression plasmid pC-mEG used in the Examples is depicted in Figure 12b.

Fig. 13a shows the amino acid sequence of the mature form of mouse IL-12 subunit beta. The amino acid sequence of the mature interleukin-12 subunit alpha isoform 2 is shown in Figure 13b. The amino acid sequence of the mature form of the recombinant interleukin-12 fusion protein used in the Examples is depicted in Fig. 13c. The amino acid sequence of the recombinant mIL-12 precursor protein used in the Examples is shown in Figure 13d.

Fig. 14a shows the amino acid sequence of the mature mouse IFNa2. The amino acid sequence of the variant of mIFNa2 used in the Examples is depicted in Figure 14b. The amino acid sequence of the synthetic mIFNa2 precursor protein used in the Examples is shown in Figure 14c.

Fig. 15a shows the nucleic acid sequence of the synthetic mTEA construct used in the Examples. The nucleic acid sequence of the expression plasmid designated pC-mTEA is shown in Figure 15c. A schematic representation of the expression plasmid pC-mTEA is depicted in Figure 15b.

Fig. 16a shows the nucleic acid sequence of the synthetic mGTE construct used in the Examples. The nucleic acid sequence of the expression plasmid designated pC-mGTE is shown in Figure 16c. A schematic representation of the corresponding expression plasmid pC-mEG used in the Examples is depicted in Figure 16b.

Fig. 17a shows a schematic representation of a synthetic mCherry construct used in the Examples.The corresponding nucleic acid sequence of the synthetic mCherry construct is shown in Figure 17b.

The nucleic acid sequence of the expression plasmid designated pC-mCherry used in the Examples is shown in Figure 18b. A schematic representation of the expression plasmid pC-mCherry is depicted in Figure 18a.

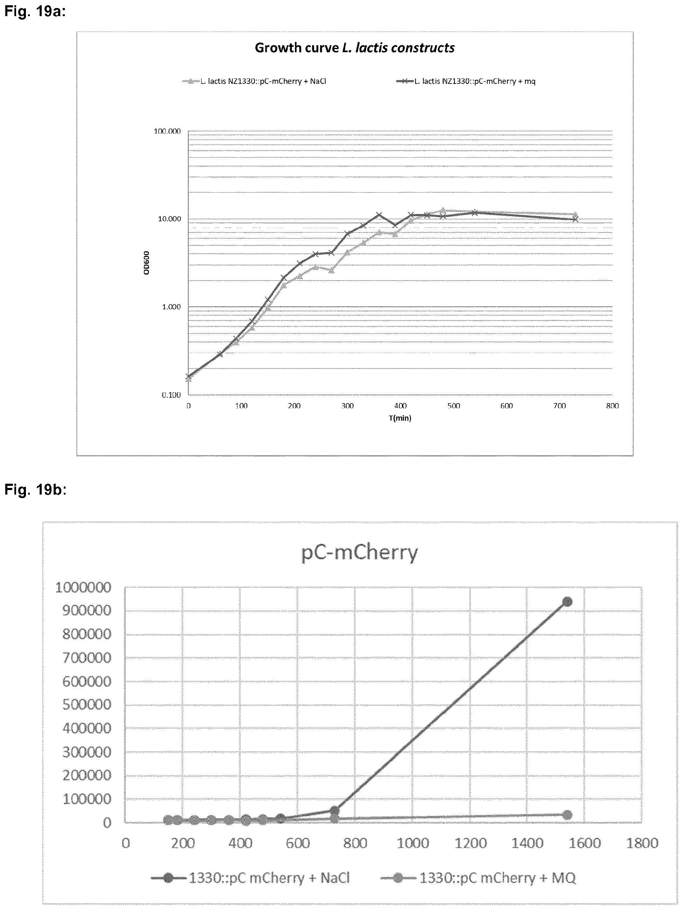

Fig.19a shows a comparison of growth curves of L. lactis NZ1330 (pC-mCherry) after induction of expression of the mCherry gene with 100 mM NaCl ("L. lactis NZ1330::pC-mCherry + NaCl") and without induction ("L. lactis NZ1330::pC-mCherry + mq") each determined in Example 3 by measuring the optical density at a wavelength of 600 nm (OD600) at the indicated time points (T).

Fig. 19b shows a comparison of mCherry fluorescence obtained from L. lactis NZ1330 (pC-mCherry) after induction of expression of the mCherry gene with 100 mM NaCl ("NZ1330::pC-mCherry + NaCl") and without induction ("NZ1330::pC-mCherry + MQ") in artificial units measured under the conditions described in Example 3 at the indicated time points in minutes.

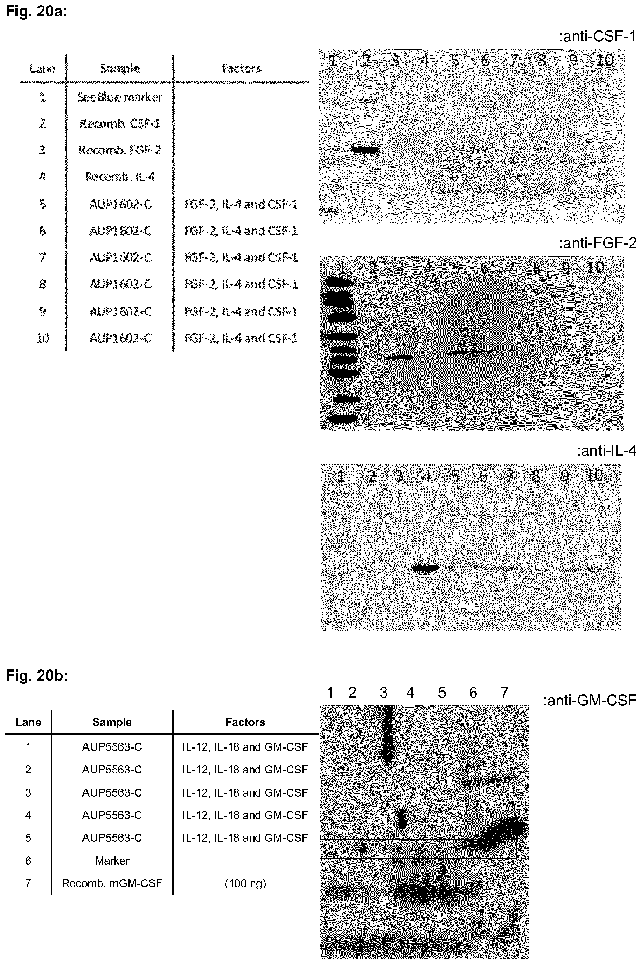

Fig. 20a shows a Western blot analysis of recombinant bacteria designated AUP1602-C expressing human CSF-1, human FGF-2 and human IL-4 from the expression plasmid pC-CFI obtained in Example 1.4. "anti-CSF-1", "anti-FGF-2" and "anti-IL-4" denotes the primary antibody used in Example 3 for detecting expression of human CSF-1, human FGF-2 and human IL-4, respectively.

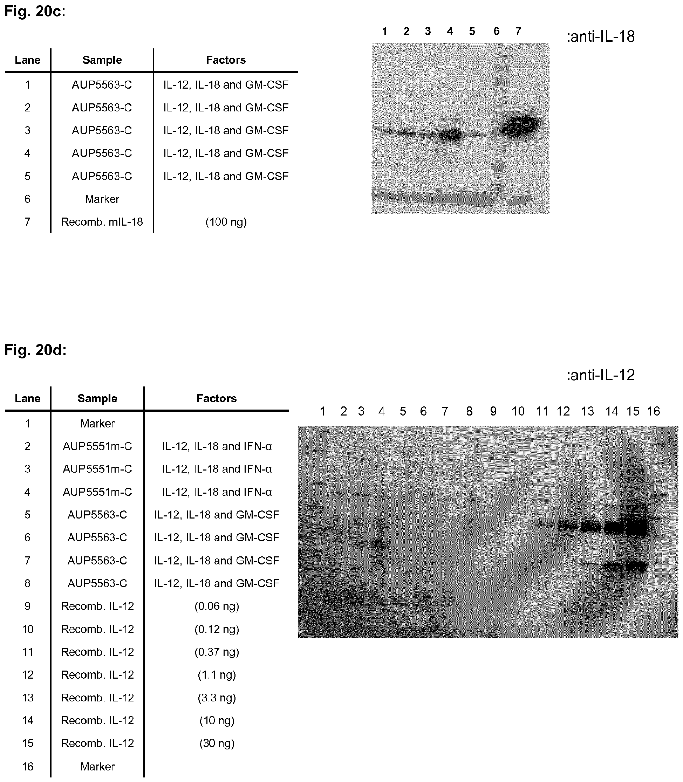

Fig. 20b and Fig. 20c show a Western blot analysis of recombinant bacteria designated AUP5563-C expressing mouse IL-12, mouse IL-18 and mouse GM-CSF from the expression plasmid pC-mGTE obtained in Example 1.7. "anti-GM-CSF" and "anti-IL-18" denotes the primary antibody used in Example 3 for detecting expression of mouse GM-CSF and mouse IL-18, respectively.

Fig. 20d shows a Western blot analysis of recombinant bacteria designated AUP555m-C, which express mouse IL-12, mouse IL-18 and mouse IFNa from the expression plasmid pC-mTEA obtained in Example 1.6, and AUP5563-C, which express mouse IL-12, mouse IL-18 and mouse GM-CSF from the expression plasmid pC-mGTE obtained in Example 1.7. "anti-IL-12" denotes the primary antibody used in Example 3 for detecting expression of mouse IL-12.

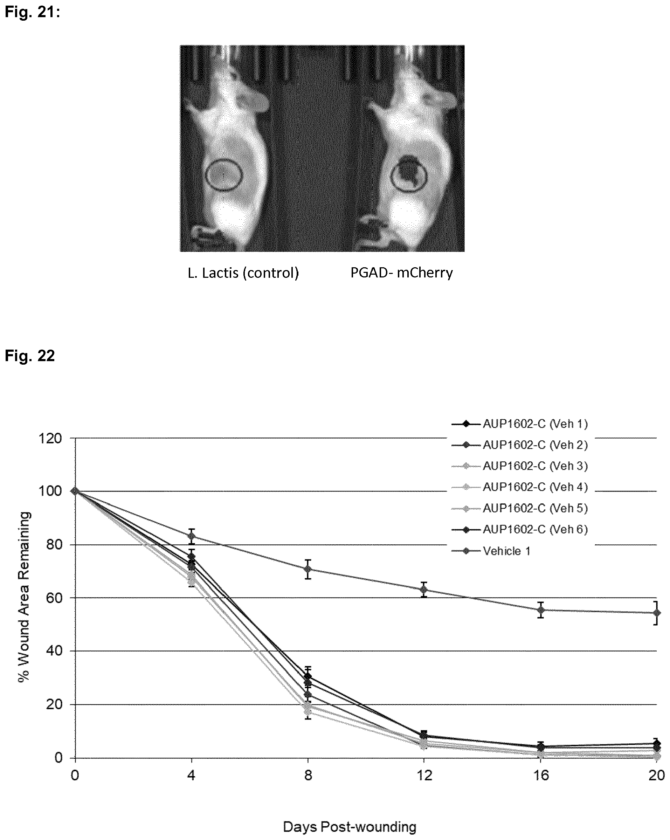

Fig. 21 shows the fluorescent imaging of tumor-induced BALB/c mice 48 hours after i.t. injection of recombinant *L. lactis* containing the AUC1000 (pC-mCherry) construct ("PGAD-mCherry") as well as control bacteria without expression plasmid ("L. lactis (control)") under the conditions described in Example 4. Blue circles indicate the position of the tumor

Fig. 22 shows the mean percentage wound area for all treatment groups of the wound closure experiments described in Example 5.

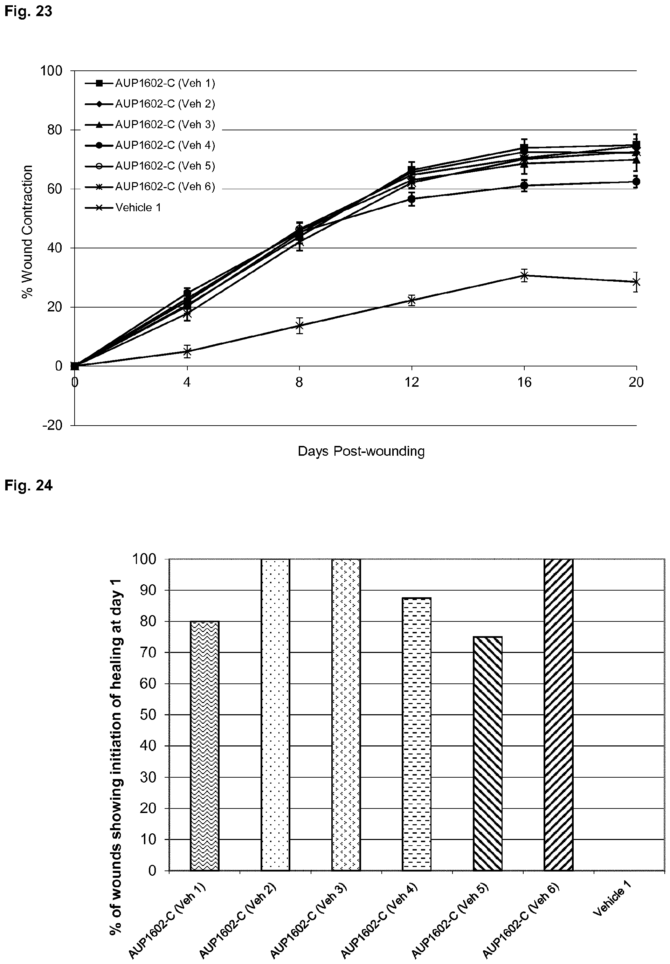

Fig. 23 shows mean percentage wound contraction for all treatment groups of the wound closure experiments described in Example 5.

Fig. 24 shows the % of wounds responding, for each treatment group, on day 1 of the wound closure experiments described in Example 5.

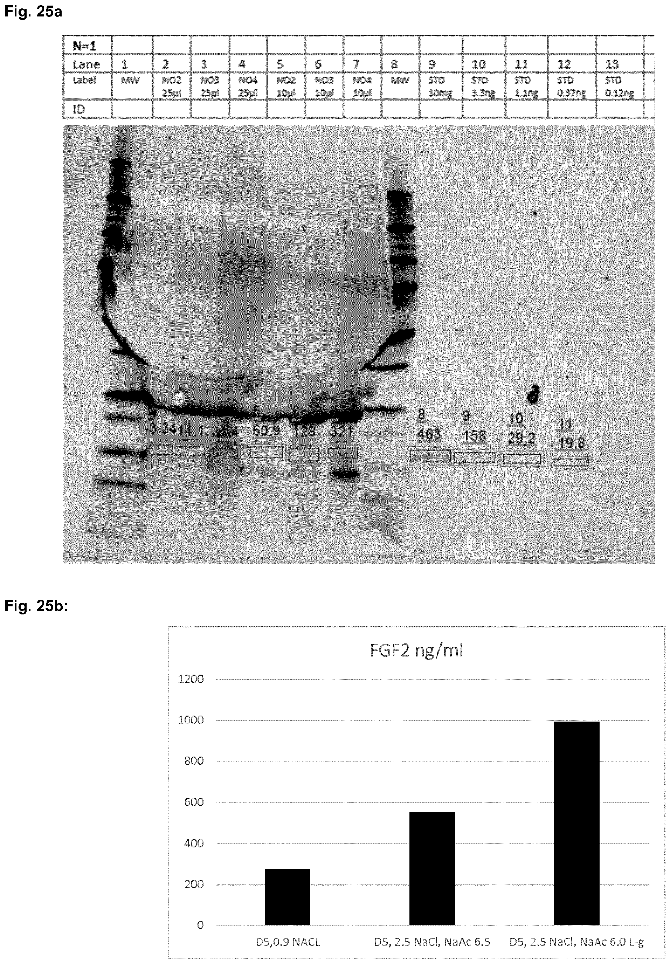

Fig. 25a to Fig. 27b show the results described in Example 6 of the detection of human FGF-2, human IL-4 and human CSF-1 in wound fluids of mice treated in the wound closure experiments described in Example 5.

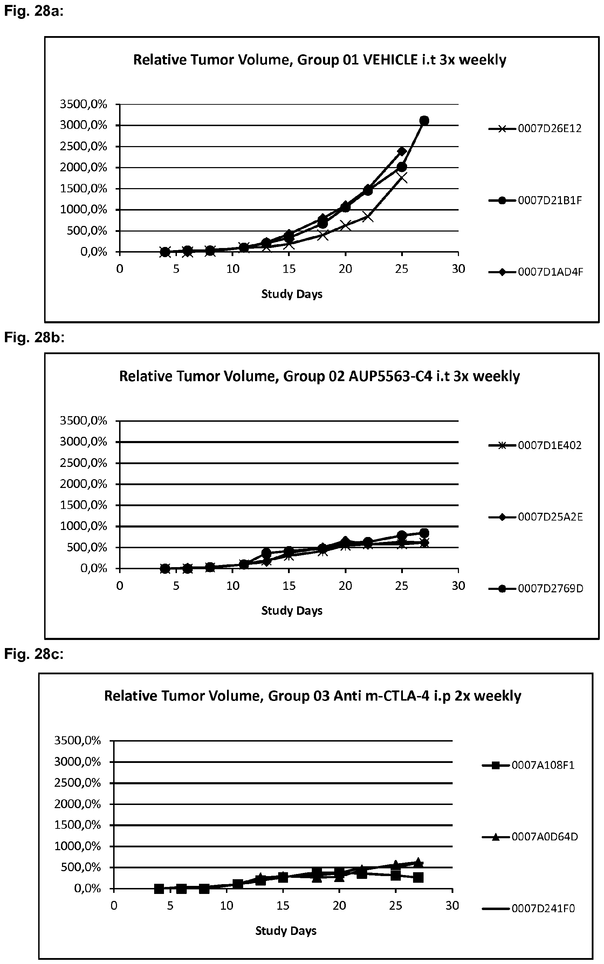

Fig. 28a to Fig. 28c show the results described in Example 7 of the relative tumor volume of vehicle-treated controll mice, mice treated with AUP5563-C4 and anti-m-CTLA-4-treated contoll mice at the indicated time points.

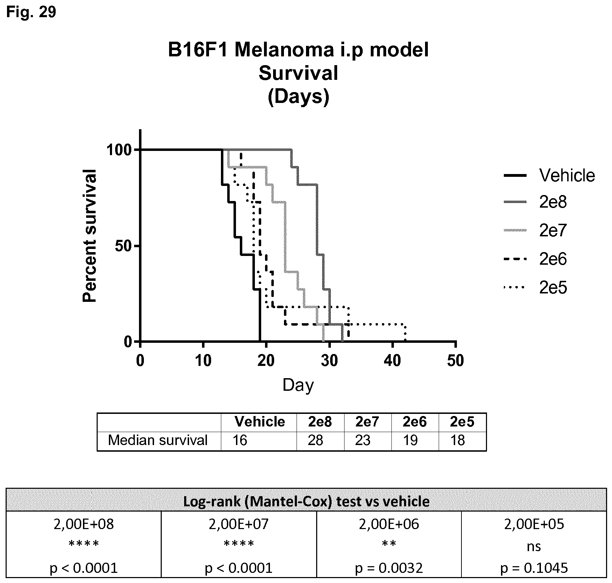

Fig. 29 shows the survival curve of C57BL76 mice treated in Example 8 with a combination of drug products AUP2059 (mIL18/mGM-CSF) and AUP5551-C (mIL12/mIL18/mIFNa2b) each obtained in Example 3 and vehicle-treated control mice.

## Examples:

### I) General experimental procedures:

Unless otherwise stated, Examples were carried out following the protocols of the manufacturer of the analytical systems. Unless otherwise stated, indicated chemicals were commercially obtained from Sigma-Aldrich Chemie Gmbh (Munich, DE), Merck KGaA (Darmstadt, DE), Thermo Fisher Scientific Inc. (Waltham, MA, US) or Becton, Dickinson and Company (Franklin Lakes, NJ, US).

### I.1 Growth media

For different purposes cells were grown in different media.
For general cloning procedures M17 medium (Oxoid Deutschland GmbH, Wesel, DE) was used containing 1 wt.-% glucose or lactose, respectively.
E. coli strains were grown in TY medium. The recipe for the TY medium used is as follows:

| | |
|---|---|
| 1 wt.-% | Trypton |
| 0.5 wt.-% | Yeast extract |
| 0.5 wt.-% | NaCl |

For fermentations and other functional growth experiments the IM1 medium was used, which is free from animal derived ingredients. Lactose, the only remaining animal derived ingredient, can be obtained in pharmaceutical quality.

The recipe for the IM1 medium used is as follows:

| | |
|---|---|
| 1 wt.-% | Lactose |
| 2 wt.-% | Na-β-glycerophosphate |

| | |
|---|---|
| 1.5 wt.-% | Soy peptone |
| 1 wt.-% | Yeast extract |
| 1 mM | $MgSO_4 \times 7\ H_2O$ |
| 0.1 mM | $MnSO_4 \times 4\ H_2O$ |
| pH 6.7 | |
| Sterilisation: | 110 °C 15 min |

During fermentation no buffer (e.g. Na-β-glycerophosphate) is added since the pH was automatically regulated using 2.5 M NaOH. This buffer neutralizes the produced lactate and allows the culture to reach a cell density of $OD_{600}$ = 10 - 15.

For the growth of the strains *L. lactis* NZ1330 and NZ9130 D-alanine was added to the media to a final concentration of 200 μg/mL.

**I.2 Bacterial strains**

The following commercially bacterial strains were used. *Lactococcus lactis* strains NZ3900, and NZ1330 were obtained from MoBiTec GmbH (Goettingen, DE).

*L. lactis* strains NZ1330 contains a deleted alanine racemase (*alr*) encoding gene (Δ*alr*). Deletion of the *alr* gene resulted in auxotrophy for the essential component D-alanine. The respective strain is unable to grow on media without D-alanine unless the *alr* gene is provided on a plasmid.

**I.3 Molecular cloning techniques**

For molecular cloning standard techniques were used as, for example, described in Green and Sambrook (2012): "Molecular cloning: a laboratory manual", fourth edition, Cold Spring Harbour Laboratory Press (Cold Spring Harbor, NY, US).

Different synthetic DNA constructs were produced by BaseClear (Leiden, NL). The constructs were obtained as purified plasmids as well as clones in *E. coli.*

Plasmid DNAs were isolated using standard DNA isolation according to Birnboim, H.C.and Doly, J. (1979). Synthetic gene constructs were excised from their plasmids using selected restriction enzymes, purified and ligated into the respective target plasmid. Ligation was performed using Anza T4 ligase master mix (ThermoFischer Scientific Inc.), according to the protocols supplied.

The different ligation mixtures were transformed by electroporation to the selected *L. lactis* strains (*L. lactis* NZ1330 or *L. lactis* NZ 9130) and plated on appropriate media.

**I.4 Preparation of electro-competent cells of strains and electroporation**

Electro-competent cells were prepared according to the following protocol. *L. lactis* NZ1330 and NZ9130 were grown overnight at 30 °C in 10 mL SMGG-medium, that was used to inoculate 100 mL SMGG-medium.

Cells were grown untill an $OD_{600}$ of ~0.5 was reached. Cells were washed three times with ice-cold washing buffer and resuspended in 1 mL washing buffer. Aliquots of 40 μL were stocked at - 80 °C. Electro-competent properties of the cells were tested using the standard electroporation procedure.

To that goal 40 μL cells were thawed on ice and mixed with 0.5 μL plasmid DNA in an ice-cold electroporation cuvette and 2500 volt, 25 μF, 200 Ohm was applied using an Eporator® - electroporator (Eppendorf AG, Hamburg, DE).
Cells were resuspended in 4 mL SMG17MC-medium and incubated at 30 °C for 2 hours. Different amounts of the culture were plated on GSM17-agar and incubated at 30 °C.

Buffers used:

SMGG-medium: commercially available M17 medium,

| | |
|---|---|
| + 0.5 M | sucrose |
| + 0.5 wt.-% | glucose |
| + 1 wt.-% | glycine |

SMG17MC-medium: commercially available M17 medium

| | |
|---|---|
| + 0.5 M | sucrose |
| + 0.5 wt.-% | glucose |
| + 20 mM | $MgCl_2$ |
| + 2 mM | $CaCl_2$ |

GSM17 agar: M17-agar obtained from Oxoid Deutschland GmbH (Wesel, DE)

| | |
|---|---|
| + 0.5 M | sucrose |
| + 0.5 wt.-% | glucose |

| | | |
|---|---|---|
| Washing buffer: | 0.5 M sucrose | |
| | + 10 wt.-% | glycerol |

### I.5 Selection of transformants

Transformants were transferred from plates to new plates and to tubes with 3 ml medium M17 (supplemented with 1 % glucose or lactose), incubated at 30 °C and DNA was isolated as outlined above under item I.3. Clones were screened for the presence and orientation of inserts using selected restriction endonucleases and agarose gel electrophoresis.
Positive clones were selected, cultivated and stocked at -80 °C.

### I.6 Cultivation and induction of gene expression

Selected clones were inoculated in IM1 medium supplemented with 0.5 % glucose or lactose and grown overnight at 30 °C. At t = 0 cultures were 1:100 inoculated in 45 mL medium and incubated. At $OD_{600}$ = 0.5 the cultures were split in three separate 15 ml cultures that were induced by the addition of NaCl (0 mM, 100 mM or 500 mM).
After 3 hours of gene induction, cells and supernatants were separated by centrifugation (10 min. at 6,000 rpm).
Cells were frozen at -20 °C prior to further processing. Supernatants were mixed with one third volume of TCA (see below) and stored at -20 °C.

### I.7 Preparation of cell free extracts and trichloroacetic acid (TCA) precipitation

Cell free extracts were prepared by bead beating according to a standard operation protocol. The procedure is based on using 50-100 μm glass beads. Samples were kept on ice as much as possible to prevent proteolytic breakdown of proteins. After the final centrifugation step, cell free extracts were collected and stored at -20 °C.
TCA precipitation was performed by the addition of 1 volume TCA to 4 volumes of culture supernatant. The mixture was incubated at -20 °C till further use. To obtain TCA precipitated protein 1-mL samples were taken and centrifuged for 10 min. at 14,000 rpm. The supernatant was decanted, and the pellets were dried in a stove at 65-70 °C. Subsequently, SDS-PAGE sample buffer containing 2 % β-mercaptoethanol was added and the samples were denatured by incubation at 100 °C for 10 min.

**I.8 Sodium dodecyl sulphate polyacrylamide gel electrophoresis (SDS-PAGE) and Western blotting**

SDS-PAGE (15 wt.-% running gel) was performed essentially according to Laemmli, U.K. (1970). After electrophoresis gels were stained using Coomassie R-250 (Bio-Rad Laboratories, Inc., Hercules, CA, US), or the proteins were transferred to Polyvinylidene fluoride (PVDF) or nitrocellulose membranes by means of semi-dry blotting system (Owl®, Thermo Fisher Scientific, Inc.), according to the manufacturer's instructions.

PVDF or nitrocellulose membranes were processed by blocking with Tris-buffered saline with 0.05 wt.-% Tween 20 (TBST) including 2 wt.-% bovine serum albumin (BSA), followed by incubation with specific primary antibodies raised against the respective protein diluted in TBST including 1 wt-% BSA for 30 minutes.

Subsequently, blots were washed 3 times 10 minutes with TBST. After washing, a secondary, alkaline phosphatase conjugated anti-rabbit antibody derived from goat, such as from Santa Cruz Biotechnology, Inc. (Dallas, TX, US, catalogue nr. sc-2004)was applied (1:7500 in TBST + 1 wt-% BSA). Unbound antibody was washed away with TBST (3 $\times$ 10 min.), followed by enzymatic staining according to standard procedures using NBT and BCIP.

After sufficient colour development the reaction was terminated by rinsing with water, membranes were dried and photographed or scanned.

Alternatively, a donkey anti-goat antibody IRDye® 680RD from LI-COR, Inc. (Lincoln, NE, US), which is conjugated with a near-infrared (NIR) fluorescent dye, was used according to the manufacturer's protocol. Detection of bound secondary antibody was perfomend using an Odyssey® imaging system from LI-COR, Inc. according to the manufacturer's protocol. Tris-buffered saline with 0.05 wt.-% Tween 20 was prepared using Roti®fair TBST 7.6 tablets obtained from Carl Roth GmbH + Co. KG (Karlsruhe, DE) according to the manufacturer's instructions.

For detection of the respective recombinant proteins the following primary antibodies from Abeam PLC (Cambridge, UK), Bio-Rad Laboratories Inc. (Hercules, CA, US), and LifeSpan BioSciences, Inc. (Seattle, WA, US), were used,

- anti-FGF-2 antibody - rabbit polyclonal antibody to FGF-2 (Abeam PLC, ab126861),
- anti-IL-4 antibody - rabbit polyclonal antibody to IL-4 (Abeam PLC, ab9622),
- anti-M-CSF antibody - rabbit polyclonal antibody to M-CSF (Abeam PLC, ab9693),
- anti-mouse GM-CSF antibody - rabbit polyclonal antibody to mouse GM-CSF (Bio-Rad Laboratories Inc., AAM16G),
- anti-mouse IL-18 antibody - rabbit polyclonal antibody to mouse IL-18 (LifeSpan BioSciences, Inc., LS-C147100),
- anti-mouse IL-12 antibody - goat anti mouse antibody to IL-12,(Biorad Laboratories Inc., AAM33).

**I.9 Fermentations**

Fermentations were carried out in a 0.5-L Multifors 6-fermenter array (Infors Benelux, Velp, NL). Inoculation was carried out with 1% pre-culture in IM-1 medium; stirrer speed was 200 rpm and the incubation temperature was 30 °C. During the runs pH was controlled using 2.5 M NaOH. pH, temperature and the addition of NaOH were continuously monitored.

**I.10 Fluorescent protein measurements**

Strains that contained constructs with the mCherry reporter gene under control of the different promoters were grown and processed as described above. mCherry activity was measured in a Synergy HT (BioTek Instruments, Inc., Winooski, VT, US) fluorispectrophotometer.

Excitation was done at 530 nm and emission was measured at 590 nm and a gain of 120 was applied as described by Tauer et al. (2014).

**Example 1 Generation of expression plasmids**

**1.1 Generation of plasmid pAUC1010**

Plasmid pAUC1010 was used in the following experiments for the expression of heterologous genes in *L. lactis.* Plasmid pAUC1010 is based on the native rolling circle plasmid of Lactococcus lactis pSH71, as described in de Vos, W.M. (1987) as well as de Vos, W.M. and Simons, G. (1994).

Plasmid pAUC1010 contains the alanine racemase (*alr*) gene including its terminator of L. *lactis* as a selection marker that can be used with *L. lactis alr* knock-out mutant strains such as NZ1330 (Bron, P.A. et al. (2002), Hols, P. et al. (1999)). The plasmid also includes the artificial promoter Pcp14 (Ruhdal Jensen, P. and Hammer, K. (1998)).

Furthermore, plasmid pAUC1010 contains a terminator sequence of the aminopeptidase N (*pepN*) gene of *L.*

*lactis* (Tan, P.S.T. et al. (1992)) downstream of the promoter and a multiple cloning site (MCS). The terminator sequence allows for a termination of transcription.

The nucleic acid sequence of *Lactococcus lactis* subsp. *cremoris alr gene* for alanine racemase (EC 5.1.1.1) is available, for example, under the NCBI accession number Y18148.2.

The respective amino acid sequence of alanine racemase of *Lactococcus lactis* subsp. *cremoris* can, for example, be found under UniProt accession number Q9RLU5 - version 99 or under NCBI Reference Sequence number WP_011835506.1.

The nucleic acid sequence of pAUC1010 is provided in SEQ ID No.1 and Figure 1b.

Figure 1a shows a schematic overview of plasmid pAUC1010. "air" denotes the alanine racemase gene used for growth of the host strain in the absence of d-alanine; "T" denotes a terminator sequence; "repC" and "repA" are prokaryotic genes necessary for replication of the plasmid in a bacterial cell.

**1.2 Generation of a chloride-inducible expression cassette**

In the following experiments a chloride-inducible gene expression cassette was generated in which the *PgadC* promoter from *L. lactis* subsp. *cremoris* was used to control expression of different heterologous genes in *L. lactis.*

The PgadC promoter was used as described in Sanders et al. (1997) and Sanders et al. (1998) in combination with the activator gene gadR, and the ribosome binding site and start codon of the *gadC* gene, which are both arranged downstream of the *PgadC* promoter.

Promoter PgadC is regulated by the gadR protein, which is a positive regulator of Pgad. The gadR protein activates the PgadC promoter in the presence of at chloride ions.

Expression of the gadR gene itself is not activated by chloride ions and instead is controlled by a constitutive promoter, which was also provided in the chloride-inducible gene expression cassette used in the present invention together with a ribosome binding site (RBS) located upstream of the ATG start codon of the gadR encoding nucleic acid sequence.

The nucleic acid sequence of the PgadC promoter including the gadR gene and the ribosome binding site and start codon of the gadC gene was extracted from the genome sequence of strain L. lactis subsp. cremoris MG1363. The respective genome sequence is available, for example, under the NCBI accession number NC_009004.1.

The nucleic acid sequence of the chloride-inducible promoter, which includes the PgadC promoter region, the gadR gene and the ribosome binding site and start codon of the gadC gene is provided in SEQ ID No.2 and Figure 2b. The ATG-start codon of gadC is denoted in Figure 2b by underlining and in bold capital letters.

A schematic representation of the chloride-inducible promoter including regulatory elements is shown in Fig.2a.

The respective target gene or genes encoding for the at least one protein to be expressed by the recombinant bacteria of the present invention was/were attached at the ATG start codon of gadC. Furthermore, the genes encoding for the respective protein to be expressed by the recombinant bacteria of the present invention was provided with a secretion signal for protein secretion derived from the expression host.

The secretion signal was preferably derived from the Lactococcus protein Usp45, which is described, for example, in van Asseldonk et al. (1993) and van Asseldonk et al. (1990). The amino acid sequence of the secretion signal of the Lactococcus protein Usp45 is shown in Figure 3, and in SEQ ID No. 3.

In the cell a mechanism exists that ensures that after the secretion of a protein this signal sequence is cleaved off from the target protein. This reaction is catalysed by the signal peptidase which has a specific preference for certain amino acids in the positions around the cleavage site.

The at least one protein to be expressed by the recombinant bacteria of the present invention was expressed as a recombinant precursor protein comprising the respective secretion signal, preferably of the Lactococcus protein Usp45, at the N-Terminus of the respective precursor protein.

The codon usage for the respective gene and/or genes encoding for the at least one protein to be expressed by the recombinant bacteria of the present invention was/were adapted to the general codon usage of L. lactis.

Furthermore, if more than one protein is to be expressed by the recombinant bacteria of the present invention the same Usp45 signal peptide sequence was used for the respective proteins.

In order to avoid large identical nucleotide regions on the plasmid in close proximity, which could lead to intraplasmid recombination/deletions, the same signal peptide was encoded by different codons on the basis of codon degeneration and the codon usage of L. lactis.

Furthermore, in order to optimize the cleavage site, where the signal peptide is cleaved from the synthetic precursor protein generating the mature protein, the web-based program SignalP 4.1 (http://www.cbs.dtu.dk/services/SignalP/) was used.

Also, if more than one protein is to be expressed by the recombinant bacteria of the present invention, preferably in at least one operon, each of the respective genes was provided with a ribosome binding site (RBS) in order to improve protein expression.

Preferably, the first gene of an operon comprising two or more protein encoding nucleic acid sequences the ribosome binding site of the gadC gene was used. For the other genes of the operon the ribosome binding sites of an endogenous L. lactis gene, for example the ATP synthase subunit gamma (atpG) gene and/or the galactoside O-acetyltransferase (lacA) gene, was used.

Suitable ribosome binding sites can, for example, be chosen on the basis of good fits with the 3'-end of the 16S rRNA of L. lactis, as described in Bolotin et al. (2001), from known L. lactis nucleic acid sequences, for example from the nucleotide sequence of the *L. lactis subsp. lactis* IL1403 genome, which is available from NCBI under the accession no. AE005176.1, or *L. lactis subsp. cremoris* MG1363 genome, which is available under the NCBI accession number NC_009004.1.

The 3' end of 16S rRNA of L. lactis (5' GGAUCACCUCCUUUCU 3') is shown in SEQ ID No. 4 and in Figure 4.

A synthetic nucleic acid construct comprising the chloride-inducible promoter as well as at least one nucleic acid sequence encoding for at least one protein to be expressed by the recombinant bacteria of the present invention were de novo synthesized and, as outlined above, were then ligated into the respective expression plasmid, which was then transformed into a suitable bacterial strain, preferably L. lactis.

The following expression plasmids were generated:

### 1.3 Generation of an expression plasmid for expression of mCherry

Different constructs have been prepared that contain the mCherry gene under control of PgadC. mCherry is a fluorescent protein first described by Beilharz K, et al. (2015).

The nucleic acid sequence encoding for the mCherry protein is available under NCBI ascension number KJ908190.1. The corresponding amino acid sequence is available und NCBI ascension number AIL28759.1.

The mCherry coding sequence was fused to the PgadC promoter. A schematic representation of the respective inserts is shown in Figure 17a.

The nucleic acid sequence of the synthetic mCherry construct is shown in Figure 17b and in SEQ ID No. 33. A shematic representation of synthetic mCherry construct is shown in Figure 17a.

The finished gene synthesis products were obtained from BaseClear as a fragment cloned in the E. coli vector pUC57.

pUC57 plasmids with the respective gene synthesis products were digested with the restriction enzymes SphI and BglII. The SphI and BglII fragments containing the respective gene synthesis products were isolated by phenol extraction and ethanol precipitation and ligated into SphI and BglII cut plasmids pAUC1010 resulting in the generation of an expression plasmids designated pC-mCherry

The nucleic acid sequence of the expression plasmid designated pC-mCherry is shown in Figure 18b and SEQ ID No. 34. A schematic representation of the expression plasmid pC-mCherry is depicted in Figure 18a.

### 1.4 Generation of an expression plasmid for expression of hFGF-2, hIL-4 and hCSF-1 from a single operon

In order to express human fibroblast growth factor 2 (hFGF-2), human interleukin 4 (hIL-4) and human colony stimulating factor 1 (hCSF-1) in L. lactis NZ1330 the following nucleic acid sequences were generated.

#### 1.4.1 human fibroblast growth factor 2 (hFGF-2)

The amino acid sequence used for expression of hFGF-2 is derived from the 288 amino acid sequence of the human FGF-2 precursor available under the NCBI accession number NP_001997.5.

The mature form of hFGF-2 contains 155 amino acids and is designated in the following as hFGF-2-155. It has a molecular weight of 17.3 kDa and a pl of 9.85. The molecule contains 4 cysteine residues.

The hFGF-2 variant that is to be expressed in L. lactis was lacking the first two amino acids methionine and alanine and contains 153 amino acids. Thus, this variant was designated in the following as hFGF-2-153. hFGF-2-153 has a molecular weight of 17.1 kDa and a pl of 9.85. This variant contains four cysteine residues.

The corresponding amino acid sequence of the human FGF-2-155 is shown in Figure 5a and in SEQ ID No 5 The underlined amino acids shown in Figure 5a denote the first two amino acids methionine and alanine, which are missing in the variant hFGF2-153. The amino acid sequence of the variant of hFGF2-153 is depicted in Figure 5b and in SEQ ID No 6.

The sequence of FGF-2-155 is the mature human FGF-2 sequence after secretion. In vivo this sequence is further processed. However, various existing recombinant products have used this 155 amino acid sequence with or without the N-terminal methionine residue.

In order to express and secrete hFGF2, for example, into the supernatant of the growth medium a signal peptide was added to the N-terminus as described above, generating a recombinant hFGF-2-153 precursor protein.

The respective amino acid sequence of the recombinant hFGF-2-153 precursor protein is shown in Figure 5c and in SEQ ID No. 7. The underlined amino acids shown in Figure 5c denote the secretion signal of the Lactococcus protein Usp45.

### 1.4.2 Human interleukin-4 (hIL-4)

The mature protein of hIL-4 contains 129 amino acids, which correspond to the amino acids 25 to 153 of the amino acid sequence available under the NCBI accession number NP_000580.1. The mature protein has a molecular weight of 14.96 kDa and a pl of 9.36. The molecule contains 6 cysteine residues.
The amino acid sequence of the mature hIL-4 is shown in Figure 6a and in SEQ ID No. 8.
The hIL-4 variant that is to be expressed in L. lactis contains an additional alanine residue at the N-terminus of the mature protein and thus contains 130 amino acids. It has a molecular weight of 15.03 kDa and a pl of 9.36. This variant also contains 6 cysteine residues.
The corresponding amino acid sequence of the human hIL-4 variant that is to be expressed is shown in Figure 6b and in SEQ ID No. 9. The underlined amino acid shown in Figure 6b denotes the additional alanine residue at the N-terminus of the amino acid sequence.
In order to express and secrete hIL4, for example, into the supernatant of the growth medium a signal peptide was added to the N-Terminus as described above, generating a recombinant hIL-4 precursor protein. The respective amino acid sequence of the recombinant hIL-4 precursor protein is shown in Figure 6c and in SEQ ID No. 10. The underlined amino acids shown in Figure 6c denote the secretion signal of the Lactococcus protein Usp45.

### 1.4.3 Human colony stimulating factor 1 (hCSF1)

The amino acid sequence of hCSF1 used for expression is derived from the 554 amino acid sequence of the human CSF1 precursor isoform a, which is available under the NCBI accession number NP_000748.3.
The mature form of hCSF1 spans the amino acids 33 to 450 of the precursor. The amino acid sequence is shown in Figure 7a and in SEQ ID No. 11.
The amino acid sequence that is to be expressed starts at amino acid 33 (E), which is the first amino acid of the mature hCSF1, and ends at amino acid 181 (Q).
Furthermore, an additional 9 amino acids have been added at the C-terminus that occupy in the native protein the positions 480 - 488 (GHERQSEGS). In order to improve the removal of the signal peptide by the signal peptidase an additional alanine residue was added at the N-terminus. The resulting amino acid sequence is shown in Figure 7b and in SEQ ID No. 12.
The additional alanine residue at the N-terminus in Figure 7b is underlined. The additional 9 amino acids added at the C-terminus are depicted in Figure 7b in bold. The protein encoded by this sequence contains 159 amino acids, has a molecular weight of 18.5 kDa and a pl of 4.72. The protein contains 7 cysteine residues.
In order to express and secrete hCSF1, for example, into the supernatant of the growth medium a signal peptide was added to the N-Terminus as described above, generating a recombinant hCSF1 precursor protein. The respective amino acid sequence of the synthetic hCSF1 precursor protein is shown in Figure 7c and in SEQ ID No. 13. The underlined amino acids shown in Figure 7c denote the secretion signal of the Lactococcus protein Usp45

### 1.4.4 Generation of expression constructs

The amino acid sequences of the synthetic hFGF-2-153 precursor protein shown in Figure 5c and SEQ ID No. 7, the synthetic hIL-4 precursor protein shown in Figure 6c and in SEQ ID No. 10 and the synthetic hCSF1 precursor protein shown in Figure 7c and in SEQ ID No. 13, were translated into a corresponding nucleic acid sequence, whereby the codon usage was adapted to the general codon usage of L. lactis.
2 constructs were made with 2 of the 6 permutation of the three genes (FGF2, IL4, CSF1):

a) hCSF1, hFGF2, hIL4 (CFI)
b) hIL4, hCSF1, hFGF2 (ICF)

For the design of the operons each gene was provided with its own ribosome binding site for translational initiation and its own signal peptide for protein secretion, using the same signal sequence (ssUsp45), which was coded by different codons on the basis of codon degeneration and the codon usage of L. lactis.
For each of the first genes of the 2 constructs the ribosome binding site of the gadC gene was used. For the other two genes of each of the 2 constructs the ribosome binding sites of the ATP synthase subunit gamma (atpG) gene and the galactoside O-acetyltransferase (lacA) gene were used.

The nucleic acid sequence of the de novo synthesized CFI construct is shown in Figure 8b and SEQ ID No. 14. A schematic representation of the de novo synthesized CFI construct is depicted in Figure 8a.

The finished gene synthesis products were obtained from BaseClear each as a fragment cloned in the E. coli vector pUC57.

pUC57 plasmids with the respective gene synthesis products were digested with the restriction enzymes SphI and BglII each obtained from New England Biolabs (Ipswich, MA, US). The SphI and BglII fragments containing the respective gene synthesis products were isolated by phenol extraction and ethanol precipitation and ligated into SphI and BglII cut plasmid pAUC1010 resulting in the generation of an expression plasmid designated pC-CFI.

The nucleic acid sequence of the expression plasmid designated pC-CFI is shown in Figure 9b and SEQ ID No. 15. A schematic representation of the expression plasmid pC-CFI is depicted in Figure 9a.

**1.5 Generation of an expression plasmid for expression of mIL-18 and mGM-CSF from a single operon**

In order to express mouse interleukin 18 (mIL18) and mouse granulocyte-macrophage colony-stimulating factor (mGM-CSF) in L. lactis NZ1330 the following nucleic acid sequences were generated.

**1.5.1 Mouse interleukin-18 (mIL-18)**

The mature protein of mIL-18 contains 157 amino acids, which correspond to the amino acids 36 to 192 of the amino acid sequence of the mouse interleukin-18 isoform a precursor available under the NCBI accession number NP_032386.1.

The amino acid sequence of the mature mouse IL-18 is shown in Figure 10a and in SEQ ID No. 16.

The mIL-18 variant that is to be expressed in L. lactis contains an additional alanine residue at the N-terminus of the mature protein. The corresponding amino acid sequence of the mouse IL-18 variant that is to be expressed is shown in Figure 10b and in SEQ ID No. 17. The underlined amino acid shown in Figure 10b denotes the additional alanine residue at the N-terminus of the amino acid sequence.

In order to express and secrete mIL-18, for example, into the supernatant of the growth medium, a signal peptide was added to the N-Terminus as described above, generating a synthetic mIL-18 precursor protein. The respective amino acid sequence of the synthetic mIL-18 precursor protein is shown in Figure 10c and in SEQ ID No. 18. The underlined amino acids shown in Figure 10c denote the secretion signal of the Lactococcus protein Usp45.

**1.5.2 Mouse granulocyte-macrophage colony-stimulating factor (mGM-CSF)**

The mature mGM-CSF contains 129 amino acids, which correspond to the amino acids 18 to 141 of the amino acid sequence of the mouse granulocyte-macrophage colony-stimulating factor precursor available under the NCBI Reference Sequence: NP_034099.2.

The amino acid sequence of the mature mouse GM-CSF is shown in Figure 11a and in SEQ ID No. 19.

The mGM-CSF variant that is to be expressed in L. lactis contains an additional alanine residue at the N-terminus of the mature protein, which replaces the serine at the N-terminus in order to optimize the cleavage of a signal peptide. The corresponding amino acid sequence of the mouse GM-CSF variant that is to be expressed is shown in Figure 11b and in SEQ ID No. 20. The underlined amino acid shown in Figure 11b denotes the additional alanine residue at the N-terminus of the amino acid sequence.

In order to express and secrete mGM-CSF, for example, into the supernatant of the growth medium, a signal peptide was added to the N-Terminus as described above, generating a recombinant mGM-CSF precursor protein. The respective amino acid sequence of the synthetic mGM-CSF precursor protein is shown in Figure 11c and in SEQ ID No. 21. The underlined amino acids shown in Figure 11c denote the secretion signal of the Lactococcus protein Usp45.

**1.5.3 Generation of expression constructs**

The amino acid sequences of the recombinant mIL-18 precursor protein shown in Figure 10c and SEQ ID No. 18and the recombinant mGM-CSF precursor protein shown in Figure 11c and in SEQ ID No. 21 were translated into a corresponding nucleic acid sequence, whereby the codon usage was adapted to the general codon usage of L. lactis.

For the design of the operon each of the two genes encoding for the respective precursor proteins was provided with its own ribosome binding site for translational initiation and its own signal peptide for protein secretion, using the same signal sequence (ssUsp45), which was coded by different codons on the basis of codon degeneration and the codon usage of L. lactis, as outlined above.

For the first gene (mIL-18) of the operon the ribosome binding site of the gadC gene was used. For the second gene (mGM-CSF) the ribosome binding site of the ATP synthase subunit gamma (atpG) gene was used.

The nucleic acid sequence of the synthetic mEG construct is shown in Figure 12a and in SEQ ID No. 22.

The finished gene synthesis product was obtained from BaseClear as a fragment cloned in the E. coli vector pUC57.

pUC57 plasmid with the respective gene synthesis product was digested with the restriction enzymes SphI and BglII each obtained from New England Biolabs (Ipswich, MA, US). The SphI and BglII fragment containing the respective gene synthesis product was isolated by phenol extraction and ethanol precipitation and ligated into SphI and BglII cut plasmid pAUC1010 resulting in the generation of an expression plasmid designated pC-mEG.

The nucleic acid sequence of the expression plasmid designated pC-mEG is shown in Figure 12c and SEQ ID No. 23. A schematic representation of the expression plasmid pC-mEG is depicted in Figure 12b.

### 1.6 Generation of an expression plasmid for expression of mIL-12, mIL-18 and mIFNa from a single operon

In order to express mouse interleukin 12 (mIL-12), mouse interleukin 18 (mIL-18) and mouse interferon alpha-2 (mIFNa) in L. lactis NZ1330 the following nucleic acid sequences were generated.

#### 1.6.1 Mouse interleukin-12 (mIL-12)

Interleukin-12 (IL-12) is a heterodimeric cytokine encoded by two separate genes, IL-12A (p35) and IL-12B (p40).

The mIL-12 variant that is to be expressed in L. lactis was designed as a fusion protein of the mature interleukin-12 subunit beta, which forms the N-terminal part of the fusion protein, and the mature interleukin-12 subunit alpha, which forms the C-terminal part of the fusion protein, both separated by a peptide linker.

The mature protein of mouse interleukin-12 subunit beta contains 313 amino acids, which correspond to the amino acids 23 to 335 of the amino acid sequence of the mouse interleukin-12 subunit beta precursor available under the NCBI accession number NP_001290173.1.

The amino acid sequence of the mature interleukin-12 subunit beta is shown in Figure 13a and in SEQ ID No. 24.

Mouse interleukin 12 subunit alpha exists in at least two isoforms 1 and 2. The encoded isoform 2 is shorter at the N-terminus compared to isoform 1.

The amino acid sequence of the mouse interleukin 12 subunit alpha isoform 1 precursor available under the NCBI accession number NP_001152896.1.

The mature protein of mouse interleukin-12 subunit alpha isoform 2, the amino acid sequence of which was used for expression in the following examples, contains 199 amino acids, which correspond to the amino acids 23 to 215 of the amino acid sequence of the mouse interleukin 12 subunit alpha isoform 2 precursor available under the NCBI accession number NP_032377.1.

The amino acid sequence of the mature interleukin-12 subunit alpha isoform 2 is shown in Figure 13b and in SEQ ID No. 25.

The amino acid sequence of the mature form of the recombinant interleukin-12 fusion protein is depicted in Fig. 13c and SEQ ID No. 26. Furthermore, an additional two amino acids (alanine and aspartic acid) were added to the N-terminus of the recombinant fusion protein, which are shown in Fig. 13c by underlining. The linker sequence, separating the two IL.12 subunits, are marked in 13c by bold letters.

In order to express and secrete the mIL-12 fusion protein, for example, into the supernatant of the growth medium, a signal peptide was added to the N-Terminus as described above, generating a recombinant mIL-12 precursor protein.

The respective amino acid sequence of the recombinant mIL-12 fusion protein precursor is shown in Figure 13d and in SEQ ID No. 27. The underlined amino acids shown in Figure 13d mark the secretion signal of the Lactococcus protein Usp45.

#### 1.6.2 Mouse interleukin-18 (mIL-18)

The amino acid sequence of the mIL-18 variant that is to be expressed in L. lactis is shown in Figure 10b and in SEQ ID No. 17. The respective amino acid sequence of the recombinant mIL-18 precursor protein is shown in Figure 10c and in SEQ ID No. 18. The underlined amino acids shown in Figure 10c denote the secretion signal of the Lactococcus protein Usp45.

#### 1.6.3 Mouse interferon alpha-2 (mIFNa2)

The mature form of mIFNa2 contains 167 amino acids, which correspond to the amino acids 24 to 190 of the amino acid sequence of the mouse interferon alpha-2 precursor available under the NCBI Reference Sequence: NP_034633.2.

The amino acid sequence of the mature mouse IFNa2 is shown in Figure 14a and in SEQ ID No. 28.

The mIFNa2 variant that is to be expressed in L. lactis contains an additional alanine residue at the N-terminus of the mature protein, in order to optimize the cleavage of the signal peptide. The corresponding amino acid sequence of the recombinant mouse IFNa2 variant that is to be expressed is shown in Figure 14b and in SEQ ID No. 29. The underlined amino acid shown in Figure 14b denotes the additional alanine residue at the N-terminus of the amino acid sequence.

In order to express and secrete mIFNa2, for example, into the supernatant of the growth medium, a signal peptide was added to the N-terminus as described above, generating a recombinant mIFNa2 precursor protein. The respective amino acid sequence of the synthetic mIFNa2 precursor protein is shown in Figure 14c and in SEQ ID No. 30. The underlined amino acids shown in Figure 14c denote the secretion signal of the Lactococcus protein Usp45.

**1.6.4 Generation of expression constructs**

The amino acid sequences of the recombinant mIL-12 protein precursor shown in Figure 13d and SEQ ID No. 27, the amino acid sequences of the recombinant mIL-18 precursor protein shown in Figure 10c and in SEQ ID No. 18 and the amino acid sequences of the recombinant mIFNa2 precursor protein shown in Figure 14c and in SEQ ID No. 30 were translated into a corresponding nucleic acid sequence, whereby the codon usage was adapted to the general codon usage of L. lactis.

For the design of the operon each of the three genes encoding for the respective precursor proteins was provided with its own ribosome binding site for translational initiation and its own signal peptide for protein secretion, using the same signal sequence (ssUsp45), which was coded by different codons on the basis of codon degeneration and the codon usage of L. lactis, as outlined above.

For the first gene (mIL-12) of the operon the ribosome binding site of the gadC gene was used. For the second gene (mIL-18) the ribosome binding sites of the ATP synthase subunit gamma (atpG) gene and for the third gene (mIFNa2) the ribosome binding sites of the galactoside O-acetyltransferase (lacA) gene were used.

The nucleic acid sequence of the synthetic mTEA construct is shown in Figure 15 and in SEQ ID No. 31.

The finished gene synthesis product was obtained from BaseClear as a fragment cloned in the E. coli vector pUC57.

pUC57 plasmid with the gene synthesis product was digested with the restriction enzymes SphI and BglII. The SphI and BglII fragment containing the respective gene synthesis product was isolated by phenol extraction and ethanol precipitation and ligated into SphI and BglII cut plasmid pAUC1010 resulting in the generation of an expression plasmid designated pC-mTEA.

The nucleic acid sequence of the expression plasmid designated pC-mTEA is shown in Figure 16b and SEQ ID No. 32. A schematic representation of the expression plasmid pC-mTEA is depicted in Figure 16a.

**1.7. Generation of an expression plasmid for expression of mGM-CSF, mIL-12 and mIL-18 from a single operon**

In order to express mouse granulocyte-macrophage colony-stimulating factor (mGM-CSF), mouse interleukin-12 (mIL-12) and mouse interleukin-18 (mIL-18) in L. lactis NZ1330 the following nucleic acid sequences were generated.

**1.7.1 Mouse granulocyte-macrophage colony-stimulating factor (mGM-CSF)**

The mGM-CSF variant that is to be expressed in L. lactis is shown in Figure 11b and in SEQ ID No. 20. The underlined amino acid shown in Figure 11b denotes the additional alanine residue at the N-terminus of the amino acid sequence as described above under item 1.5.2. The respective amino acid sequence of the synthetic mGM-CSF precursor protein is shown in Figure 11c and in SEQ ID No. 21. The underlined amino acids shown in Figure 11c denote the secretion signal of the Lactococcus protein Usp45.

**1.7.2 Mouse interleukin-12 (mIL-12)**

The mIL-12 variant that is to be expressed in L. lactis was designed as a fusion protein of the mature interleukin-12 subunit beta, which forms the N-terminal part of the fusion protein, and the mature interleukin-12 subunit alpha, which forms the C-terminal part of the fusion protein, both separated by a peptide linker as described above under item 1.6.1. The respective amino acid sequence of the recombinant mIL-12 fusion protein precursor is shown in Figure 13d and in SEQ ID No. 27. The underlined amino acids shown in Figure 13d mark the secretion signal of the Lactococcus protein Usp45.

### 1.7.3 Mouse interleukin-18 (mIL-18)

The amino acid sequence of the mIL-18 variant that is to be expressed in L. lactis is shown in Figure 10b and in SEQ ID No. 17. The respective amino acid sequence of the recombinant mIL-18 precursor protein is shown in Figure 10c and in SEQ ID No. 18. The underlined amino acids shown in Figure 10c denote the secretion signal of the Lactococcus protein Usp45.

### 1.7.4 Generation of expression constructs

The amino acid sequences of the recombinant mGM-CSF precursor protein shown in Figure 11c and in SEQ ID No. 21 and the amino acid sequences of the recombinant mIL-12 fusion protein precursor is shown in Figure 13d and in SEQ ID No. 27 were translated into a corresponding nucleic acid sequence, whereby the codon usage was adapted to the general codon usage of L. lactis.

For the design of the operon each of the two genes encoding for the respective precursor proteins was provided with its own ribosome binding site for translational initiation and its own signal peptide for protein secretion, using the same signal sequence (ssUsp45), which was coded by different codons on the basis of codon degeneration and the codon usage of L. lactis, as outlined above.

For the first gene (mGM-CSF) of the operon the ribosome binding site of the gadC gene was used. For the second gene (mIL-12) the ribosome binding site of the ATP synthase subunit gamma (atpG) gene and for the third gene (mIL-18) the ribosome binding sites of the galactoside O-acetyltransferase (lacA) gene were used.

The nucleic acid sequence of the synthetic mGTE construct is shown in Figure 16a and in SEQ ID No. 33.

The finished gene synthesis product was obtained from BaseClear as a fragment cloned in the E. coli vector pUC57.

pUC57 plasmid with the respective gene synthesis product was digested with the restriction enzymes SphI and BglII each obtained from New England Biolabs (Ipswich, MA, US). The SphI and BglII fragment containing the respective gene synthesis product was isolated by phenol extraction and ethanol precipitation and ligated into SphI and BglII cut plasmid pAUC1010 resulting in the generation of an expression plasmid designated pC-mGTE.

The nucleic acid sequence of the expression plasmid designated pC-mGTE is shown in Figure 16c and SEQ ID No. 34. A schematic representation of the expression plasmid pC-mGTE is depicted in Figure 16b.

### Example 2 Generation of recombinant bacteria

The expression plasmids obtained in Example 2 were transformed in L. lactis strains by means of electroporation as outlined above. The respective L. lactis strains used in the following Examples are summarized in Table 1 below.

**Table 1:** Summary of recombinant bacteria used in following Examples

| Recombinant bacteria | | Expression plasmid | | | |
|---|---|---|---|---|---|
| Designation | *L. lactis* strain | Designation | Proteins | Sequence | SEQ ID No |
| AUP1602-C | NZ1330 | pC-CFI | FGF2:IL4:CSF1 | Fig. 9b | 15 |
| AUP5591m-C | NZ1330 | pC-mEG | IL18:GM-CSF | Fig. 12c | 23 |
| AUP5551m-C | NZ1330 | pC-mTEA | IL12:IL18:IFN-$\alpha$2b | Fig. 15c | 32 |
| AUP5563-C | NZ1330 | pC-mGTE | GM-CSF:IL12:IL18 | Fig. 16c | 34 |
| | | | | | |
| NZ1330 (pC-mCherry) | NZ1330 | pC-mCherry | mCherry | Fig. 18b | 36 |

For further testing stocks of the respective recombinant bacteria with a cell density of approximately $1\times10^{11}$ colony forming units (CFU)/ml were prepared after growth in IM1 or CDM3 medium with lactose and Na-beta-glycerophosphate by the addition of glycerol to a final concentration of 20 wt.-%. Stocks were stored at -80°C.

The composition of the CDM3 medium is based on ZMB3 medium described in Zhang, G. and Block, DE ("Using highly efficient nonlinear experimental design methods for optimization of Lactococcus lactis fermentation in chemically defined media", Biotechnol. Prog. 2009, 25(6): pages 1587 to 1597, doi: 10.1002/btpr.277).

**Example 3 Activity determination of Pgad promoter in vitro**

For the induction studies IM1 medium was used for growth of the respective *L. lactis* NZ1330 strains indicated in Table 1. The IM1 medium was supplemented in the following experiment as described below:

2 wt.-% β-glycerophosphate (Sigma, Catalog no. 50020-500G)
1.5 wt.-% soy peptone (BD, catalog no. 211906)
1 wt.-% Yeast extract (BD, catalog no. 212750)
1 mM $MgSO_4.7H_2O$ (Merck, catalog no. 1058860500)
0,1 mM $MgSO_4.7H_2O$ (Sigma, catalog no. M-7634)
3 wt.-% glucose (Natural Spices B.V., catalog no. ES212)

For pH-controlled fermentations 3.5 M NHsOH was used for base addition.

**Induction protocol**

Frozen stocks from the cell banks were used to inoculate 50 ml of IM1-medium, supplemented with 3 % glucose, followed by incubation for 16 hours at 30 °C (pre-culture). These overnight cultures were used to inoculate (1 vol.-% inoculum) 100 ml fermenters, preassembled with IM1 medium with 3 % glucose, pH 6.5, 30 °C. The cultures were mixed at continuous speed of 200 rpm using magnetic stirring.

The expression of the respective genes was induced with 100 mM NaCl at $OD_{600}$ = 0.5. The control cells were grown in medium without additional NaCl and, thus, expression of the respective genes was not induced.

The influence of the chloride-induced expression of a heterologous gene on the viability of *L. lactis* NZ1330 was assessed by comparing the growth curve of NaCl-induced *L. lactis* NZ1330 expressing mCherry under control of Pgad and an uninduced control.

The cultures were sampled at 0.5, 1, 2, 3, 4, 5 and 6 hours. These samples were used for $OD_{600}$ measurement and mCherry activity determination. Growth of the bacteria was monitored by measuring the optical density at 600 nm (OD600) of either induced bacterial cells or uninduced controlls. A comparison of the respective growth curves is shown in Figure 19a.

mCherry fluorescence activity was measured using a Synergy HT fluorispectrophotometer (Biotek, Winooski, Vermont, United States). Excitation was done at 530 nm and emission was measured at 590 nm and a gain of 120 was applied.

The results are shown in Figure 19b. As can be seen in Fig. 19b, induction of mCherry gene expression by addition of 100 mM NaCl resulted in a significant increase of fluorescence, which corresponded to an encreased amount of mCherry protein generated by the chlorideinduced expression of the heterologous gene in *L. lactis* NZ 1330. The increased amount of mCherry protein expression did not significantly impact viability of the respective bacteria after induction of mCherry gene expression, since the growth curve of induced bacteria did not show a significantly decreased grofth rate compared to uninduced bacteria, as can be seen in Fig. 19a.

Chlorid-induced expression of FGF-2, IL-4 and CSF-1 from the recombinant strain designated AUP-1602-C was determined by western blotting using the the above indicated protocol from TCA precipitates of 1 ml supernatants obtained by centrifugation of the respective culture at 14000 g for 10 min. at +4°C.

Purified preparation of commercially available recombinant human CSF-1 protein (Abcam PLC, 5 ng), recombinant human FGF-2 protein (R&D Systems, 7.5 ng), and recombinant human IL-4 protein (Sigma Aldrich, 5 ng) were used as positive control standards. The respective primary antibodies used are indicated above. As secondary antibody an anti-rabbit IgG, HRP-linked antibody from New England BioLabs (catalog Nr. #7074) was used.

The respective Western blots are shown in Fig. 20a. As can be seen in Fig. 20a all three recombinant proteins were expressed by AUP-1602-C and released into the supernatant.

Chlorid-induced expression of GM-CSF from the recombinant strain designated AUP5563-C was determined by western blotting using the the above indicated protocol from TCA precipitates of 1 ml supernatants obtained by centrifugation of the respective cultures at 14000 g for 10 min. at +4°C. Purified preparation of commercially available recombinant mouse GM-CSF protein (Sigma Aldrich) was used as positive control standard.

The respective Western blots is shown in Fig. 20b. As can be seen in Fig. 20b GM-CSF is expressed by AUP5563-C and released into the supernatant.

Chlorid-induced expression of IL-18 from the recombinant strain designated AUP5563-C was determined by western blotting using the the above indicated protocol from TCA precipitates of 1 ml supernatants obtained by centrifugation of the respective cultures at 14000 g for 10 min. at +4°C. Purified preparation of commercially available recombinant mouse IL-18 protein (R&D Systems) was used as positive control standard.

The respective Western blot is shown in Fig. 20c. As can be seen in Fig. 20c IL-18 is expressed by AUP5563-C and released into the supernatant.

Chlorid-induced expression of IL-12 from the recombinant strains designated AUP5551m-C and AUP5563-C was determined by western blotting using the the above indicated protocol from TCA precipitates of 1 ml supernatants obtained by centrifugation of the respective cultures at 14000 g for 10 min. at +4°C. Purified preparation of commercially available recombinant mouse IL-12 protein (R&D Systems) was used as positive control standard.

The respective Western blot is shown in Fig. 20d. As can be seen in Fig. 20d IL-12 is expressed by AUP5551m-C and AUP5563-C and released into the supernatant.

**Example 4 Activity determination of Pgad promoter in vivo**

The activity of the Pgad promoter system in vivo was assesed by utilizing the chloride-inducible mCherry construct and measuring the fluorescent signal in situ following intratumoral (i.t.) injection.

Mouse CT26 is an N-nitroso-N-methylurethane-(NNMU) induced, undifferentiated colon carcinoma cell line from Mus musculus. It was cloned to generate the cell line designated CT26.WT (ATCC® CRL-2638™), which is commercially available from LGC Standards GmbH.

CT26.WT cells can induce lethal tumors when inoculated into BALB/c mice.

According to Wang, M. et al. (1995) BALB/c mice inoculated, subcutaneously, with CT26 cells developed lethal tumors at 80% frequency with $10^3$ cells and at 100% with $10^4$ cells. Pulmonary metastases developed when mice were inoculated, intravenously, with $10^4$ cells. BALB/cJ mice (Genotype: A/A Tyrp1b/Tyrp1b Tyrc/Tyrc, Stock Code 000651), commercially obtained from The Jackson Laboratory (Bar Harbor, ME, USA), were used in this study.

BALB/c mice were inoculated with CT26.WT cells by the following procedure:

$1 \times 10^{-5}$ of tumor cells in 0.1 ml PBS were injected subcutaneously into the left flank of mice. Treatment was commenced when tumors reached a mean volume of ~100 - 200 $mm^3$, after which mice were allocated to their treatment groups with uniform mean tumour volume between groups.

Following a single intratumoral injection of the L. lactis NZ1330 containing the pC-mCherry construct, tumor-bearing mice were imaged using the the IVIS Spectrum CT (Excitation filters: 535-570 nm/ Emission filters: 580-680nm). Animals were imaged at 48 hours following i.t. injection of L. lactis NZ1330 (pC-mCherry) bacteria or control bacteria, i.e. L. lactis NZ1330 without mCherry plasmid . Duration and binning (sensitivity) of the image acquisition was dependent upon the intensity of the lesions present and was captured and processed using Living Image 4.3.1 software (PerkinElmer, Inc., Waltham, MS, US).

The results are shown in Figure 21, which show fluorescent imaging 48 hours after i.t. injection of the L. lactis NZ1330 (pC-mCherry) bacteria as well as of control bacteria. Black circles indicate the position of the tumor. Higher mCherry activity is indicated by a black area.

As can be seen in Figure 21 a single 50 μl intratumoral injection of $2 \times 10^{10}$ CFU/ml recombinant bacteria obtained in Example 3 and expressing the mCherry protein under the control of the PgadC promoter system provided for a sufficient localisation and survival of the respective bacteria in the tumor environment.

The results clearly show that recombinant bacteria of the present invention can survive in the tumor environment long enough to express and secrete at least one therapeutic protein, thereby influencing cells of the innate and/or adaptive immune system of the tumor environment to achieve an anti-tumor response.

Furthermore, the chloride concentration within the tumor environment is sufficently high to provide for an expression of the respective at least one therapeutic protein to be expressed by the recombinant bacteria of the present invention.

**Example 5: Wound closure experiments**

Patients with diabetes are prone to impaired wound healing, with foot ulceration being particularly prevalent. This delay in wound healing also extends to diabetic animals, including the spontaneously diabetic (db/db) mouse - which was commercially obtained from The Jackson Laboratory (Bar Harbor, ME, US).

60 diabetic mice (strain name BKS.Cg-Dock7m +/+ Leprdb/J - Stock Code 00642), all male and aged approximately 10 weeks were used in a first study.

The consequence of application of bacteria expressing a combination of human FGF-2, CSF1 and IL-4 each under the control of the PgadC promoter to full-thickness excisional wounds on db/db diabetic mice was examined in a combined pharmacokinetic (PK) / pharmacodynamic (PD) - efficacy study.

Therein, recombinant bacteria designated AUP16-C obtained in Example 3 expressing FGF-2, IL-4 and CSF-1 in a single bacterial cell was evaluated and compared to a vehicle treatment. The composition of the respective vehicles used for application is summarized below:

Vehicle 1: 5 % dextrose in 0.9 % saline.
Vehicle 2: 5 % dextrose + 0.9 % saline + 200 mM Na Acetate (pH 6.5)
Vehicle 3: 5 % dextrose + 2.5 % saline
Vehicle 4: 5 % dextrose + 2.5 % saline + 200 mM Na Acetate (pH 6.5)
Vehicle 5: 10 % dextrose + 0.9 % saline
Vehicle 6: 10 % dextrose + 0.9 % saline + 200 mM Na Acetate (pH 6.5)

Animals were randomized to one of the 7 treatment regimens according to Table 2.

**Table 2:** Experimental treatment groups and regimes

| Group no. | Treatment (all with film dressing) | CFU/ml | Dose | Regimen | Number of applications | Animals per group |
|---|---|---|---|---|---|---|
| **1** | AUP1602-C in Vehicle 1) | $5\times10^8$ | 50μl | Daily to day 6 | 7 | 10 |
| **2** | AUP1602-C in Vehicle 2) | $5\times10^8$ | 50μl | Daily to day 6 | 7 | 8 |
| **3** | AUP1602-C in Vehicle 3 | $5\times10^8$ | 50μl | Daily to day 6 | 7 | 8 |
| **4** | AUP1602-C in Vehicle 4) | $5\times10^8$ | 50μl | Daily to day 6 | 7 | 8 |
| **5** | AUP1602-C in Vehicle 5 | $5\times10^8$ | 50μl | Daily to day 6 | 7 | 8 |
| **6** | AUP1602-C in Vehicle 6 | $5\times10^8$ | 50μl | Daily to day 6 | 7 | 8 |
| **7** | Vehicle 1 | 0 | 50μl | Daily to day 6 | 7 | 10 |

Recombinant bacteria expressing FGF-2, IL-4 and CSF-1 in a single bacterial cell were applied to wounds on the day of wounding (day 0) and their survival was examined 6 hours after application.

In order to determine the production of FGF-2, CSF-1 and/or IL-4 by bacteria applied to wounds, wound fluid samples were taken from wounds after 6 hours, and 1, 2 & 7 days. Systemic blood and key organs were harvested after 6 & 24 hours and 7 days to facilitate PK/PD analysis. Wounds in receipt of vehicle 1 alone were used as controls for the PK/PD component of this study.

In order to examine the impact of these recombinant bacteria on the process of wound healing, bacteria expressing human FGF-2, CSF-1 and IL-4 were applied to wounds on the day of wounding (day 0) and daily thereafter until post-wounding day 6. The healing of wounds in receipt of these recombinant bacteria was compared to that of similar wounds exposed to fresh vehicle 1 only.

Wound healing was studied at both the macroscopic and histological levels. Wound healing was studied at the macroscopic level in terms of initiation of neo-dermal repair responses, wound contraction and wound closure.

Wound closure, and its components wound contraction and wound re-epithelialisation, were determined from digital photographs taken on post-wounding days 0, 4 & 7 post-wounding. Histological assessments of granulation tissue formation (depth) and wound width (craniocaudal contraction) were undertaken on routine (H&E) stained sections. These histological assessments were undertaken on tissues harvested on post-wounding day 7.

The development of adverse effects was monitored and fully documented.

## Creation of full-thickness experimental wounds and application of treatments

All mice were anaesthetised using isofluorane and air; and their dorsal flank skin was clipped and cleansed. A single standardised full-thickness wound (10mm × 10mm) was created on the left flank approximately 10mm from the spine. Each wound was then photographed with an identification plate and calibration rule.

All wounds were then dressed with the transparent film dressing Tegaderm® Film (3M Deutschland GmbH, Neuss, DE). Animals were then allowed to recover in a warmed environment (34°C). Animals were later restrained and dosed with one of the treatments described in Table 2, each applied by injection through the Tegaderm® film using a 27-gauge needle.

Treatments were reapplied likewise to wounds on a daily basis until post-wounding day 6, thus each wound received 7 applications. All wounds were closely monitored for excessive build-up of applied agents and excessive wound site hydration; and excess product/fluid was removed by aspiration on post-wounding days 4, 6 & 8 as well as prior to re-application on day 4 & 6, and prior to dressing removal on day 8.

On post-wounding days 4, 8, 12 and 16 all animals were re-anaesthetised, their film dressings and any free debris removed gently, and their wounds cleaned using sterile salinesoaked gauze. Wounds were then photographed, re-dressed (as above) with Bioclusive® film dressing - and animals were allowed to recover in a warmed environment (34°C).

Immediately after wounding, and subsequently on days 4, 8, and 12, all wounds were digitally photographed together with a calibration/identity plate following film dressing removal and wound cleaning.

All animals were terminated on day 20, following wound photography. Termination was achieved by a UK Home Office 'Schedule 1' compliant method.

**Image Analysis of Wound Closure**

Each wound was digitally photographed, along with an identification/calibration plate, immediately after injury and subsequently on days 4, 8, 12, 16 and 20 and open wound area was measured and expressed in terms of % wound area relative to day 0.

Image Pro Plus image analysis software (version 4.1.0.0, Media Cybernetics, Inc., Rockville, MD, US) was used to calculate wound closure from scaled wound images taken at each assessment point.

The results for each treatment group are summarized in Table 3 and Figure 22.

For a given wound at a given time point, wound closure was expressed as the percentage wound area remaining relative to the initial wound area immediately after injury (i.e. day 0). Table 3 shows the mean percentage of the remaining wound area for all treatment groups.

**Table 3.** Percentage "Wound Area Remaining" Data for all study group.

| % wound area remaining with time - open wound area (mean +/- standard error) | | | | | | | |
|---|---|---|---|---|---|---|---|
| Treatment | | n | Days post-wounding | | | | |
| | | | 4 | 8 | 12 | 16 | 20 |
| 1 | AUP1602-C (Veh 1) | 10 | 72.5 ± 1.6 | 30.7 ± 3.5 | 8.0 ± 1.6 | 4.2 ± 1.5 | 5.3 ± 1.9 |
| 2 | AUP1602-C (Veh 2) | 8 | 71.5 ± 2.2 | 23.8 ± 2.6 | 4.5 ± 0.9 | 1.2 ± 0.8 | 0.5 ± 0.4 |
| 3 | AUP1602-C (Veh 3) | 8 | 68.7 ± 1.8 | 19.3 ± 1.6 | 6.5 ± 0.6 | 1.9 ± 0.7 | 2.7 ± 1.5 |
| 4 | AUP1602-C (Veh 4) | 8 | 65.8 ± 1.7 | 17.1 ± 2.5 | 4.2 ± 0.7 | 1.1 ± 0.4 | 0.3 ± 0.2 |
| 5 | AUP1602-C (Veh 5) | 8 | 67.8 ± 1.6 | 19.8 ± 3.1 | 5.2 ± 0.7 | 1.8 ± 0.7 | 0.7 ± 0.4 |
| 6 | AUP1602-C (Veh 6) | 8 | 75.6 ± 2.6 | 28.2 ± 5.0 | 8.4 ± 1.6 | 3.7 ± 1.0 | 3.7 ± 1.2 |
| 7 | Vehicle 1 | 10 | 83.2 ± 2.7 | 70.7 ± 3.5 | 63.2 ± 2.7 | 55.4 ± 2.9 | 54.3 ± 4.4 |

As can be seen from the data presented in Table 3 all groups in receipt of AUP1602-C bacteria irrespective of delivery vehicle were found to close significantly more rapidly than the control group (5 % dextrose in 0.9 % saline) at all time points.

**Wound Contraction**

Contraction is the centripetal movement of the wound margins - due to the compaction of granulation tissue within the "body" of the wound.

The "compactional" forces, that drive this process, are thought to reside in cells of the fibroblast lineage. In this study, % contraction was calculated as:-

$$\%\ \text{contraction} = \frac{\text{The area defined by the boundary of normal dermis and the “repairing neo-dermis”}}{\text{The original wound area (day 0)}} \times 100$$

Mean percentage wound contraction data for all treatment groups are described in Table 4 and in Figure 23.

**Table 4:** Summary of "percentage wound contraction" data.

| % of original wound area closed by contraction (mean +/- standard error) | | | | | | | |
|---|---|---|---|---|---|---|---|
| Treatment | | n | Days post-wounding | | | | |
| | | | 4 | 8 | 12 | 16 | 20 |
| 1 | AUP1602-C (Veh 1) | 10 | 20.7 ± 1.3 | 43.8 ± 2.2 | 66.4 ± 2.8 | 73.9 ± 2.9 | 74.9 ± 3.5 |
| 2 | AUP1602-C (Veh 2) | 8 | 20.3 ± 2.5 | 45.0 ± 1.0 | 65.7 ± 1.6 | 72.5 ± 1.7 | 72.3 ± 2.1 |
| 3 | AUP1602-C (Veh 3) | 8 | 22.9 ± 2.1 | 46.0 ± 2.8 | 63.0 ± 2.9 | 68.6 ± 3.4 | 69.9 ± 3.9 |
| 4 | AUP1602-C (Veh 4) | 8 | 24.7 ± 1.7 | 45.3 ± 1.5 | 56.6 ± 2.2 | 61.1 ± 1.9 | 62.5 ± 1.9 |
| 5 | AUP1602-C (Veh 5) | 8 | 22.1 ± 1.8 | 46.3 ± 2.3 | 64.8 ± 2.4 | 70.5 ± 2.3 | 74.5 ± 2.4 |
| 6 | AUP1602-C (Veh 6) | 8 | 17.8 ± 2.4 | 42.1 ± 2.9 | 62.2 ± 1.7 | 70.1 ± 2.3 | 72.6 ± 2.8 |
| 7 | Vehicle 1 | 10 | 4.9 ± 2.1 | 13.7 ± 2.7 | 22.2 ± 1.8 | 30.7 ± 2.1 | 28.5 ± 3.4 |

As can be seen from Table 4 all groups in receipt of AUP1602-C bacteria, irrespective of delivery vehicle, were found to contract significantly more rapidly than the control group (5 % dextrose in 0.9 % saline) at all time points ($p \leq 0.007$).

Wounds on non-diabetic mice close predominantly by contraction, while those on diabetic mice have a significantly reduced ability to contract, probably due to impoverished granulation tissue formation. As a result, wounds on diabetic animals tend to close by re-epithelialisation to a greater extent than those on non-diabetic animals. The forces, that drive the process of contraction, are thought to derive from the activities of fibroblasts that populate the neo-dermal compartment of cutaneous wounds.

The observation of enhanced contraction after application of AUP1602-C clearly suggests improvement in granulation tissue function; which may in turn be explained by an increase in the amount of granulation tissue formed, an increase in the speed at which it is formed, and/or increased contractile capacity of the tissue.

**Initiation of Wound Healing (neo-dermal tissue generation)**

Wounds were visually assessed on a daily basis until day 8, and then on alternate days - to establish their "healing" status. Each wound was scored as to whether it was displaying "neodermal tissue generation activity" or not, i.e. whether the wound had initiated the dermal healing process or not. Each wound was assessed by two independent observers and the percentage of wounds displaying "neo-dermal tissue generation activity" was compared between treatment groups at each assessment point.

The number of wounds responding for each treatment group, each day is displayed in Table 5.

The % of wounds responding, for each treatment group, on day 1, is displayed in Figure 24. A healing response was evident in the majority of wounds in receipt of AUP1602-C regardless of the delivery vehicle used following the first day of dosing on day 1 post-wounding.

On day 1 post-wounding, 100% of wounds were found to have initiated a healing response in treatment groups AUP1602-C (Veh 2), AUP1602-C (Veh 3) & AUP1602-C (Veh 6), 88% with AUP1602-C (Veh 4), 80% with AUP1602-C (Veh 1) and 75% with AUP1602-C (Veh 5).

No significant differences were detected between these treatment groups (Fisher Exact Test).

100% of wounds in receipt of AUP1602-C were found to have demonstrated a healing response by day 2 post-wounding.

None of the wounds in receipt of vehicle 1 only (5 % dextrose in 0.9 % saline) demonstrated a healing response during the 20-day study period.

A significantly greater proportion of wounds were found to have responded in all AUP1602-C treatment groups compared to the vehicle control group at all time points assessed ($p \leq 0.001$, Fisher Exact Test).

**Table 5:** Number of wounds displaying "Initiation of neo-dermal tissue formation' after indicated day post-wounding.

| **Group** | **Treatment** | **Day** | | **n** |
|---|---|---|---|---|
| | | **1** | **2 onwards** | |
| 1 | AUP1602-C (Veh 1) | 8 | 10 | 10 |
| 2 | AUP1602-C (Veh 2) | 8 | 8 | 8 |

(continued)

| Group | Treatment | Day | | n |
|---|---|---|---|---|
| | | 1 | 2 onwards | |
| 3 | AUP1602-C (Veh 3) | 8 | 8 | 8 |
| 4 | AUP1602-C (Veh 4) | 7 | 8 | 8 |
| 5 | AUP1602-C (Veh 5) | 6 | 8 | 8 |
| 6 | AUP1602-C (Veh 6) | 8 | 8 | 8 |
| 7 | Vehicle 1 | 0 | 0 | 10 |

A significantly greater proportion of wounds in receipt of AUP1602-C bacteria, regardless of formulation, initiated 'neodermal tissue formation' compared to that observed with the vehicle 1 control group (5% dextrose in 0.9% saline alone) i.e. 100% of wounds in receipt of AUP bacteria responded; whereas, no wounds in receipt of vehicle 1 alone appeared to respond.

For all formulation vehicles AUP1602-C treatment initiated a response in the wound bed very early on after application (within 1 to 2 days).

**Angiogenic response**

Day 4 and day 8 wound images were visually assessed to quantify the level of angiogenesis. The mean score for angiogenic response on day 4 and day 8 for all groups is shown in Table 6 Scores were averaged from 2 independent observers.

**Table 6:** Angiogenic response score on days 4 and 8 post-wounding.

| | | Average score from 2 independent observers | |
|---|---|---|---|
| Group | Treatment | Day 4 | Day 8 |
| 1 | AUP1602-C (Veh 1) | 2.30 $\pm$ 0.2 | 2.93 $\pm$ 0.2 |
| 2 | AUP1602-C (Veh 2) | 2.25 $\pm$ 0.4 | 3.03 $\pm$ 0.1 |
| 3 | AUP1602-C (Veh 3) | 2.31 $\pm$ 0.3 | 3.06 $\pm$ 0.1 |
| 4 | AUP1602-C (Veh 4) | 2.13 $\pm$ 0.3 | 2.66 $\pm$ 0.3 |
| 5 | AUP1602-C (Veh 5) | 2.25 $\pm$ 0.3 | 2.97 $\pm$ 0.2 |
| 6 | AUP1602-C (Veh 6) | 2.81 $\pm$ 0.2 | 3.00 $\pm$ 0.1 |
| 7 | Vehicle 1 | 0.90 $\pm$ 0.1 | 0.90 $\pm$ 0.1 |

All treatments involving AUP1602-C scored significantly higher for angiogenesis than vehicle 1 alone, when observed on days 4 and 8 ($p \leq 0.002$).

Wounds in receipt of AUP1602-C bacteria regardless of vehicle demonstrated significantly increased angiogenesis compared to the vehicle 1 control group (5% dextrose in 0.9% saline alone) at both the day 4 and the day 8 assessment points.

All bacterial treatment regimes investigated in this experiment, regardless of vehicle, were found to promote wound repair in the diabetic db/db mouse, which is a widely accepted and one of the best validated animal model of delayed wound healing in humans.

Excisional wounds in db/db mice show a statistically significant delay in wound closure, decreased granulation tissue formation, decreased wound bed vascularity, and markedly diminished proliferation, as for example described in Michaels et al. (2007) ("db/db mice exhibit severe wound-healing impairments compared with other murine diabetic strains in a siliconesplinted excisional wound model", Wound Rep. Reg. 15, pages 665 to 670).

The experimental results clearly show that application of recombinant bacteria of the present invention provides a significant improvement in the treatment of wounds in humans, especially of chronic wounds.

Application of recombinant bacteria of the present invention induces granulation tissue formation, increased wound bed vascularity and proliferation, thus leading to an improved and accelerated wound closure even in a model exhibiting severe wound-healing impairments.

These results, thus, show that inflammatory, preferably chronic inflammatory, skin dysfunctions such as frostbite, eczema, psoriasis, dermatitis, ulcer, wound, lupus eritematosus, neurodermitis, and combinations thereof, preferably dermatitis, ulcer, wound, and combinations thereof, further preferably ulcer, benefit from the application of recombinant bacteria of the present invention.

For example, chronic wounds such as chronic venous ulcers, chronic arterial ulcers, chronic diabetic ulcers, and chronic pressure ulcers, can be treated by application of the recombinant bacteria of the present invention, since the wound-healing impairments observed in the respective chronic wounds are overcome after application of the recombinant bacteria of the present invention, preferably expressing human FGF-2, human IL-4 and human CSF-1.

**Example 6: Detection of human FGF-2, human IL-4 and human CSF-1 in wound fluids.**

As in Example 5 diabetic mice (strain name BKS.Cg-Dock7m +/+ Leprdb/J - Stock Code 00642), all male and aged approximately 10 weeks were used in a subsequent study.

Detection of the respective recombinant proteins after application of bacteria expressing a combination of human FGF2, CSF1 and IL4 each under the control of the PgadC promoter to full-thickness excisional wounds on db/db diabetic mice was examined in this study. Therein, recombinant bacteria designated AUP16-C obtained in Example 3 expressing FGF-2, IL-4 and CSF-1 in a single bacterial cell were evaluated with respect to the composition of the vehicle. The composition of the respective vehicles used for application is summarized below:

| | |
|---|---|
| Vehicle 1: | 5 % dextrose in 0.9 % saline |
| Vehicle 4: | 5 % dextrose in 2.5 % saline<br>+ 200 mM Na Acetate (pH 6.5) |
| Vehicle 7: | 5 % dextrose in 2.5 % saline<br>+ 200 mM Na Acetate<br>+ 50 mM L-glutamic acid (pH 6.0) |

Wound fluid samples were prepared with 5 kDa cut off concentrators (Vivaspin® sample concentrator) commercially available from Sigma-Aldrich with 0.5% SDS according the manufacturer's protocol.

Samples were separated on SDS PAGE gels and blotted to nitrocellulose as described above under item I.8.

Detection of the recombinant proteins was performed by using the following antibodies:
For detection of human FGF-2 a purified mouse anti-human FGF-2 monoclonal antibody (BD Transduction laboratories, Heidelberg, DE) was used at a dilution of 1:250. The second antibody was horseradish peroxidase (HRP)-linked antibody anti-mouse IgG at a dilution of 1:10,000 obtained from Bioké B.V. (Leiden, NL).

For detection of human IL-4 a monoclonal mouse anti-human IL-4 IgG1 from R&D systems (clone #3007) was used at a dilution of 1:500. The second antibody was HRP-linked antibody anti-mouse IgG at a dilution of 1:10,000 obtained from Bioké B.V. (Leiden, NL).

For detection of human CSF-1 a rabbit polyclonal antibody to human MCSF from Abcam PLC (clone ab9693) was used at a dilution of 1:2,000. The second antibody was HRP-linked anti-rabbit IgG antibody at a dilution of 1:2,000 obtained from Bioké B.V. (Leiden, NL).

The amounts of the respective recombinant proteins in the wound fluid were determined by comparison with a respective reference protein.

Human FGF-2 reference protein was obtained from Sigma Aldrich and was dissolved in 5 mM Tris pH 7.5 to give a final concentration of 100 $\mu$g/$\mu$l. As reference a total amount of 10 mg, 3,3 ng, 1,1 ng, 0,37 ng and 0,12 ng were applied to different lanes for Western blots. The results are shown in Figure 25a and 25b.

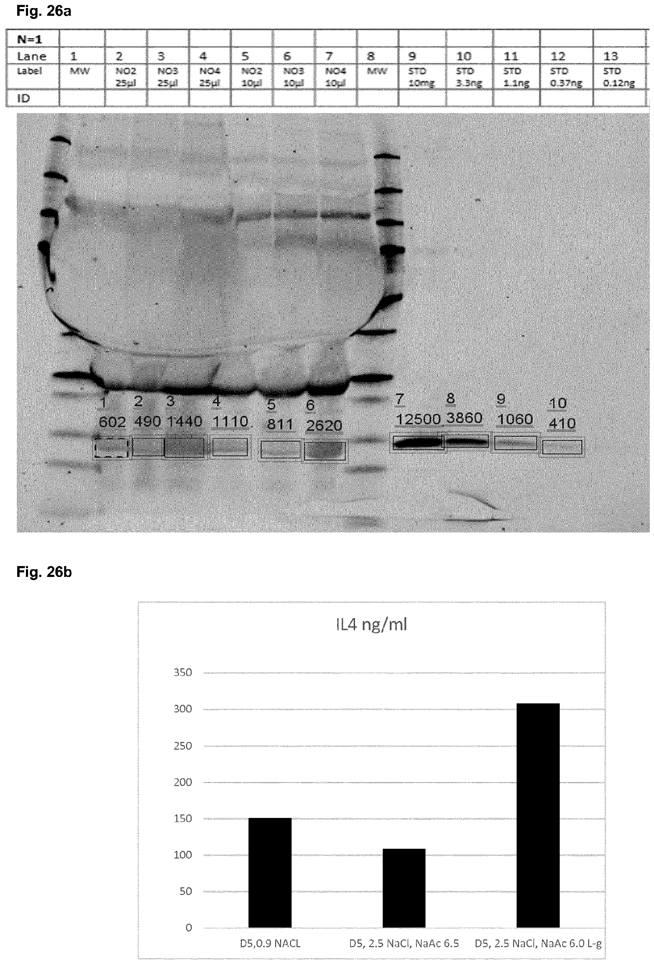

Human IL-4 reference protein was obtained from R&D systems (Minneapolis, MN, US). Human IL-4 was dissolved in PBS (5.8 mM $Na_2HPO_4$, 4.2 mM $NaH_2PO_4$, 145 mM NaCl) to give a final concentration of 100 $\mu$g/mL. As reference a total amount of 10 mg, 3,3 ng, 1,1 ng, 0,37 ng and 0,12 ng were applied to different lanes for Western blots. The results are shown in Figure 26a and 26b.

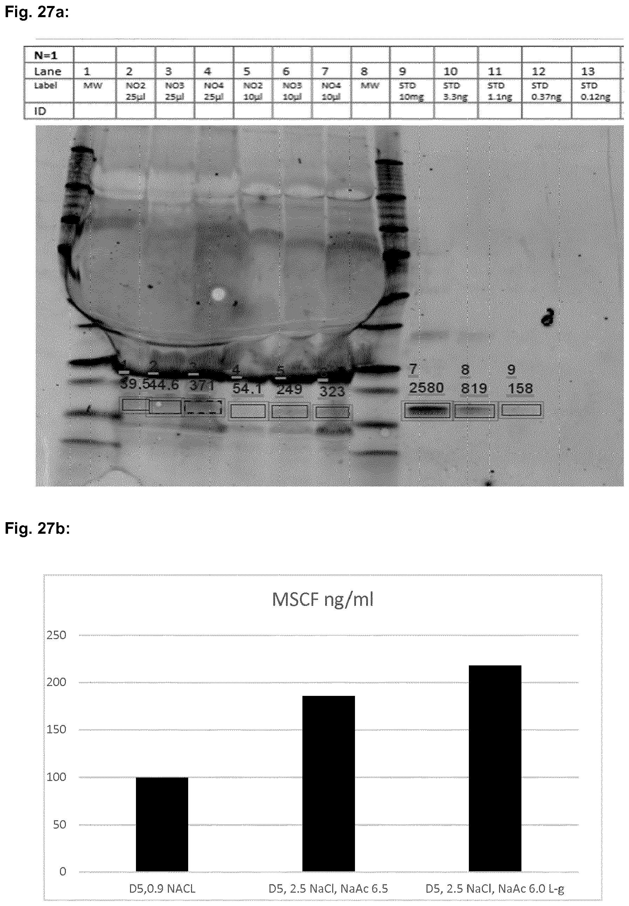

Human CSF-1 reference protein was obtained from Abcam PLC (Cambridge, GB). Human CSF-1 was dissolved in sterile water to give a final concentration of 100 $\mu$g/mL. As reference a total amount of 10 mg, 3,3 ng, 1,1 ng, 0,37 ng and 0,12 ng were applied to different lanes for Western blots. The results are shown in Figure 27a and 27b.

Recombinant bacteria using PgadC system in vivo properly express human FGF-2, human IL-4 and human CSF-1 and all three target proteins could be detected in wound fluids.

AUP1602-C with PgadC promoter system produced sufficient amount of the respective target proteins under in vivo conditions after topical application of the respective recombinant bacteria without the requirement of additional administration of an external inducer and/or excipient such as $\beta$-galactosidase or nisin.

**Example 7 Inhibition of carcinoma cell growth in vivo**

A prerequisite to successful immunotherapy is the existing of a fully functional immune system. Therein, the immunogenicity of a tumors is considered the dominant feature predicting response to immunotherapy.

Among different mouse models developed to assess properties of anti-cancer agents and/or treatments, syngeneic models are mice bearing tumors of genetically same murine strain. These models offer an intact immune and microenvironment system for testing immunotherapeutic and/or antiangiogenic agents as well as treatment regimenes.

The background mouse strain used for generating the respective murine model has been shown to impact tumor immunogenicity with tumors established in BALB/c mice generally being more immunogenic than for example tumors established in C57BL/6 mice.

Furthermore, a study on murine solid tumor models performed by Lechner, M.G. et al. (2013) demonstrated that among different cell lines CT26, RENCA, and 4T1 tumors showed the greatest positive response to immunotherapy regimens, including significantly decreased tumor growth and increased survival.

Thus, CT26 cells were selected as a cell line to be used withe BALB/C mice to initially evaluate the efficacy of administration of AUP5563-C4 in vivo.

An efficacy study with AUP5563-C4, expressing IL-12, IL-18 and GM-CSF, was carried out evaluating the effect of an intratumoral administration of the bacterial preparation AUP5563-C4 in the treatment of the subcutaneous murine CT-26 cancer model.

As described above in Example 4, CT26.WT cells were used to induce lethal tumors in BALB/c mice, by injecting $1 \times 10^5$ of CT26.WT cells in 0.1 ml PBS subcutaneously into the left flank of mice with a 27-gauge needle.

Body weight, dosing and any comments relating to clinical condition was captured in realtime using the study management software, StudyDirector (StudyLog Systems, Inc., San Francisco, CA, US).

Tumor were measured 3 times a week during treatment and the apparent tumor volume was estimated using the formula

$$V = 0.5 \text{ x } (\text{Length x Width}^2),$$

where V is the tumor volume, L is the tumor length, and W is the tumor width, by measuring the tumor in two dimensions using electronic callipers as, for example, described in Tomayko, M. and Reynolds, C. (1989).

Mice were randomly allocated to treatment groups. Treatment was commenced when tumours reached a mean volume of 50 - 100 $mm^3$. Mice were allocated to their treatment groups 1 to 3 with uniform mean tumour volume between groups. Duration of treatment was up to 2 weeks. The treatment groups are summarized in Table 7.

**Table 7:** Summary of treatment groups

| Group | Treatment Description | Dose Route | Dose Frequency & Duration | Dose Vol. |
|---|---|---|---|---|
| 1 | Vehicle (5% dextrose in 0.9% saline) | i.t. | Three times weekly; for two weeks | 25 μl |
| 2 | AUP5563-C 4 | i.t. | Three times weekly; for two weeks | 25 μl |
| 3 | Anti-mCTLA4 (clone 9D9) | i.p. | BIW (Twice weekly); for two weeks | 10 ml/kg |

Efficacy was evaluated after intratumoral (i.t.) injection of AUP5563-C4 ($1 \times 10^8$ CFU) 3 × weekly for 2 weeks.

Vehicle treated group served as negative control and as positive control anti-CTLA-4 treated mice. Monoclonal anti-mouse CTLA-4 antibody, clone 9D9, was obtained from Bio X Cell (West Lebanon, NH, US), which is directed against mouse cytotoxic T-lymphocyte-associated protein 4 (CTLA-4) a protein receptor that downregulates the immune system.

The results are summarized in Figs. 28a to 28c, which show evaluation of tumor growth after intratumoral (i.t.) administrations of (a) saline (negative control), (b) AUP5563-C4 and (c) mouse anti-CTLA4 (positive control), respectively.

As can be seen in Fig. 28a and 28b i.t. injection of AUP5563-C4 ($1 \times 10^8$ CFU) 3 × weekly for 2 weeks significantly reduced tumor growth compared to vehicle-treated control mice.

Administration of AUP5563-C4 ($1 \times 10^8$ CFU) 3 × weekly for 2 weeks resulted in a comparable reduction in tumor growth as a treatment with anti-m-CTLA-4 antibody used as positive control.

The results demonstrate that i.t. administration of AUP5563-C4 had similar efficacy as the immune checkpoint inhibitor anti-CTLA4.

Tumors that received intratumoral injection of saline (negative control) where growing normally.

**Example 8 Treatment of intraperitoneal tumor in mice**

To evaluate the feasibility as well as efficacy after intraperitoneal administration of recombinant bacteria of the present invention expressing at least one therapeutic protein under control of the Pgad promoter, B16-F1 cells were inoculated intraperitoneally into C57BL/6 mice and survival as primary endpoint was measured.
The B16 melanoma cells were selected for tumor inoculation, as C57BL/6 mice is the syngeneic hosts for B16-F1 cells with high tumor intake rate as described by Fu, Q. et al.(2016).
Furthermore, this model has been shown to respond to immunotherapeutic treatment regimens as described by Lesinski, G.B. et al (2003).
Inoculation of mouse B16-F1 cells into C57BL/6 mice lead to the development of malignant tumors that can kill the mice before the 20$^{th}$ day after implantation without any treatments.
Mouse B16-F1 (KCLB no 80007) were obtained from Korea Cell Line Bank. The cells were seeded in 96-well microplates and incubated for 24 hours under the following conditions:

- Culture conditions: 37°C.
- Culture medium: Dulbecco's Modified Eagle's Medium (DMEM) (Life Technologies Corporation, Carlsbad, CA, US) supplemented with penicillin 50 U/ml, streptomycin 50 μg/ml, and fetal bovine serum to a final concentration of 10% by volume.

B16-F1 cells are a well-known model for the study of metastasis and solid tumor formation.
A total of 66 C57BL/6N mice, which were commercially obtained from Orient Bio Inc. (Gyeonggi-do, KR), were used in this study.
1 × 10$^5$ B16F1 cells were inoculated in each individual C57BL/6 mouse by intraperitoneal (i.p.) injection (Day 0). The treatment with either vehicle (5 % dextrose in 0.9 % saline) or with AUP2059/AUP5551-C (1:1) was administered intraperitoneally (300 μl per injection) three times a week up to five weeks starting on Day 0 (up to 15x administrations between Day 0 and Day 35).
Animals were randomized to one of the 6 treatment regimens according to Table 8.

**Table 8:** Experimental treatment groups and regimes

| Group no. | Treatment | total CFU/ dose | Dose | Regimen | Animals per group |
|---|---|---|---|---|---|
| **1** | Vehicle (5 % dextrose in 0.9% saline) | 0 | 300μl | three times a week | 11 |
| **3** | AUP2059/ AUP5551-C (1:1) | 2x10$^8$ | 300μl | three times a week | 11 |
| **4** | AUP2059/ AUP5551-C (1:1) | 2x10$^7$ | 300μl | three times a week | 11 |
| **5** | AUP2059/ AUP5551-C (1:1) | 2×10$^6$ | 300μl | three times a week | 11 |
| 6 | AUP2059/ AUP5551-C (1:1) | 2×10$^5$ | 300μl | three times a week | 11 |

Treatment was conducted by administration of a combination of drug products AUP2059 (mIL18/mGM-CSF) and AUP5551-C (mIL12/mIL18/mIFNa2b) each obtained in Example 3.
Recombinant bacteria were mixed in a 1 : 1 ratio, based on CFU/mL in a total amount of 2×10$^5$, 2×10$^6$, 2×10$^7$ or 2×10$^8$ CFU per 300μl dose.
Treatment was administered by i.p injection of a 1:1 combination of AUP2059 (mIL18/mGM-CSF) and AUP5551-C2 (mIL12/mIL18/mIFNa2b) in commercial 5% dextrose in 0.9 % saline solution obtained from Dai Han Pharm. CO. (Dai Han, KR).
Dosing was initiated either on Day 0 after inoculation of B16F1 cells.
The mean survival times compared to vehicle treatment are summarized in Figure 29.
As can be seen in Fig. 29 treatment of combination of AUP2059 (mIL18/mGM-CSF) and AUP5551-C2 (mIL12/mIL18/mIFNa2b) lead to a significant increase in survival time compared to vehicle-treated mice.
Syngeneic cancer cell transplantation induced rapid mortality in the vehicle treated control group with all animals dead by day 19.

In comparison, the treatment with a combination of AUP5551 -C2 and AUP2059 increased the survival of mice in a dose-dependent manner. The survival was improved more than 70% with the mean and median survival of 28.3 and 28 days, respectively. These results clearly show that administration of recombinant bacteria of the present invention, e.g. a combination of AUP5551-C2 andAUP2059, is highly effective in delaying tumor growth in syngeneic mice with intraperitoneal (IP) tumors.

**Literature**

Beilharz K, et al. (2015): "Red fluorescent proteins for gene expression and protein localization studies in Streptococcus pneumoniae and efficient transformation with DNA assembled via the gibson assembly method", Appl. Environ. Microbiol. 81(20), pages 7244 to 7252.

Birnboim, H.C.and Doly, J. (1979): "A rapid alkaline extraction procedure for screening recombinant plasmid DNA", Nucleic Acids Res. 7(6), pages 1513 to 1523.

Bron, P.A. et al. (2002): "Use of the air gene as a food-grade selection marker in lactic acid bacteria", Appl. Environ. Microbiol. 68(11), pages 5663 to 5670

Bolotin et al. (2001): "The complete genome sequence of the lactic acid bacterium lactococcus lactis ssp. lactis IL1403", Genome Res. Volume 11, pages 731 to 753.

de Vos, W.M. (1987): "Gene cloning and expression in lactic streptococci", FEMS Microbiol. Lett. 46 (3), page 281 to 295.

de Vos, W.M. and Simons, G. (1994): Gene cloning and expression systems in Lactococci. In Genetics and Biotechnology of Lactic Acid Bacteria. Edited by Gasson MJ, de Vos WM. Oxford: Chapman and Hall; pages 52 to 105 - ISBN: 978-0-7514-0098-4.

Hols, P. et al. (1999): "Conversion of Lactococcus lactis from homolactic to homoalanine fermentation through metabolic engineering", Nat. Biotechnol. 17(6), pages 588 to 592.

Ruhdal Jensen, P. and Hammer, K. (1998): "The Sequence of Spacers between the Consensus Sequences", Appl. Environ. Microbiol. 64(1), pages 82 to 87.

Tan, P.S.T. et al. (1992): "Characterization of the Lactococcus lactis pepN gene encoding an aminopeptidase homologous to mammalian aminopeptidase N", FEBS Lett. 306(1), pages 9 to 16.

Sanders, J.W. et al. (1998): "A chloride-inducible acid resistance mechanism in Lactococcus lactis and its regulation", Mol Microbiol. 27(2) pages 299 to 310.

Sanders, J.W. et al. (1997): "A chloride-inducible gene expression cassette and its use in induced lysis of Lactococcus lactis", Appl. Environ. Microbiol. 63(12), pages 4877 to 4882. van Asseldonk et al. (1993): "Functional analysis of the Lactococcus lactis usp45 secretion signal in the secretion of a homologous proteinase and a heterologous alpha-amylase", Mol. Gen. Genet. 240 (3), 1993, pages 428-434.

van Asseldonk et al. (1990): "Cloning of usp45, a gene encoding a secreted protein from Lactococcus lactis subsp. lactis MG1363", Gene 95 (1), 1990, pages 155 to 160.

Laemmli, U.K. (1970): "Cleavage of structural proteins during the assembly of the head of bacteriophage T4", Nature 227 (5259), pages 680 to 685.

Tauer, C. et al. (2014), "Tuning constitutive recombinant gene expression in Lactobacillus plantarum", Microbial. Cell Factories 13,150, doi: 10.1186/s12934-014-0150-z:

Wang, M. et al. (1995): "Active immunotherapy of cancer with a nonreplicating recombinant fowlpox virus encoding a model tumor-associated antigen", J. Immunol. 154(9), pages 4685 to 4692.

Tomayko, M. and Reynolds, C. (1989): "Determination of subcutaneous tumor size in athymic (nude) mice", Cancer Chemother. Pharmacol. 24(3), pages 148 to 154.

Lechner, M.G. (2013); "Immunogenicity of murine solid tumor models as a defining feature of in vivo behavior and response to immunotherapy", J. Immunother. 36(9), pages 477 to 489.

Fu, Q. et.al (2016): "Novel murine tumour models depend on strain and route of inoculation", Int. J. Exp. Path. (2016), 97, pages 351 to 356.

Lesinski, G.B. et al (2003: "The antitumor effects of IFN-α are abrogated in a STAT1-deficient mouse", J. Clin. Invest. 112(2), pages 170 to 180.

## Claims

**1.** Recombinant bacteria, comprising

a) at least one nucleic acid sequence functionally coupled to a prokaryotic, chloride-inducible promoter and encoding for at least one heterologous factor, said heterologous factor is independently a heterologous polypeptide, or a complex thereof, and
b) at least one prokaryotic regulator gene, which controls activity of said chloride-inducible promoter,
wherein said heterologous polypeptide comprises at least one eukaryotic polypeptide, at least one fragment thereof or a combination thereof, and
wherein said heterologous polypeptide is selected from the group consisting of growth factors, cytokines, chemokines, enzymes, polypeptide hormones, neuropeptides, antibodies, cell surface receptors, soluble receptors, receptor ligands, intrabodies, co-factors, transcription factors, adhesion molecules, tumor antigens, precursors thereof, biologically active fragments thereof and combinations thereof from an eukaryotic species.

**2.** A recombinant nucleic acid, comprising

a) at least one nucleic acid sequence functionally coupled to a prokaryotic, chloride-inducible promoter and encoding for at least one heterologous factor, said heterologous factor is independently a heterologous polypeptide, or a complex thereof, and
b) at least one prokaryotic regulator gene controlling activity of said chloride-inducible promoter,
wherein said heterologous polypeptide comprises a eukaryotic polypeptide, at least one fragment thereof or a combination thereof, and
wherein said heterologous polypeptide is selected from the group consisting of growth factors, cytokines, chemokines, enzymes, polypeptide hormones, neuropeptides, antibodies, cell surface receptors, soluble receptors, receptor ligands, intrabodies, co-factors, transcription factors, adhesion molecules, tumor antigens, precursors thereof, biologically active fragments thereof and combinations thereof from an eukaryotic species.

**3.** The recombinant bacteria according to claim 1, wherein said at least one nucleic acid sequence functionally coupled to a prokaryotic, chloride-inducible promoter and encoding for at least one heterologous factor and/or said at least one procaryotic regulator gene, which controls activity of said chloride-inducible promoter, is/are each independently located on a chromosome and/or at least one plasmid of said recombinant bacteria.

**4.** The recombinant bacteria according to any one of claims 1 or 3 or the recombinant nucleic acid according to claim 2, wherein said chloride-inducible promoter and/or said at least one regulator gene is/are each independently from a, preferably gram-positive or gramnegative, bacterial species.

**5.** The recombinant bacteria according to any one of claims 1, or 3 to 4 or the recombinant nucleic acid according to any one of claims 2 or 4, wherein said chloride-inducible promoter is PgadC from a bacterial species of the taxonomic order Lactobacillales, preferably Lactococcus lactis.

**6.** The recombinant bacteria according to any one of claims 1, or 3 to 4 or the recombinant nucleic acid according to any one of claims 2 or 4, wherein said regulator gene encodes for gadR from a bacterial species of the taxonomic order Lactobacillales, preferably Lactococcus lactis.

7. The recombinant bacteria according to any one of claims 1, or 3 to 6 or the recombinant nucleic acid according to any one of claims 2, or 4 to 6, wherein said chloride-inducible promoter and said at least one regulator gene are arranged in a chloride-inducible gene expression cassette, wherein said chloride-inducible promoter is arranged downstream from said at least one regulator gene, and wherein said chloride-inducible gene expression cassette controls transcription of the at least one nucleic acid sequence encoding for the at least one heterologous factor.

8. The recombinant bacteria or the recombinant nucleic acid according to claim 7, wherein said chloride-inducible gene expression cassette comprises or consists of the nucleic acid sequence of SEQ ID No 2 and controls transcription of the at least one nucleic acid sequence encoding for the at least one heterologous factor.

9. The recombinant bacteria according to any one of claims 1, 3, or 7 to 8 or the recombinant nucleic acid according to any one of claims 2, or 7 to 8, wherein said at least one heterologous factor is selected from the group consisting of polypeptide hormones, growth factors, cytokines, chemokines, enzymes, neuropeptides, antibodies, precursors thereof, fragments thereof and combinations thereof from a mammalian species, further preferably human.

10. The recombinant bacteria according to any one of claims 1, or 3 to 9, further comprising at least one inactivated gene encoding for an essential protein necessary for viability of the recombinant bacteria.

11. The recombinant bacteria according to claim 10, wherein said gene encoding for an essential protein is inactivated by deletion of said gene, mutation of said gene, RNA interference (RNAi) mediated gene silencing of said gene, translational inhibition of said gene, or combinations thereof.

12. The recombinant nucleic acid according to any one of claims 2, or 4 to 9, further comprising at least one gene encoding for an essential protein necessary for viability of a recombinant bacteria.

13. The recombinant bacteria according to any one of claims 10 to 11 or the recombinant nucleic acid according to claim 12, wherein said at least one gene necessary for viability is selected from the group consisting of alanine racemase (alr), thymidylate synthase (thyA), asparagine synthase (asnH), CTP synthase (pyrG), tryptophan synthase (trpBA), and combinations thereof.

14. The nucleic acid according to any one of claims 2, 4 to 9, 12, or 13 wherein said recombinant nucleic acid is in the form of a plasmid.

15. The recombinant bacteria according to any one of claims 1, 3 to 11, or 13, comprising at least one recombinant nucleic acid according to any one of claims 2, 4 to 9, 13 or 14.

16. The recombinant bacteria according to any one of claims 1, 3 to 11, 13 or 15, wherein said recombinant bacteria are non-pathogenic bacteria.

17. The recombinant bacteria according to any one of claims 1, 3 to 11, or 13 to 16, wherein said recombinant bacteria are recombinant bacteria, preferably lactic acid bacteria, further preferably lactobacillus or lactococcus species.

18. The recombinant bacteria according to claim 17, wherein said lactococcus species is *Lactococcus lactis,* preferably *Lactococcus lactis* subspecies cremoris.

19. The recombinant bacteria according to any one of claims 1, 3 to 11, 13 or 15 to 19, for use in medicine.

20. The recombinant bacteria according to any one of claims 1, 3 to 11, 13 or 15 to 19, for use in treatment of a, preferably chronic, inflammatory wound.

21. The recombinant bacteria according to any one of claims 1, 3 to 11, 13 or 15 to 19, for use in treatment of a tumor, preferably a malignant tumor.

22. A pharmaceutical composition comprising recombinant bacteria according to any one of claims 1, 3 to 11, 13 or 15 to 19 and at least one pharmaceutically acceptable excipient.

**23.** Kit for use in medicine, comprising

a) recombinant bacteria according to any one of claims 1, 3 to 11, 13 or 15 to 19, capable of expressing the at least one heterologous factor under the control of the prokaryotic, chloride-inducible promoter, and
b) at least one inducer comprising chloride ions.

**24.** A medical device, comprising

a) recombinant bacteria according to any one of claims 1, 3 to 11, 13 or 15 to 19, capable of expressing the at least one heterologous factor under the control of the prokaryotic, chloride-inducible promoter.

**25.** The pharmaceutical composition according to claim 22 or the kit according to claim 23 or the medical device according to claim 24, wherein said recombinant bacteria are in solution, frozen, or dried, preferably lyophilised or spray dried.

**26.** The pharmaceutical composition according to any one of claims 22 or 25 or the kit according to any one of claims 23 or 25 or the medical device according to any one of claims 24 or 25, wherein said recombinant bacteria are reconstituted in a liquid, preferably culture medium, comprising chloride ions.

**27.** The kit according to any one of claims 23, 25, or 26, wherein the kit is provided as a combined preparation for separate, sequential or simultaneous use in treatment of a, preferably chronic, inflammatory wound.

**28.** The kit according to any one of claims 23, 25, or 27 wherein the kit is provided as a combined preparation for separate, sequential or simultaneous use in treatment of a tumor, preferably a malignant tumor.

**29.** Use of a reconstitution medium comprising chloride ions to reconstitute recombinant bacteria according to any one of claims 1, 3 to 11, 13 or 15 to 18.

## Patentansprüche

**1.** Rekombinante Bakterien, umfassend

a) mindestens eine Nucleinsäuresequenz, die funktionell an einen prokaryotischen, chloridinduzierbaren Promotor gekoppelt ist und für mindestens einen heterologen Faktor kodiert, wobei es sich beim heterologen Faktor in unabhängiger Weise um ein heterologes Polypeptid oder einen Komplex davon handelt, und
b) mindestens ein prokaryotisches Regulatorgen, das die Aktivität des chloridinduzierbaren Promotors steuert, wobei das heterologe Polypeptid mindestens ein eukaryotisches Polypeptid, mindestens ein Fragment davon oder eine Kombination davon umfasst und
wobei das heterologe Polypeptid aus der Gruppe ausgewählt ist, die besteht aus Wachstumsfaktoren, Cytokinen, Chemokinen, Enzymen, Polypeptidhormonen, Neuropeptiden, Antikörpern, Zelloberflächenrezeptoren, löslichen Rezeptoren, Rezeptorliganden, Intrabodies, Cofaktoren, Transkriptionsfaktoren, Adhäsionsmolekülen, Tumorantigenen, Vorläufern davon, biologisch aktiven Fragmenten davon und Kombinationen davon von einer eukaryotischen Spezies.

**2.** Rekombinante Nucleinsäure, umfassend

a) mindestens eine Nucleinsäuresequenz, die funktionell an einen prokaryotischen, chloridinduzierbaren Promotor gekoppelt ist und für mindestens einen heterologen Faktor kodiert, wobei es sich beim heterologen Faktor in unabhängiger Weise um ein heterologes Polypeptid oder einen Komplex davon handelt, und
b) mindestens ein prokaryotisches Regulatorgen, das die Aktivität des chloridinduzierbaren Promotors steuert, wobei das heterologe Polypeptid mindestens ein eukaryotisches Polypeptid, mindestens ein Fragment davon oder eine Kombination davon umfasst und
wobei das heterologe Polypeptid aus der Gruppe ausgewählt ist, die besteht aus Wachstumsfaktoren, Cytokinen, Chemokinen, Enzymen, Polypeptidhormonen, Neuropeptiden, Antikörpern, Zelloberflächenrezeptoren, löslichen Rezeptoren, Rezeptorliganden, Intrabodies, Cofaktoren, Transkriptionsfaktoren, Adhäsionsmolekülen, Tumorantigenen, Vorläufern davon, biologisch aktiven Fragmenten davon und Kombinationen davon von einer eukaryotischen Spezies.

3. Rekombinante Bakterien nach Anspruch 1, wobei die mindestens eine Nucleinsäuresequenz, die funktionell an einen prokaryotischen, chloridinduzierbaren Promotor gekoppelt ist und für mindestens einen heterologen Faktor kodiert, und/oder das mindestens eine prokaryotische Regulatorgen, das die Aktivität des chloridinduzierbaren Promotors steuert, sich jeweils unabhängig voneinander auf einem Chromosom und/oder auf mindestens einem Plasmid der rekombinanten Bakterien befinden

4. Rekombinante Bakterien nach einem der Ansprüche 1 oder 3 oder die rekombinante Nucleinsäure nach Anspruch 2, wobei der chloridinduzierbare Promotor und/oder das mindestens eine Regulatorgen jeweils unabhängig voneinander von einer vorzugsweise gram-positiven oder gram-negativen Bakterienspezies stammen.

5. Rekombinante Bakterien nach einem der Ansprüche 1, 3 oder 4 oder die rekombinante Nucleinsäure nach einem der Ansprüche 2 oder 4, wobei es sich beim chloridinduzierbaren Promotor um PgadC von einer bakteriellen Spezies der taxonomischen Ordnung Lactobacilllales, vorzugsweise Lactococcus lactis, handelt.

6. Rekombinante Bakterien nach einem der Ansprüche 1, 3 oder 4 oder die rekombinante Nucleinsäure nach einem der Ansprüche 2 oder 4, wobei das Regulatorgen für gadR aus einer bakteriellen Spezies der taxonomischen Ordnung Lactobacilllales, vorzugsweise Lactococcus lactis, kodiert.

7. Rekombinante Bakterien nach einem der Ansprüche 1 oder 3 bis 6 oder die rekombinante Nucleinsäure nach einem der Ansprüche 2 oder 4 bis 6, wobei der chloridinduzierbare Promotor und das mindestens eine Regulatorgen in einer chloridinduzierbaren Genexpressionskassette angeordnet sind, wobei der chloridinduzierbare Promotor strangabwärts von dem mindestens einen Regulatorgen angeordnet ist und wobei die chloridinduzierbare Genexpressionskassette die Transkription der mindestens einen Nucleinsäuresequenz, die für den mindestens einen heterologen Faktor kodiert, steuert.

8. Rekombinante Bakterien oder rekombinante Nucleinsäure nach Anspruch 7, wobei die chloridinduzierbare Genexpressionskassette die Nucleinsäuresequenz von SEQ No. 2 umfasst oder aus dieser besteht und die Transkription der mindestens einen Nucleinsäuresequenz, die für den mindestens einen heterologen Faktor kodiert, steuert.

9. Rekombinante Bakterien nach einem der Ansprüche 1, 3 oder 7 bis 8 oder die rekombinante Nucleinsäure nach einem der Ansprüche 2 oder 7 bis 8, wobei der mindestens eine heterologe Faktor aus der Gruppe ausgewählt ist, die besteht aus Polypeptidhormonen, Wachstumsfaktoren, Cytokinen, Chemokinen, Enzymen, Neuropeptiden, Antikörpern, Vorläufern davon, Fragmenten davon und Kombinationen davon von einer Säugetierspezies, vorzugsweise vom Menschen..

10. Rekombinante Bakterien nach einem der Ansprüche 1 oder 3 bis 9, ferner umfassend mindestens ein inaktiviertes Gen, das für ein essentielles Protein, das für die Lebensfähigkeit der rekombinanten Bakterien notwendig ist, kodiert.

11. Rekombinante Bakterien nach Anspruch 10, wobei das für ein essentielles Protein kodierende Gen durch Deletion des Gens, Mutation des Gens, durch RNA-Interferenz (RNAi) vermittelte Genstilllegung des Gens, translationale Hemmung des Gens oder Kombinationen davon inaktiviert worden ist..

12. Rekombinante Nucleinsäure nach einem der Ansprüche 2 oder 4 bis 9, ferner umfassend mindestens ein Gen, das für ein essentielles Protein, das für die Lebensfähigkeit der rekombinanten Bakterien notwendig ist, kodiert.

13. Rekombinante Bakterien nach einem der Ansprüche 10 bis 11 oder die rekombinante Nucleinsäure nach Anspruch 12, wobei das mindestens eine Gen, das für die Lebensfähigkeit notwenidg ist, aus der Gruppe ausgewählt ist, die besteht aus Alanin-racemase (alr), Thymidylat-synthase (thyA), Asparaginsynthase (asnH), CTP-synthase (pyrG), Tryptophan-synthase (trpBA) und Kombinationen davon.

14. Nucleinsäure nach einem der Ansprüche 2, 4 bis 9, 12 oder 13, wobei die rekombinante Nucleinsäure in Form eines Plasmids vorliegt.

15. Rekombinante Bakterien nach einem der Ansprüche 1, 3 bis 11 oder 13, umfassend mindestens eine rekombinante Nucleinsäure nach einem der Ansprüche 2, 4 bis 9, 13 oder 14.

16. Rekombinante Bakterien nach einem der Ansprüche 1, 3 bis 11, 13 oder 15, wobei es sich bei den rekombinanten Bakterien um nicht-pathogene Bakterien handelt.

**17.** Rekombinante Bakterien nach einem der Ansprüche 1, 3 bis 11 oder 13 bis 16, wobei es sich bei den rekombinanten Bakterien um rekombinante Bakterien, vorzugsweise um Milchsäurebakterien und insbesondere um Lactobacillus- oder Lactococcus-Spezies handelt.

**18.** Rekombinante Bakterien nach Anspruch 17, wobei es sich bei der Lactococcus-Spezies um Lactococcus lactis und vorzugsweise um Lactococcus lactis Subspezies cremoris handelt.

**19.** Rekombinante Bakterien nach einem der Ansprüche 1, 3 bis 11, 13 oder 15 bis 19 zur Verwendung in der Medizin.

**20.** Rekombinante Bakterien nach einem der Ansprüche 1, 3 bis 11, 13 oder 15 bis 19 zur Verwendung bei der Behandlung einer, vorzugsweise chronischen, inflammatorischen Wunde.

**21.** Rekombinante Bakterien nach einem der Ansprüche 1, 3 bis 11, 13 oder 15 bis 19 zur Verwendung bei der Behandlung eines Tumors, vorzugsweise eines malignen Tumors.

**22.** Pharmazeutische Zusammensetzung, umfassend rekombinante Bakterien nach einem der Ansprüche 1, 3 bis 11, 13 oder 15 bis 19 und mindestens ein pharmazeutisch verträgliches Exzipiens.

**23.** Kit zur Verwendung in der Medizin, umfassend

a) rekombinante Bakterien nach einem der Ansprüche 1, 3 bis 11, 13 oder 15 bis 19, die zur Expression des mindestens einen heterologen Faktors unter der Steuerung des prokaryotischen, chloridinduzierbaren Promotors befähigt sind, und
b) mindestens einen Chloridionen umfassenden Induktor.

**24.** Medizinische Vorrichtung, umfassend

a) rekombinante Bakterien nach einem der Ansprüche 1, 3 bis 11, 13 oder 15 bis 19, die zur Expression des mindestens einen heterologen Faktors unter der Steuerung des prokaryotischen, chloridinduzierbaren Promotors befähigt sind.

**25.** Pharmazeutische Zusammensetzung nach Anspruch 22 oder Kit nach Anspruch 23 oder medizinische Vorrichtung nach Anspruch 24, wobei sich die rekombinanten Bakterien in Lösung, in gefrorenem Zustand oder in getrockneten Zustand, vorzugsweise in lyophilisiertem oder sprühgetrocknetem Zustand, befinden.

**26.** Pharmazeutische Zusammensetzung nach einem der Ansprüche 22 oder 25 oder Kit nach einem der Ansprüche 23 oder 25 oder medizinische Vorrichtung nach einem der Ansprüche 24 oder 25, wobei die rekombinanten Bakterien in einem Chloridionen enthaltenden Medium, vorzugsweise einem Kulturmedium, rekonstituiert werden.

**27.** Kit nach einem der Ansprüche 23, 25 oder 26, wobei das Kit in Form eines kombinierten Präparats zur getrennten, sequentiellen oder gleichzeitigen Verwendung bei der Behandlung einer, vorzugsweisen chronischen, inflammatorischen Wunde bereitgestellt wird.

**28.** Kit nach einem der Ansprüche 23, 25 oder 27, wobei das Kit in Form eines kombinierten Präparats zur getrennten, sequentiellen oder gleichzeitigen Verwendung bei der Behandlung eines Tumors, vorzugsweise eines malignen Tumors, bereitgestellt wird.

**29.** Verwendung eines Rekonstitutionsmediums, das Chloridionen umfasst, zur Rekonstitution von rekombinanten Bakterien nach einem der Ansprüche 1, 3 bis 11, 13 oder 15 bis 18.

**Revendications**

**1.** Bactéries recombinantes, comprenant

a) au moins une séquence d’acide nucléique couplée de manière fonctionnelle à un promoteur procaryote inductible par le chlore et codant pour au moins un facteur hétérologue, ledit facteur hétérologue étant indépendamment un polypeptide hétérologue, ou un complexe de celui-ci, et

b) au moins un gène de régulation procaryote, qui contrôle l'activité dudit promoteur inductible par le chlore, dans lesquelles ledit polypeptide hétérologue comprend au moins un polypeptide eucaryote, au moins un fragment de celui-ci ou une combinaison de celui-ci, et
dans lesquelles ledit polypeptide hétérologue est sélectionné dans le groupe consistant en des facteurs de croissance, des cytokines, des chimiokines, des enzymes, des hormones polypeptidiques, des neuropeptides, des anticorps, des récepteurs de surface cellulaire, des récepteurs solubles, des ligands de récepteurs, des intracorps, des cofacteurs, des facteurs de transcription, des molécules d'adhésion, des antigènes tumoraux, des précurseurs de ceux-ci, des fragments biologiquement actifs de ceux-ci et des combinaisons de ceux-ci issus d'une espèce eucaryote.

**2.** Un acide nucléique recombinant, comprenant

a) au moins une séquence d'acide nucléique couplée de manière fonctionnelle à un promoteur procaryote inductible par le chlore et codant pour au moins un facteur hétérologue, ledit facteur hétérologue étant indépendamment un polypeptide hétérologue, ou un complexe de celui-ci, et
b) au moins un gène de régulation procaryote contrôlant l'activité dudit promoteur inductible par le chlore, dans lequel ledit polypeptide hétérologue comprend un polypeptide eucaryote, au moins un fragment de celui-ci ou une combinaison de celui-ci, et
dans lequel ledit polypeptide hétérologue est sélectionné dans le groupe consistant en des facteurs de croissance, des cytokines, des chimiokines, des enzymes, des hormones polypeptidiques, des neuropeptides, des anticorps, des récepteurs de surface cellulaire, des récepteurs solubles, des ligands de récepteurs, des intracorps, des cofacteurs, des facteurs de transcription, des molécules d'adhésion, des antigènes tumoraux, des précurseurs de ceux-ci, des fragments biologiquement actifs de ceux-ci et des combinaisons de ceux-ci issus d'une espèce eucaryote.

**3.** Les bactéries recombinantes selon la revendication 1, dans lesquelles ladite au moins une séquence d'acide nucléique couplée de manière fonctionnelle à un promoteur procaryote inductible par le chlore et codant pour au moins un facteur hétérologue et/ou ledit au moins un gène de régulation procaryote, qui contrôle l'activité dudit promoteur inductible par le chlore, est/sont chacun indépendamment localisé(e)(s) sur un chromosome et/ou au moins un plasmide desdites bactéries recombinantes.

**4.** Les bactéries recombinantes selon l'une quelconque des revendications 1 ou 3 ou acide nucléique recombinant selon la revendication 2, dans lesquelles/lequel ledit promoteur inductible par le chlore et/ou ledit au moins un gène de régulation est/sont chacun indépendamment issu(s) d'une espèce bactérienne, de préférence à Gram-positif ou à Gram-négatif.

**5.** Les bactéries recombinantes selon l'une quelconque des revendications 1, ou 3 à 4 ou acide nucléique recombinant selon l'une quelconque des revendications 2 ou 4, dans lesquelles/lequel ledit promoteur inductible par le chlore est PgadC issu d'une espèce bactérienne de l'ordre taxonomique des Lactobacillales, de préférence Lactococcus lactis.

**6.** Les bactéries recombinantes selon l'une quelconque des revendications 1, ou 3 à 4 ou acide nucléique recombinant selon l'une quelconque des revendications 2 ou 4, dans lesquelles/lequel ledit gène de régulation code pour gadR issu d'une espèce bactérienne de l'ordre taxonomique des Lactobacillales, de préférence Lactococcus lactis.

**7.** Les bactéries recombinantes selon l'une quelconque des revendications 1, ou 3 à 6 ou acide nucléique recombinant selon l'une quelconque des revendications 2 ou 4 à 6, dans lesquelles/lequel ledit promoteur inductible par le chlore et ledit au moins un gène de régulation sont arrangés dans une cassette d'expression génique inductible par le chlore, dans lesquelles/lequel ledit promoteur inductible par le chlore est arrangé en aval dudit au moins un gène de régulation, et dans lesquelles/lequel ladite cassette d'expression génique inductible par le chlore contrôle la transcription de la au moins une séquence d'acide nucléique codant pour le au moins un facteur hétérologue.

**8.** Les bactéries recombinantes ou acide nucléique recombinant selon la revendication 7, dans lesquelles/lequel ladite cassette d'expression génique inductible par le chlore comprend ou consiste en la séquence d'acide nucléique de SEQ ID No 2 et contrôle la transcription de la au moins une séquence d'acide nucléique codant pour le au moins un facteur hétérologue.

**9.** Les bactéries recombinantes selon l'une quelconque des revendications 1, 3, ou 7 à 8 ou acide nucléique recombinant selon l'une quelconque des revendications 2, ou 7 à 8, dans lesquelles/lequel ledit au moins un facteur hétérologue est sélectionné dans le groupe consistant en des hormones polypeptidiques, des facteurs de croissance, des cytokines, des chimiokines, des enzymes, des neuropeptides, des anticorps, des précurseurs de ceux-ci, des fragments de ceux-ci et des combinaisons de ceux-ci issus d'une espèce de mammifère, en outre de préférence d'un humain.

**10.** Les bactéries recombinantes selon l'une quelconque des revendications 1, ou 3 à 9, comprenant en outre au moins un gène inactivé codant pour une protéine essentielle nécessaire à la viabilité des bactéries recombinantes.

**11.** Les bactéries recombinantes selon la revendication 10, dans lesquelles ledit gène codant pour une protéine essentielle est inactivé par une délétion dudit gène, une mutation dudit gène, une extinction génique dudit gène médiée par une interférence par ARN (ARNi), une inhibition de la traduction dudit gène, ou des combinaisons de celles-ci.

**12.** L'acide nucléique recombinant selon l'une quelconque des revendications 2, ou 4 à 9, comprenant en outre au moins un gène codant pour une protéine essentielle nécessaire à la viabilité des bactéries recombinantes.

**13.** Les bactéries recombinantes selon l'une quelconque des revendications 10 à 11 ou acide nucléique recombinant selon la revendication 12, dans lesquelles/lequel ledit au moins un gène nécessaire à la viabilité est sélectionné dans le groupe consistant en l'alanine racémase (alr), la thymidylate synthase (thyA), l'asparagine synthase (asnH), la CTP synthase (pyrG), la tryptophane synthase (trpBA), et des combinaisons de ceux-ci.

**14.** L'acide nucléique selon l'une quelconque des revendications 2, 4 à 9, 12, ou 13 dans lequel ledit acide nucléique recombinant est sous la forme d'un plasmide.

**15.** Les bactéries recombinantes selon l'une quelconque des revendications 1, 3 à 11, ou 13, comprenant au moins un acide nucléique recombinant selon l'une quelconque des revendications 2, 4 à 9, 13 ou 14.

**16.** Les bactéries recombinantes selon l'une quelconque des revendications 1, 3 à 11, 13 ou 15, dans lesquelles lesdites bactéries recombinantes sont des bactéries non pathogènes.

**17.** Les bactéries recombinantes selon l'une quelconque des revendications 1, 3 à 11, ou 13 à 16, dans lesquelles lesdites bactéries recombinantes sont des bactéries recombinantes, de préférence des bactéries lactiques, en outre de préférence une espèce lactobacillus ou lactococcus.

**18.** Les bactéries recombinantes selon la revendication 17, dans lesquelles ladite espèce lactococcus est *Lactococcus lactis,* de préférence *Lactococcus lactis* sous-espèce cremoris.

**19.** Les bactéries recombinantes selon l'une quelconque des revendications 1, 3 à 11, 13 ou 15 à 19, pour une utilisation en médecine.

**20.** Les bactéries recombinantes selon l'une quelconque des revendications 1, 3 à 11, 13 ou 15 à 19, pour une utilisation dans le traitement d'une plaie inflammatoire, de préférence chronique.

**21.** Les bactéries recombinantes selon l'une quelconque des revendications 1, 3 à 11, 13 ou 15 à 19, pour une utilisation dans le traitement d'une tumeur, de préférence une tumeur maligne.

**22.** Une composition pharmaceutique comprenant des bactéries recombinantes selon l'une quelconque des revendications 1, 3 à 11, 13 ou 15 à 19 et au moins un excipient pharmaceutiquement acceptable.

**23.** Kit destiné à être utilisé en médecine, comprenant

a) des bactéries recombinantes selon l'une quelconque des revendications 1, 3 à 11, 13 ou 15 à 19, capables d'exprimer le au moins un facteur hétérologue sous le contrôle du promoteur procaryote inductible par le chlore, et
b) au moins un inducteur comprenant des ions chlore.

**24.** Un dispositif médical, comprenant

a) des bactéries recombinantes selon l'une quelconque des revendications 1, 3 à 11, 13 ou 15 à 19, capables d'exprimer le au moins un facteur hétérologue sous le contrôle du promoteur procaryote inductible par le chlore.

**25.** La composition pharmaceutique selon la revendication 22 ou kit selon la revendication 23 ou dispositif médical selon la revendication 24, dans laquelle/lequel lesdites bactéries recombinantes sont en solution, congelées, ou séchées, de préférence lyophilisées ou séchées par atomisation.

**26.** La composition pharmaceutique selon l'une quelconque des revendications 22 ou 25 ou kit selon l'une quelconque des revendications 23 ou 25 ou dispositif médical selon l'une quelconque des revendications 24 ou 25, dans laquelle/lequel lesdites bactéries recombinantes sont reconstituées dans un liquide, de préférence un milieu de culture, comprenant des ions chlore.

**27.** Le kit selon l'une quelconque des revendications 23, 25, ou 26, dans lequel le kit est fourni sous la forme d'une préparation combinée pour une utilisation séparée, séquentielle ou simultanée dans le traitement d'une plaie inflammatoire, de préférence chronique.

**28.** Le kit selon l'une quelconque des revendications 23, 25, ou 27, dans lequel le kit est fourni sous la forme d'une préparation combinée pour une utilisation séparée, séquentielle ou simultanée dans le traitement d'une tumeur, de préférence une tumeur maligne.

**29.** Utilisation d'un milieu de reconstitution comprenant des ions chlore pour reconstituer des bactéries recombinantes selon l'une quelconque des revendications 1, 3 à 11, 13 ou 15 à 18.

**Fig 1a:**

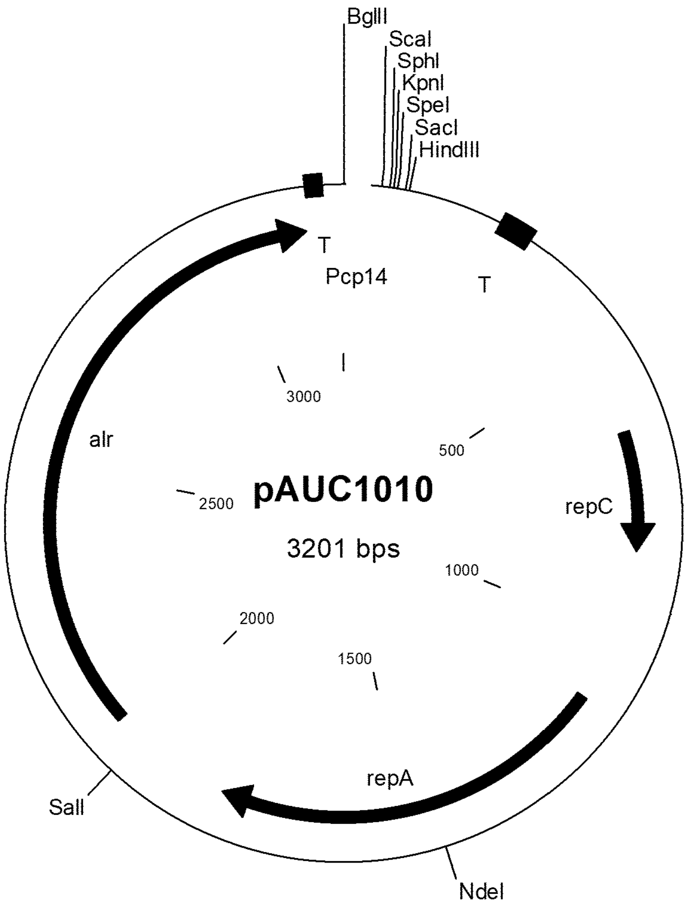


**Fig. 1b:**

```
      BglII
     -+----
  1  agatctcatg acggagttta ttcttgacac aggtatggac ttatgatata ataaaacagt
         >>...............Pcp14...............>>

                       KpnI                 HindIII
                     -----+-               --+----
   ScaI        SphI         SpeI        SacI
  ---+--      ------+      -+----      -----+
 61  actctgcagg catgcggtac cactagttct agagagctca agctttcttt gaaccaaaat

121  tagaaaacca aggcttgaaa cgttcaattg aaatggcaat taaacaaatt acagcacgtg

181  ttgctttgat tgatagccaa aaagcagcag ttgataaagc aattactgat attgctgaaa

241  aattgtaatt tataaataaa aatcaccttt tagaggtggt ttttttattt ataaattatt
          >>.........................T.........................>>

301  cgtttgattt cgctttcgat agaacaatca aatcgtttct gagacgtttt agcgtttatt

361  tcgtttagtt atcggcataa tcgttaaaac aggcgttatc gtagcgtaaa agcccttgag
```

```
 421  cgtagcgtgg ctttgcagcg aagatgttgt ctgttagatt atgaaagccg atgactgaat

 481  gaaataataa gcgcagcgtc cttctatttc ggttggagga ggctcaaggg agtttgaggg

 541  aatgaaattc cctcatgggt ttgattttaa aaattgcttg caattttgcc gagcggtagc

 601  gctggaaaat ttttgaaaaa aatttggaat ttggaaaaaa atgggggaaa aggaagcgaa
                                                  >>......repC........>

 661  ttttgcttcc gtactacgac cccccattaa gtgccgagtg ccaatttttg tgccaaaaac
      >.............................repC..............................>

 721  gctctatccc aactggctca agggtttgag ggtttttca atcgccaacg aatcgccaac
      >.............................repC..............................>

 781  gttttcgcca acgtttttta taaatctata tttaagtagc tttatttttg tttttatgat
      >.............................repC..............................>

 841  tacaaagtga tacactaatt ttataaaatt atttgattgg agtttttaa atggtgattt
      >..repC.>>

 901  cagaatcgaa aaaaagagtt atgatttctc tgacaaaaga gcaagataaa aaattaacag

 961  atatggcgaa acaaaaagat tttcaaaat ctgcggttgc ggcgttagct atagaagaat

1021  atgcaagaaa ggaatcagaa caaaaaaaat aagcgaaagc tcgcgttttt agaaggatac

1081  gagttttcgc tacttgtttt tgataaggta attatatcat ggctattaaa aatactaaag
                                               >>........repA.........>

1141  ctagaaattt tggattttta ttatatcctg actcaattcc taatgattgg aaagaaaaat
      >.............................repA..............................>

1201  tagagagttt gggcgtatct atggctgtca gtcctttaca cgatatggac gaaaaaaaag
      >.............................repA..............................>

1261  ataaagatac atggaatagt agtgatgtta tacgaaatgg aaagcactat aaaaaaccac
      >.............................repA..............................>

1321  actatcacgt tatatatatt gcacgaaatc ctgtaacaat agaaagcgtt aggaacaaga
      >.............................repA..............................>

1381  ttaagcgaaa attggggaat agttcagttg ctcatgttga gatacttgat tatatcaaag
      >.............................repA..............................>

          NdeI
         --+---
1441  gttcatatga atatttgact catgaatcaa aggacgctat tgctaagaat aaacatatat
      >.............................repA..............................>

1501  acgacaaaaa agatattttg aacattaatg attttgatat tgaccgctat ataacacttg
      >.............................repA..............................>

1561  atgaaagcca aaaaagagaa ttgaagaatt tacttttaga tatagtggat gactataatt
      >.............................repA..............................>

1621  tggtaaatac aaaagattta atggctttta ttcgccttag gggagcggag tttggaattt
      >.............................repA..............................>

1681  taaatacgaa tgatgtaaaa gatattgttt caacaaactc tagcgccttt agattatggt
      >.............................repA..............................>
```

```
1741  ttgagggcaa ttatcagtgt ggatatagag caagttatgc aaaggttctt gatgctgaaa
      >.............................repA..............................>

1801  cgggggaaat aaaatgacaa acaaagaaaa agagttattt gctgaaaatg aggaattaaa
      >......repA.....>>

1861  aaaagaaatt aaggacttaa aagagcgtat tgaaagatac agagaaatgg aagttgaatt

1921  aagtacaaca atagatttat tgagaggagg gattattgaa taaataaaag ccccccctgac

           SalI
          -+----
1981  gaaagtcgac atggtcgatg tctagatgct taaactagag aaaggtttaa aagatgaaaa
                                                               >>alr.>

2041  cttcaccaca tcgtaatact tcagctattg ttgatttaaa agcgattaga aataatattg
      >..............................alr..............................>

2101  aaaaatttaa aaagcatatt aaccctaatg cagagatttg gccagcagtg aaagcagatg
      >..............................alr..............................>

2161  cttatggtca tggctcgatt gaggtttcta aagcggtgag cgatttggta ggtggttttt
      >..............................alr..............................>

2221  gtgtatcaaa cctagatgag gcaattgaat tacgaaatca tctggtgact aaaccgattt
      >..............................alr..............................>

2281  tagttttatc cggaattgtt ccagaagatg ttgatattgc agctgccctt aatattagtc
      >..............................alr..............................>

2341  ttactgcccc gagtttagaa tggttgaaat tggttgttca agaagaagca gaactttcag
      >..............................alr..............................>

2401  atttaaaaat tcatattggt gtagattctg gtatgggtcg gattggtatt cgtgatgttg
      >..............................alr..............................>

2461  aagaagctaa tcagatgatt gaacttgctg ataaatatgc gattaatttt gaaggaattt
      >..............................alr..............................>

2521  tcactcattt tgcgactgcg gatatggctg atgaaacaaa atttaaaaat caacaggcaa
      >..............................alr..............................>

2581  gatttaacaa aattatggcc ggattatcac gtcaaccaaa atttatacac tcaactaata
      >..............................alr..............................>

2641  cggccgctgc tttatggcat aaggaacaag ttcaagctat tgaacgttta gggatttcaa
      >..............................alr..............................>

2701  tgtatggctt gaatccaagt ggtaaaactt tggaacttcc ttttgaaatt gaacccgctc
      >..............................alr..............................>

2761  tctctttagt ttctgaattg actcatataa aaaaaatagc tgcaggtgaa acggttggtt
      >..............................alr..............................>

2821  atggtgcaac ttatgagacg agtgaagaaa cttggattgg aactgttcca attggttacg
      >..............................alr..............................>

2881  ctgacgggtg gacccgtcaa atgcaaggtt tcaaagtgct tgttgatgga aagttttgtg
      >..............................alr..............................>

2941  agattgttgg tcgagtttgt atggatcaaa tgatgataaa acttgataag tcttaccctt
      >..............................alr..............................>
```

```
3001  tgggaacgaa ggtcactttg attggtcgag ataaggctaa tgaaatcacg acaacagacg
      >..........................alr............................>

3061  ttgctgattg gcgtggaacg attaattatg aagtgctttg cttactttct gatagaatca
      >..........................alr............................>

3121  aaagaatcta taaataaaat taaaaaaact gtatttttac agttttttttg ttttctgtta
      >......alr......>>
                           >>...........T............>>

3181  aaagcagatg ataacctcac t
```

**Fig. 2a**

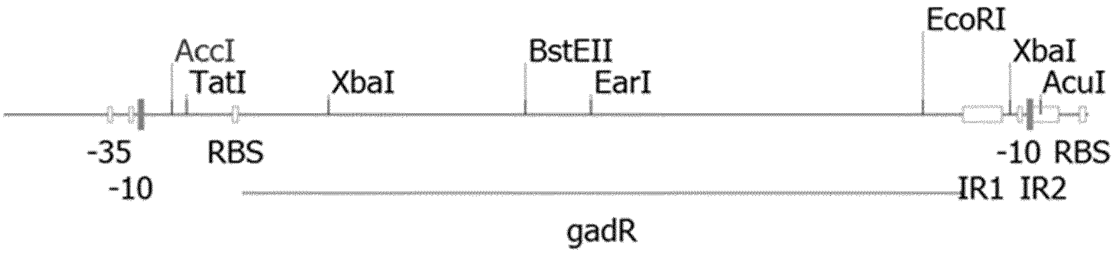


**Fig. 2b:**

```
   1  tgagcgttgt ataagctttt atgtctttct atatcaactt ttaatagaaa tataaagtaa

  61  tataaatgtt tttataataa attatgtgag atatattttt ttgtccgtac tggtatagat

 121  ttgacgatta agtcttaaat aagttataat ctcaattgcg taatttctta aatacagaaa
      >>..>> -35
                          -10 >>..>>
                                       Start <

 181  taacaactac attggtagac tgattaaaaa gtgtacttga tgaactgtta taaaccttaa

 241  aaaaataaaa ataatagttt gggggatgtt aaagatgtat aaaaaatatg gagattgttt
                             >>RBS>>
                                          >>.........gadR..........>

 301  taaaaagttg cgaaaccaaa agaatttagg gttatcatac tttagtaaac ttggaataga
      >..........................gadR............................>

 361  ccgttcaaat atatctagat ttgaacatgg aaaatgtatg atgagttttg agcgtataga
      >..........................gadR............................>

 421  tttgatgtta gaagaaatgc aagttccgtt atctgagtac gaattgattg taaataattt
      >..........................gadR............................>

 481  tatgccgaat ttccaagaat tttttatatt agaattggaa aaagctgaat ttagccaaaa
      >..........................gadR............................>

 541  tcgagataaa ataaaagagt tgtattctga ggtcaaagaa acggggaatc atttactgac
      >..........................gadR............................>

 601  ggttaccgtg aaaacgaagc ttgggaatat aagtcagaca gaagttaaag aaattgaagc
```

```
      >.............................gadR..............................>

 661  ttatctttgc aatattgaag agtggggata ttttgaactt actttatttt attttgtatc
      >.............................gadR..............................>

 721  tgattatctc aatgtcaatc aattagaatt gctgcttttt aattttgata aaagatgtga
      >.............................gadR..............................>

 781  aaattactgt agagtcttaa aatatagaag gagactattg caaatagcct ataaaagtgt
      >.............................gadR..............................>

 841  tgcgatatac gcggctaaag gagaaagaaa aaaagccgaa aatattttag aaatgactaa
      >.............................gadR..............................>

 901  aaaatatcga actgtgggag tcgatttata ttcagaagta ttaagacatc ttgctagagc
      >.............................gadR..............................>

 961  tatcattatt tttaattttg aaaatgcaga gattggggaa gaaaaaataa attatgctct
      >.............................gadR..............................>

1021  tgagattttg gaagaatttg gaggaaagaa gataaaagaa ttctatcaga ataaaatgga
      >.............................gadR..............................>

1081  aaagtatttg aaaaggtcaa tttagtctct tttgagctgt tgctttaaag caacagctca
      >..........gadR..........>>
                               >>................IR1..................>

1141  aaagagattt tctttattct agagcatata ctagagggtg aagataggtt gtctgaagca
      >..IR1.>>
                                  >>-10>>
                                                  >>.......IR2........>
                                        Start >

1201  ttataacttg tcttttaaaa aattcaatca taaatataag gaggtATG
      >.....IR2....>>
                                          >>.RBS.>>
```

## Fig.3

MKKKIISAIL MSTVILSAAA PLSGVYA

## Fig.4

5′ GGAUCACCUCCUUUCU 3′

## Fig. 5a

MAAGSITTLP ALPEDGGSGA FPPGHFKDPK RLYCKNGGFF LRIHPDGRVD
GVREKSDPHI KLQLQAEERG VVSIKGVCAN RYLAMKEDGR LLASKCVTDE
CFFFERLESN NYNTYRSRKY TSWYVALKRT GQYKLGSKTG PGQKAILFLP
MSAKS

## Fig. 5b

AGSITTLPAL PEDGGSGAFP PGHFKDPKRL YCKNGGFFLR IHPDGRVDGV
REKSDPHIKL QLQAEERGVV SIKGVCANRY LAMKEDGRLL ASKCVTDECF
FFERLESNNY NTYRSRKYTS WYVALKRTGQ YKLGSKTGPG QKAILFLPMS
AKS

**Fig. 5c**

MKKKIISAIL MSTVILSAAA PLSGVYAAGS ITTLPALPED GGSGAFPPGH
FKDPKRLYCK NGGFFLRIHP DGRVDGVREK SDPHIKLQLQ AEERGVVSIK
GVCANRYLAM KEDGRLLASK CVTDECFFFE RLESNNYNTY RSRKYTSWYV
ALKRTGQYKL GSKTGPGQKA ILFLPMSAKS

**Fig. 6a**

HKCDITLQEI IKTLNSLTEQ KTLCTELTVT DIFAASKNTT EKETFCRAAT
VLRQFYSHHE KDTRCLGATA QQFHRHKQLI RFLKRLDRNL WGLAGLNSCP
VKEANQSTLE NFLERLKTIM REKYSKCSS

**Fig. 6b**

AHKCDITLQE IIKTLNSLTE QKTLCTELTV TDIFAASKNT TEKETFCRAA
TVLRQFYSHH EKDTRCLGAT AQQFHRHKQL IRFLKRLDRN LWGLAGLNSC
PVKEANQSTL ENFLERLKTI MREKYSKCSS

**Fig. 6c**

MKKKIISAIL MSTVILSAAA PLSGVYAAHK CDITLQEIIK TLNSLTEQKT
LCTELTVTDI FAASKNTTEK ETFCRAATVL RQFYSHHEKD TRCLGATAQQ
FHRHKQLIRF LKRLDRNLWG LAGLNSCPVK EANQSTLENF LERLKTIMRE
KYSKCSS

**Fig. 7a**

EEVSEYCSHM IGSGHLQSLQ RLIDSQMETS CQITFEFVDQ EQLKDPVCYL
KKAFLLVQDI MEDTMRFRDN TPNAIAIVQL QELSLRLKSC FTKDYEEHDK
ACVRTFYETP LQLLEKVKNV FNETKNLLDK DWNIFSKNCN NSFAECSSQD
VVTKPDCNCL YPKAIPSSDP ASVSPHQPLA PSMAPVAGLT WEDSEGTEGS
SLLPGEQPLH TVDPGSAKQR PPRSTCQSFE PPETPVVKDS TIGGSPQPRP

SVGAFNPGME DILDSAMGTN WVPEEASGEA SEIPVPQGTE LSPSRPGGGS
MQTEPARPSN FLSASSPLPA SAKGQQPADV TGTALPRVGP VRPTGQDWNH
TPQKTDHPSA LLRDPPEPGS PRISSLRPQG LSNPSTLSAQ PQLSRSHSSG
SVLPLGELEG RRSTRDRR

## Fig. 7b

AEEVSEYCSH MIGSGHLQSL QRLIDSQMET SCQITFEFVD QEQLKDPVCY
LKKAFLLVQD IMEDTMRFRD NTPNAIAIVQ LQELSLRLKS CFTKDYEEHD
KACVRTFYET PLQLLEKVKN VFNETKNLLD KDWNIFSKNC NNSFAECSSQ
**GHERQSEGS**

## Fig. 7c

MKKKIISAIL MSTVILSAAA PLSGVYAAEE VSEYCSHMIG SGHLQSLQRL
IDSQMETSCQ ITFEFVDQEQ LKDPVCYLKK AFLLVQDIME DTMRFRDNTP
NAIAIVQLQE LSLRLKSCFT KDYEEHDKAC VRTFYETPLQ LLEKVKNVFN
ETKNLLDKDW NIFSKNCNNS FAECSSQGHE RQSEGS

## Figure 8a

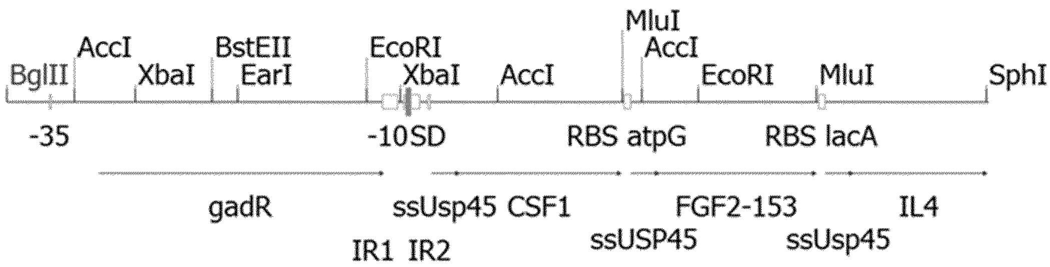


## Figure 8b

```
      BglII
     -+----
  1  agatctgagc gttgtataag cttttatgtc tttctatatc aacttttaat agaaatataa agtaatataa

 71  atgtttttat aataaattat gtgagatata ttttttgtc cgtactggta tagatttgac gattaagtct
                                                                  >>-35>>

141  taaataagtt ataatctcaa ttgcgtaatt tcttaaatac agaaataaca actacattgg tagactgatt

211  aaaaagtgta cttgatgaac tgttataaac cttaaaaaaa taaaaataat agtttggggg atgttaaaga
                                                                        >>.gadR..>

281  tgtataaaaa atatggagat tgttttaaaa agttgcgaaa ccaaaagaat ttagggttat catactttag
     >...............................gadR..................................>
```

```
 351  taaacttgga atagaccgtt caaatatatc tagatttgaa catggaaaat gtatgatgag ttttgagcgt
      >...............................gadR...................................>

 421  atagatttga tgttagaaga aatgcaagtt ccgttatctg agtacgaatt gattgtaaat aattttatgc
      >...............................gadR...................................>

 491  cgaatttcca agaatttttt atattagaat tggaaaaagc tgaatttagc caaaatcgag ataaaataaa
      >...............................gadR...................................>

 561  agagttgtat tctgaggtca aagaaacggg gaatcattta ctgacggtta ccgtgaaaac gaagcttggg
      >...............................gadR...................................>

                                                                  EarI
                                                                 ------
 631  aatataagtc agacagaagt taaagaaatt gaagcttatc tttgcaatat tgaagagtgg ggatattttg
      >...............................gadR...................................>

 701  aacttacttt attttatttt gtatctgatt atctcaatgt caatcaatta gaattgctgc tttttaattt
      >...............................gadR...................................>

 771  tgataaaaga tgtgaaaatt actgtagagt cttaaaatat agaaggagac tattgcaaat agcctataaa
      >...............................gadR...................................>

 841  agtgttgcga tatacgcggc taaaggagaa agaaaaaaag ccgaaaatat tttagaaatg actaaaaaat
      >...............................gadR...................................>

 911  atcgaactgt gggagtcgat ttatattcag aagtattaag acatcttgct agagctatca ttatttttaa
      >...............................gadR...................................>

 981  ttttgaaaat gcagagattg gggaagaaaa aataaattat gctcttgaga ttttggaaga atttggagga
      >...............................gadR...................................>

1051  aagaagataa aagaattcta tcagaataaa atggaaaagt atttgaaaag gtcaatttag tctcttttga
      >..........................gadR............................>>
                                                                    >>...IR1....>

1121  gctgttgctt taaagcaaca gctcaaaaga gattttcttt attctagagc atatactaga gggtgaagat
      >...............IR1..............>>
                                                         -10 >>..>>
                                                                    Start >
                                                                      IR2 >>..>

1191  aggttgtctg aagcattata acttgtcttt taaaaaattc aatcataaat ataaggaggt atgatgaaaa
      >............IR2...........>>
                                                              >>.SD.>>
                                                                 ssUsp45 >>....>

1261  agaaaatcat ttcagcgatt ttgatgtcaa cggttatttt aagcgcagca gctccattat ctggagttta
      >.............................ssUsp45..................................>

                                           NdeI
                                          --+---
1331  tgcagcagaa gaagttagtg agtactgtag tcatatgatt ggttctggac acttacaatc acttcagcgt
      >.>> ssUsp45
         >>..............................CSF1.................................>

1401  cttattgata gtcaaatgga aacctcttgt caaattacat ttgaatttgt agaccaagaa caacttaaag
      >...............................CSF1...................................>

1471  atccagtatg ttatcttaag aaagcttttc ttttagtcca agacataatg gaagatacaa tgagattcag
      >...............................CSF1...................................>

1541  agacaatact cctaacgcta tcgccattgt ccaattacaa gaactttctt taagattgaa aagttgcttc
      >...............................CSF1...................................>

1611  actaaagatt atgaggaaca tgataaagct tgtgttcgaa cattttatga aactcctttg caattattgg
      >...............................CSF1...................................>

1681  aaaaagtgaa aaatgttttc aatgagacga agaatttgtt ggataaagat tggaatatat tcagtaagaa
```

```
      >.................................CSF1.................................>

MluI
                                                                          -+-
---
1751  ttgtaataac tcatttgccg aatgttcaag ccagggtcat gaacgtcaat cagaaggctc ttaataaacg
      >..............................CSF1.............................>>

1821  cgtattaata aggaggctaa ctaatgaaaa aaaagattat ctcagctatt ttaatgtcta cagtgatact
        >>.....RBS atpG......>>
                                >>..................ssUSP45....................>

1891  ttctgctgca gccccgttgt caggtgttta cgctgctggt tccattacga ccttgccggc tttaccagag
      >.............ssUSP45............>>
                                       >>.............FGF2-153...............>

1961  gacggaggtt caggagcctt tccaccaggg cactttaaag atcccaaacg tctatattgt aaaaatggag
      >...............................FGF2-153...............................>

2031  gcttctttct gcgaattcat cctgatggac gtgtagatgg tgtgcgtgag aaaagtgatc ctcatatcaa
      >...............................FGF2-153...............................>

2101  actccaactt caggcagaag aaagaggcgt cgtaagtata aaaggagttt gcgcgaatcg ttacttagct
      >...............................FGF2-153...............................>

2171  atgaaagaag acggtcgatt attggcctct aagtgtgtta ctgatgaatg ttttttttt gaacggcttg
      >...............................FGF2-153...............................>

2241  aatctaataa ttataacact tatagaagca gaaaatatac atcatggtac gttgcactta aaaggacagg
      >...............................FGF2-153...............................>

2311  tcaatataaa ttagggtcta agacaggacc tggtcaaaaa gcaattttgt tcttaccaat gtcggctaaa
      >...............................FGF2-153...............................>

          MluI
        --+----
2381  agttaataaa cgcgtgaaat ttaggaggta gtccaaatga agaaaaagat tattagtgca attttaatgt
      >.....>> FGF2-153
                 >>.....RBS lacA.....>>
                                        >>.............ssUsp45.............>

2451  caacggtcat cttaagcgct gctgccccat tgtcaggtgt ttatgcagca cataagtgtg atataacatt
      >..................ssUsp45..................>>
                                                  >>.........IL4..........>

2521  acaagaaatt atcaaaaccc ttaatagttt aactgaacag aagactttgt gtaccgaatt aactgtaact
      >.................................IL4.................................>

2591  gatatttttg ctgcttctaa aaatacaact gaaaaagaga cattttgtcg agctgccaca gtgttaagac
      >.................................IL4.................................>

2661  aattttacag tcatcatgaa aaagacacaa gatgtcttgg tgctacggca caacaatttc atagacacaa
      >.................................IL4.................................>

2731  acaacttatc cgttttctta aacgtttgga tcgtaatctg tggggcttgg caggattgaa cagttgtcct
      >.................................IL4.................................>

2801  gttaaagaag ccaatcaatc tactcttgaa aatttcttag agagattgaa aacaattatg cgagaaaaat
      >.................................IL4.................................>

                          SphI
                        -----+
2871  attctaagtg ttcatcttaa taagcatgc
```

>..........IL4.........>>

**Fig. 9a**

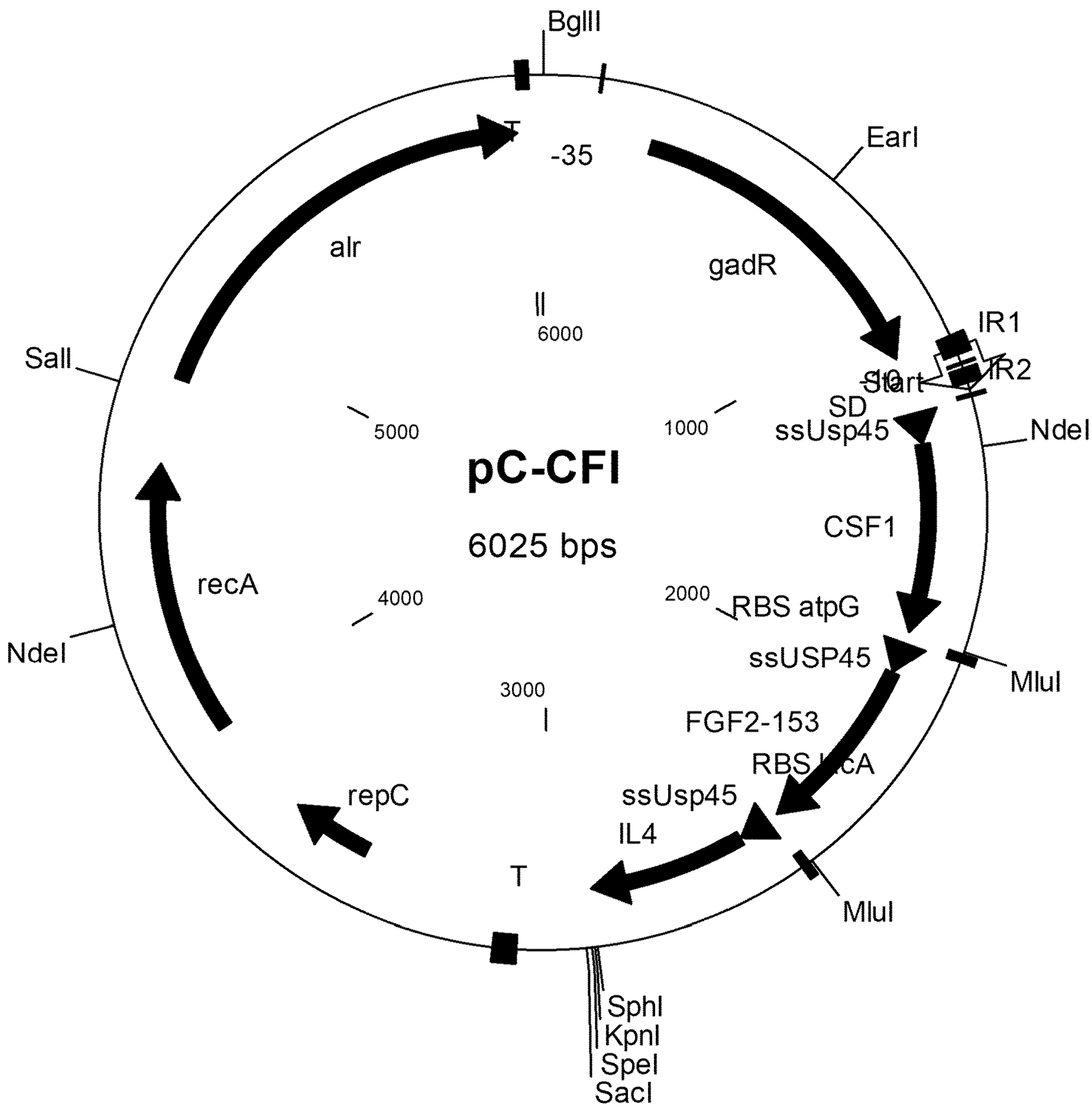


**Fig. 9b**

```
     BglII
    -+----
  1 agatctgagc gttgtataag cttttatgtc tttctatatc aacttttaat agaaatataa agtaatataa

 71 atgtttttat aataaattat gtgagatata ttttttttgtc cgtactggta tagatttgac gattaagtct
                                                                  >>-35>>

141 taaataagtt ataatctcaa ttgcgtaatt tcttaaatac agaaataaca actacattgg tagactgatt

211 aaaaagtgta cttgatgaac tgttataaac cttaaaaaaa taaaaataat agtttggggg atgttaaaga
                                                                        >>.gadR..>

281 tgtataaaaa atatggagat tgttttaaaa agttgcgaaa ccaaaagaat ttagggttat catactttag
    >..............................gadR.................................>

351 taaacttgga atagaccgtt caaatatatc tagatttgaa catggaaaat gtatgatgag ttttgagcgt
    >..............................gadR.................................>
```

```
 421  atagatttga tgttagaaga aatgcaagtt ccgttatctg agtacgaatt gattgtaaat aattttatgc
      >...................................gadR...................................>

 491  cgaatttcca agaatttttt atattagaat tggaaaaagc tgaatttagc caaaatcgag ataaaataaa
      >...................................gadR...................................>

 561  agagttgtat tctgaggtca aagaaacggg gaatcattta ctgacggtta ccgtgaaaac gaagcttggg
      >...................................gadR...................................>

                                                               EarI
                                                              ------
 631  aatataagtc agacagaagt taaagaaatt gaagcttatc tttgcaatat tgaagagtgg ggatattttg
      >...................................gadR...................................>

 701  aacttacttt attttatttt gtatctgatt atctcaatgt caatcaatta gaattgctgc tttttaattt
      >...................................gadR...................................>

 771  tgataaaaga tgtgaaaatt actgtagagt cttaaaatat agaaggagac tattgcaaat agcctataaa
      >...................................gadR...................................>

 841  agtgttgcga tatacgcggc taaaggagaa agaaaaaaag ccgaaaatat tttagaaatg actaaaaaat
      >...................................gadR...................................>

 911  atcgaactgt gggagtcgat ttatattcag aagtattaag acatcttgct agagctatca ttatttttaa
      >...................................gadR...................................>

 981  ttttgaaaat gcagagattg gggaagaaaa aataaattat gctcttgaga ttttggaaga atttggagga
      >...................................gadR...................................>

1051  aagaagataa aagaattcta tcagaataaa atggaaaagt atttgaaaag gtcaatttag tctcttttga
      >............................gadR...............................>>
                                                                     >>...IR1....>

1121  gctgttgctt taaagcaaca gctcaaaaga gattttcttt attctagagc atatactaga gggtgaagat
      >................IR1................>>
                                                          -10 >>..>>
                                                                       Start >
                                                                         IR2 >>..>

1191  aggttgtctg aagcattata acttgtcttt taaaaaattc aatcataaat ataaggaggt atgatgaaaa
      >.............IR2.............>>
                                                               >>.SD.>>
                                                                   ssUsp45 >>....>

1261  agaaaatcat ttcagcgatt ttgatgtcaa cggttatttt aagcgcagca gctccattat ctggagttta
      >................................ssUsp45...................................>

                                         NdeI
                                        --+---
1331  tgcagcagaa gaagttagtg agtactgtag tcatatgatt ggttctggac acttacaatc acttcagcgt
      >.>> ssUsp45
          >>................................CSF1.................................>

1401  cttattgata gtcaaatgga aacctcttgt caaattacat ttgaatttgt agaccaagaa caacttaaag
      >...................................CSF1...................................>

1471  atccagtatg ttatcttaag aaagcttttc ttttagtcca agacataatg gaagatacaa tgagattcag
      >...................................CSF1...................................>

1541  agacaatact cctaacgcta tcgccattgt ccaattacaa gaactttctt taagattgaa aagttgcttc
      >...................................CSF1...................................>

1611  actaaagatt atgaggaaca tgataaagct tgtgttcgaa cattttatga aactcctttg caattattgg
      >...................................CSF1...................................>

1681  aaaaagtgaa aaatgttttc aatgagacga agaatttgtt ggataaagat tggaatatat tcagtaagaa
      >...................................CSF1...................................>
```

```
MluI
                                                                                   -+-
---
 1751   ttgtaataac tcatttgccg aatgttcaag ccagggtcat gaacgtcaat cagaaggctc ttaataaacg
        >.............................CSF1..............................>>

 1821   cgtattaata aggaggctaa ctaatgaaaa aaaagattat ctcagctatt ttaatgtcta cagtgatact
          >>.....RBS atpG......>>
                                >>..................ssUSP45.....................>

 1891   ttctgctgca gccccgttgt caggtgttta cgctgctggt tccattacga ccttgccggc tttaccagag
        >.............ssUSP45............>>
                                          >>............FGF2-153...............>

 1961   gacggaggtt caggagcctt tccaccaggg cactttaaag atcccaaacg tctatattgt aaaaatggag
        >..............................FGF2-153.................................>

 2031   gcttctttct gcgaattcat cctgatggac gtgtagatgg tgtgcgtgag aaaagtgatc ctcatatcaa
        >..............................FGF2-153.................................>

 2101   actccaactt caggcagaag aaagaggcgt cgtaagtata aaaggagttt gcgcgaatcg ttacttagct
        >..............................FGF2-153.................................>

 2171   atgaaagaag acggtcgatt attggcctct aagtgtgtta ctgatgaatg tttttttttt gaacggcttg
        >..............................FGF2-153.................................>

 2241   aatctaataa ttataacact tatagaagca gaaaatatac atcatggtac gttgcactta aaaggacagg
        >..............................FGF2-153.................................>

 2311   tcaatataaa ttagggtcta agacaggacc tggtcaaaaa gcaattttgt tcttaccaat gtcggctaaa
        >..............................FGF2-153.................................>

                 MluI
               --+----
 2381   agttaataaa cgcgtgaaat ttaggaggta gtccaaatga agaaaaagat tattagtgca attttaatgt
        >......>> FGF2-153
                       >>.....RBS lacA.....>>
                                               >>............ssUsp45.............>

 2451   caacggtcat cttaagcgct gctgccccat tgtcaggtgt ttatgcagca cataagtgtg atataacatt
        >...................ssUsp45...................>>
                                                       >>.........IL4..........>

 2521   acaagaaatt atcaaaaccc ttaatagttt aactgaacag aagactttgt gtaccgaatt aactgtaact
        >................................IL4....................................>

 2591   gatatttttg ctgcttctaa aaatacaact gaaaaagaga cattttgtcg agctgccaca gtgttaagac
        >................................IL4....................................>

 2661   aattttacag tcatcatgaa aaagacacaa gatgtcttgg tgctacggca caacaatttc atagacacaa
        >................................IL4....................................>

 2731   acaacttatc cgttttctta aacgtttgga tcgtaatctg tggggcttgg caggattgaa cagttgtcct
        >................................IL4....................................>

 2801   gttaaagaag ccaatcaatc tactcttgaa aatttcttag agagattgaa aacaattatg cgagaaaaat
        >................................IL4....................................>

                                          KpnI
                                        ------+
                                  SphI          SpeI          SacI
                                -----+       -+-----       ------+
 2871   attctaagtg ttcatcttaa taagcatgcg gtaccactag ttctagagag ctcaagcttt ctttgaacca
        >.........IL4........>>

 2941   aaattagaaa accaaggctt gaaacgttca attgaaatgg caattaaaca aattacagca cgtgttgctt

 3011   tgattgatag ccaaaaagca gcagttgata aagcaattac tgatattgct gaaaaattgt aatttataaa
                                                                          >>...T.....>
```

```
3081  taaaaatcac cttttagagg tggttttttt atttataaat tattcgtttg atttcgcttt cgatagaaca
      >...................T...................>>

3151  atcaaatcgt ttctgagacg ttttagcgtt tatttcgttt agttatcggc ataatcgtta aaacaggcgt

3221  tatcgtagcg taaaagccct tgagcgtagc gtggctttgc agcgaagatg ttgtctgtta gattatgaaa

3291  gccgatgact gaatgaaata ataagcgcag cgtccttcta tttcggttgg aggaggctca agggagtttg

3361  agggaatgaa attccctcat gggtttgatt ttaaaaattg cttgcaattt tgccgagcgg tagcgctgga

3431  aaatttttga aaaaaatttg gaatttggaa aaaaatgggg ggaaaggaag cgaattttgc ttccgtacta
                                       >>..............repC................>

3501  cgacccccca ttaagtgccg agtgccaatt tttgtgccaa aaacgctcta tcccaactgg ctcaagggtt
      >...............................repC.................................>

3571  tgaggggttt ttcaatcgcc aacgaatcgc caacgttttc gccaacgttt tttataaatc tatatttaag
      >...............................repC.................................>

3641  tagctttatt tttgttttta tgattacaaa gtgatacact aattttataa aattatttga ttggagtttt
      >..............repC..............>>

3711  ttaaatggtg atttcagaat cgaaaaaaag agttatgatt tctctgacaa aagagcaaga taaaaaatta

3781  acagatatgg cgaaacaaaa agatttttca aaatctgcgg ttgcggcgtt agctatagaa gaatatgcaa

3851  gaaaggaatc agaacaaaaa aaataagcga aagctcgcgt tttagaagg atacgagttt tcgctacttg

3921  tttttgataa ggtaattata tcatggctat taaaaatact aaagctagaa attttggatt tttattatat
                           >>...................recA......................>

3991  cctgactcaa ttcctaatga ttggaaagaa aaattagaga gtttgggcgt atctatggct gtcagtcctt
      >...............................recA.................................>

4061  tacacgatat ggacgaaaaa aaagataaag atacatggaa tagtagtgat gttatacgaa atggaaagca
      >...............................recA.................................>

4131  ctataaaaaa ccacactatc acgttatata tattgcacga aatcctgtaa caatagaaag cgttaggaac
      >...............................recA.................................>


NdeI
                                                                           --
+---
4201  aagattaagc gaaaattggg gaatagttca gttgctcatg ttgagatact tgattatatc aaaggttcat
      >...............................recA.................................>

4271  atgaatattt gactcatgaa tcaaaggacg ctattgctaa gaataaacat atatacgaca aaaaagatat
      >...............................recA.................................>

4341  tttgaacatt aatgattttg atattgaccg ctatataaca cttgatgaaa gccaaaaaag agaattgaag
      >...............................recA.................................>

4411  aatttacttt tagatatagt ggatgactat aatttggtaa atacaaaaga tttaatggct tttattcgcc
      >...............................recA.................................>

4481  ttaggggagc ggagtttgga attttaaata cgaatgatgt aaaagatatt gtttcaacaa actctagcgc
      >...............................recA.................................>

4551  ctttagatta tggtttgagg gcaattatca gtgtggatat agagcaagtt atgcaaaggt tcttgatgct
      >...............................recA.................................>

4621  gaaacggggg aaataaaatg acaaacaaag aaaaagagtt atttgctgaa aatgaggaat taaaaaaaga
      >.......recA.......>>

4691  aattaaggac ttaaaagagc gtattgaaag atacagagaa atggaagttg aattaagtac aacaatagat

                                                                SalI
```

```
                                                                            -+-----
4761  ttattgagag gagggattat tgaataaata aaagcccccc tgacgaaagt cgacatggtc gatgtctaga

4831  tgcttaaact agagaaaggt ttaaaagatg aaaacttcac cacatcgtaa tacttcagct attgttgatt
                                  >>..................alr....................>

4901  taaaagcgat tagaaataat attgaaaaat ttaaaaagca tattaaccct aatgcagaga tttggccagc
      >...............................alr..................................>

4971  agtgaaagca gatgcttatg gtcatggctc gattgaggtt tctaaagcgg tgagcgattt ggtaggtggt
      >...............................alr..................................>

5041  ttttgtgtat caaacctaga tgaggcaatt gaattacgaa atcatctggt gactaaaccg attttagttt
      >...............................alr..................................>

5111  tatccggaat tgttccagaa gatgttgata ttgcagctgc ccttaatatt agtcttactg ccccgagttt
      >...............................alr..................................>

5181  agaatggttg aaattggttg ttcaagaaga agcagaactt tcagatttaa aaattcatat tggtgtagat
      >...............................alr..................................>

5251  tctggtatgg gtcggattgg tattcgtgat gttgaagaag ctaatcagat gattgaactt gctgataaat
      >...............................alr..................................>

5321  atgcgattaa ttttgaagga attttcactc attttgcgac tgcggatatg gctgatgaaa caaaatttaa
      >...............................alr..................................>

5391  aaatcaacag gcaagattta acaaaattat ggccggatta tcacgtcaac caaaatttat acactcaact
      >...............................alr..................................>

5461  aatacggccg ctgctttatg gcataaggaa caagttcaag ctattgaacg tttagggatt tcaatgtatg
      >...............................alr..................................>

5531  gcttgaatcc aagtggtaaa actttggaac ttccttttga aattgaaccc gctctctctt tagtttctga
      >...............................alr..................................>

5601  attgactcat ataaaaaaaa tagctgcagg tgaaacggtt ggttatggtg caacttatga gacgagtgaa
      >...............................alr..................................>

5671  gaaacttgga ttggaactgt tccaattggt tacgctgacg ggtggacccg tcaaatgcaa ggtttcaaag
      >...............................alr..................................>

5741  tgcttgttga tggaaagttt tgtgagattg ttggtcgagt ttgtatggat caaatgatga taaaacttga
      >...............................alr..................................>

5811  taagtcttac cctttgggaa cgaaggtcac tttgattggt cgagataagg ctaatgaaat cacgacaaca
      >...............................alr..................................>

5881  gacgttgctg attggcgtgg aacgattaat tatgaagtgc tttgcttact ttctgataga atcaaaagaa
      >...............................alr..................................>

5951  tctataaata aaattaaaaa aactgtattt ttacagtttt tttgttttct gttaaaagca gatgataacc
      >...alr...>>
                    >>............T............>>

6021  tcact
```

**Fig. 10a**

NFGRLHCTTA VIRNINDQVL FVDKRQPVFE DMTDIDQSAS EPQTRLIIYM YKDSEVRGLA

VTLSVKDSKM STLSCKNKII SFEEMDPPEN IDDIQSDLIF FQKRVPGHNK MEFESSLYEG

HFLACQKEDD AFKLILKKKD ENGDKSVMFT LTNLHQS

**Fig. 10b**

ANFGRLHCTT AVIRNINDQV LFVDKRQPVF EDMTDIDQSA SEPQTRLIIY MYKDSEVRGL
AVTLSVKDSK MSTLSCKNKI ISFEEMDPPE NIDDIQSDLI FFQKRVPGHN KMEFESSLYE
GHFLACQKED DAFKLILKKK DENGDKSVMF TLTNLHQS

**Fig. 10c**

MKKKIISAIL MSTVILSAAA PLSGVYAANF GRLHCTTAVI RNINDQVLFV DKRQPVFEDM
TDIDQSASEP QTRLIIYMYK DSEVRGLAVT LSVKDSKMST LSCKNKIISF EEMDPPENID
DIQSDLIFFQ KRVPGHNKME FESSLYEGHF LACQKEDDAF KLILKKKDEN GDKSVMFTLT
NLHQS

**Fig. 11a**

SAPTRSPITV TRPWKHVEAI KEALNLLDDM PVTLNEEVEV VSNEFSFKKL TCVQTRLKIF
EQGLRGNFTK LKGALNMTAS YYQTYCPPTP ETDCETQVTT YADFIDSLKT FLTDIPFECK
KPGQK

**Fig. 11b**

AAPTRSPITV TRPWKHVEAI KEALNLLDDM PVTLNEEVEV VSNEFSFKKL TCVQTRLKIF
EQGLRGNFTK LKGALNMTAS YYQTYCPPTP ETDCETQVTT YADFIDSLKT FLTDIPFECK
KPGQK

**Fig. 11c**

MKKKIISAIL MSTVILSAAA PLSGVYAAAP TRSPITVTRP WKHVEAIKEA LNLLDDMPVT
LNEEVEVVSN EFSFKKLTCV QTRLKIFEQG LRGNFTKLKG ALNMTASYYQ TYCPPTPETD
CETQVTTYAD FIDSLKTFLT DIPFECKKPG QK

**Fig. 12a**

```
      BglII
     -+----
  1  agatctgagc gttgtataag cttttatgtc tttctatatc aacttttaat agaaatataa agtaatataa

 71  atgtttttat aataaattat gtgagatata tttttttgtc cgtactggta tagatttgac gattaagtct
                                                                   >>-35>>

141  taaataagtt ataatctcaa ttgcgtaatt tcttaaatac agaaataaca actacattgg tagactgatt

211  aaaaagtgta cttgatgaac tgttataaac cttaaaaaaa taaaaataat agtttggggg atgttaaaga
```

```
                                                                            >>.gadR..>

 281  tgtataaaaa atatggagat tgttttaaaa agttgcgaaa ccaaaagaat ttagggttat catactttag
      >...................................gadR...................................>

 351  taaacttgga atagaccgtt caaatatatc tagatttgaa catggaaaat gtatgatgag ttttgagcgt
      >...................................gadR...................................>

 421  atagatttga tgttagaaga aatgcaagtt ccgttatctg agtacgaatt gattgtaaat aattttatgc
      >...................................gadR...................................>

 491  cgaatttcca agaatttttt atattagaat tggaaaaagc tgaatttagc caaaatcgag ataaaataaa
      >...................................gadR...................................>

 561  agagttgtat tctgaggtca aagaaacggg gaatcattta ctgacggtta ccgtgaaaac gaagcttggg
      >...................................gadR...................................>

                                                               EarI
                                                              ------
 631  aatataagtc agacagaagt taaagaaatt gaagcttatc tttgcaatat tgaagagtgg ggatattttg
      >...................................gadR...................................>

 701  aacttacttt attttatttt gtatctgatt atctcaatgt caatcaatta gaattgctgc tttttaattt
      >...................................gadR...................................>

 771  tgataaaaga tgtgaaaatt actgtagagt cttaaaatat agaaggagac tattgcaaat agcctataaa
      >...................................gadR...................................>

 841  agtgttgcga tatacgcggc taaaggagaa agaaaaaaag ccgaaaatat tttagaaatg actaaaaaat
      >...................................gadR...................................>

 911  atcgaactgt gggagtcgat ttatattcag aagtattaag acatcttgct agagctatca ttatttttaa
      >...................................gadR...................................>

 981  ttttgaaaat gcagagattg gggaagaaaa aataaattat gctcttgaga ttttggaaga atttggagga
      >...................................gadR...................................>

1051  aagaagataa aagaattcta tcagaataaa atggaaaagt atttgaaaag gtcaatttag tctcttttga
      >.............................gadR..............................>>
                                                                     >>...IR1....>

1121  gctgttgctt taaagcaaca gctcaaaaga gattttcttt attctagagc atatactaga gggtgaagat
      >................IR1...............>>
                                                          -10 >>..>>
                                                                     Start >
                                                                       IR2 >>..>

1191  aggttgtctg aagcattata acttgtcttt taaaaaattc aatcataaat ataaggaggt atgatgaaga
      >............IR2...........>>
                                                               >>RBS.>>
                                                                   ssUsp45 >>....>

1261  aaaagattat tagtgcaatt ttaatgtcaa cggtcatctt aagcgctgct gccccattgt caggtgttta
      >.................................ssUsp45..................................>

1331  tgcagcaaat tttggtagat tacattgtac aacagcagta atacgtaata ttaatgatca agttttattt
      >.>> ssUsp45
          >>............................mIL18....................................>

1401  gttgataaaa gacaacctgt ttttgaagat atgactgata ttgatcaatc tgcatctgaa ccacaaacta
      >.................................mIL18....................................>

1471  gattaataat ttatatgtat aaagatagtg aagttagagg attagctgta acattaagtg ttaaagatag
      >.................................mIL18....................................>

1541  taaaatgagt acattatcat gtaaaaacaa aataatttca tttgaagaaa tggacccacc tgaaaatatt
      >.................................mIL18....................................>

1611  gatgatattc aatcagattt aattttcttt caaaaacgtg ttccaggtca taacaaaatg gaatttgaat
      >.................................mIL18....................................>
```

```
1681  ctagtttata tgaaggtcac tttttagctt gtcaaaaaga agatgatgct tttaaattaa ttttaaagaa
      >..............................mIL18..................................>

1751  aaaagatgaa aatggagata aatcagttat gtttacatta actaatttac atcaatcata ataaacgcgt
      >..............................mIL18.........................>>
                                                                     RBS atpG >

1821  attaataagg aggctaacta atgaaaaaga aaatcatttc agcgattttg atgtcaacgg ttattttaag
      >......RBS atpG....>>
                          >>.....................ssUsp45..................>

1891  cgcagcagct ccattatctg gagtttatgc agccccaaca cgtagtccaa taacagtaac tagaccttgg
      >............ssUsp45..........>>
                                     >>..............mGM-CSF................>

1961  aaacatgttg aagcaattaa agaagcatta aatttattag atgatatgcc agtaacatta aatgaagaag
      >..............................mGM-CSF................................>

2031  ttgaagtagt aagtaatgaa tttagtttta aaaaattaac atgtgttcaa actagattaa aaatttttga
      >..............................mGM-CSF................................>

2101  acaaggatta agaggtaatt ttactaaatt aaaaggtgct ttaaatatga cagctagtta ttatcaaaca
      >..............................mGM-CSF................................>

2171  tattgtccac caacacctga aactgattgt gaaacacaag taactacata tgctgatttt attgattcat
      >..............................mGM-CSF................................>

                                                                          SphI
                                                                        -----+
2241  taaaaacatt tttaactgat attccttttg aatgtaaaaa accaggacaa aaataataat aaggcatgc
      >........................mGM-CSF.....................>>
```

**Fig. 12b**

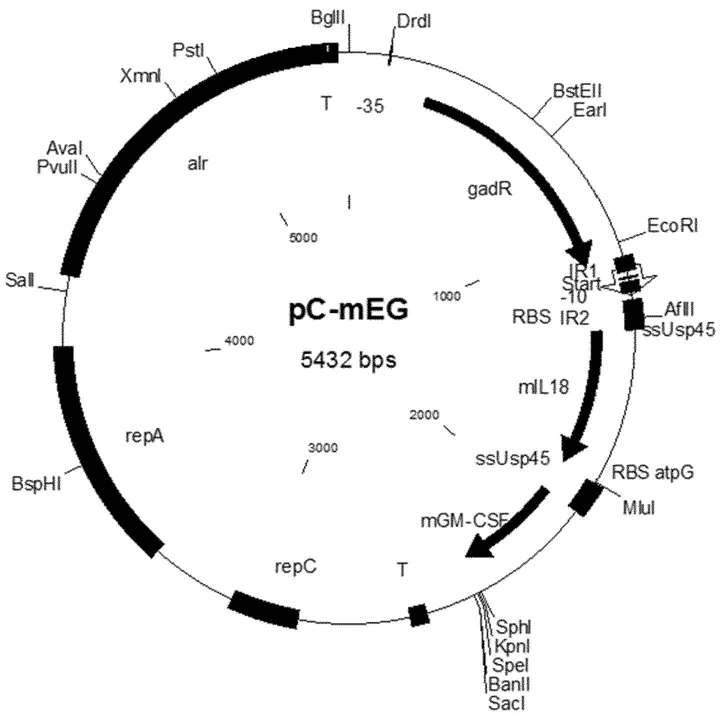


**Fig. 12c**

```
   1  gatctgagcg ttgtataagc ttttatgtct ttctatatca acttttaata gaaatataaa

  61  gtaatataaa tgtttttata ataaattatg tgagatatat tttttgtcc gtactggtat

 121  agatttgacg attaagtctt aaataagtta taatctcaat tgcgtaattt cttaaataca
      -35 >>..>>

 181  gaaataacaa ctacattggt agactgatta aaaagtgtac ttgatgaact gttataaacc

 241  ttaaaaaaat aaaaataata gtttggggga tgttaaagat gtataaaaaa tatggagatt
                                     >>.............gadR..............>

 301  gttttaaaaa gttgcgaaac caaaagaatt tagggttatc atactttagt aaacttggaa
      >.............................gadR..............................>

 361  tagaccgttc aaatatatct agatttgaac atggaaaatg tatgatgagt tttgagcgta
      >.............................gadR..............................>

 421  tagatttgat gttagaagaa atgcaagttc cgttatctga gtacgaattg attgtaaata
      >.............................gadR..............................>

 481  attttatgcc gaatttccaa gaatttttta tattagaatt ggaaaaagct gaatttagcc
      >.............................gadR..............................>

 541  aaaatcgaga taaaataaaa gagttgtatt ctgaggtcaa agaaacgggg aatcatttac
      >.............................gadR..............................>

 601  tgacggttac cgtgaaaacg aagcttggga atataagtca gacagaagtt aaagaaattg
      >.............................gadR..............................>

 661  aagcttatct ttgcaatatt gaagagtggg gatattttga acttacttta ttttattttg
      >.............................gadR..............................>

 721  tatctgatta tctcaatgtc aatcaattag aattgctgct ttttaatttt gataaaagat
      >.............................gadR..............................>

 781  gtgaaaatta ctgtagagtc ttaaaatata gaaggagact attgcaaata gcctataaaa
      >.............................gadR..............................>

 841  gtgttgcgat atacgcggct aaaggagaaa gaaaaaaagc cgaaaatatt ttagaaatga
      >.............................gadR..............................>

 901  ctaaaaaata tcgaactgtg ggagtcgatt tatattcaga agtattaaga catcttgcta
      >.............................gadR..............................>

 961  gagctatcat tatttttaat tttgaaaatg cagagattgg ggaagaaaaa ataaattatg
      >.............................gadR..............................>

1021  ctcttgagat tttggaagaa tttggaggaa agaagataaa agaattctat cagaataaaa
      >.............................gadR..............................>

1081  tggaaaagta tttgaaaagg tcaatttagt ctcttttgag ctgttgcttt aaagcaacag
      >............gadR............>>
                                   >>..............IR1................>

1141  ctcaaaagag atttctttta ttctagagca tatactagag ggtgaagata ggttgtctga
      >....IR1....>>
                                   -10 >>..>>
                                                Start >
                                                      >>.....IR2......>
```

```
1201  agcattataa cttgtctttt aaaaaattca atcataaata taaggaggta tgatgaagaa
      >.......IR2......>>
                                                   >>RBS.>>
                                                       ssUsp45 >>.....>

1261  aaagattatt agtgcaattt taatgtcaac ggtcatctta agcgctgctg ccccattgtc
      >............................ssUsp45............................>

1321  aggtgtttat gcagcaaatt ttggtagatt acattgtaca acagcagtaa tacgtaatat
      >..ssUsp45..>>
                    >>.....................mIL18......................>

1381  taatgatcaa gttttatttg ttgataaaag acaacctgtt tttgaagata tgactgatat
      >.............................mIL18.............................>

1441  tgatcaatct gcatctgaac cacaaactag attaataatt tatatgtata aagatagtga
      >.............................mIL18.............................>

1501  agttagagga ttagctgtaa cattaagtgt taaagatagt aaaatgagta cattatcatg
      >.............................mIL18.............................>

1561  taaaaacaaa ataatttcat ttgaagaaat ggacccacct gaaaatattg atgatattca
      >.............................mIL18.............................>

1621  atcagattta attttctttc aaaaacgtgt tccaggtcat aacaaaatgg aatttgaatc
      >.............................mIL18.............................>

1681  tagtttatat gaaggtcact tttagcttg tcaaaaagaa gatgatgctt ttaaattaat
      >.............................mIL18.............................>

1741  tttaaagaaa aaagatgaaa atggagataa atcagttatg tttacattaa ctaatttaca
      >.............................mIL18.............................>

1801  tcaatcataa taaacgcgta ttaataagga ggctaactaa tgaaaaagaa aatcatttca
      >....>> mIL18
                         >>.....RBS atpG......>>
                                                 >>......ssUsp45......>

1861  gcgattttga tgtcaacggt tattttaagc gcagcagctc cattatctgg agtttatgca
      >............................ssUsp45...........................>>

1921  gccccaacac gtagtccaat aacagtaact agaccttgga aacatgttga agcaattaaa
      >>...........................mGM-CSF............................>

1981  gaagcattaa atttattaga tgatatgcca gtaacattaa atgaagaagt tgaagtagta
      >............................mGM-CSF............................>

2041  agtaatgaat ttagttttaa aaaattaaca tgtgttcaaa ctagattaaa aatttttgaa
      >............................mGM-CSF............................>

2101  caaggattaa gaggtaattt tactaaatta aaaggtgctt taaatatgac agctagttat
      >............................mGM-CSF............................>

2161  tatcaaacat attgtccacc aacacctgaa actgattgtg aaacacaagt aactacatat
      >............................mGM-CSF............................>

2221  gctgatttta ttgattcatt aaaaacattt ttaactgata ttcctttttga atgtaaaaaa
      >............................mGM-CSF............................>

2281  ccaggacaaa aataataagg catgcggtac cactagttct agagagctca agctttcttt
      >..mGM-CSF.>>

2341  gaaccaaaat tagaaaacca aggcttgaaa cgttcaattg aaatggcaat taaacaaatt
```

```
2401  acagcacgtg ttgctttgat tgatagccaa aaagcagcag ttgataaagc aattactgat

2461  attgctgaaa aattgtaatt tataaataaa aatcaccttt tagaggtggt tttttattt
                      >>......................T.......................>

2521  ataaattatt cgtttgattt cgctttcgat agaacaatca aatcgtttct gagacgtttt
      >..T..>>

2581  agcgtttatt tcgtttagtt atcggcataa tcgttaaaac aggcgttatc gtagcgtaaa

2641  agcccttgag cgtagcgtgg ctttgcagcg aagatgttgt ctgttagatt atgaaagccg

2701  atgactgaat gaaataataa gcgcagcgtc cttctatttc ggttggagga ggctcaaggg

2761  agtttgaggg aatgaaattc cctcatgggt ttgattttaa aaattgcttg caattttgcc

2821  gagcggtagc gctggaaaat ttttgaaaaa aatttggaat ttggaaaaaa atgggggaa
                                                             >>.repC..>

2881  aggaagcgaa ttttgcttcc gtactacgac cccccattaa gtgccgagtg ccaatttttg
      >.............................repC..............................>

2941  tgccaaaaac gctctatccc aactggctca agggtttgag gggtttttca atcgccaacg
      >.............................repC..............................>

3001  aatcgccaac gttttcgcca acgtttttta taaatctata tttaagtagc tttattttg
      >.............................repC..............................>

3061  tttttatgat tacaaagtga tacactaatt ttataaaatt atttgattgg agttttttaa
      >.......repC.......>>

3121  atggtgattt cagaatcgaa aaaaagagtt atgatttctc tgacaaaaga gcaagataaa

3181  aaattaacag atatggcgaa acaaaaagat ttttcaaaat ctgcggttgc ggcgttagct

3241  atagaagaat atgcaagaaa ggaatcagaa caaaaaaaat aagcgaaagc tcgcgttttt

3301  agaaggatac gagttttcgc tacttgtttt tgataaggta attatatcat ggctattaaa
                                                          >>..repA....>

3361  aatactaaag ctagaaattt tggattttta ttatatcctg actcaattcc taatgattgg
      >.............................repA..............................>

3421  aaagaaaaat tagagagttt gggcgtatct atggctgtca gtcctttaca cgatatggac
      >.............................repA..............................>

3481  gaaaaaaaag ataaagatac atggaatagt agtgatgtta tacgaaatgg aaagcactat
      >.............................repA..............................>

3541  aaaaaaccac actatcacgt tatatatatt gcacgaaatc ctgtaacaat agaaagcgtt
      >.............................repA..............................>

3601  aggaacaaga ttaagcgaaa attggggaat agttcagttg ctcatgttga gatacttgat
      >.............................repA..............................>

3661  tatatcaaag gttcatatga atatttgact catgaatcaa aggacgctat tgctaagaat
      >.............................repA..............................>

3721  aaacatatat acgacaaaaa agatattttg aacattaatg attttgatat tgaccgctat
      >.............................repA..............................>

3781  ataacacttg atgaaagcca aaaaagagaa ttgaagaatt tacttttaga tatagtggat
```

```
     >.............................repA..............................>

3841 gactataatt tggtaaatac aaaagattta atggctttta ttcgccttag gggagcggag
     >.............................repA..............................>

3901 tttggaattt taaatacgaa tgatgtaaaa gatattgttt caacaaactc tagcgccttt
     >.............................repA..............................>

3961 agattatggt ttgagggcaa ttatcagtgt ggatatagag caagttatgc aaaggttctt
     >.............................repA..............................>

4021 gatgctgaaa cgggggaaat aaaatgacaa acaaagaaaa agagttattt gctgaaaatg
     >...........repA...........>>

4081 aggaattaaa aaaagaaatt aaggacttaa aagagcgtat tgaaagatac agagaaatgg

4141 aagttgaatt aagtacaaca atagatttat tgagaggagg gattattgaa taaataaaag

4201 cccccctgac gaaagtcgac atggtcgatg tctagatgct taaactagag aaaggtttaa

4261 aagatgaaaa cttcaccaca tcgtaatact tcagctattg ttgatttaaa agcgattaga
        >>...........................alr.............................>

4321 aataatattg aaaaatttaa aaagcatatt aaccctaatg cagagatttg gccagcagtg
     >..............................alr..............................>

4381 aaagcagatg cttatggtca tggctcgatt gaggtttcta aagcggtgag cgatttggta
     >..............................alr..............................>

4441 ggtggttttt gtgtatcaaa cctagatgag gcaattgaat tacgaaatca tctggtgact
     >..............................alr..............................>

4501 aaaccgattt tagttttatc cggaattgtt ccagaagatg ttgatattgc agctgccctt
     >..............................alr..............................>

4561 aatattagtc ttactgcccc gagtttagaa tggttgaaat tggttgttca agaagaagca
     >..............................alr..............................>

4621 gaactttcag atttaaaaat tcatattggt gtagattctg gtatgggtcg gattggtatt
     >..............................alr..............................>

4681 cgtgatgttg aagaagctaa tcagatgatt gaacttgctg ataaatatgc gattaatttt
     >..............................alr..............................>

4741 gaaggaattt tcactcattt tgcgactgcg gatatggctg atgaaacaaa atttaaaaat
     >..............................alr..............................>

4801 caacaggcaa gatttaacaa aattatggcc ggattatcac gtcaaccaaa atttatacac
     >..............................alr..............................>

4861 tcaactaata cggccgctgc tttatggcat aaggaacaag ttcaagctat tgaacgttta
     >..............................alr..............................>

4921 gggatttcaa tgtatggctt gaatccaagt ggtaaaactt tggaacttcc ttttgaaatt
     >..............................alr..............................>

4981 gaacccgctc tctctttagt ttctgaattg actcatataa aaaaaatagc tgcaggtgaa
     >..............................alr..............................>

5041 acggttggtt atggtgcaac ttatgagacg agtgaagaaa cttggattgg aactgttcca
     >..............................alr..............................>

5101 attggttacg ctgacgggtg gacccgtcaa atgcaaggtt tcaaagtgct tgttgatgga
```

```
     >..........................alr...........................>

5161 aagttttgtg agattgttgg tcgagtttgt atggatcaaa tgatgataaa acttgataag
     >..........................alr...........................>

5221 tcttaccctt tgggaacgaa ggtcactttg attggtcgag ataaggctaa tgaaatcacg
     >..........................alr...........................>

5281 acaacagacg ttgctgattg gcgtggaacg attaattatg aagtgctttg cttactttct
     >..........................alr...........................>

5341 gatagaatca aaagaatcta taaataaaat taaaaaaact gtatttttac agtttttttg
     >...........alr..........>>
                                  >>...........T............>>

5401 ttttctgtta aaagcagatg ataacctcac ta
```

## Fig. 13a

MWELEKDVYV VEVDWTPDAP GETVNLTCDT PEEDDITWTS DQRHGVIGSG KTLTITVKEF
LDAGQYTCHK GGETLSHSHL LLHKKENGIW STEILKNFKN KTFLKCEAPN YSGRFTCSWL
VQRNMDLKFN IKSSSSSPDS RAVTCGMASL SAEKVTLDQR DYEKYSVSCQ EDVTCPTAEE
TLPIELALEA RQQNKYENYS TSFFIRDIIK PDPPKNLQMK PLKNSQVEVS WEYPDSWSTP
HSYFSLKFFV RIQRKKEKMK ETEEGCNQKG AFLVEKTSTE VQCKGGNVCV QAQDRYYNSS
CSKWACVPCR VR

## Fig. 13b

RVIPVSGPAR CLSQSRNLLK TTDDMVKTAR EKLKHYSCTA EDIDHEDITR DQTSTLKTCL
PLELHKNESC LATRETSSTT RGSCLPPQKT SLMMTLCLGS IYEDLKMYQT EFQAINAALQ
NHNHQQIILD KGMLVAIDEL MQSLNHNGET LRQKPPVGEA DPYRVKMKLC ILLHAFSTRV
VTINRVMGYL SSA

## Fig. 13c

AD MWELEKDV YVVEVDWTPD APGETVNLTC DTPEEDDITW TSDQRHGVIG SGKTLTITVK
EFLDAGQYTC HKGGETLSHS HLLLHKKENG IWSTEILKNF KNKTFLKCEA PNYSGRFTCS
WLVQRNMDLK FNIKSSSSSP DSRAVTCGMA SLSAEKVTLD QRDYEKYSVS CQEDVTCPTA
EETLPIELAL EARQQNKYEN YSTSFFIRDI IKPDPPKNLQ MKPLKNSQVE VSWEYPDSWS
TPHSYFSLKF FVRIQRKKEK MKETEEGCNQ KGAFLVEKTS TEVQCKGGNV CVQAQDRYYN
SSCSKWACVP CRVR**SGGGGS GGGGSGGGGS** RVIPVSGPAR CLSQSRNLLK TTDDMVKTAR
EKLKHYSCTA EDIDHEDITR DQTSTLKTCL PLELHKNESC LATRETSSTT RGSCLPPQKT
SLMMTLCLGS IYEDLKMYQT EFQAINAALQ NHNHQQIILD KGMLVAIDEL MQSLNHNGET
LRQKPPVGEA DPYRVKMKLC ILLHAFSTRV VTINRVMGYL SSA

## Fig. 13d:

MKKKIISAIL MSTVILSAAA PLSGVYAADM WELEKDVYVV EVDWTPDAPG ETVNLTCDTP
EEDDITWTSD QRHGVIGSGK TLTITVKEFL DAGQYTCHKG GETLSHSHLL LHKKENGIWS
TEILKNFKNK TFLKCEAPNY SGRFTCSWLV QRNMDLKFNI KSSSSSPDSR AVTCGMASLS
AEKVTLDQRD YEKYSVSCQE DVTCPTAEET LPIELALEAR QQNKYENYST SFFIRDIIKP
DPPKNLQMKP LKNSQVEVSW EYPDSWSTPH SYFSLKFFVR IQRKKEKMKE TEEGCNQKGA
FLVEKTSTEV QCKGGNVCVQ AQDRYYNSSC SKWACVPCRV RSGGGGSGGG GSGGGGSRVI
PVSGPARCLS QSRNLLKTTD DMVKTAREKL KHYSCTAEDI DHEDITRDQT STLKTCLPLE
LHKNESCLAT RETSSTTRGS CLPPQKTSLM MTLCLGSIYE DLKMYQTEFQ AINAALQNHN
HQQIILDKGM LVAIDELMQS LNHNGETLRQ KPPVGEADPY RVKMKLCILL HAFSTRVVTI
NRVMGYLSSA

**Fig. 14a:**

GCDLPHTYNL RNKRALKVLA QMRRLPFLSC LKDRQDFGFP LEKVDNQQIQ KAQAIPVLRD
LTQQTLNLFT SKASSAAWNT TLLDSFCNDL HQQLNDLQTC LMQQVGVQEP PLTQEDALLA
VRKYFHRITV YLREKKHSPC AWEVVRAEVW RALSSSVNLL PRLSEEKE

**Fig. 14b:**

AGCDLPHTYN LRNKRALKVL AQMRRLPFLS CLKDRQDFGF PLEKVDNQQI QKAQAIPVLR
DLTQQTLNLF TSKASSAAWN TTLLDSFCND LHQQLNDLQT CLMQQVGVQE PPLTQEDALL
AVRKYFHRIT VYLREKKHSP CAWEVVRAEV WRALSSSVNL LPRLSEEKE

**Fig. 14c:**

MKKKIISAIL MSTVILSAAA PLSGVYAAGC DLPHTYNLRN KRALKVLAQM RRLPFLSCLK
DRQDFGFPLE KVDNQQIQKA QAIPVLRDLT QQTLNLFTSK ASSAAWNTTL LDSFCNDLHQ
QLNDLQTCLM QQVGVQEPPL TQEDALLAVR KYFHRITVYL REKKHSPCAW EVVRAEVWRA
LSSSVNLLPR LSEEKE

**Fig. 15a:**

```
       BglII
      -+----
  1   agatctgagc gttgtataag cttttatgtc tttctatatc aacttttaat agaaatataa

 61   agtaatataa atgtttttat aataaattat gtgagatata tttttttgtc cgtactggta

121   tagatttgac gattaagtct taaataagtt ataatctcaa ttgcgtaatt tcttaaatac
       -35 >>...>>

181   agaaataaca actacattgg tagactgatt aaaaagtgta cttgatgaac tgttataaac
```

```
 241  cttaaaaaaa taaaaataat agtttggggg atgttaaaga tgtataaaaa atatggagat
                                         >>............gadR.............>

 301  tgttttaaaa agttgcgaaa ccaaaagaat ttagggttat catactttag taaacttgga
      >.............................gadR..............................>

 361  atagaccgtt caaatatatc tagatttgaa catggaaaat gtatgatgag ttttgagcgt
      >.............................gadR..............................>

 421  atagatttga tgttagaaga aatgcaagtt ccgttatctg agtacgaatt gattgtaaat
      >.............................gadR..............................>

 481  aattttatgc cgaatttcca agaatttttt atattagaat tggaaaaagc tgaatttagc
      >.............................gadR..............................>

 541  caaaatcgag ataaaataaa agagttgtat tctgaggtca aagaaacggg gaatcattta
      >.............................gadR..............................>

 601  ctgacggtta ccgtgaaaac gaagcttggg aatataagtc agacagaagt taaagaaatt
      >.............................gadR..............................>

 661  gaagcttatc tttgcaatat tgaagagtgg ggatattttg aacttacttt attttatttt
      >.............................gadR..............................>

 721  gtatctgatt atctcaatgt caatcaatta gaattgctgc tttttaattt tgataaaaga
      >.............................gadR..............................>

 781  tgtgaaaatt actgtagagt cttaaaatat agaaggagac tattgcaaat agcctataaa
      >.............................gadR..............................>

 841  agtgttgcga tatacgcggc taaaggagaa agaaaaaaag ccgaaaatat tttagaaatg
      >.............................gadR..............................>

 901  actaaaaaat atcgaactgt gggagtcgat ttatattcag aagtattaag acatcttgct
      >.............................gadR..............................>

 961  agagctatca ttatttttaa ttttgaaaat gcagagattg gggaagaaaa aataaattat
      >.............................gadR..............................>

1021  gctcttgaga ttttggaaga atttggagga aagaagataa aagaattcta tcagaataaa
      >.............................gadR..............................>

1081  atggaaaagt atttgaaaag gtcaatttag tctcttttga gctgttgctt taaagcaaca
      >............gadR..............>>
                                    >>.............IR1................>

1141  gctcaaaaga gattttcttt attctagagc atatactaga gggtgaagat aggttgtctg
      >.....IR1....>>
                                    -10 >>..>>
                                                       >>.....IR2.....>
                                                 Start >

1201  aagcattata acttgtcttt taaaaaattc aatcataaat ataaggaggt atgatgaaga
      >.......IR2.......>>
                                                    >>.SD.>>
                                                        ssUSP45 >>....>

1261  aaaagattat tagtgcaatt ttaatgtcaa cggtcatctt aagcgctgct gccccattgt
      >............................ssUSP45............................>

1321  caggtgttta tgcagccgat atgtgggaat tagaaaaaga tgtttatgtt gtagaagttg
      >..ssUSP45...>>
```

```
                    >>....................mIL12......................>

1381  attggacacc agatgctcct ggtgaaacag ttaatttaac atgtgatact ccagaagaag
      >............................mIL12..............................>

1441  atgatattac atggacatct gatcaacgac atggagttat tggtagtgga aaaacattaa
      >............................mIL12..............................>

1501  caattacagt taaagaattt ttagatgctg gacaatatac ttgtcataaa ggaggtgaaa
      >............................mIL12..............................>

1561  cattatctca ttcacattta ttattacata aaaaagaaaa tggtatttgg tcaactgaaa
      >............................mIL12..............................>

1621  tcttaaaaaa ttttaaaaat aagacatttt taaaatgtga agctcctaat tattctggta
      >............................mIL12..............................>

1681  gatttacttg ttcatggtta gttcaaagaa atatggattt aaaatttaat attaaatcaa
      >............................mIL12..............................>

1741  gttcaagtag tcctgatagt agagctgtaa catgtggaat ggcaagttta tctgctgaaa
      >............................mIL12..............................>

1801  aagtaacact tgatcaacgt gattatgaaa aatattctgt tagttgtcaa gaagatgtta
      >............................mIL12..............................>

1861  cttgtccaac agctgaagaa acattaccaa ttgaattagc attagaagct agacaacaaa
      >............................mIL12..............................>

1921  acaaatatga aaattatagt acatctttct ttattcgtga cattattaaa cctgatccac
      >............................mIL12..............................>

1981  ctaaaaattt acaaatgaaa cctttaaaaa atagtcaagt tgaagttagt tgggaatatc
      >............................mIL12..............................>

2041  cagattcatg gtcaacacca cattcatatt tttcattaaa attctttgtt agaattcaac
      >............................mIL12..............................>

2101  gtaaaaaaga aaaaatgaaa gaaactgaag aaggttgtaa ccaaaaaggt gcatttttag
      >............................mIL12..............................>

2161  ttgaaaaaac tagtactgaa gttcaatgta aaggtggtaa tgtatgtgtt caagctcaag
      >............................mIL12..............................>

2221  atcgatatta taattctagt tgttcaaaat gggcatgtgt tccatgtaga gttcgtagtg
      >............................mIL12..............................>

2281  gaggaggtgg aagtggtgga ggtggttcag gtggtggagg ttcacgtgta attccagttt
      >............................mIL12..............................>

2341  caggacctgc tcgatgttta agtcaatctc gaaatttatt aaaaactaca gatgatatgg
      >............................mIL12..............................>

2401  ttaaaactgc tagagaaaaa ttaaaacatt atagttgtac agctgaagat attgatcatg
      >............................mIL12..............................>

2461  aagatattac acgtgatcaa acttcaacac ttaaaacatg tttaccactt gaattacata
      >............................mIL12..............................>

2521  aaaatgaatc ttgtttagct actagagaaa ctagtagtac aactagaggt tcttgtttgc
      >............................mIL12..............................>

2581  ctccacaaaa aacatcatta atgatgacat tatgtttagg ttctatttat gaagatttga
```

```
      >.............................mIL12.............................>

2641  aaatgtatca aactgaattt caagcaatta atgctgcttt acaaaatcat aatcatcaac
      >.............................mIL12.............................>

2701  aaattatttt agataaaggt atgttagttg caattgatga attaatgcaa tctttaaatc
      >.............................mIL12.............................>

2761  ataatggtga aactcttaga caaaaaccac ctgttggaga agcagaccct tatcgagtga
      >.............................mIL12.............................>

2821  aaatgaaatt atgtatcttg ttgcacgctt tttcaacacg ggtagtcaca attaatcgtg
      >.............................mIL12.............................>

2881  taatgggata tttgagtagt gcataataaa cgcgtattaa taaggaggct aactaatgaa
      >........mIL12.........>>
                                           >>.....RBS atpG......>>
                                                        ssUsp45 >>..>

2941  aaagaaaatc atttcagcga ttttgatgtc aacggttatt ttaagcgcag cagctccatt
      >...........................ssUsp45.............................>

3001  atctggagtt tatgcagcaa attttggtag attacattgt acaacagcag taatacgtaa
      >...ssUsp45....>>
                       >>..................mIL-18...................>

3061  tattaatgat caagttttat ttgttgataa aagacaacct gtttttgaag atatgactga
      >............................mIL-18.............................>

3121  tattgatcaa tctgcatctg aaccacaaac tagattaata atttatatgt ataaagatag
      >............................mIL-18.............................>

3181  tgaagttaga ggattagctg taacattaag tgttaaagat agtaaaatga gtacattatc
      >............................mIL-18.............................>

3241  atgtaaaaac aaaataattt catttgaaga aatggaccca cctgaaaata ttgatgatat
      >............................mIL-18.............................>

3301  tcaatcagat ttaattttct ttcaaaaacg tgttccaggt cataacaaaa tggaatttga
      >............................mIL-18.............................>

3361  atctagttta tatgaaggtc actttttagc ttgtcaaaaa gaagatgatg cttttaaatt
      >............................mIL-18.............................>

3421  aattttaaag aaaaaagatg aaaatggaga taaatcagtt atgtttacat taactaattt
      >............................mIL-18.............................>

3481  acatcaatca taataaacgc gtgaaattta ggaggtagtc caaatgaaaa aaaagattat
      >.......>> mIL-18
                               >>.....RBS lacA.....>>
                                                     >>....ssUSP45.....>

3541  ctcagctatt ttaatgtcta cagtgatact ttctgctgca gccccgttgt caggtgttta
      >...........................ssUSP45.............................>

3601  cgctgcctgt gatttaccac atacttataa tttaagaaat aagcgtgctt taaaagtttt
      >.>> ssUSP45
          >>.......................mIFNa.............................>

3661  agcacaaatg agacgtttac catttttaag ttgtttaaaa gatagacaag attttggttt
      >.............................mIFNa.............................>

3721  tccattagaa aaagtagata atcaacaaat tcaaaaagct caagcaattc ctgttttaag
```

```
      >..........................mIFNa............................>

3781  agatttaaca caacaaacat taaatttatt tactagtaaa gctagtagtg cagcttggaa
      >..........................mIFNa............................>

3841  tgcaacatta ttagatagtt tttgtaatga tttacatcaa caattaaatg atttacaaac
      >..........................mIFNa............................>

3901  atgtttaatg caacaagtag gagttcaaga acctccatta actcaagaag atgcattatt
      >..........................mIFNa............................>

3961  agctgttaga aaatattttc atagaataac cgtttattta agagaaaaga aacatagtcc
      >..........................mIFNa............................>

4021  ttgtgcttgg gaagtagtta gagctgaagt ttggagagct ttaagttcat cagttaattt
      >..........................mIFNa............................>

                                                           SphI
                                                          -----+
4081  attacctaga ttaagtgaag aaaaagaata ataaccatgg gtactgcag gcatgc
      >..........mIFNa...........>>
```

**Fig. 15b:**

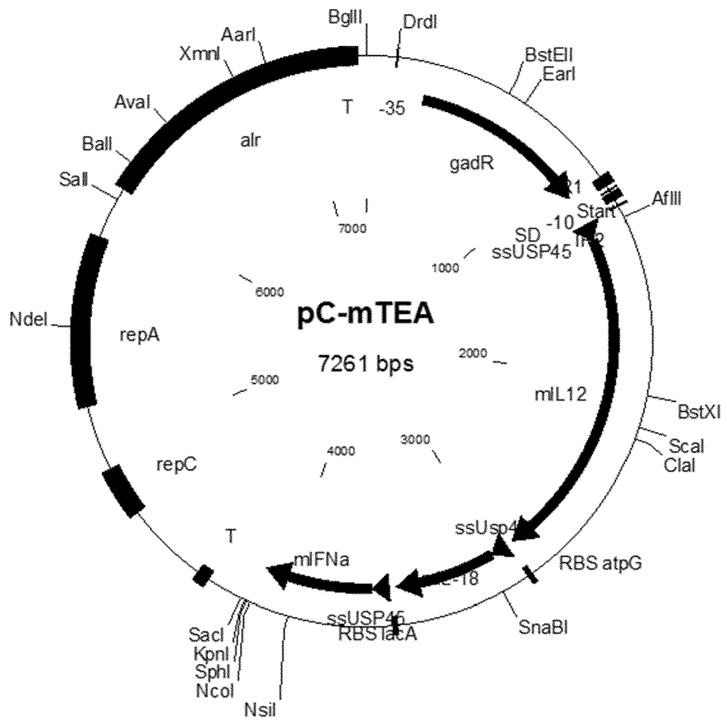


**Fig. 15c:**

```
  1  gatctgagcg ttgtataagc ttttatgtct ttctatatca acttttaata gaaatataaa

 61  gtaatataaa tgtttttata ataaattatg tgagatatat ttttttgtcc gtactggtat

121  agatttgacg attaagtctt aaataagtta taatctcaat tgcgtaattt cttaaataca
```

```
      -35 >>..>>

 181  gaaataacaa ctacattggt agactgatta aaaagtgtac ttgatgaact gttataaacc

 241  ttaaaaaaat aaaaataata gtttggggga tgttaaagat gtataaaaaa tatggagatt
                                     >>.............gadR..............>

 301  gttttaaaaa gttgcgaaac caaaagaatt tagggttatc atactttagt aaacttggaa
      >.............................gadR..............................>

 361  tagaccgttc aaatatatct agatttgaac atggaaaatg tatgatgagt tttgagcgta
      >.............................gadR..............................>

 421  tagatttgat gttagaagaa atgcaagttc cgttatctga gtacgaattg attgtaaata
      >.............................gadR..............................>

 481  attttatgcc gaatttccaa gaatttttta tattagaatt ggaaaaagct gaatttagcc
      >.............................gadR..............................>

 541  aaaatcgaga taaaataaaa gagttgtatt ctgaggtcaa agaaacgggg aatcatttac
      >.............................gadR..............................>

 601  tgacggttac cgtgaaaacg aagcttggga atataagtca gacagaagtt aaagaaattg
      >.............................gadR..............................>

 661  aagcttatct ttgcaatatt gaagagtggg gatattttga acttacttta ttttattttg
      >.............................gadR..............................>

 721  tatctgatta tctcaatgtc aatcaattag aattgctgct ttttaatttt gataaaagat
      >.............................gadR..............................>

 781  gtgaaaatta ctgtagagtc ttaaaatata gaaggagact attgcaaata gcctataaaa
      >.............................gadR..............................>

 841  gtgttgcgat atacgcggct aaaggagaaa gaaaaaaagc cgaaaatatt ttagaaatga
      >.............................gadR..............................>

 901  ctaaaaaata tcgaactgtg ggagtcgatt tatattcaga agtattaaga catcttgcta
      >.............................gadR..............................>

 961  gagctatcat tatttttaat tttgaaaatg cagagattgg ggaagaaaaa ataaattatg
      >.............................gadR..............................>

1021  ctcttgagat tttggaagaa tttggaggaa agaagataaa agaattctat cagaataaaa
      >.............................gadR..............................>

1081  tggaaaagta tttgaaaagg tcaatttagt ctcttttgag ctgttgcttt aaagcaacag
      >............gadR............>>
                                   >>..............IR1................>

1141  ctcaaaagag attttcttta ttctagagca tatactagag ggtgaagata ggttgtctga
      >....IR1....>>
                                   -10 >>..>>
                                                      >>.....IR2......>
                                                Start >

1201  agcattataa cttgtctttt aaaaaattca atcataaata taaggaggta tgatgaagaa
      >.......IR2......>>
                                                   >>.SD.>>
                                                       ssUSP45 >>.....>

1261  aaagattatt agtgcaattt taatgtcaac ggtcatctta agcgctgctg ccccattgtc
      >............................ssUSP45............................>
```

```
1321  aggtgtttat gcagccgata tgtgggaatt agaaaaagat gtttatgttg tagaagttga
      >..ssUSP45..>>
                    >>.....................mIL12......................>

1381  ttggacacca gatgctcctg gtgaaacagt taatttaaca tgtgatactc cagaagaaga
      >.............................mIL12.............................>

1441  tgatattaca tggacatctg atcaacgaca tggagttatt ggtagtggaa aaacattaac
      >.............................mIL12.............................>

1501  aattacagtt aaagaatttt tagatgctgg acaatatact tgtcataaag gaggtgaaac
      >.............................mIL12.............................>

1561  attatctcat tcacatttat tattacataa aaaagaaaat ggtatttggt caactgaaat
      >.............................mIL12.............................>

1621  cttaaaaaat tttaaaaata agacattttt aaaatgtgaa gctcctaatt attctggtag
      >.............................mIL12.............................>

1681  atttacttgt tcatggttag ttcaaagaaa tatggattta aaatttaata ttaaatcaag
      >.............................mIL12.............................>

1741  ttcaagtagt cctgatagta gagctgtaac atgtggaatg gcaagtttat ctgctgaaaa
      >.............................mIL12.............................>

1801  agtaacactt gatcaacgtg attatgaaaa atattctgtt agttgtcaag aagatgttac
      >.............................mIL12.............................>

1861  ttgtccaaca gctgaagaaa cattaccaat tgaattagca ttagaagcta gacaacaaaa
      >.............................mIL12.............................>

1921  caaatatgaa aattatagta catctttctt tattcgtgac attattaaac ctgatccacc
      >.............................mIL12.............................>

1981  taaaaattta caaatgaaac ctttaaaaaa tagtcaagtt gaagttagtt gggaatatcc
      >.............................mIL12.............................>

2041  agattcatgg tcaacaccac attcatattt ttcattaaaa ttctttgtta gaattcaacg
      >.............................mIL12.............................>

2101  taaaaaagaa aaaatgaaag aaactgaaga aggttgtaac caaaaaggtg catttttagt
      >.............................mIL12.............................>

2161  tgaaaaaact agtactgaag ttcaatgtaa aggtggtaat gtatgtgttc aagctcaaga
      >.............................mIL12.............................>

2221  tcgatattat aattctagtt gttcaaaatg ggcatgtgtt ccatgtagag ttcgtagtgg
      >.............................mIL12.............................>

2281  aggaggtgga agtggtggag gtggttcagg tggtggaggt tcacgtgtaa ttccagtttc
      >.............................mIL12.............................>

2341  aggacctgct cgatgtttaa gtcaatctcg aaatttatta aaaactacag atgatatggt
      >.............................mIL12.............................>

2401  taaaactgct agagaaaaat taaaacatta tagttgtaca gctgaagata ttgatcatga
      >.............................mIL12.............................>

2461  agatattaca cgtgatcaaa cttcaacact taaaacatgt ttaccacttg aattacataa
      >.............................mIL12.............................>

2521  aaatgaatct tgtttagcta ctagagaaac tagtagtaca actagaggtt cttgtttgcc
```

```
      >..........................mIL12............................>

2581  tccacaaaaa acatcattaa tgatgacatt atgtttaggt tctatttatg aagatttgaa
      >..........................mIL12............................>

2641  aatgtatcaa actgaatttc aagcaattaa tgctgcttta caaaatcata atcatcaaca
      >..........................mIL12............................>

2701  aattatttta gataaaggta tgttagttgc aattgatgaa ttaatgcaat ctttaaatca
      >..........................mIL12............................>

2761  taatggtgaa actcttagac aaaaaccacc tgttggagaa gcagaccctt atcgagtgaa
      >..........................mIL12............................>

2821  aatgaaatta tgtatcttgt tgcacgcttt ttcaacacgg gtagtcacaa ttaatcgtgt
      >..........................mIL12............................>

2881  aatgggatat ttgagtagtg cataataaac gcgtattaat aaggaggcta actaatgaaa
      >........mIL12........>>
                                  >>.....RBS atpG......>>
                                               ssUsp45 >>...>

2941  aagaaaatca tttcagcgat tttgatgtca acggttattt taagcgcagc agctccatta
      >.........................ssUsp45...........................>

3001  tctggagttt atgcagcaaa ttttggtaga ttacattgta caacagcagt aatacgtaat
      >...ssUsp45...>>
                    >>................mIL-18....................>

3061  attaatgatc aagttttatt tgttgataaa agacaacctg tttttgaaga tatgactgat
      >.........................mIL-18............................>

3121  attgatcaat ctgcatctga accacaaact agattaataa tttatatgta taaagatagt
      >.........................mIL-18............................>

3181  gaagttagag gattagctgt aacattaagt gttaaagata gtaaaatgag tacattatca
      >.........................mIL-18............................>

3241  tgtaaaaaca aaataatttc atttgaagaa atggacccac ctgaaaatat tgatgatatt
      >.........................mIL-18............................>

3301  caatcagatt taattttctt tcaaaaacgt gttccaggtc ataacaaaat ggaatttgaa
      >.........................mIL-18............................>

3361  tctagtttat atgaaggtca ctttttagct tgtcaaaaag aagatgatgc ttttaaatta
      >.........................mIL-18............................>

3421  attttaaaga aaaaagatga aaatggagat aaatcagtta tgtttacatt aactaattta
      >.........................mIL-18............................>

3481  catcaatcat aataaacgcg tgaaatttag gaggtagtcc aaatgaaaaa aaagattatc
      >......>> mIL-18
                       >>.....RBS lacA.....>>
                                              >>....ssUSP45.....>

3541  tcagctattt taatgtctac agtgatactt tctgctgcag ccccgttgtc aggtgtttac
      >.........................ssUSP45...........................>

3601  gctgcctgtg atttaccaca tacttataat ttaagaaata agcgtgcttt aaaagtttta
      >>> ssUSP45
        >>.......................mIFNa...........................>

3661  gcacaaatga gacgtttacc atttttaagt tgtttaaaag atagacaaga ttttggtttt
```

```
      >...........................mIFNa............................>

3721  ccattagaaa aagtagataa tcaacaaatt caaaaagctc aagcaattcc tgttttaaga
      >...........................mIFNa............................>

3781  gatttaacac aacaaacatt aaatttattt actagtaaag ctagtagtgc agcttggaat
      >...........................mIFNa............................>

3841  gcaacattat tagatagttt ttgtaatgat ttacatcaac aattaaatga tttacaaaca
      >...........................mIFNa............................>

3901  tgtttaatgc aacaagtagg agttcaagaa cctccattaa ctcaagaaga tgcattatta
      >...........................mIFNa............................>

3961  gctgttagaa aatattttca tagaataacc gtttatttaa gagaaaagaa acatagtcct
      >...........................mIFNa............................>

4021  tgtgcttggg aagtagttag agctgaagtt tggagagctt taagttcatc agttaattta
      >...........................mIFNa............................>

4081  ttacctagat taagtgaaga aaaagaataa taaccatggg tactgcaggc atgcggtacc
      >..........mIFNa..........>>

4141  actagttcta gagagctcaa gctttctttg aaccaaaatt agaaaaccaa ggcttgaaac

4201  gttcaattga aatggcaatt aaacaaatta cagcacgtgt tgctttgatt gatagccaaa

4261  aagcagcagt tgataaagca attactgata ttgctgaaaa attgtaattt ataaataaaa
                                                        >>......T.......>

4321  atcacctttt agaggtggtt tttttattta taaattattc gtttgatttc gctttcgata
      >................T.................>>

4381  gaacaatcaa atcgtttctg agacgtttta gcgtttattt cgtttagtta tcggcataat

4441  cgttaaaaca ggcgttatcg tagcgtaaaa gcccttgagc gtagcgtggc tttgcagcga

4501  agatgttgtc tgttagatta tgaaagccga tgactgaatg aaataataag cgcagcgtcc

4561  ttctatttcg gttggaggag gctcaaggga gtttgaggga atgaaattcc ctcatgggtt

4621  tgattttaaa aattgcttgc aattttgccg agcggtagcg ctggaaaatt tttgaaaaaa

4681  atttggaatt tggaaaaaaa tggggggaaa ggaagcgaat tttgcttccg tactacgacc
                           >>.................repC...................>

4741  ccccattaag tgccgagtgc caatttttgt gccaaaaacg ctctatccca actggctcaa
      >...........................repC............................>

4801  gggtttgagg ggtttttcaa tcgccaacga atcgccaacg ttttcgccaa cgttttttat
      >...........................repC............................>

4861  aaatctatat ttaagtagct ttatttttgt ttttatgatt acaaagtgat acactaattt
      >......................repC......................>>

4921  tataaaatta tttgattgga gtttttaaa tggtgatttc agaatcgaaa aaaagagtta

4981  tgatttctct gacaaaagag caagataaaa aattaacaga tatggcgaaa caaaaagatt

5041  tttcaaaatc tgcggttgcg gcgttagcta tagaagaata tgcaagaaag gaatcagaac

5101  aaaaaaaata agcgaaagct cgcgttttta gaaggatacg agttttcgct acttgttttt
```

```
5161  gataaggtaa ttatatcatg gctattaaaa atactaaagc tagaaatttt ggatttttat
                        >>...................repA.....................>

5221  tatatcctga ctcaattcct aatgattgga aagaaaaatt agagagtttg ggcgtatcta
      >.............................repA..............................>

5281  tggctgtcag tcctttacac gatatggacg aaaaaaaaga taaagataca tggaatagta
      >.............................repA..............................>

5341  gtgatgttat acgaaatgga aagcactata aaaaaccaca ctatcacgtt atatatattg
      >.............................repA..............................>

5401  cacgaaatcc tgtaacaata gaaagcgtta ggaacaagat taagcgaaaa ttggggaata
      >.............................repA..............................>

5461  gttcagttgc tcatgttgag atacttgatt atatcaaagg ttcatatgaa tatttgactc
      >.............................repA..............................>

5521  atgaatcaaa ggacgctatt gctaagaata aacatatata cgacaaaaaa gatattttga
      >.............................repA..............................>

5581  acattaatga ttttgatatt gaccgctata taacacttga tgaaagccaa aaaagagaat
      >.............................repA..............................>

5641  tgaagaattt acttttagat atagtggatg actataattt ggtaaataca aaagatttaa
      >.............................repA..............................>

5701  tggcttttat tcgccttagg ggagcggagt ttggaatttt aaatacgaat gatgtaaaag
      >.............................repA..............................>

5761  atattgtttc aacaaactct agcgccttta gattatggtt tgagggcaat tatcagtgtg
      >.............................repA..............................>

5821  gatatagagc aagttatgca aaggttcttg atgctgaaac gggggaaata aaatgacaaa
      >...........................repA............................>>

5881  caaagaaaaa gagttatttg ctgaaaatga ggaattaaaa aaagaaatta aggacttaaa

5941  agagcgtatt gaaagataca gagaaatgga agttgaatta agtacaacaa tagatttatt

6001  gagaggaggg attattgaat aaataaaagc ccccctgacg aaagtcgaca tggtcgatgt

6061  ctagatgctt aaactagaga aaggtttaaa agatgaaaac ttcaccacat cgtaatactt
                                         >>...........alr.............>

6121  cagctattgt tgatttaaaa gcgattagaa ataatattga aaaatttaaa aagcatatta
      >..............................alr..............................>

6181  accctaatgc agagatttgg ccagcagtga aagcagatgc ttatggtcat ggctcgattg
      >..............................alr..............................>

6241  aggtttctaa agcggtgagc gatttggtag gtggtttttg tgtatcaaac ctagatgagg
      >..............................alr..............................>

6301  caattgaatt acgaaatcat ctggtgacta aaccgatttt agttttatcc ggaattgttc
      >..............................alr..............................>

6361  cagaagatgt tgatattgca gctgccctta atattagtct tactgccccg agtttagaat
      >..............................alr..............................>

6421  ggttgaaatt ggttgttcaa gaagaagcag aactttcaga tttaaaaatt catattggtg
      >..............................alr..............................>
```

```
6481  tagattctgg tatgggtcgg attggtattc gtgatgttga agaagctaat cagatgattg
      >..............................alr..............................>

6541  aacttgctga taaatatgcg attaattttg aaggaatttt cactcatttt gcgactgcgg
      >..............................alr..............................>

6601  atatggctga tgaaacaaaa tttaaaaatc aacaggcaag atttaacaaa attatggccg
      >..............................alr..............................>

6661  gattatcacg tcaaccaaaa tttatacact caactaatac ggccgctgct ttatggcata
      >..............................alr..............................>

6721  aggaacaagt tcaagctatt gaacgtttag ggatttcaat gtatggcttg aatccaagtg
      >..............................alr..............................>

6781  gtaaaacttt ggaacttcct tttgaaattg aacccgctct ctctttagtt tctgaattga
      >..............................alr..............................>

6841  ctcatataaa aaaaatagct gcaggtgaaa cggttggtta tggtgcaact tatgagacga
      >..............................alr..............................>

6901  gtgaagaaac ttggattgga actgttccaa ttggttacgc tgacgggtgg acccgtcaaa
      >..............................alr..............................>

6961  tgcaaggttt caaagtgctt gttgatggaa agttttgtga gattgttggt cgagtttgta
      >..............................alr..............................>

7021  tggatcaaat gatgataaaa cttgataagt cttacccttt gggaacgaag gtcactttga
      >..............................alr..............................>

7081  ttggtcgaga taaggctaat gaaatcacga caacagacgt tgctgattgg cgtggaacga
      >..............................alr..............................>

7141  ttaattatga agtgctttgc ttactttctg atagaatcaa aagaatctat aaataaaatt
      >............................alr...........................>>

7201  aaaaaaactg tatttttaca gttttttgt tttctgttaa aagcagatga taacctcact
      >>............T............>>

7261  a
```

## Figure 16a

```
       BglII
      -+----
   1  agatctgagc gttgtataag cttttatgtc tttctatatc aacttttaat agaaatataa

  61  agtaatataa atgtttttat aataaattat gtgagatata ttttttgtc cgtactggta

 121  tagatttgac gattaagtct taaataagtt ataatctcaa ttgcgtaatt tcttaaatac
        -35 >>..>>

 181  agaaataaca actacattgg tagactgatt aaaaagtgta cttgatgaac tgttataaac

 241  cttaaaaaaa taaaaataat agtttggggg atgttaaaga tgtataaaaa atatggagat
                                      >>.............gadR.............>

 301  tgttttaaaa agttgcgaaa ccaaaagaat ttagggttat catactttag taaacttgga
      >.............................gadR..............................>

 361  atagaccgtt caaatatatc tagatttgaa catggaaaat gtatgatgag ttttgagcgt
      >.............................gadR..............................>
```

```
 421  atagatttga tgttagaaga aatgcaagtt ccgttatctg agtacgaatt gattgtaaat
      >.............................gadR..............................>

 481  aattttatgc cgaatttcca agaatttttt atattagaat tggaaaaagc tgaatttagc
      >.............................gadR..............................>

 541  caaaatcgag ataaaataaa agagttgtat tctgaggtca aagaaacggg gaatcattta
      >.............................gadR..............................>

 601  ctgacggtta ccgtgaaaac gaagcttggg aatataagtc agacagaagt taaagaaatt
      >.............................gadR..............................>

 661  gaagcttatc tttgcaatat tgaagagtgg ggatattttg aacttacttt attttatttt
      >.............................gadR..............................>

 721  gtatctgatt atctcaatgt caatcaatta gaattgctgc tttttaattt tgataaaaga
      >.............................gadR..............................>

 781  tgtgaaaatt actgtagagt cttaaaatat agaaggagac tattgcaaat agcctataaa
      >.............................gadR..............................>

 841  agtgttgcga tatacgcggc taaaggagaa agaaaaaaag ccgaaaatat tttagaaatg
      >.............................gadR..............................>

 901  actaaaaaat atcgaactgt gggagtcgat ttatattcag aagtattaag acatcttgct
      >.............................gadR..............................>

 961  agagctatca ttatttttaa ttttgaaaat gcagagattg gggaagaaaa aataaattat
      >.............................gadR..............................>

1021  gctcttgaga ttttggaaga atttggagga aagaagataa aagaattcta tcagaataaa
      >.............................gadR..............................>

1081  atggaaaagt atttgaaaag gtcaatttag tctcttttga gctgttgctt taaagcaaca
      >............gadR..............>>
                                    >>..............IR1...............>

1141  gctcaaaaga gattttcttt attctagagc atatactaga gggtgaagat aggttgtctg
      >....IR1.....>>
                                    -10 >>..>>
                                                       >>.....IR2.....>
                                                 Start >

1201  aagcattata acttgtcttt taaaaaattc aatcataaat ataaggaggt atgatgaaga
      >.......IR2.......>>
                                                    >>.SD.>>
                                                        ssUsp45 >>....>

1261  aaaagattat tagtgcaatt ttaatgtcaa cggtcatctt aagcgctgct gccccattgt
      >............................ssUsp45............................>

1321  caggtgttta tgcagcccca acacgtagtc caataacagt aactagacct tggaaacatg
      >...ssUsp45..>>
                     >>....................mGM-CSF....................>

1381  ttgaagcaat taaagaagca ttaaatttat tagatgatat gccagtaaca ttaaatgaag
      >............................mGM-CSF............................>

1441  aagttgaagt agtaagtaat gaatttagtt ttaaaaaatt aacatgtgtt caaactagat
      >............................mGM-CSF............................>

1501  taaaaatttt tgaacaagga ttaagaggta attttactaa attaaaaggt gctttaaata
```

```
      >............................mGM-CSF............................>

1561  tgacagctag ttattatcaa acatattgtc caccaacacc tgaaactgat tgtgaaacac
      >............................mGM-CSF............................>

1621  aagtaactac atatgctgat tttattgatt cattaaaaac atttttaact gatattcctt
      >............................mGM-CSF............................>

1681  ttgaatgtaa aaaaccagga caaaaataat aaacgcgtat taataaggag gctaactaat
      >.........mGM-CSF.........>>
                                              >>.....RBS atpG......>>
                                                             ssUsp45 >>

1741  gaaaaagaaa atcatttcag cgattttgat gtcaacggtt attttaagcg cagcagctcc
      >............................ssUsp45............................>

1801  attatctgga gtttatgcag ccgatatgtg ggaattagaa aaagatgttt atgttgtaga
      >......ssUsp45....>>
                          >>..................mIL12...................>

1861  agttgattgg acaccagatg ctcctggtga aacagttaat ttaacatgtg atactccaga
      >.............................mIL12.............................>

1921  agaagatgat attacatgga catctgatca acgacatgga gttattggta gtggaaaaac
      >.............................mIL12.............................>

1981  attaacaatt acagttaaag aatttttaga tgctggacaa tatacttgtc ataaaggagg
      >.............................mIL12.............................>

2041  tgaaacatta tctcattcac atttattatt acataaaaaa gaaaatggta tttggtcaac
      >.............................mIL12.............................>

2101  tgaaatctta aaaaatttta aaaataagac atttttaaaa tgtgaagctc ctaattattc
      >.............................mIL12.............................>

2161  tggtagattt acttgttcat ggttagttca aagaaatatg gatttaaaat ttaatattaa
      >.............................mIL12.............................>

2221  atcaagttca agtagtcctg atagtagagc tgtaacatgt ggaatggcaa gtttatctgc
      >.............................mIL12.............................>

2281  tgaaaaagta acacttgatc aacgtgatta tgaaaaatat tctgttagtt gtcaagaaga
      >.............................mIL12.............................>

2341  tgttacttgt ccaacagctg aagaaacatt accaattgaa ttagcattag aagctagaca
      >.............................mIL12.............................>

2401  acaaaacaaa tatgaaaatt atagtacatc tttctttatt cgtgacatta ttaaacctga
      >.............................mIL12.............................>

2461  tccacctaaa aatttacaaa tgaaaccttt aaaaaatagt caagttgaag ttagttggga
      >.............................mIL12.............................>

2521  atatccagat tcatggtcaa caccacattc atatttttca ttaaaattct ttgttagaat
      >.............................mIL12.............................>

2581  tcaacgtaaa aaagaaaaaa tgaaagaaac tgaagaaggt tgtaaccaaa aaggtgcatt
      >.............................mIL12.............................>

2641  tttagttgaa aaaactagta ctgaagttca atgtaaaggt ggtaatgtat gtgttcaagc
      >.............................mIL12.............................>

2701  tcaagatcga tattataatt ctagttgttc aaaatgggca tgtgttccat gtagagttcg
```

>.............................mIL12.............................>

2761 tagtggagga ggtggaagtg gtggaggtgg ttcaggtggt ggaggttcac gtgtaattcc
>.............................mIL12.............................>

2821 agtttcagga cctgctcgat gtttaagtca atctcgaaat ttattaaaaa ctacagatga
>.............................mIL12.............................>

2881 tatggttaaa actgctagag aaaaattaaa acattatagt tgtacagctg aagatattga
>.............................mIL12.............................>

2941 tcatgaagat attacacgtg atcaaacttc aacacttaaa acatgtttac cacttgaatt
>.............................mIL12.............................>

3001 acataaaaat gaatcttgtt tagctactag agaaactagt agtacaacta gaggttcttg
>.............................mIL12.............................>

3061 tttgcctcca caaaaaacat cattaatgat gacattatgt ttaggttcta tttatgaaga
>.............................mIL12.............................>

3121 tttgaaaatg tatcaaactg aatttcaagc aattaatgct gctttacaaa atcataatca
>.............................mIL12.............................>

3181 tcaacaaatt attttagata aaggtatgtt agttgcaatt gatgaattaa tgcaatcttt
>.............................mIL12.............................>

3241 aaatcataat ggtgaaactc ttagacaaaa accacctgtt ggagaagcag acccttatcg
>.............................mIL12.............................>

3301 agtgaaaatg aaattatgta tcttgttgca cgctttttca acacgggtag tcacaattaa
>.............................mIL12.............................>

3361 tcgtgtaatg ggatatttga gtagtgcata ataaacgcgt gaaatttagg aggtagtcca
>..........mIL12............>>
>>....RBS lacA....>>

3421 aatgaaaaaa aagattatct cagctatttt aatgtctaca gtgatacttt ctgctgcagc
>>...........................ssUSP45............................>

3481 cccgttgtca ggtgtttacg ctgcaaattt tggtagatta cattgtacaa cagcagtaat
>......ssUSP45........>>
>>................mIL-18................>

3541 acgtaatatt aatgatcaag ttttatttgt tgataaaaga caacctgttt ttgaagatat
>............................mIL-18.............................>

3601 gactgatatt gatcaatctg catctgaacc acaaactaga ttaataattt atatgtataa
>............................mIL-18.............................>

3661 agatagtgaa gttagaggat tagctgtaac attaagtgtt aaagatagta aaatgagtac
>............................mIL-18.............................>

3721 attatcatgt aaaaacaaaa taatttcatt tgaagaaatg gacccacctg aaaatattga
>............................mIL-18.............................>

3781 tgatattcaa tcagatttaa ttttctttca aaaacgtgtt ccaggtcata acaaaatgga
>............................mIL-18.............................>

3841 atttgaatct agtttatatg aaggtcactt tttagcttgt caaaaagaag atgatgcttt
>............................mIL-18.............................>

3901 taaattaatt ttaaagaaaa aagatgaaaa tggagataaa tcagttatgt ttacattaac
>............................mIL-18.............................>

```
                                               SphI
                                              -----+
 3961  taatttacat caatcataat aaccatgggt actgcaggca tgc
       >....mIL-18....>>
```

## Figure 16b

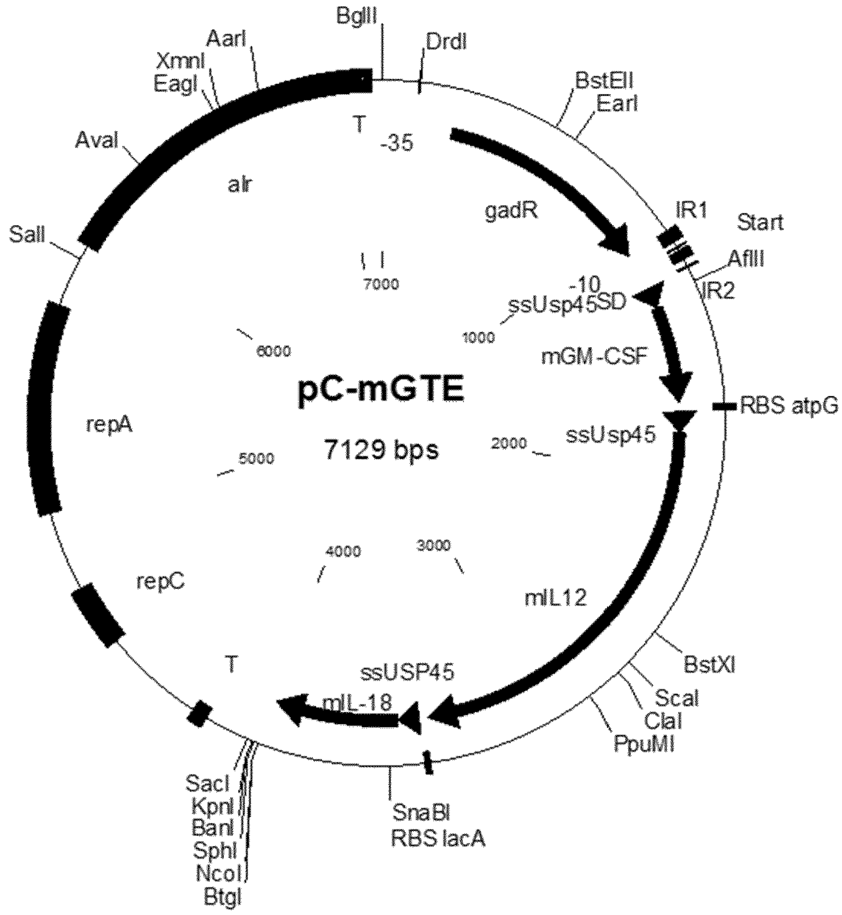


## Figure 16c

```
   1  gatctgagcg ttgtataagc ttttatgtct ttctatatca acttttaata gaaatataaa

  61  gtaatataaa tgtttttata ataaattatg tgagatatat ttttttgtcc gtactggtat

 121  agatttgacg attaagtctt aaataagtta taatctcaat tgcgtaattt cttaaataca
      -35 >>..>>

 181  gaaataacaa ctacattggt agactgatta aaaagtgtac ttgatgaact gttataaacc

 241  ttaaaaaaat aaaaataata gtttggggga tgttaaagat gtataaaaaa tatggagatt
                                    >>............gadR..............>

 301  gttttaaaaa gttgcgaaac caaaagaatt tagggttatc atactttagt aaacttggaa
      >..........................gadR.............................>
```

```
 361  tagaccgttc aaatatatct agatttgaac atggaaaatg tatgatgagt tttgagcgta
      >.............................gadR..............................>

 421  tagatttgat gttagaagaa atgcaagttc cgttatctga gtacgaattg attgtaaata
      >.............................gadR..............................>

 481  attttatgcc gaatttccaa gaatttttta tattagaatt ggaaaaagct gaatttagcc
      >.............................gadR..............................>

 541  aaaatcgaga taaaataaaa gagttgtatt ctgaggtcaa agaaacgggg aatcatttac
      >.............................gadR..............................>

 601  tgacggttac cgtgaaaacg aagcttggga atataagtca gacagaagtt aaagaaattg
      >.............................gadR..............................>

 661  aagcttatct ttgcaatatt gaagagtggg gatattttga acttacttta ttttattttg
      >.............................gadR..............................>

 721  tatctgatta tctcaatgtc aatcaattag aattgctgct tttaattttt gataaaagat
      >.............................gadR..............................>

 781  gtgaaaatta ctgtagagtc ttaaaatata gaaggagact attgcaaata gcctataaaa
      >.............................gadR..............................>

 841  gtgttgcgat atacgcggct aaaggagaaa gaaaaaaagc cgaaaatatt ttagaaatga
      >.............................gadR..............................>

 901  ctaaaaaata tcgaactgtg ggagtcgatt tatattcaga agtattaaga catcttgcta
      >.............................gadR..............................>

 961  gagctatcat tatttttaat tttgaaaatg cagagattgg ggaagaaaaa ataaattatg
      >.............................gadR..............................>

1021  ctcttgagat tttggaagaa tttggaggaa agaagataaa agaattctat cagaataaaa
      >.............................gadR..............................>

1081  tggaaaagta tttgaaaagg tcaatttagt ctcttttgag ctgttgcttt aaagcaacag
      >............gadR............>>
                                   >>..............IR1................>

1141  ctcaaaagag attttcttta ttctagagca tatactagag ggtgaagata ggttgtctga
      >....IR1....>>
                                   -10 >>..>>
                                                      >>.....IR2......>
                                            Start >

1201  agcattataa cttgtctttt aaaaaattca atcataaata taaggaggta tgatgaagaa
      >......IR2........>>
                                                   >>.SD.>>
                                                       ssUsp45 >>.....>

1261  aaagattatt agtgcaattt taatgtcaac ggtcatctta agcgctgctg ccccattgtc
      >............................ssUsp45............................>

1321  aggtgtttat gcagccccaa cacgtagtcc aataacagta actagacctt ggaaacatgt
      >..ssUsp45..>>
                    >>....................mGM-CSF.....................>

1381  tgaagcaatt aaagaagcat taaatttatt agatgatatg ccagtaacat taaatgaaga
      >............................mGM-CSF............................>

1441  agttgaagta gtaagtaatg aatttagttt taaaaaatta acatgtgttc aaactagatt
      >............................mGM-CSF............................>
```

```
1501  aaaaattttt gaacaaggat taagaggtaa ttttactaaa ttaaaaggtg ctttaaatat
      >..........................mGM-CSF..........................>

1561  gacagctagt tattatcaaa catattgtcc accaacacct gaaactgatt gtgaaacaca
      >..........................mGM-CSF..........................>

1621  agtaactaca tatgctgatt ttattgattc attaaaaaca tttttaactg atattccttt
      >..........................mGM-CSF..........................>

1681  tgaatgtaaa aaaccaggac aaaaataata aacgcgtatt aataaggagg ctaactaatg
      >.........mGM-CSF........>>
                                           >>.....RBS atpG......>>
                                                         ssUsp45 >>>

1741  aaaaagaaaa tcatttcagc gattttgatg tcaacggtta ttttaagcgc agcagctcca
      >..........................ssUsp45..........................>

1801  ttatctggag tttatgcagc cgatatgtgg gaattagaaa aagatgttta tgttgtagaa
      >.....ssUsp45....>>
                        >>.................mIL12...................>

1861  gttgattgga caccagatgc tcctggtgaa acagttaatt taacatgtga tactccagaa
      >...........................mIL12...........................>

1921  gaagatgata ttacatggac atctgatcaa cgacatggag ttattggtag tggaaaaaca
      >...........................mIL12...........................>

1981  ttaacaatta cagttaaaga atttttagat gctggacaat atacttgtca taaaggaggt
      >...........................mIL12...........................>

2041  gaaacattat ctcattcaca tttattatta cataaaaaag aaaatggtat ttggtcaact
      >...........................mIL12...........................>

2101  gaaatcttaa aaaattttaa aaataagaca tttttaaaat gtgaagctcc taattattct
      >...........................mIL12...........................>

2161  ggtagattta cttgttcatg gttagttcaa agaaatatgg atttaaaatt taatattaaa
      >...........................mIL12...........................>

2221  tcaagttcaa gtagtcctga tagtagagct gtaacatgtg gaatggcaag tttatctgct
      >...........................mIL12...........................>

2281  gaaaaagtaa cacttgatca acgtgattat gaaaaatatt ctgttagttg tcaagaagat
      >...........................mIL12...........................>

2341  gttacttgtc caacagctga agaaacatta ccaattgaat tagcattaga agctagacaa
      >...........................mIL12...........................>

2401  caaaacaaat atgaaaatta tagtacatct ttctttattc gtgacattat taaacctgat
      >...........................mIL12...........................>

2461  ccacctaaaa atttacaaat gaaaccttta aaaaatagtc aagttgaagt tagttgggaa
      >...........................mIL12...........................>

2521  tatccagatt catggtcaac accacattca tatttttcat taaaattctt tgttagaatt
      >...........................mIL12...........................>

2581  caacgtaaaa aagaaaaaat gaaagaaact gaagaaggtt gtaaccaaaa aggtgcattt
      >...........................mIL12...........................>

2641  ttagttgaaa aaactagtac tgaagttcaa tgtaaaggtg gtaatgtatg tgttcaagct
      >...........................mIL12...........................>
```

```
2701  caagatcgat attataattc tagttgttca aaatgggcat gtgttccatg tagagttcgt
      >.............................mIL12.............................>

2761  agtggaggag gtggaagtgg tggaggtggt tcaggtggtg gaggttcacg tgtaattcca
      >.............................mIL12.............................>

2821  gtttcaggac ctgctcgatg tttaagtcaa tctcgaaatt tattaaaaac tacagatgat
      >.............................mIL12.............................>

2881  atggttaaaa ctgctagaga aaaattaaaa cattatagtt gtacagctga agatattgat
      >.............................mIL12.............................>

2941  catgaagata ttacacgtga tcaaacttca acacttaaaa catgtttacc acttgaatta
      >.............................mIL12.............................>

3001  cataaaaatg aatcttgttt agctactaga gaaactagta gtacaactag aggttcttgt
      >.............................mIL12.............................>

3061  ttgcctccac aaaaaacatc attaatgatg acattatgtt taggttctat ttatgaagat
      >.............................mIL12.............................>

3121  ttgaaaatgt atcaaactga atttcaagca attaatgctg ctttacaaaa tcataatcat
      >.............................mIL12.............................>

3181  caacaaatta ttttagataa aggtatgtta gttgcaattg atgaattaat gcaatcttta
      >.............................mIL12.............................>

3241  aatcataatg gtgaaactct tagacaaaaa ccacctgttg gagaagcaga cccttatcga
      >.............................mIL12.............................>

3301  gtgaaaatga aattatgtat cttgttgcac gctttttcaa cacgggtagt cacaattaat
      >.............................mIL12.............................>

3361  cgtgtaatgg gatatttgag tagtgcataa taaacgcgtg aaatttagga ggtagtccaa
      >..........mIL12..........>>
                                                  >>....RBS lacA.....>>

3421  atgaaaaaaa agattatctc agctatttta atgtctacag tgatactttc tgctgcagcc
      >>...........................ssUSP45............................>

3481  ccgttgtcag gtgtttacgc tgcaaatttt ggtagattac attgtacaac agcagtaata
      >.......ssUSP45......>>
                             >>...............mIL-18.................>

3541  cgtaatatta atgatcaagt ttatttgtt gataaaagac aacctgtttt tgaagatatg
      >............................mIL-18.............................>

3601  actgatattg atcaatctgc atctgaacca caaactagat taataattta tatgtataaa
      >............................mIL-18.............................>

3661  gatagtgaag ttagaggatt agctgtaaca ttaagtgtta aagatagtaa aatgagtaca
      >............................mIL-18.............................>

3721  ttatcatgta aaaacaaaat aatttcattt gaagaaatgg acccacctga aaatattgat
      >............................mIL-18.............................>

3781  gatattcaat cagatttaat ttctttcaa aaacgtgttc caggtcataa caaaatggaa
      >............................mIL-18.............................>

3841  tttgaatcta gtttatatga aggtcacttt ttagcttgtc aaaaagaaga tgatgctttt
      >............................mIL-18.............................>
```

```
3901  aaattaattt taaagaaaaa agatgaaaat ggagataaat cagttatgtt tacattaact
      >..........................mIL-18............................>

3961  aatttacatc aatcataata accatgggta ctgcaggcat gcggtaccac tagttctaga
      >....mIL-18...>>

4021  gagctcaagc tttctttgaa ccaaaattag aaaaccaagg cttgaaacgt tcaattgaaa

4081  tggcaattaa acaaattaca gcacgtgttg ctttgattga tagccaaaaa gcagcagttg

4141  ataaagcaat tactgatatt gctgaaaaat tgtaatttat aaataaaaat cacctttttag
                                    >>............T..............>

4201  aggtggtttt tttatttata aattattcgt ttgatttcgc tttcgataga acaatcaaat
      >............T...........>>

4261  cgtttctgag acgttttagc gtttatttcg tttagttatc ggcataatcg ttaaaacagg

4321  cgttatcgta gcgtaaaagc ccttgagcgt agcgtggctt tgcagcgaag atgttgtctg

4381  ttagattatg aaagccgatg actgaatgaa ataataagcg cagcgtcctt ctatttcggt

4441  tggaggaggc tcaagggagt ttgagggaat gaaattccct catgggtttg attttaaaaa

4501  ttgcttgcaa ttttgccgag cggtagcgct ggaaaatttt tgaaaaaaat ttggaatttg

4561  gaaaaaaatg gggggaaagg aagcgaattt tgcttccgta ctacgacccc ccattaagtg
              >>.......................repC.........................>

4621  ccgagtgcca atttttgtgc caaaaacgct ctatcccaac tggctcaagg gtttgagggg
      >..........................repC.............................>

4681  tttttcaatc gccaacgaat cgccaacgtt ttcgccaacg ttttttataa atctatattt
      >..........................repC.............................>

4741  aagtagcttt atttttgttt ttatgattac aaagtgatac actaatttta taaaattatt
      >................repC...............>>

4801  tgattggagt tttttaaatg gtgatttcag aatcgaaaaa aagagttatg atttctctga

4861  caaaagagca agataaaaaa ttaacagata tggcgaaaca aaaagatttt tcaaaatctg

4921  cggttgcggc gttagctata gaagaatatg caagaaagga atcagaacaa aaaaaataag

4981  cgaaagctcg cgtttttaga aggatacgag ttttcgctac ttgtttttga taaggtaatt

5041  atatcatggc tattaaaaat actaaagcta gaaattttgg atttttatta tatcctgact
            >>.......................repA..........................>

5101  caattcctaa tgattggaaa gaaaaattag agagtttggg cgtatctatg gctgtcagtc
      >..........................repA.............................>

5161  ctttacacga tatggacgaa aaaaaagata aagatacatg gaatagtagt gatgttatac
      >..........................repA.............................>

5221  gaaatggaaa gcactataaa aaaccacact atcacgttat atatattgca cgaaatcctg
      >..........................repA.............................>

5281  taacaataga aagcgttagg aacaagatta agcgaaaatt ggggaatagt tcagttgctc
      >..........................repA.............................>

5341  atgttgagat acttgattat atcaaaggtt catatgaata tttgactcat gaatcaaagg
      >..........................repA.............................>
```

```
5401  acgctattgc taagaataaa catatatacg acaaaaaaga tattttgaac attaatgatt
      >.............................repA..............................>

5461  ttgatattga ccgctatata acacttgatg aaagccaaaa aagagaattg aagaatttac
      >.............................repA..............................>

5521  ttttagatat agtggatgac tataatttgg taaatacaaa agatttaatg gcttttattc
      >.............................repA..............................>

5581  gccttagggg agcggagttt ggaattttaa atacgaatga tgtaaaagat attgtttcaa
      >.............................repA..............................>

5641  caaactctag cgcctttaga ttatggtttg agggcaatta tcagtgtgga tatagagcaa
      >.............................repA..............................>

5701  gttatgcaaa ggttcttgat gctgaaacgg gggaaataaa atgacaaaca aagaaaaaga
      >.....................repA....................>>

5761  gttatttgct gaaaatgagg aattaaaaaa agaaattaag gacttaaaag agcgtattga

5821  aagatacaga gaaatggaag ttgaattaag tacaacaata gatttattga gaggagggat

5881  tattgaataa ataaaagccc ccctgacgaa agtcgacatg gtcgatgtct agatgcttaa

5941  actagagaaa ggtttaaaag atgaaaactt caccacatcg taatacttca gctattgttg
                            >>..................alr...................>

6001  atttaaaagc gattagaaat aatattgaaa aatttaaaaa gcatattaac cctaatgcag
      >..............................alr..............................>

6061  agatttggcc agcagtgaaa gcagatgctt atggtcatgg ctcgattgag gtttctaaag
      >..............................alr..............................>

6121  cggtgagcga tttggtaggt ggtttttgtg tatcaaacct agatgaggca attgaattac
      >..............................alr..............................>

6181  gaaatcatct ggtgactaaa ccgattttag ttttatccgg aattgttcca gaagatgttg
      >..............................alr..............................>

6241  atattgcagc tgcccttaat attagtctta ctgccccgag tttagaatgg ttgaaattgg
      >..............................alr..............................>

6301  ttgttcaaga agaagcagaa ctttcagatt taaaaattca tattggtgta gattctggta
      >..............................alr..............................>

6361  tgggtcggat tggtattcgt gatgttgaag aagctaatca gatgattgaa cttgctgata
      >..............................alr..............................>

6421  aatatgcgat taattttgaa ggaattttca ctcattttgc gactgcggat atggctgatg
      >..............................alr..............................>

6481  aaacaaaatt taaaaatcaa caggcaagat ttaacaaaat tatggccgga ttatcacgtc
      >..............................alr..............................>

6541  aaccaaaatt tatacactca actaatacgg ccgctgcttt atggcataag gaacaagttc
      >..............................alr..............................>

6601  aagctattga acgtttaggg atttcaatgt atggcttgaa tccaagtggt aaaactttgg
      >..............................alr..............................>

6661  aacttccttt tgaaattgaa cccgctctct ctttagtttc tgaattgact catataaaaa
      >..............................alr..............................>
```

```
6721   aaatagctgc aggtgaaacg gttggttatg gtgcaactta tgagacgagt gaagaaactt
       >..............................alr..............................>

6781   ggattggaac tgttccaatt ggttacgctg acgggtggac ccgtcaaatg caaggtttca
       >..............................alr..............................>

6841   aagtgcttgt tgatggaaag ttttgtgaga ttgttggtcg agtttgtatg gatcaaatga
       >..............................alr..............................>

6901   tgataaaact tgataagtct taccctttgg gaacgaaggt cactttgatt ggtcgagata
       >..............................alr..............................>

6961   aggctaatga aatcacgaca acagacgttg ctgattggcg tggaacgatt aattatgaag
       >..............................alr..............................>

7021   tgctttgctt actttctgat agaatcaaaa gaatctataa ataaaattaa aaaaactgta
       >.....................alr......................>>
                                                           >>....T.....>

7081   tttttacagt ttttttgttt tctgttaaaa gcagatgata acctcacta
       >.......T......>>
```

Fig. 17a.

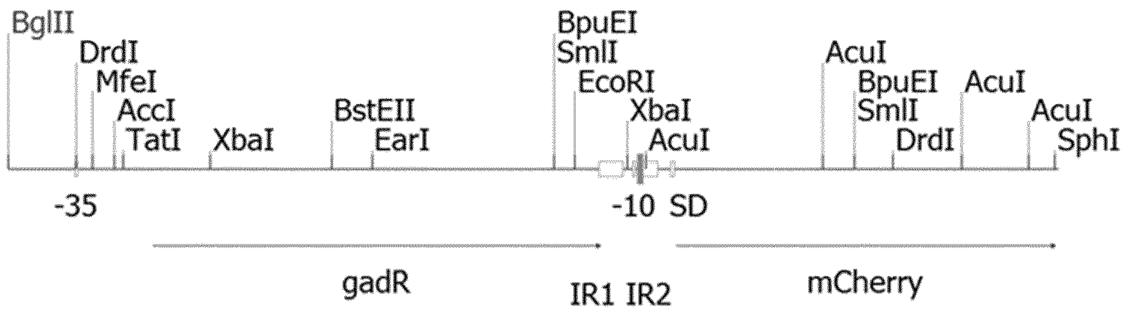


Fig. 17b.

```
        BglII
       -+----
   1   agatctgagc gttgtataag cttttatgtc tttctatatc aacttttaat agaaatataa

  61   agtaatataa atgtttttat aataaattat gtgagatata ttttttttgtc cgtactggta

 121   tagatttgac gattaagtct taaataagtt ataatctcaa ttgcgtaatt tcttaaatac
        -35 >>...>>

 181   agaaataaca actacattgg tagactgatt aaaaagtgta cttgatgaac tgttataaac

 241   cttaaaaaaa taaaaataat agtttggggg atgttaaaga tgtataaaaa atatggagat
                                        >>.............gadR............>

 301   tgttttaaaa agttgcgaaa ccaaaagaat ttagggttat catactttag taaacttgga
       >.............................gadR..............................>

 361   atagaccgtt caaatatatc tagatttgaa catggaaaat gtatgatgag ttttgagcgt
       >.............................gadR..............................>
```

```
 421  atagatttga tgttagaaga aatgcaagtt ccgttatctg agtacgaatt gattgtaaat
      >.............................gadR..............................>

 481  aattttatgc cgaatttcca agaatttttt atattagaat tggaaaaagc tgaatttagc
      >.............................gadR..............................>

 541  caaaatcgag ataaaataaa agagttgtat tctgaggtca aagaaacggg gaatcattta
      >.............................gadR..............................>

 601  ctgacggtta ccgtgaaaac gaagcttggg aatataagtc agacagaagt taaagaaatt
      >.............................gadR..............................>

 661  gaagcttatc tttgcaatat tgaagagtgg ggatattttg aacttacttt attttatttt
      >.............................gadR..............................>

 721  gtatctgatt atctcaatgt caatcaatta gaattgctgc tttttaattt tgataaaaga
      >.............................gadR..............................>

 781  tgtgaaaatt actgtagagt cttaaaatat agaaggagac tattgcaaat agcctataaa
      >.............................gadR..............................>

 841  agtgttgcga tatacgcggc taaaggagaa agaaaaaaag ccgaaaatat tttagaaatg
      >.............................gadR..............................>

 901  actaaaaaat atcgaactgt gggagtcgat ttatattcag aagtattaag acatcttgct
      >.............................gadR..............................>

 961  agagctatca ttatttttaa ttttgaaaat gcagagattg gggaagaaaa aataaattat
      >.............................gadR..............................>

1021  gctcttgaga ttttggaaga atttggagga aagaagataa aagaattcta tcagaataaa
      >.............................gadR..............................>

1081  atggaaaagt atttgaaaag gtcaatttag tctcttttga gctgttgctt taaagcaaca
      >............gadR............ >>
                                    >>.............IR1................>

1141  gctcaaaaga gattttcttt attctagagc atatactaga gggtgaagata ggttgtctg
      >.....IR1....>>
                                    -10 >>..>>
                                                       >>.....IR2.....>
                                             Start >

1201  aagcattata acttgtcttt taaaaaattc aatcataaat ataaggaggt atgatgagca
      >........IR2......>>
                                                  >>.SD.>>
                                                      mCherry >>....>

1261  aaggagaaga agataacatg gcaatcatca aagaatttat gcgtttcaaa gttcacatgg
      >............................mCherry............................>

1321  aaggttctgt aaacggacac gaatttgaaa ttgaaggtga aggtgaaggc cgtccttatg
      >............................mCherry............................>

1381  aaggaacaca aacggcaaag ctgaaagtaa caaaaggcgg accgcttccg tttgcatggg
      >............................mCherry............................>

1441  atatcctttc tccgcaattc atgtacggtt caaaagcata cgtgaagcat ccggctgata
      >............................mCherry............................>

1501  ttcctgatta tttgaagctg tcattccctg aaggcttcaa atgggagcgt gtgatgaact
      >............................mCherry............................>
```

```
1561  ttgaagatgg cggtgttgtt actgttactc aagattcaag ccttcaagac ggtgaattta
      >.........................mCherry...........................>

1621  tttacaaagt gaagctgcgc ggaacaaact tcccatctga cggacctgtc atgcaaaaga
      >.........................mCherry...........................>

1681  aaacaatggg ctgggaagca agctctgaac gcatgtatcc agaggacggt gctttaaaag
      >.........................mCherry...........................>

1741  gagaaatcaa acagcgtttg aagctgaaag acggcggaca ctatgacgct gaagtgaaaa
      >.........................mCherry...........................>

1801  caacttacaa agcgaaaaag ccggttcagc ttccaggtgc ttacaacgta aacatcaaac
      >.........................mCherry...........................>

1861  ttgatattac aagccacaat gaagattata cgattgttga acaatatgaa cgcgctgaag
      >.........................mCherry...........................>

                                                    SphI
                                                   -----+
1921  gccgtcattc aactggcgga atggatgagc tttacaaata ataagcatgc
      >.................mCherry................>>
```

**Fig. 18a:**

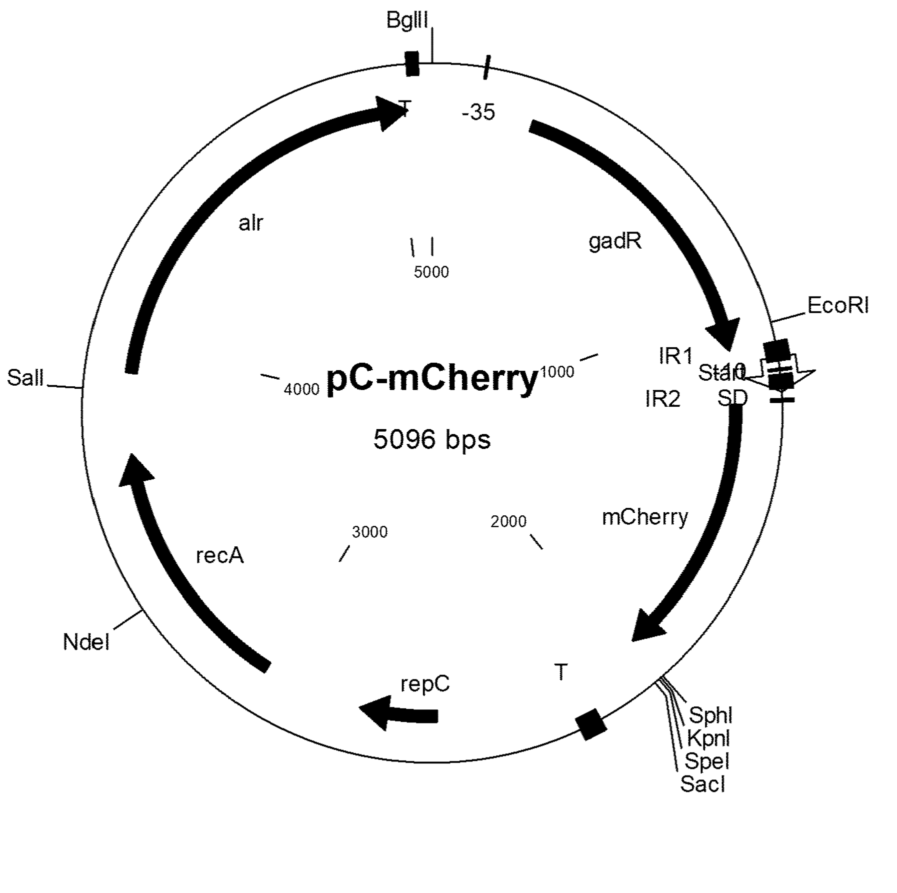


**Fig. 18b:**

```
   1  gatctgagcg ttgtataagc ttttatgtct ttctatatca acttttaata gaaatataaa

  61  gtaatataaa tgtttttata ataaattatg tgagatatat ttttttgtcc gtactggtat

 121  agatttgacg attaagtctt aaataagtta taatctcaat tgcgtaattt cttaaataca
      -35 >>..>>

 181  gaaataacaa ctacattggt agactgatta aaaagtgtac ttgatgaact gttataaacc

 241  ttaaaaaaat aaaaataata gtttgggga tgttaaagat gtataaaaaa tatggagatt
                                     >>.............gadR..............>

 301  gttttaaaaa gttgcgaaac caaaagaatt tagggttatc atactttagt aaacttggaa
      >.............................gadR..............................>

 361  tagaccgttc aaatatatct agatttgaac atggaaaatg tatgatgagt tttgagcgta
      >.............................gadR..............................>

 421  tagatttgat gttagaagaa atgcaagttc cgttatctga gtacgaattg attgtaaata
      >.............................gadR..............................>

 481  attttatgcc gaatttccaa gaatttttta tattagaatt ggaaaaagct gaatttagcc
      >.............................gadR..............................>

 541  aaaatcgaga taaaataaaa gagttgtatt ctgaggtcaa agaaacgggg aatcatttac
      >.............................gadR..............................>

 601  tgacggttac cgtgaaaacg aagcttggga atataagtca gacagaagtt aaagaaattg
      >.............................gadR..............................>

 661  aagcttatct ttgcaatatt gaagagtggg gatattttga acttacttta ttttattttg
      >.............................gadR..............................>

 721  tatctgatta tctcaatgtc aatcaattag aattgctgct tttaatttt gataaaagat
      >.............................gadR..............................>

 781  gtgaaaatta ctgtagagtc ttaaaatata gaaggagact attgcaaata gcctataaaa
      >.............................gadR..............................>

 841  gtgttgcgat atacgcggct aaaggagaaa gaaaaaaagc cgaaaatatt ttagaaatga
      >.............................gadR..............................>

 901  ctaaaaaata tcgaactgtg ggagtcgatt tatattcaga agtattaaga catcttgcta
      >.............................gadR..............................>

 961  gagctatcat tatttttaat tttgaaaatg cagagattgg ggaagaaaaa ataaattatg
      >.............................gadR..............................>

1021  ctcttgagat tttggaagaa tttggaggaa agaagataaa agaattctat cagaataaaa
      >.............................gadR..............................>

1081  tggaaaagta tttgaaaagg tcaatttagt ctcttttgag ctgttgcttt aaagcaacag
      >............gadR............>>
                                   >>..............IR1................>

1141  ctcaaaagag atttctttta ttctagagca tatactagag ggtgaagata ggttgtctga
      >....IR1....>>
                                   -10 >>..>>
                                                      >>.....IR2......>
                                            Start >

1201  agcattataa cttgtctttt aaaaaattca atcataaata taaggaggta tgatgagcaa
```

>.......IR2......>>
>>.SD.>>
mCherry >>.....>

1261 aggagaagaa gataacatgg caatcatcaa agaatttatg cgtttcaaag ttcacatgga
>.........................mCherry...........................>

1321 aggttctgta aacggacacg aatttgaaat tgaaggtgaa ggtgaaggcc gtccttatga
>.........................mCherry...........................>

1381 aggaacacaa acggcaaagc tgaaagtaac aaaaggcgga ccgcttccgt ttgcatggga
>.........................mCherry...........................>

1441 tatcctttct ccgcaattca tgtacggttc aaaagcatac gtgaagcatc cggctgatat
>.........................mCherry...........................>

1501 tcctgattat ttgaagctgt cattccctga aggcttcaaa tgggagcgtg tgatgaactt
>.........................mCherry...........................>

1561 tgaagatggc ggtgttgtta ctgttactca agattcaagc cttcaagacg gtgaatttat
>.........................mCherry...........................>

1621 ttacaaagtg aagctgcgcg gaacaaactt cccatctgac ggacctgtca tgcaaaagaa
>.........................mCherry...........................>

1681 aacaatgggc tgggaagcaa gctctgaacg catgtatcca gaggacggtg ctttaaaagg
>.........................mCherry...........................>

1741 agaaatcaaa cagcgtttga agctgaaaga cggcggacac tatgacgctg aagtgaaaac
>.........................mCherry...........................>

1801 aacttacaaa gcgaaaaagc cggttcagct tccaggtgct tacaacgtaa acatcaaact
>.........................mCherry...........................>

1861 tgatattaca agccacaatg aagattatac gattgttgaa caatatgaac gcgctgaagg
>.........................mCherry...........................>

1921 ccgtcattca actggcggaa tggatgagct ttacaaataa taagcatgcg gtaccactag
>.................mCherry.................>>

1981 ttctagagag ctcaagcttt ctttgaacca aaattagaaa accaaggctt gaaacgttca

2041 attgaaatgg caattaaaca aattacagca cgtgttgctt tgattgatag ccaaaaagca

2101 gcagttgata aagcaattac tgatattgct gaaaaattgt aatttataaa taaaaatcac
>>.........T..........>

2161 cttttagagg tggttttttt atttataaat tattcgtttg atttcgcttt cgatagaaca
>...............T..............>>

2221 atcaaatcgt ttctgagacg ttttagcgtt tatttcgttt agttatcggc ataatcgtta

2281 aaacaggcgt tatcgtagcg taaaagccct tgagcgtagc gtggctttgc agcgaagatg

2341 ttgtctgtta gattatgaaa gccgatgact gaatgaaata ataagcgcag cgtccttcta

2401 tttcggttgg aggaggctca agggagtttg agggaatgaa attccctcat gggtttgatt

2461 ttaaaaattg cttgcaattt tgccgagcgg tagcgctgga aaatttttga aaaaaatttg

2521 gaatttggaa aaaaatgggg ggaaaggaag cgaattttgc ttccgtacta cgacccccca
>>...................repC.....................>

```
2581  ttaagtgccg agtgccaatt tttgtgccaa aaacgctcta tcccaactgg ctcaagggtt
      >..........................repC............................>

2641  tgagggggttt ttcaatcgcc aacgaatcgc caacgttttc gccaacgttt tttataaatc
      >..........................repC............................>

2701  tatatttaag tagctttatt tttgttttta tgattacaaa gtgatacact aattttataa
      >...................repC...................>>

2761  aattatttga ttggagtttt ttaaatggtg atttcagaat cgaaaaaaag agttatgatt

2821  tctctgacaa aagagcaaga taaaaaatta acagatatgg cgaaacaaaa agatttttca

2881  aaatctgcgg ttgcggcgtt agctatagaa gaatatgcaa gaaaggaatc agaacaaaaa

2941  aaataagcga aagctcgcgt ttttagaagg atacgagttt tcgctacttg tttttgataa

3001  ggtaattata tcatggctat taaaaatact aaagctagaa attttggatt tttattatat
                  >>...................recA......................>

3061  cctgactcaa ttcctaatga ttggaaagaa aaattagaga gtttgggcgt atctatggct
      >..........................recA............................>

3121  gtcagtcctt tacacgatat ggacgaaaaa aaagataaag atacatggaa tagtagtgat
      >..........................recA............................>

3181  gttatacgaa atggaaagca ctataaaaaa ccacactatc acgttatata tattgcacga
      >..........................recA............................>

3241  aatcctgtaa caatagaaag cgttaggaac aagattaagc gaaaattggg gaatagttca
      >..........................recA............................>

3301  gttgctcatg ttgagatact tgattatatc aaaggttcat atgaatattt gactcatgaa
      >..........................recA............................>

3361  tcaaaggacg ctattgctaa gaataaacat atatacgaca aaaaagatat tttgaacatt
      >..........................recA............................>

3421  aatgattttg atattgaccg ctatataaca cttgatgaaa gccaaaaaag agaattgaag
      >..........................recA............................>

3481  aatttacttt tagatatagt ggatgactat aatttggtaa atacaaaaga tttaatggct
      >..........................recA............................>

3541  tttattcgcc ttaggggagc ggagtttgga attttaaata cgaatgatgt aaaagatatt
      >..........................recA............................>

3601  gtttcaacaa actctagcgc ctttagatta tggtttgagg gcaattatca gtgtggatat
      >..........................recA............................>

3661  agagcaagtt atgcaaaggt tcttgatgct gaaacggggg aaataaaatg acaaacaaag
      >......................recA......................>>

3721  aaaaagagtt atttgctgaa aatgaggaat taaaaaaaga aattaaggac ttaaaagagc

3781  gtattgaaag atacagagaa atggaagttg aattaagtac aacaatagat ttattgagag

3841  gagggattat tgaataaata aaagcccccc tgacgaaagt cgacatggtc gatgtctaga

3901  tgcttaaact agagaaaggt ttaaaagatg aaaacttcac cacatcgtaa tacttcagct
                                    >>.............alr...............>

3961  attgttgatt taaaagcgat tagaaataat attgaaaaat ttaaaaagca tattaaccct
```

```
     >...........................alr.............................>

4021 aatgcagaga tttggccagc agtgaaagca gatgcttatg gtcatggctc gattgaggtt
     >...........................alr.............................>

4081 tctaaagcgg tgagcgattt ggtaggtggt tttgtgtat caaacctaga tgaggcaatt
     >...........................alr.............................>

4141 gaattacgaa atcatctggt gactaaaccg attttagttt tatccggaat tgttccagaa
     >...........................alr.............................>

4201 gatgttgata ttgcagctgc ccttaatatt agtcttactg ccccgagttt agaatggttg
     >...........................alr.............................>

4261 aaattggttg ttcaagaaga agcagaactt tcagatttaa aaattcatat tggtgtagat
     >...........................alr.............................>

4321 tctggtatgg gtcggattgg tattcgtgat gttgaagaag ctaatcagat gattgaactt
     >...........................alr.............................>

4381 gctgataaat atgcgattaa ttttgaagga attttcactc attttgcgac tgcggatatg
     >...........................alr.............................>

4441 gctgatgaaa caaaatttaa aaatcaacag gcaagattta acaaaattat ggccggatta
     >...........................alr.............................>

4501 tcacgtcaac caaaatttat acactcaact aatacggccg ctgctttatg gcataaggaa
     >...........................alr.............................>

4561 caagttcaag ctattgaacg tttagggatt tcaatgtatg gcttgaatcc aagtggtaaa
     >...........................alr.............................>

4621 actttggaac ttccttttga aattgaaccc gctctctctt tagtttctga attgactcat
     >...........................alr.............................>

4681 ataaaaaaaa tagctgcagg tgaaacggtt ggttatggtg caacttatga gacgagtgaa
     >...........................alr.............................>

4741 gaaacttgga ttggaactgt tccaattggt tacgctgacg ggtggacccg tcaaatgcaa
     >...........................alr.............................>

4801 ggtttcaaag tgcttgttga tggaaagttt tgtgagattg ttggtcgagt ttgtatggat
     >...........................alr.............................>

4861 caaatgatga taaaacttga taagtcttac cctttgggaa cgaaggtcac tttgattggt
     >...........................alr.............................>

4921 cgagataagg ctaatgaaat cacgacaaca gacgttgctg attggcgtgg aacgattaat
     >...........................alr.............................>

4981 tatgaagtgc tttgcttact ttctgataga atcaaaagaa tctataaata aaattaaaaa
     >.......................alr........................>>
                                                             >>T.>

5041 aactgtattt ttacagtttt tttgttttct gttaaaagca gatgataacc tcacta
     >..........T.........>>
```

Fig. 19a:

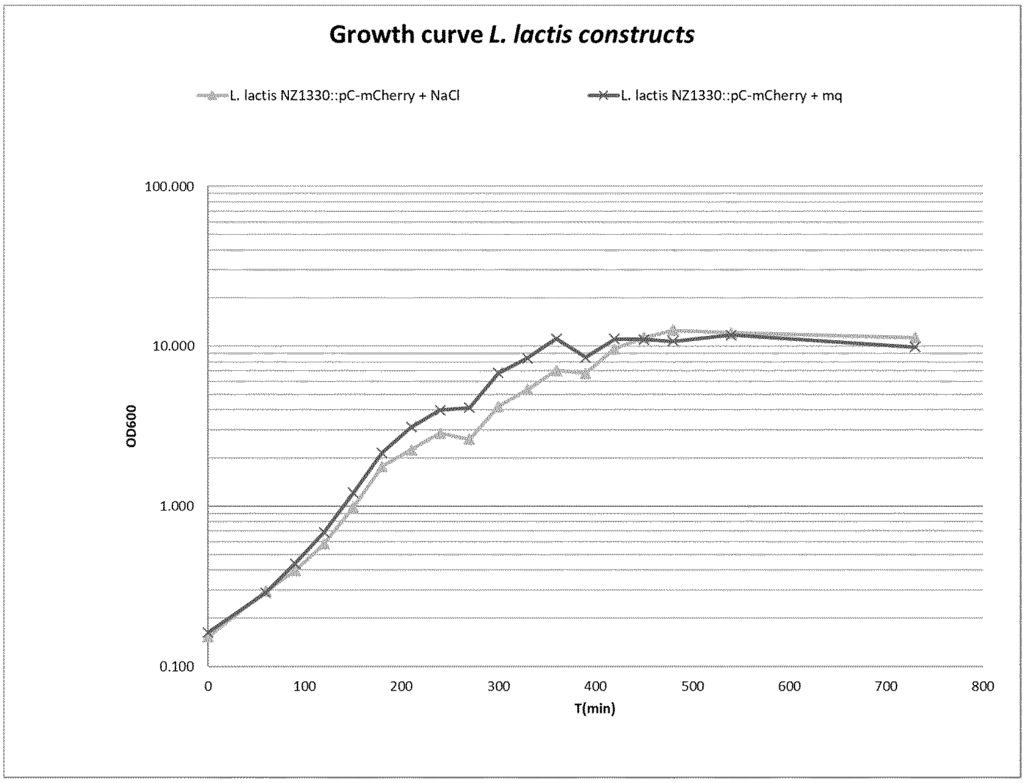


Fig. 19b:

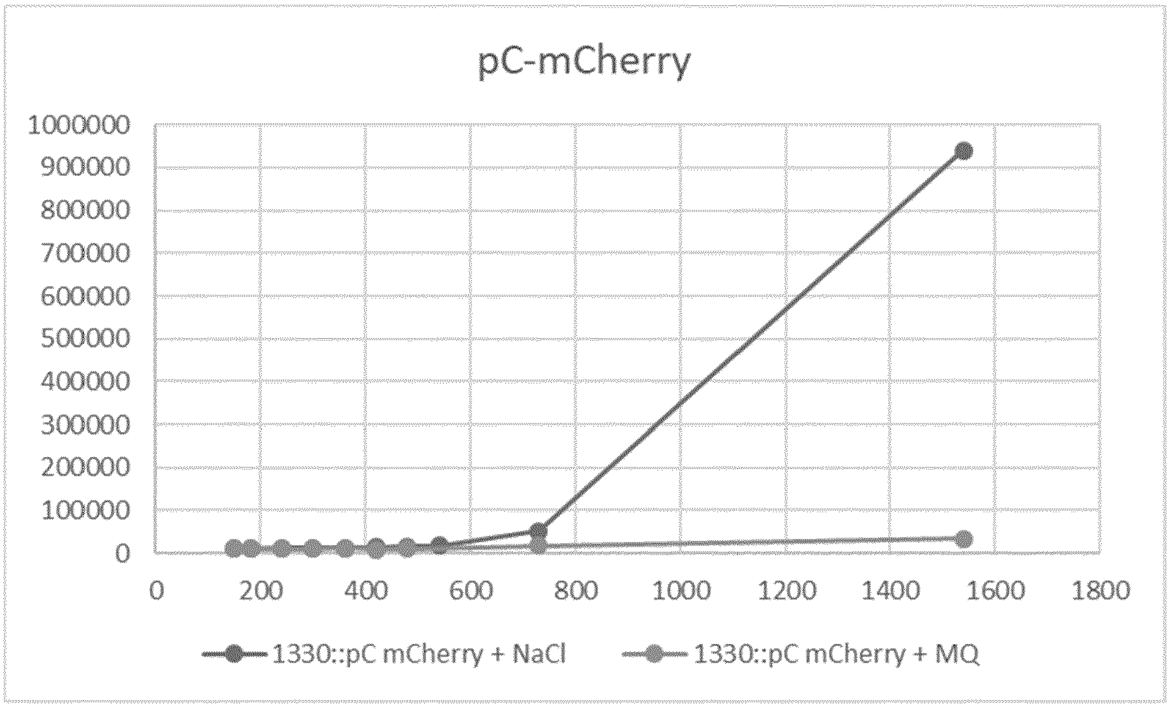

**Fig. 20a:**

| Lane | Sample | Factors |
|---|---|---|
| 1 | SeeBlue marker | |
| 2 | Recomb. CSF-1 | |
| 3 | Recomb. FGF-2 | |
| 4 | Recomb. IL-4 | |
| 5 | AUP1602-C | FGF-2, IL-4 and CSF-1 |
| 6 | AUP1602-C | FGF-2, IL-4 and CSF-1 |
| 7 | AUP1602-C | FGF-2, IL-4 and CSF-1 |
| 8 | AUP1602-C | FGF-2, IL-4 and CSF-1 |
| 9 | AUP1602-C | FGF-2, IL-4 and CSF-1 |
| 10 | AUP1602-C | FGF-2, IL-4 and CSF-1 |

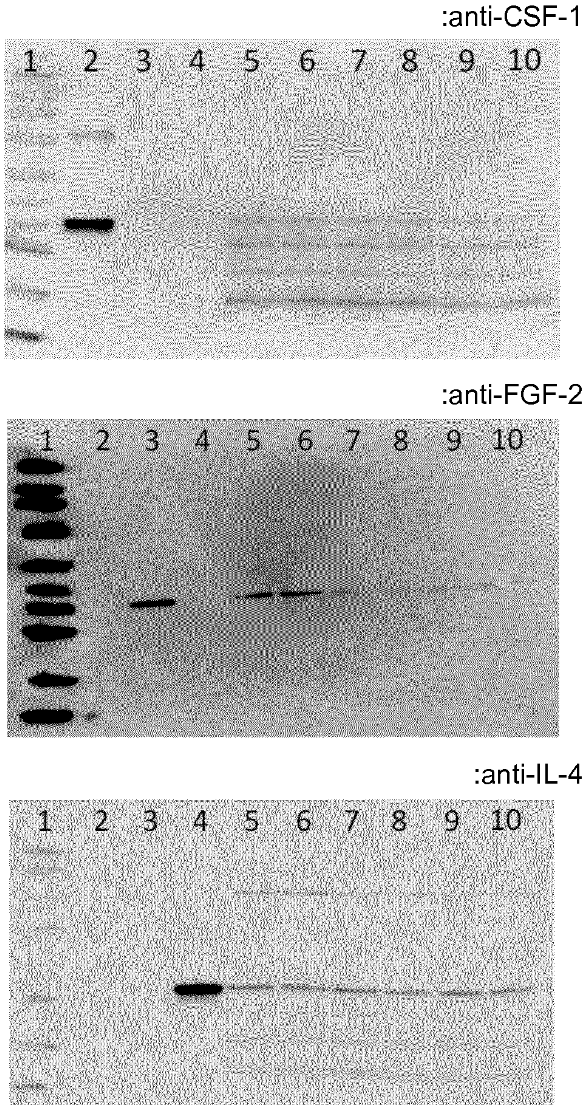


**Fig. 20b:**

| Lane | Sample | Factors |
|---|---|---|
| 1 | AUP5563-C | IL-12, IL-18 and GM-CSF |
| 2 | AUP5563-C | IL-12, IL-18 and GM-CSF |
| 3 | AUP5563-C | IL-12, IL-18 and GM-CSF |
| 4 | AUP5563-C | IL-12, IL-18 and GM-CSF |
| 5 | AUP5563-C | IL-12, IL-18 and GM-CSF |
| 6 | Marker | |
| 7 | Recomb. mGM-CSF | (100 ng) |

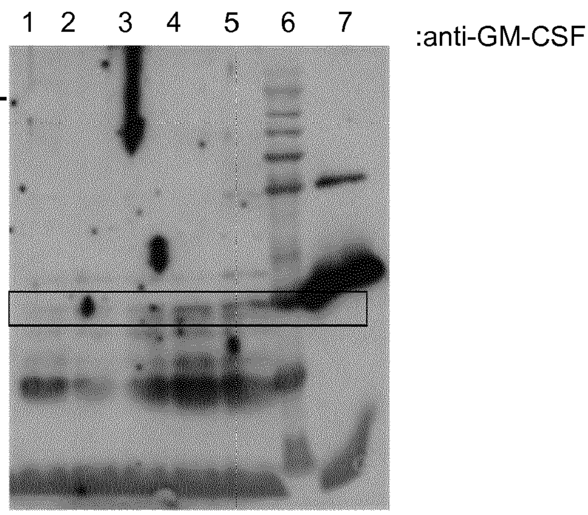

**Fig. 20c:**

| Lane | Sample | Factors |
|---|---|---|
| 1 | AUP5563-C | IL-12, IL-18 and GM-CSF |
| 2 | AUP5563-C | IL-12, IL-18 and GM-CSF |
| 3 | AUP5563-C | IL-12, IL-18 and GM-CSF |
| 4 | AUP5563-C | IL-12, IL-18 and GM-CSF |
| 5 | AUP5563-C | IL-12, IL-18 and GM-CSF |
| 6 | Marker | |
| 7 | Recomb. mIL-18 | (100 ng) |

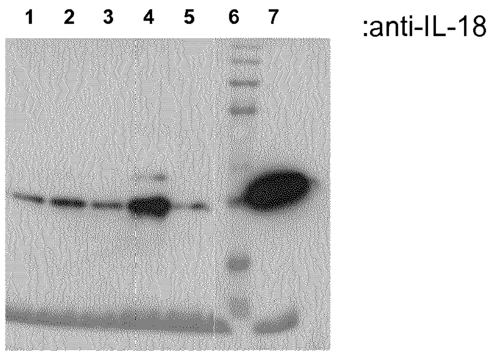


**Fig. 20d:**

| Lane | Sample | Factors |
|---|---|---|
| 1 | Marker | |
| 2 | AUP5551m-C | IL-12, IL-18 and IFN-α |
| 3 | AUP5551m-C | IL-12, IL-18 and IFN-α |
| 4 | AUP5551m-C | IL-12, IL-18 and IFN-α |
| 5 | AUP5563-C | IL-12, IL-18 and GM-CSF |
| 6 | AUP5563-C | IL-12, IL-18 and GM-CSF |
| 7 | AUP5563-C | IL-12, IL-18 and GM-CSF |
| 8 | AUP5563-C | IL-12, IL-18 and GM-CSF |
| 9 | Recomb. IL-12 | (0.06 ng) |
| 10 | Recomb. IL-12 | (0.12 ng) |
| 11 | Recomb. IL-12 | (0.37 ng) |
| 12 | Recomb. IL-12 | (1.1 ng) |
| 13 | Recomb. IL-12 | (3.3 ng) |
| 14 | Recomb. IL-12 | (10 ng) |
| 15 | Recomb. IL-12 | (30 ng) |
| 16 | Marker | |

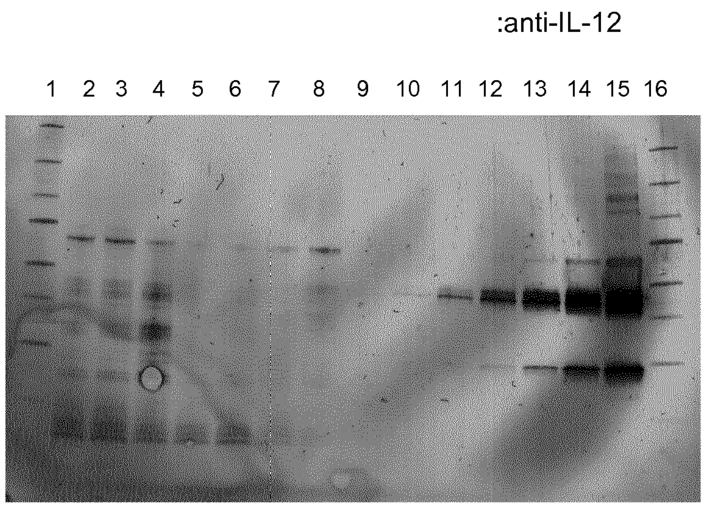

Fig. 21:

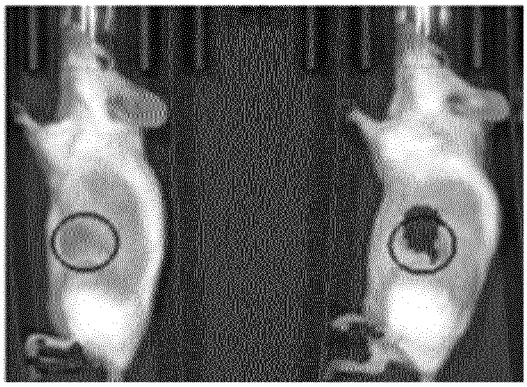

L. Lactis (control) PGAD- mCherry

Fig. 22

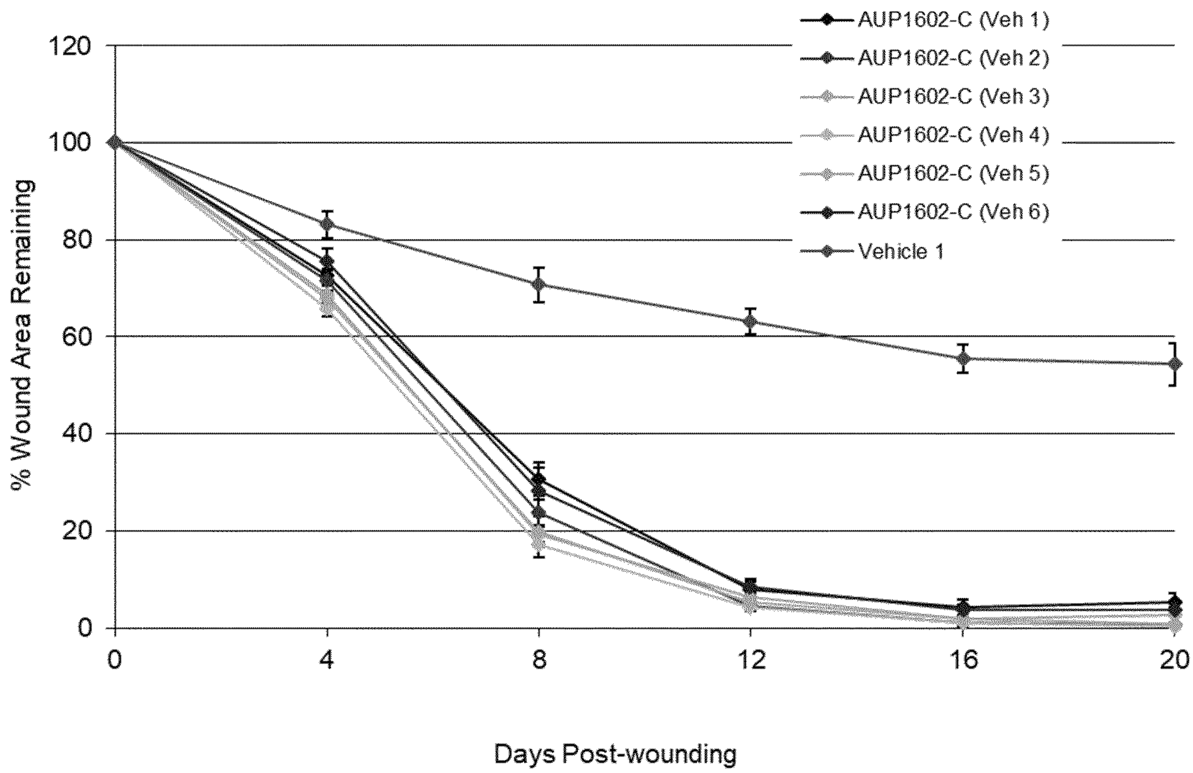

**Fig. 23**

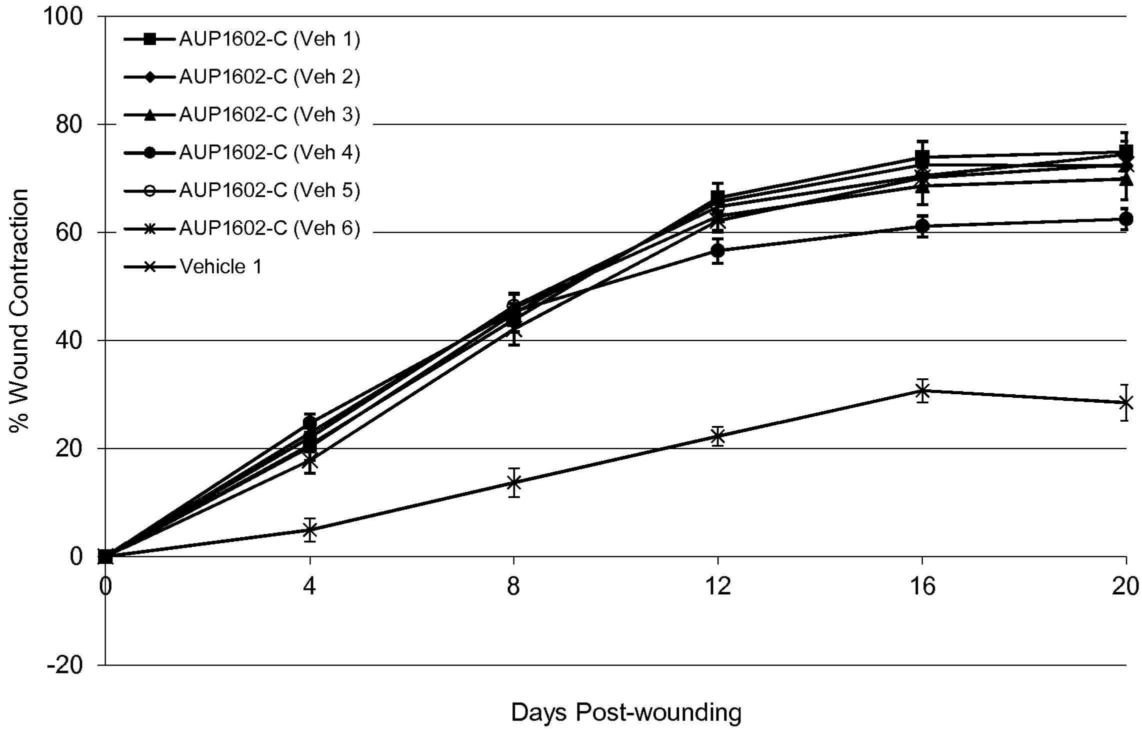

% Wound Contraction
Days Post-wounding
AUP1602-C (Veh 1)
AUP1602-C (Veh 2)
AUP1602-C (Veh 3)
AUP1602-C (Veh 4)
AUP1602-C (Veh 5)
AUP1602-C (Veh 6)
Vehicle 1
100
80
60
40
20
0
-20
0
4
8
12
16
20


**Fig. 24**

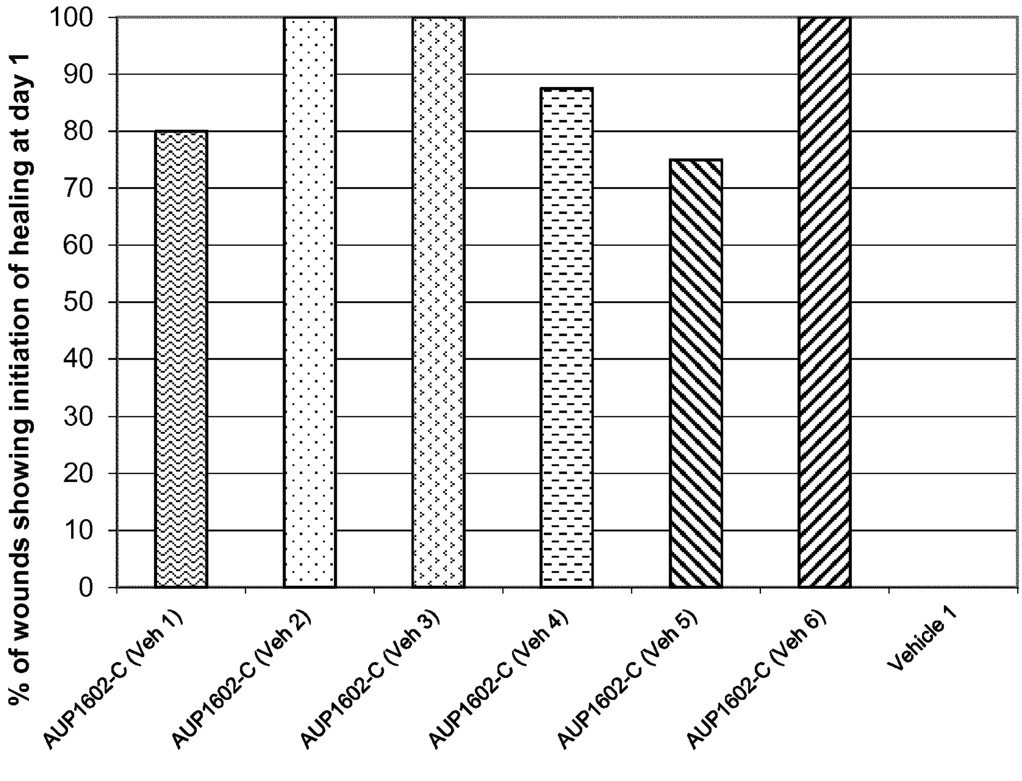

% of wounds showing initiation of healing at day 1
100
90
80
70
60
50
40
30
20
10
0
AUP1602-C (Veh 1)
AUP1602-C (Veh 2)
AUP1602-C (Veh 3)
AUP1602-C (Veh 4)
AUP1602-C (Veh 5)
AUP1602-C (Veh 6)
Vehicle 1

Fig. 25a

| N=1 | | | | | | | | | | | | | |
|---|---|---|---|---|---|---|---|---|---|---|---|---|---|
| Lane | 1 | 2 | 3 | 4 | 5 | 6 | 7 | 8 | 9 | 10 | 11 | 12 | 13 |
| Label | MW | NO2 25µl | NO3 25µl | NO4 25µl | NO2 10µl | NO3 10µl | NO4 10µl | MW | STD 10mg | STD 3.3ng | STD 1.1ng | STD 0.37ng | STD 0.12ng |
| ID | | | | | | | | | | | | | |

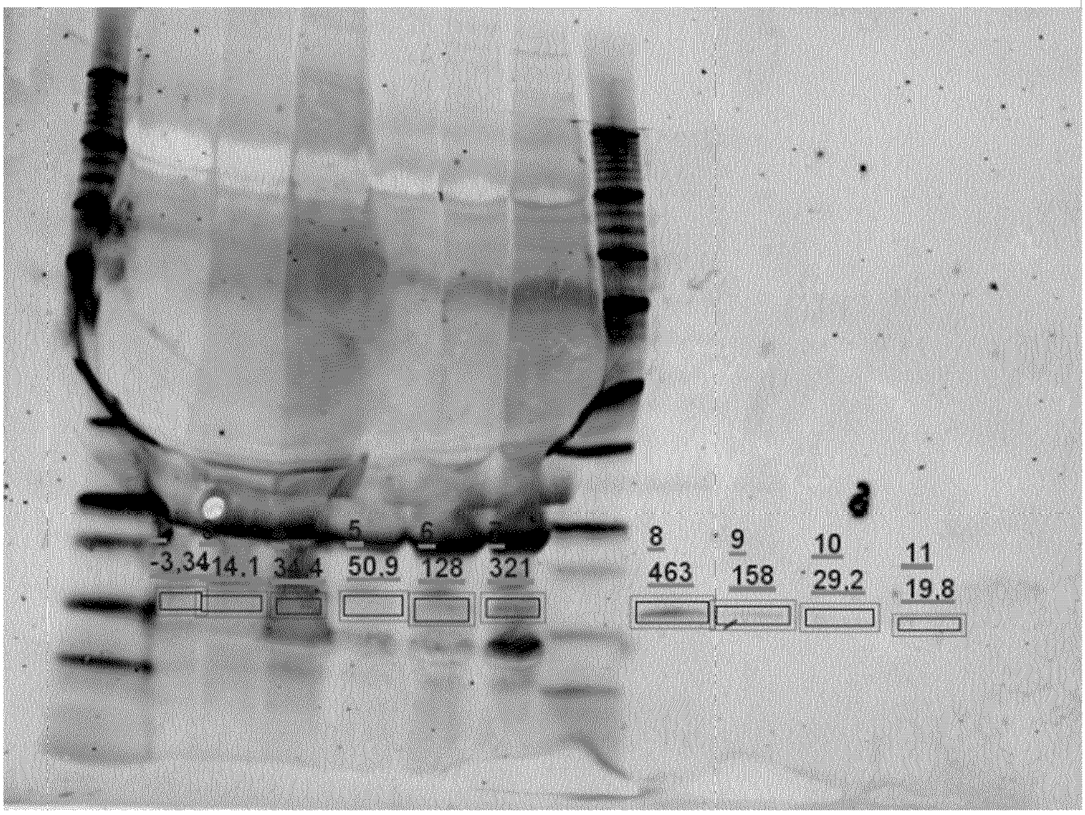


Fig. 25b:

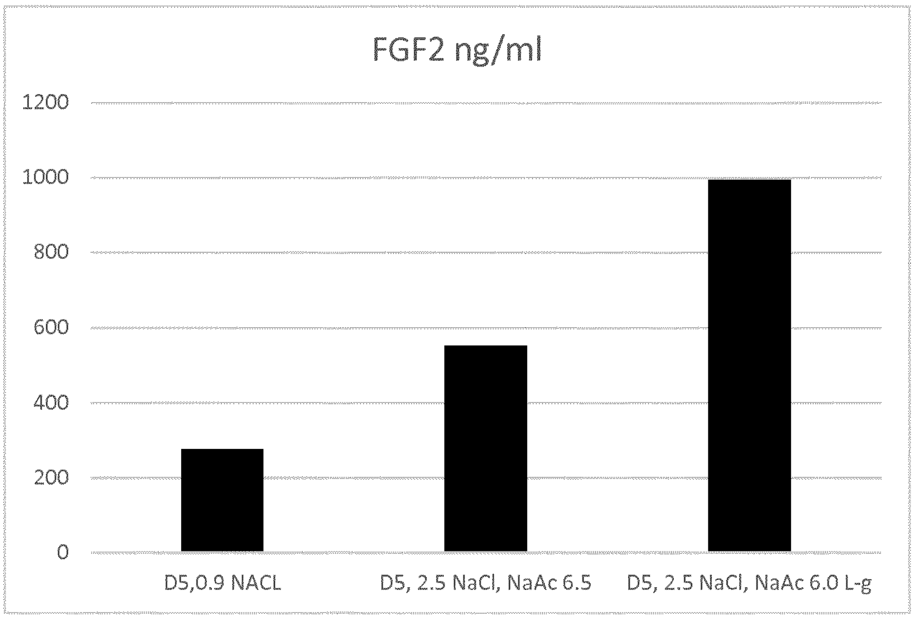

Fig. 26a

| N=1 | | | | | | | | | | | | | |
|---|---|---|---|---|---|---|---|---|---|---|---|---|---|
| Lane | 1 | 2 | 3 | 4 | 5 | 6 | 7 | 8 | 9 | 10 | 11 | 12 | 13 |
| Label | MW | NO2 25µl | NO3 25µl | NO4 25µl | NO2 10µl | NO3 10µl | NO4 10µl | MW | STD 10mg | STD 3.3ng | STD 1.1ng | STD 0.37ng | STD 0.12ng |
| ID | | | | | | | | | | | | | |

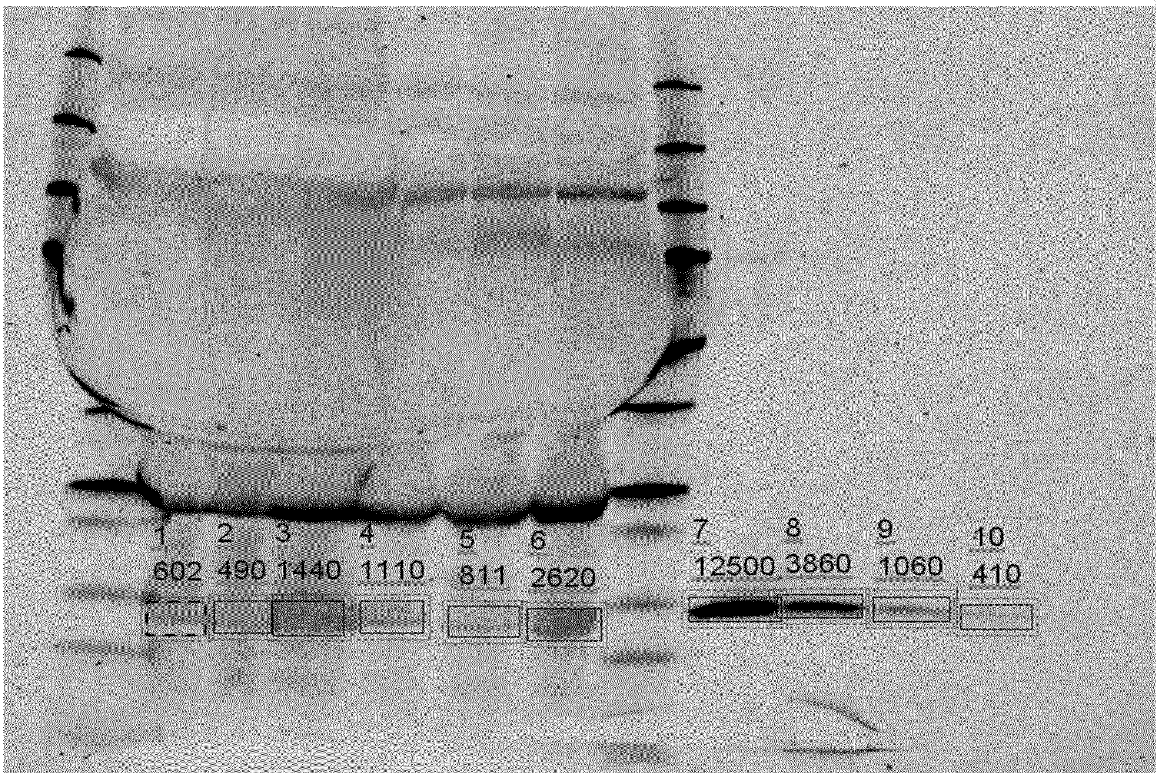


Fig. 26b

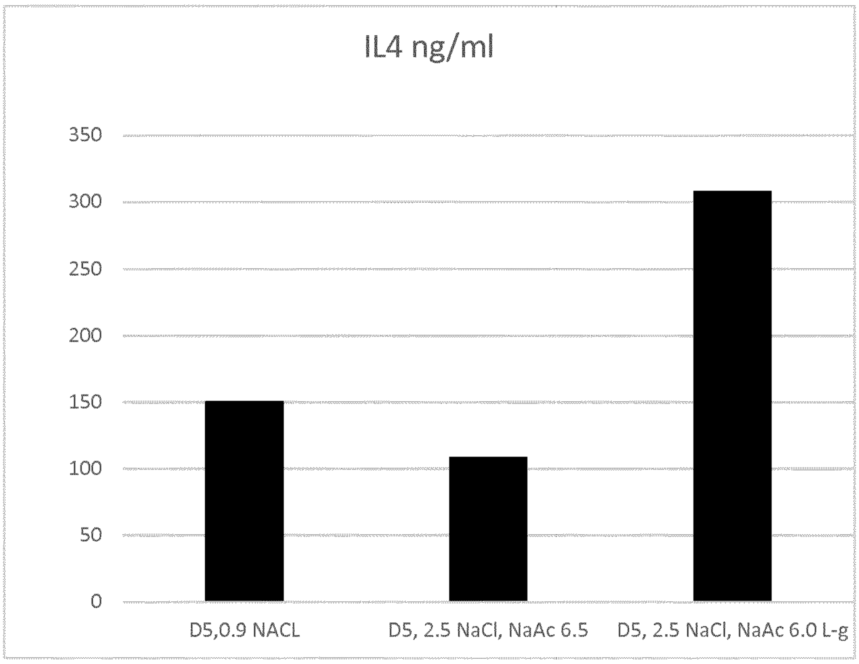

Fig. 27a:

| N=1 | | | | | | | | | | | | | |
|---|---|---|---|---|---|---|---|---|---|---|---|---|---|
| Lane | 1 | 2 | 3 | 4 | 5 | 6 | 7 | 8 | 9 | 10 | 11 | 12 | 13 |
| Label | MW | NO2 25µl | NO3 25µl | NO4 25µl | NO2 10µl | NO3 10µl | NO4 10µl | MW | STD 10mg | STD 3.3ng | STD 1.1ng | STD 0.37ng | STD 0.12ng |
| ID | | | | | | | | | | | | | |

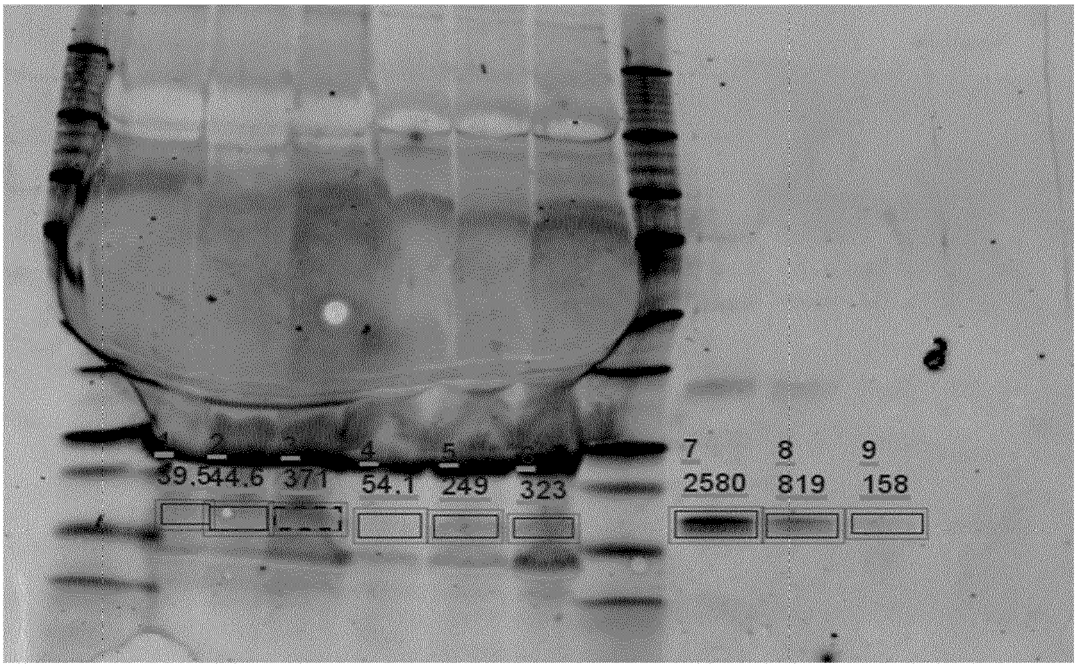


Fig. 27b:

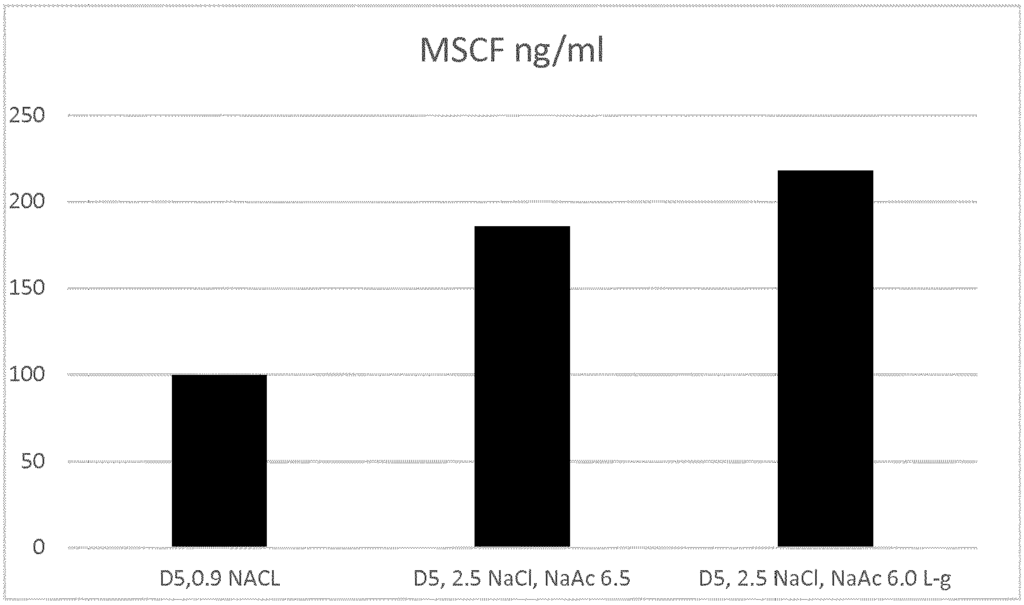

Fig. 28a:

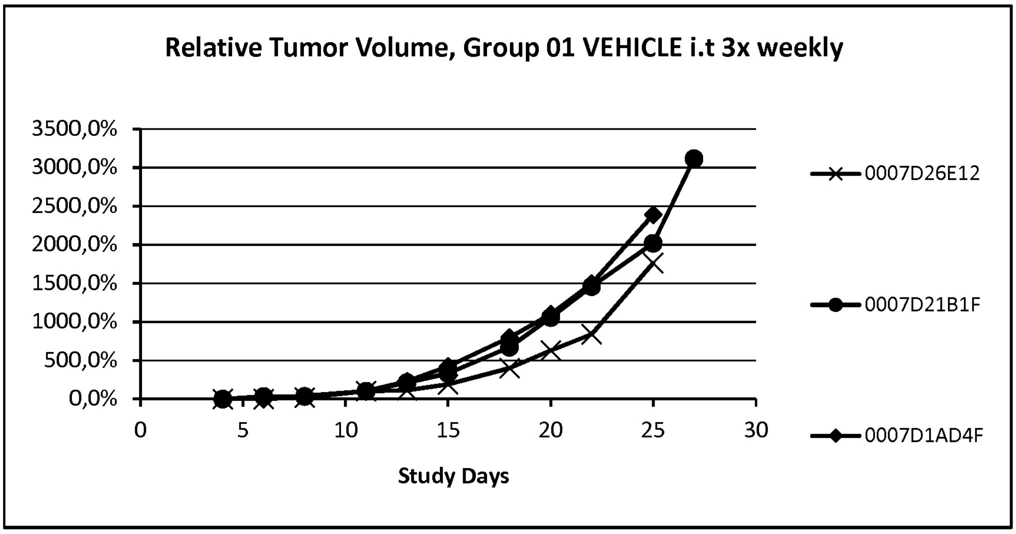


Fig. 28b:

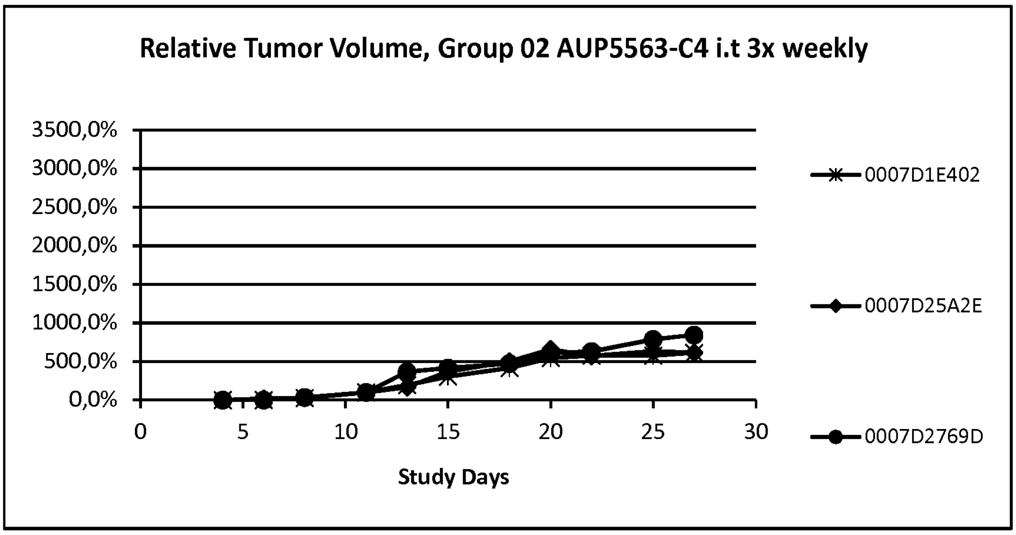


Fig. 28c:

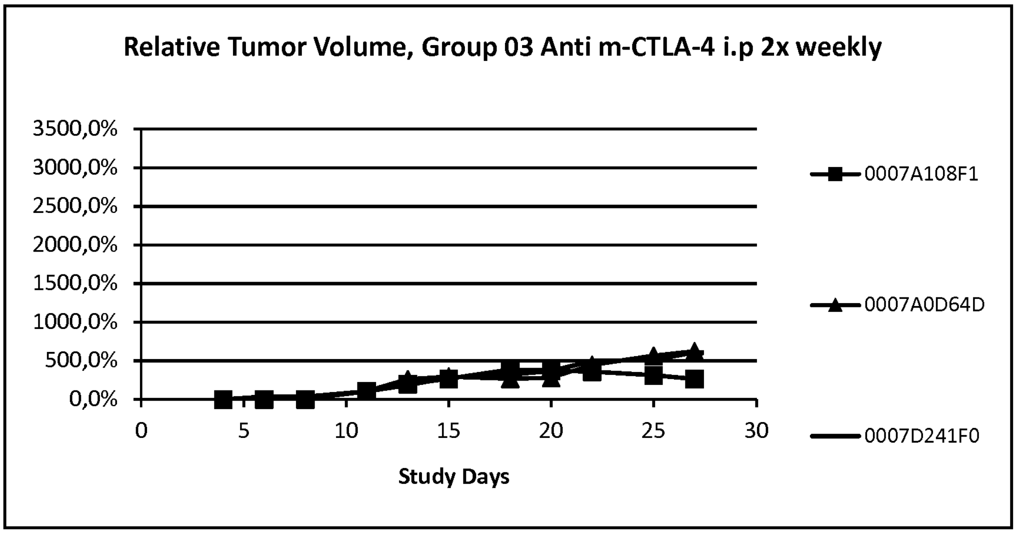

Fig. 29

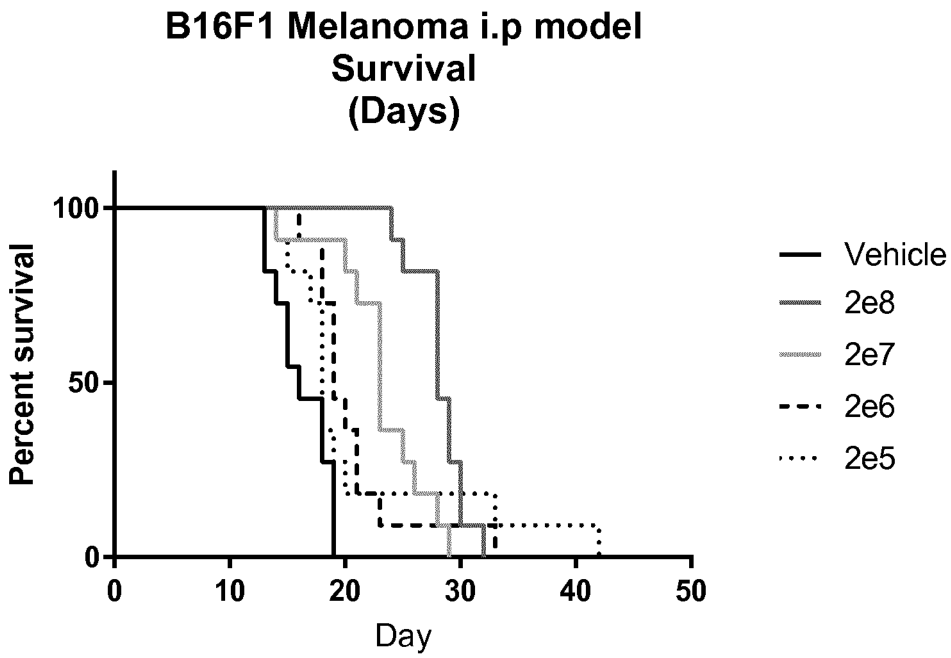


| | Vehicle | 2e8 | 2e7 | 2e6 | 2e5 |
|---|---|---|---|---|---|
| Median survival | 16 | 28 | 23 | 19 | 18 |

| Log-rank (Mantel-Cox) test vs vehicle | | | |
|---|---|---|---|
| 2,00E+08<br>****<br>p < 0.0001 | 2,00E+07<br>****<br>p < 0.0001 | 2,00E+06<br>**<br>p = 0.0032 | 2,00E+05<br>ns<br>p = 0.1045 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9714806 A2
- WO 9611277 A1
- WO 2011160062 A2
- US 2013209407 A


### Non-patent literature cited in the description


- **KATHRYN GELDART et al.** *Applied and Environmental Microbiology,* 01 June 2015, vol. 81 (11), 3889-3897
- **CHANG SHIAO-MING et al.** *Biotechnology Letters Feb,* February 2014, vol. 36 (2), 327-335
- **SANDERS J. W. et al.** APPLIED AND ENVIRONMENTAL MICROBIOLOGY. American Society for Microbiology, 01 December 1997, vol. 63, 4877-4882
- **SANDERS J. W. et al.** *Molecular Microbiology,* January 1998, vol. 27 (2), 299-310
- **DJORDJEVIC G. M. et al.** Molecular Miotechnology. Humana Press, 01 January 1998, vol. 9, 127-139
- **MASSO-SILVA, J. et al.** *Pharmaceuticals,* 03 March 2014, vol. 7 (3), 265-310
- **CHEEVER, M.A. et al.** The Prioritization of Cancer Antigens: A National Cancer Institute Pilot Project for the Acceleration of Translational Research. *Clin. Cancer Res.,* 2009, vol. 15 (17), 5323-5337
- *EFSA Journal,* 2007, vol. 587, 1-16
- **GREEN ; SAMBROOK.** Molecular cloning: a laboratory manual. Cold Spring Harbour Laboratory Press, 2012
- **SINGH, S. et al.** Peritoneal Carcinomatosis: Pictorial Review of Computed Tomography Findings. *International Journal of Advanced Research,* 2016, vol. 4 (7), 735-748
- **COCCOLINI, F. et al.** *World J Gastroenterol.,* 2013, vol. 19 (41), 6979-6994
- **ZHANG, G. ; BLOCK, DE.** Using highly efficient nonlinear experimental design methods for optimization of Lactococcus lactis fermentation in chemically defined media. *Biotechnol. Prog.,* 2009, vol. 25 (6), 1587-1597
- **MICHAELS et al.** db/db mice exhibit severe wound-healing impairments compared with other murine diabetic strains in a siliconesplinted excisional wound model. *Wound Rep. Reg.,* 2007, vol. 15, 665-670
- **BEILHARZ K et al.** Red fluorescent proteins for gene expression and protein localization studies in Streptococcus pneumoniae and efficient transformation with DNA assembled via the gibson assembly method. *Appl. Environ. Microbiol.,* 2015, vol. 81 (20), 7244-7252
- **BIRNBOIM, H.C. ; DOLY, J.** A rapid alkaline extraction procedure for screening recombinant plasmid DNA. *Nucleic Acids Res.,* 1979, vol. 7 (6), 1513-1523
- **BRON, P.A. et al.** Use of the air gene as a food-grade selection marker in lactic acid bacteria. *Appl. Environ. Microbiol.,* 2002, vol. 68 (11), 5663-5670
- **BOLOTIN et al.** The complete genome sequence of the lactic acid bacterium lactococcus lactis ssp. lactis IL1403. *Genome Res.,* 2001, vol. 11, 731-753
- **DE VOS, W.M.** Gene cloning and expression in lactic streptococci. *FEMS Microbiol. Lett.,* 1987, vol. 46 (3), 281-295
- Gene cloning and expression systems in Lactococci. **DE VOS, W.M. ; SIMONS, G.** Genetics and Biotechnology of Lactic Acid Bacteria. 1994, 52-105
- **HOLS, P. et al.** Conversion of Lactococcus lactis from homolactic to homoalanine fermentation through metabolic engineering. *Nat. Biotechnol.,* 1999, vol. 17 (6), 588-592
- **RUHDAL JENSEN, P. ; HAMMER, K.** The Sequence of Spacers between the Consensus Sequences. *Appl. Environ. Microbiol.,* 1998, vol. 64 (1), 82-87
- **TAN, P.S.T. et al.** Characterization of the Lactococcus lactis pepN gene encoding an aminopeptidase homologous to mammalian aminopeptidase N. *FEBS Lett.,* 1992, vol. 306 (1), 9-16
- **SANDERS, J.W. et al.** A chloride-inducible acid resistance mechanism in Lactococcus lactis and its regulation. *Mol Microbiol.,* 1998, vol. 27 (2), 299-310
- **SANDERS, J.W. et al.** A chloride-inducible gene expression cassette and its use in induced lysis of Lactococcus lactis. *Appl. Environ. Microbiol.,* 1997, vol. 63 (12), 4877-4882

- **VAN ASSELDONK et al.** Functional analysis of the Lactococcus lactis usp45 secretion signal in the secretion of a homologous proteinase and a heterologous alpha-amylase. *Mol. Gen. Genet.,* 1993, vol. 240 (3), 428-434
- **VAN ASSELDONK et al.** Cloning of usp45, a gene encoding a secreted protein from Lactococcus lactis subsp. lactis MG1363. *Gene,* 1990, vol. 95 (1), 155-160
- **LAEMMLI, U.K.** Cleavage of structural proteins during the assembly of the head of bacteriophage T4. *Nature,* 1970, vol. 227 (5259), 680-685
- **TAUER, C. et al.** Tuning constitutive recombinant gene expression in Lactobacillus plantarum. *Microbial. Cell Factories,* 2014, vol. 13, 150
- **WANG, M. et al.** Active immunotherapy of cancer with a nonreplicating recombinant fowlpox virus encoding a model tumor-associated antigen. *J. Immunol.,* 1995, vol. 154 (9), 4685-4692
- **TOMAYKO, M. ; REYNOLDS, C.** Determination of subcutaneous tumor size in athymic (nude) mice. *Cancer Chemother. Pharmacol.,* 1989, vol. 24 (3), 148-154
- **LECHNER, M.G.** Immunogenicity of murine solid tumor models as a defining feature of in vivo behavior and response to immunotherapy. *J. Immunother.,* 2013, vol. 36 (9), 477-489
- **FU, Q.** Novel murine tumour models depend on strain and route of inoculation. *Int. J. Exp. Path.,* 2016, vol. 97, 351-356
- **LESINSKI, G.B. et al.** The antitumor effects of IFN-$\alpha$ are abrogated in a STAT1-deficient mouse. *J. Clin. Invest.,* 2003, vol. 112 (2), 170-180